# EUROPEAN PATENT SPECIFICATION

(11) **EP 2 155 769 B1**
(45) Date of publication and mention of the grant of the patent: **27.06.2012**
(21) Application number: 08748050.5
(22) Date of filing: 05.05.2008
(51) Int. Cl.: C07H 15/18

(54) **TISSUE DEGENERATION PROTECTION**
SCHUTZ GEGEN GEWEBEDEGENERATION
PROTECTION CONTRE LA DÉGÉNÉRESCENCE TISSULAIRE

(30) Priority: 04.05.2007 GB 0708713; 18.12.2007 GB 0724588; 29.01.2008 US 62895; 08.02.2008 GB 0802433; 21.02.2008 GB 0803173; 12.03.2008 GB 0804561; 04.05.2007 US 927791 P
(43) Date of publication of application: 24.02.2010
(73) Proprietor: Katholieke Universiteit Leuven KU Leuven Research & Development, 3000 Leuven (BE)
(72) Inventor: GEUNS, Joannes, B-3001 Heverlee (BE); HOLVOET, Paul, B-3010 Kessel-Lo (BE)
(74) Representative: Laenen, Bart Roger Albert
(86) International application number: PCT/BE2008/000035
(87) International publication number: WO 2008/134828

(56) References cited:
- WO-A-02/060419
- WO-A-2006/116815
- XU, DEYI ET AL: "The cardioprotective effect of isosteviol on rats with heart ischemia-reperfusion injury" LIFE SCIENCES , 80(4), 269-274 CODEN: LIFSAK; ISSN: 0024-3205, 2007, XP002540771
- LI, YONGFANG ET AL: "The mechanism of the cardioprotective effect of isostevioside in anesthetized rats" HUAXI YAOXUE ZAZHI , 21(4), 331-334 CODEN: HYZAE2; ISSN: 1006-0103, 2006, XP001539527

## Description

### Background and Summary

### BACKGROUND OF THE INVENTION

### A. Field of the Invention

The present invention is concerned with compositions for use in the medical art and generally to glycoside stevioside and its aglycone component, steviol, and its derivatives such as the rebaudiosides, dulcosides and rubusosides with PPAR agonist activities.
It also relates to essentially pure diterpenoic compounds such as isosteviol, dihydrosteviol, steviol and the diterpene-O-tetrahydro-pyran derivatives, such as isosteviol-19-0-β-D-glucuronide, dihydrosteviol-19-0-β-D-glucuronide, steviol-19-0-β-D-glucuronide or its glycoside stevioside, steviol-O-β-D-Glucopyranosiduronic acid, steviol-O-β-D-Glucopyranoside, steviol-O-α-D-Glucopyranoside, steviol-O-β-D-Glucopyranose, steviol-O-β-D-gluco-Hexodialdo-1,5-pyranoside, steviol-O-β-D-Glucopyranoside, steviol-O-β-L-Mannopyranose, steviol-O-β-D-Glucopyranose, steviol-O-β-D-Xylopyranoside, steviol-O-β-L-Xylopyranose, steviol-O-β-D-galacto-Heptopyranoside, steviol-O-β-D-Galactopyranoside, rebaudioside A, rebaudioside C, Dulcoside A, steviolbioside, rebaudioside B, rebaudioside D and rebaudioside E and pharmaceutically acceptable salts or esters thereof for treatment of a PPAR deficiency disorder or to the use of these diterpenoic or diterpenoic or diterpenoic tetrahydropyran PPAR agonists for the manufacture of a medicament to increase the expression of the peroxisome proliferator activated receptor (PPAR) genes and/or activity of peroxisome proliferator activated receptor (PPAR) and to treat PPAR deficiency-related disorders. It has been found that such compounds with or without the glycoside groups still have a PPAR activity or act as PPAR agonists.

The invention relates to diterpenoic compounds such as isosteviol, dihydrosteviol, steviol and the diterpene-O-tetrahydro-pyran derivatives, such as isosteviol-19-0-β-D-glucuronide, dihydrosteviol-19-0-β-D-glucuronide, steviol-19-0-β-D-glucuronide or stevioside, steviol-O-β-D-Glucopyranosiduronic acid, steviol-O-β-D-Glucopyranoside, steviol-O-α-D-Glucopyranoside, steviol-O-β-D-Glucopyranose,, steviol-O-β-D-gluco-Hevodialdo-1,5-pyranoside, steviol-O-β-D-Glucopyranoside, steviol-O-β-L-Mannopyranose, steviol-O-β-D-Glucopyranose, steviol-O-β-D-Xylopyranoside, steviol-O-β-L-Xylopyranose, steviol-O-β-D-galacto-Heptopyranoside, steviol-O-β-D-Galactopyranoside, rebaudioside A, rebaudioside C, Dulcoside A, steviolbioside, rebaudioside B, rebaudioside D and rebaudioside E and pharmaceutically acceptable salts or esters thereof and/or mixtures thereof for use in a treatment of PPAR activation to improve adipocyte differentiation and adipogenesis, to lower free fatty acids (FFA), triglycerides and/or cholesterol, glucose and insulin in plasma or blood circulation or to treat hypercholesterolemia and/or dyslipidemia and/or glucose intolerance and/or insulin resistance and diabetes, effects that have been observed experimentally and described in the examples and figures of this application. These diterpenoic or diterpenoic tetrahydropyran PPAR agonists may also be used to manufacture a medicament to treat hypercholesterolemia and/or dyslipidemia.

The invention also relates to processes for the preparation of the same, and to the use thereof in the preparation of pharmaceutical compositions for the therapeutic treatment of warm-blooded animals, including humans. The invention applies to human and veterinary applications.

### B. Description of the Related Art

Several documents are cited throughout the text of this specification. However, there is no admission that any document cited is indeed prior art of the present invention.

The natural sweetener stevioside is a diterpene glycoside extracted from the plant *Stevia rebaudiana* (Bertoni) Bertoni which belongs to the *Asteraceae* family and is native to Brazil and Paraguay. Stevioside tastes about 300 times sweeter than 0.4 M sucrose and is non-calorigenic. In many parts of the world, including Japan, South East Asia, and South America, stevioside is used to sweeten food products and beverages. In the USA, powdered *Stevia* leaves and refined extracts from the leaves have been used as a dietary supplement since 1995. Recently the Joint FAO/WHO

Expert Committee on Food Additives (JECFA) accepted a temporary allowable daily intake (ADI) of 2 mg steviol equivalents/kg BW.

*In vitro* stevioside has been demonstrated to act directly on pancreatic β-cells to secrete insulin. For instance studies with incubated mouse pancreatic islets have indicated that hypoglycaemic effects of stevioside and steviol result from the stimulation of insulin secretion *via* direct action of these compounds on β-cells and the β-cell line INS-1 (Jeppesen, P.B.; et al. Metabolism. 2000, 49, 208-214). This effect is independent of cyclic adenosine monophosphate and adenosine triphosphate-sensitive K+-channel activity. By direct intravenous injection, stevioside and aqueous extracts of *Stevia* have been demonstrated to induce direct *in vivo* blood pressure reduction in hypertensive rats (Chan, P. et al. Life Sci. 1998, 63, 1679-84). In addition, increased diuresis and natriuresis were observed during infusion experiments in male Wistar rats with both normal blood pressure and experimental renal hypertension (Melis, M.S. et al. Journal of Ethnopharmacology 1991, 33, 257-262 and Melis, M.S. Journal of Ethnopharmacology. 1992, 36, 213-217.). However, after gastrointestinal administration only hypotensive effects can be achieved after long-term oral administration of stevioside or after nasogastric delivery of very high doses. For instance, in double blind, placebo controlled studies in Chinese hypertensive men and women taking 750 mg (Chan, P.; et al. Br J Clin Pharmacol. 2000, 50, 215-220) or 1500 mg (Hsieh, M.H.; et al. Clin Ther. 2003, 25, 2797-808) of stevioside a day for one (Chan, P.; et al. Br J Clin Pharmacol. 2000, 50, 215-220) or two years (Hsieh, M.H.; et al. Clin Ther. 2003, 25, 2797-808). In both studies, the systolic and diastolic blood pressure of the stevioside group was significantly lower (about 7%) and the blood pressure lowering effect persisted throughout the whole study. This long-term human administration studies did not reveal effects of stevioside on fasting glucose concentrations in volunteers with normal glucose levels. Only very high peroral doses (200 mg stevioside powder/kg) can induce short-term blood pressure reduction as has been demonstrated in anaesthetised dogs (Liu, J.C et al. Pharmacology 2003, 67, 14-20). These high doses (about 94 mg steviol equivalents/kg BW), which are considerably higher than the allowable daily intake (ADI) of stevioside for humans (2 mg steviol equivalents/kg BW), should be avoided for a save pharmaceutical or nutriceutical treatment.

It was an unexpected finding that oral administration of stevioside (10 mg/kg) or steviol (5mg/kg) to mice which are obese and diabetic, have dyslipidemia and hypertension, and show accelerated atherosclerosis and loss of heart function can increase PPARalpha and PPARgamma, and exerts clear PPARalpha and PPARgamma agonist effects at least in the adipose tissues. These changes in PPARalpha and PPAR gamma expression were associated with improved adipocyte differentiation and maturation associated with reduced dyslipidemia evidenced by decreased plasma FFA, plasma triglycerides and plasma cholesterol, and lowered glucose and insulin associated with improved glucose tolerance and insulin signalling. As the underlying mechanism of the decrease in FFA, we identified an increased expression of the free acid binding protein 4 (FABP-4). The increased glucose transport was explained by expression of the glucose transporter 4 (GLUT-4) and of LXRalpha that regulates GLUT-4 expression. The improved insulin signalling was explained by increased expression of the insulin receptor substrates 1 and 2 (IRS1 and IRS2). Treatment of mice with steviol (5 mg/kg) reduced dyslipidemia and improved glucose tolerance and insulin sensitivity to a similar extent as stevioside. The direct effect of stevioside and of its metabolite steviol glucuronide on adipocyte differention was further supported by *in vitro* experiments that they inhibited triglyceride accumulation in adipocytes.

An other novel finding was that oral delivery of stevioside or steviol for 12 weeks resulted in significantly lower plaque area (atherosclerotic plaque formation) and macrophage area (macrophage infiltration in atherosclerotic plaque) and oxidized LDL area (oxidative stress in atherosclerotic plaque) and lipids (foam cell generation and impaired lipid efflux from macrophages) in de atherosclerotic plaques than in mice deprived from said stevioside or steviol treatment.
As the underlying mechanism, we identified improved insulin signalling in the arterial wall as evidenced by increased IRS2 (insulin receptor substrate 2) expression in mice treated with stevioside. This increase was associated with increased expression of the antioxidants superoxide dismutase (SOD)-1 and SOD2, and SOD3, which was associated with decreased accumulation of oxLDL. It was also associated with decreased expression of the inflammatory inducible NO synthase (iNOS).

### SUMMARY OF THE INVENTION

The present invention is concerned with compositions for use in the medical art.

It also relates to essentially pure diterpenoic compounds such as isosteviol, dihydrosteviol, steviol and the diterpene-O-tetrahydro-pyran derivatives, such as isosteviol-19-0-β-D-glucuronide, dihydrosteviol-19-0-β-D-glucuronide, steviol-19-0-β-D-glucuronide or stevioside, steviol-O-β-D-Glucopyranosiduronic acid, steviol-O-β-D-Glucopyranoside, steviol-O-α-D-Glucopyranoside, steviol-O-β-D-Glucopyranose,, steviol-O-β-D-gluco-Hexodialdo-1,5-pyranoside, steviol-O-β-D-Glucopyranoside, steviol-O-β-L-Mannopyranose, steviol-O-β-D-Glucopyranose, steviol-O-β-D-Xylopyranoside, steviol-O-β-L-Xylopyranose, steviol-O-β-D-galacto-Heptopyranoside, steviol-O-β-D-Galactopyranoside, rebaudioside A, rebaudioside C, Dulcoside A, steviolbioside, rebaudioside B, rebaudioside D and rebaudioside E and pharmaceutically acceptable salts or esters thereof and/or mixtures thereof for treatment of a PPAR deficiency disorder or to the use of these diterpenoic or diterpenoic or diterpenoic tetrahydropyran PPAR agonists for the manufacture of a medicament or a dosage form to increase the expression of the PPAR genes and/or activity of the PPARs and to treat PPAR deficiencies disorders.

The invention relates to diterpenoic compounds such as isosteviol, dihydrosteviol, steviol and the diterpene-O-tetrahydro-pyran derivatives, such as isosteviol-19-0-β-D-glucuronide, dihydrosteviol-19-0-β-D-glucuronide, steviol-19-0-β-D-glucuronide or stevioside, steviol-O-β-D-Glucopyranosiduronic acid, steviol-O-β-D-Glucopyranoside, steviol-O-α-D-Glucopyranoside, steviol-O-β-D-Glucopyranose,, steviol-O-β-D-gluco-Hexodialdo-1,5-pyranoside, steviol-O-β-D-Glucopyranoside, steviol-O-β-L-Mannopyranose, steviol-O-β-D-Glucopyranose, steviol-O-β-D-Xylopyranoside, steviol-O-β-L-Xylopyranose, steviol-O-β-D-galacto-Heptopyranoside, steviol-O-β-D-Galactopyranoside, rebaudioside A, rebaudioside C, Dulcoside A, steviolbioside, rebaudioside B, rebaudioside D and rebaudioside E and pharmaceutically acceptable salts or esters thereof and/or mixtures thereof for use in a treatment of PPAR activation to restore adipocyte differentiation, to lower FFA, triglycerides and/or cholesterol in plasma or blood circulation or to treat hypercholesterolemia and/or dyslipidemia, and to lower glucose and increase glucose intolerance and/or to lower insulin and improve insulin signalling and/or treat insulin resistance and diabetes, effects that have been observed experimentally and described in the examples and figures of this application. These diterpenoic or diterpenoic tetrahydropyran PPAR agonists may also be used to manufacture a medicament or a dosage form to treat hypercholesterolemia and/or dyslipidemia.

The invention also relates to processes for the preparation of the same, and to the use thereof in the preparation of pharmaceutical compositions for the therapeutic treatment of warm-blooded animals, including humans. The invention applies to human and veterinary applications.

The present invention solves the problems of the related art by providing a novel class of PPAR agonists, the diterpenoic PPAR agonists, that also as diterpenoic tetrahydropyran compounds have been proved to exert PPAR against activity or to induce increased PPAR expression in adipose tissues and that have been demonstrated to induce PPARalpha/PPARgamma system biological effects in a subject treated with such diterpenoic or diterpenoic tetrahydropyran PPAR agonists. These diterpenoic or diterpenoic tetrahydropyran PPAR agonist agents can be for instance pditerpene-O-tetrahydro-pyran, steviol like compounds or stevioside like which are useful for treating PPAR related disorders.

In accordance with the purpose of the invention, as embodied and broadly described herein, the invention is broadly drawn to diterpene-O-tetrahydro-pyrans such as for instance steviol-O-glucuronide, isosteviol-O-glucuronoide, dihydroisosteviol-O-glucuronide, steviol-O-galacturonide, isosteviol-O-galacturonide, dihydroisosteviol-O- galacturonide, stevioside, steviol, isostevioside, isosteviol and the pharmaceutically acceptable salts or esters thereof.

The invention also relates to a system and method for obtaining such diterpene-O-tetrahydro-pyran compounds and the use of said compounds for the preparation method thereof of a medicament or a dosage form and the uses of same as in therapy to treat arteriosclerosis and/or atherosclerosis by inhibiting glucose intolerance and/or insulin resistance, and/or diabetes, and/or hypercholesterolemia (to lower total and LDL cholesterol) and/or dyslipidemia (to lower FFA and triglycerides); and/or to improve endothelial dysfunction and to increase myocardial perfusion; and/or infiltration of monocytes/macrophages in the vessel wall and/or adipose tissues, and/or accumulation of oxidized LDL in the vessel wall and/or adipose tissues, and their use to manufacture a medicament or a dosage form to treat such disorders.

One aspect of the invention is that the steviol- glucuronide is such a therapy. Steviol-glucuronide can be isolated from urine of warm-blooded animals such as human or it can be chemical synthesized as well as other diterpenoic compounds for instance by the hydrogen atom replacement with a glucuronide or galacturonide or other tetrahydropyran moiety. The galacturonides and glucuronides of this invention are similarly prepared. The invention concerns these compounds for use in a treatment by improving adipocyte differentiation and adipogenesis and/or decreasing dyslipidemia (lower FFA and triglycerides) and or inhibiting glucose intolerance and/or insulin resistance, and/or diabetes, and/or hypercholesterolemia (to lower total and LDL cholesterol); and/or to improve endothelial dysfunction and to increase myocardial perfusion; and/or infiltration of monocytes/macrophages in the vessel wall and/or adipose tissues, and/or accumulation of oxidized LDL in the vessel wall and/or adipose tissues and their use to manufacture a medicament or a dosage form to treat such disorders. The anti-oxidative effects are due at least in part to induction of genes, and/or activation of corresponding gene products, that are involved in cellular anti-oxidative defense mechanisms, such as for instance superoxide dismutase or catalase genes.Still another aspect of the invention, involves the preparation of pharmaceutically acceptable salts and esters thereof for use in a treatment of arteriosclerosis and/or atherosclerosis by improving adipocyte differentiation and adipogenesis and/or decreasing dyslipidemia (lower FFA and triglycerides) and or inhibiting glucose intolerance and/or insulin resistance, and/or diabetes, and/or hypercholesterolemia (to lower total and LDL-cholesterol); and/or to improve endothelial dysfunction and to increase myocardial perfusion; and/or infiltration of monocytes/macrophages in the vessel wall and/or adipose tissues, and/or accumulation of oxidized LDL in the vessel wall and/or adipose tissues, and their use to manufacture a medicament or a dosage form to treat such disorders, and their use to manufacture a medicament or a dosage form to treat such disorders. For instance stable hydrobromide salt (steviol-19-glucuronide.HBr) and other steviol-19-glucuronide salts, in particular the hydrochloride (steviol-19-glucuronide.HCl) salt, sulphate (steviol-19-glucuronide.H2SO4) salt and phosphate (steviol-19-glucuronide.H3PO4) salts or the same for the other diterpenoic tetrahydropyrans.

"Medicated" for this application means that it contains a medicinal substance for instance a functional ingredient. A medicated feed for instance is a feed containing a medicines or a functional ingredient for the purpose of treating or controlling disease or disorders in animals or reduce the risk of, to prevent, to treat or to manage a number of health disorders. A medicated food for instance is a food containing a medicines or a functional ingredient for the purpose of treating or controlling disease or disorders in human or reduce the risk of, to prevent, to treat or to manage a number of health disorders. The medicament of the dosage form of present invention can be comprised in a medicated feed or a medicated food.

"Functional ingredients" offer potential health benefits beyond basic nutrition when incorporated into foods, beverages, and other orally ingested products. Such ingredients have been shown to help reduce the risk of or manage a number of health concerns, including cancer, heart and cardiovascular disease, gastrointestinal health, menopausal symptoms, osteoporosis, and vision. Since 1993, the United States Food and Drug Administration (FDA) has approved numerous health claims for the labelling of food products with information related to the health benefits of functional food (U.S. Food and Drug Administration, A Food Labelling Guide (2000)). Although not yet approved by the FDA for the purposes of labelling, numerous other functional foods are believed to provide health benefits beyond those listed above, such as reduced inflammation. Functional ingredients generally are classified into categories such as carotenoids, dietary fibre, fatty acids, flavonoids, isothiocyanates, phenols, plant sterols and stanols (phytosterols and phytostanols); polyols; prebiotics/probiotics; phytoestrogens; soy protein; sulfides/thiols; amino acids; proteins; vitamins; and minerals. Functional ingredients also may be classified based on their health benefits, such as cardiovascular, cholesterol reducing, and anti-inflammatory.

The invention furthermore concerns the following treatments.

Furthermore the present invention concerns diterpenoic tetrahydropyrans such as isosteviol, dihydrosteviol, steviol and the diterepene-O-tetrahydro-pyran derivatives, such as isosteviol-19-0-β-D-glucuronide, dihydrosteviol-19-0-β-D-glucuronide, steviol-19-0-β-D-glucuronide or stevioside and pharmaceutically acceptable salts or esters thereof and/or mixtures thereof for use in a treatment of or the use of such diterpenoic tetrahydropyrans PPAR-α agonists for the manufacture of a medicament to increase the expression and/or activity of the peroxisome proliferator activated receptor (PPAR) gene, more particular the PPARalpha gene or for PPAR-α activation to treat PPAR deficiencies disorders.

Furthermore the present invention concerns diterpenoic tetrahydropyrans such as isosteviol, dihydrosteviol, steviol and the diterepene-O-tetrahydro-pyran derivatives, such as isosteviol-19-0-β-D-glucuronide, dihydrosteviol-19-0-β-D-glucuronide, steviol-19-0-β-D-glucuronide or stevioside and pharmaceutically acceptable salts or esters thereof and/or mixtures thereof for use in a treatment of or the use of such diterpenoic tetrahydropyrans PPAR-α agonists for the manufacture of a medicament to include normalisation or improvement of the lipid homeostasis of the group consisting of induction the formation of less atherogenic LDL, lowering free fatty acids, triglycerides and/or cholesterol in plasma or blood circulation, inducing increased expression of PPARalpha (PPAR-α) in adipose tissue, increasing the levels of lipoprotein lipase in adipose tissue, inducing hydrolysis of the lipids very low density lipoproteins (VLDL) into three fatty acids and one glycerol molecule, increasing the production of apoB100 while decreasing the biosynthesis of triglycerides, inducing a shift of apoB100 from VLDL to denser particles or to smaller, less triglyceride rich VLDL, increasing diversion of fatty acids into beta-oxidation, decreasing the availability of fatty-acyl-coenzyme A substrates for triglyceride synthesis, reducing secretion of very-low-density lipoprotein by the liver, increasing mitochondrial fatty acid oxidation in patients in need thereof for instance paediatric burn trauma patients, decreasing triglyceride levels by increasing the expression of lipoprotein lipase in the liver and in macrophages, increasing plasma HDL-cholesterol, increasing PPAR-alpha or PPAR-alpha/gamma activity in adipose tissue, heart, or aortic arch, decreasing levels of intracellular triglycerides, diacylglycerol and increasing fat beta-oxidation and increasing the oxidation rate of fatty acids.

Furthermore the present invention concerns diterpenoic compounds such as isosteviol, dihydrosteviol, steviol and the diterpene-O-tetrahydro-pyran derivatives, such as isosteviol-19-0-β-D-glucuronide, dihydrosteviol-19-0-β-D-glucuronide, steviol-19-0-β-D-glucuronide or stevioside, steviol-O-β-D-Glucopyranosiduronic acid, steviol-O-β-D-Glucopyranoside, steviol-O-α-D-Glucopyranoside, steviol-O-β-D-Glucopyranose,, steviol-O-β-D-gluco-Hexodialdo-1,5-pyranoside, steviol-O-β-D-Glucopyranoside, steviol-O-β-L-Mannopyranose, steviol-O-β-D-Glucopyranose, steviol-O-β-D-Xylopyranoside, steviol-O-β-L-Xylopyranose, steviol-O-β-D-galacto-Heptopyranoside, steviol-O-β-D-Galactopyranoside, rebaudioside A, rebaudioside C, Dulcoside A, steviolbioside, rebaudioside B, rebaudioside D and rebaudioside E and pharmaceutically acceptable salts or esters thereof and/or mixtures thereof for use in a treatment of or the use of such diterpenoic or diterpenoic or diterpenoic tetrahydropyran PPAR agonists for the manufacture of a medicament or a dosage form to increase the expression and/or activity of the PPAR genes to treat PPAR deficiencies disorders.

Furthermore the present invention concerns diterpenoic compounds such as isosteviol, dihydrosteviol, steviol and the diterpene-O-tetrahydro-pyran derivatives, such as isosteviol-19-0-β-D-glucuronide, dihydrosteviol-19-0-β-D-glucuronide, steviol-19-0-β-D-glucuronide or stevioside and pharmaceutically acceptable salts or esters thereof and/or mixtures thereof for use in a treatment of or the use of such diterpenoic or diterpenoic or diterpenoic tetrahydropyran PPAR agonists for the manufacture of a medicament or a dosage form to include normalisation or improvement of the lipid homeostasis of the group consisting of induction the formation of less atherogenic LDL, lowering FFA, triglycerides and/or cholesterol in plasma or blood circulation, inducing increased expression of PPARs in adipose tissue, increasing the levels of lipoprotein lipase in adipose tissue, inducing hydrolysis of the lipids very low density lipoproteins (VLDL) into three fatty acids and one glycerol molecule, increasing the production of apoB100 while decreasing the biosynthesis of triglycerides, inducing a shift of apoB100 from VLDL to denser particles or to smaller, less triglyceride rich VLDL, increasing diversion of fatty acids into beta-oxidation, decreasing the availability of fatty-acyl-coenzyme A substrates for triglyceride synthesis, reducing secretion of very-low-density lipoprotein by the liver, increasing mitochondrial fatty acid oxidation in patients in need thereof for instance paediatric burn trauma patients, decreasing triglyceride levels by increasing the expression of lipoprotein lipase in the liver and in macrophages, increasing plasma HDL-cholesterol, increasing PPARalpha or PPARalpha/gamma activity in adipose tissue, heart, or aortic arch, decreasing levels of intracellular FFA, triglycerides, diacylglycerol and increasing fat beta-oxidation and increasing the oxidation rate of fatty acids.

Furthermore, the present invention concerns diterpenoic compounds such as isosteviol, dihydrosteviol, steviol and the diterpene-O-tetrahydro-pyran derivatives, such as isosteviol-19-0-β-D-glucuronide, dihydrosteviol-19-0-β-D-glucuronide, steviol-19-0-β-D-glucuronide or stevioside, steviol-O-β-D-Glucopyranosiduronic acid, steviol-O-β-D-Glucopyranoside, steviol-O-α-D-Glucopyranoside, steviol-O-β-D-Glucopyranose,, steviol-O-β-D-gluco-Hexodialdo-1,5-pyranoside, steviol-O-β-D-Glucopyranoside, steviol-O-β-L-Mannopyranose, steviol-O-β-D-Glucopyranose, steviol-O-β-D-Xylopyranoside, steviol-O-β-L-Xylopyranose, steviol-O-β-D-galacto-Heptopyranoside, steviol-O-β-D-Galactopyranoside, rebaudioside A, rebaudioside C, Dulcoside A, steviolbioside, rebaudioside B, rebaudioside D and rebaudioside E and pharmaceutically acceptable salts or esters thereof and/or mixtures thereof for use in a treatment of or the use of such diterpenoic or diterpenoic or diterpenoic tetrahydropyran PPAR agonists for the manufacture of a medicament or a dosage form to treat hypercholesterolemia and/or dyslipidemia, to lower total cholesterol, triglycerides & FFA, to lower plasma triglycerides, FFA and cholesterol to modulate the whole-body sterol homeostasis, including fatty acid, triglyceride, and lipoprotein metabolism and reverse cholesterol transport.

Furthermore the present invention concerns diterpenoic compounds such as isosteviol, dihydrosteviol, steviol and the diterpene-O-tetrahydro-pyran derivatives, such as isosteviol-19-0-β-D-glucuronide, dihydrosteviol-19-0-β-D-glucuronide, steviol-19-0-β-D-glucuronide or stevioside, steviol-O-β-D-Glucopyranosiduronic acid, steviol-O-β-D-Glucopyranoside, steviol-O-α-D-Glucopyranoside, steviol-O-β-D-Glucopyranose,, steviol-O-β-D-gluco-Hexodialdo-1,5-pyranoside, steviol-O-β-D-Glucopyranoside, steviol-O-β-L-Mannopyranose, steviol-O-β-D-Glucopyranose, steviol-O-β-D-Xylopyranoside, steviol-O-β-L-Xylopyranose, steviol-O-β-D-galacto-Heptopyranoside, steviol-O-β-D-Galactopyranoside, rebaudioside A, rebaudioside C, Dulcoside A, steviolbioside, rebaudioside B, rebaudioside D and rebaudioside E and pharmaceutically acceptable salts or esters thereof for use in a treatment of or the use of such diterpenoic or diterpenoic or diterpenoic tetrahydropyran PPAR agonists for the manufacture of a medicament or a dosage form to treat hypercholesterolemia.

Furthermore the present invention concerns diterpenoic compounds such as isosteviol, dihydrosteviol, steviol and the diterpene-O-tetrahydro-pyran derivatives, such as isosteviol-19-0-β-D-glucuronide, dihydrosteviol-19-0-β-D-glucuronide, steviol-19-0-β-D-glucuronide or stevioside and pharmaceutically acceptable salts or esters thereof and/or mixtures thereof for use in a treatment of or the use of such diterpenoic or diterpenoic or diterpenoic tetrahydropyran PPAR agonists for the manufacture of a medicament or a dosage form to treat dyslipidemia.

Furthermore, the present invention concerns diterpenoic compounds such as isosteviol, dihydrosteviol, steviol and the diterpene-O-tetrahydro-pyran derivatives, such as isosteviol-19-0-β-D-glucuronide, dihydrosteviol-19-0-β-D-glucuronide, steviol-19-0-β-D-glucuronide or stevioside, steviol-O-β-D-Glucopyranosiduronic acid, steviol-O-β-D-Glucopyranoside, steviol-O-α-D-Glucopyranoside, steviol-O-β-D-Glucopyranose,, steviol-O-β-D-gluco-Hexodialdo-1,5-pyranoside, steviol-O-β-D-Glucopyranoside, steviol-O-β-L-Mannopyranose, steviol-O-β-D-Glucopyranose, steviol-O-β-D-Xylopyranoside, steviol-O-β-L-Xylopyranose, steviol-O-β-D-galacto-Heptopyranoside, steviol-O-β-D-Galactopyranoside, rebaudioside A, rebaudioside C, Dulcoside A, steviolbioside, rebaudioside B, rebaudioside D and rebaudioside E and pharmaceutically acceptable salts or esters thereof for use in a treatment of or the use of such diterpenoic or diterpenoic or diterpenoic tetrahydropyran PPAR agonists for the manufacture of a medicament or a dosage form to treat cholesterol disorders.

Furthermore the present invention concerns diterpenoic compounds such as isosteviol, dihydrosteviol, steviol and the diterpene-O-tetrahydro-pyran derivatives, such as isosteviol-19-0-β-D-glucuronide, dihydrosteviol-19-0-β-D-glucuronide, steviol-19-0-β-D-glucuronide or stevioside, steviol-O-β-D-Glucopyranosiduronic acid, steviol-O-β-D-Glucopyranoside, steviol-O-α-D-Glucopyranoside, steviol-O-β-D-Glucopyranose,, steviol-O-β-D-gluco-Hexodialdo-1,5-pyranoside, steviol-O-β-D-Glucopyranoside, steviol-O-β-L-Mannopyranose, steviol-O-β-D-Glucopyranose, steviol-O-β-D-Xylopyranoside, steviol-O-β-L-Xylopyranose, steviol-O-β-D-galacto-Heptopyranoside, steviol-O-β-D-Galactopyranoside, rebaudioside A, rebaudioside C, Dulcoside A, steviolbioside, rebaudioside B, rebaudioside D and rebaudioside E and pharmaceutically acceptable salts or esters thereof and/or mixtures thereof as hypolipidemic agents for use in a treatment of or the use of such diterpenoic or diterpenoic or diterpenoic tetrahydropyran PPAR agonists for the manufacture of a medicament or a dosage form to reduce circulating triglycerides or liver triglyceride deposition.

Furthermore, the present invention concerns diterpenoic compounds such as isosteviol, dihydrosteviol, steviol and the diterpene-O-tetrahydro-pyran derivatives, such as isosteviol-19-0-β-D-glucuronide, dihydrosteviol-19-0-β-D-glucuronide, steviol-19-0-β-D-glucuronide or stevioside and pharmaceutically acceptable salts or esters thereof and/or mixtures thereof for use in a treatment of or the use of such diterpenoic or diterpenoic or diterpenoic tetrahydropyran PPAR agonists for the manufacture of a medicament or a dosage form to induce weight loss or to decrease, retard or reduce increase in adipose tissue formation .

Furthermore, the present invention concerns diterpenoic compounds such as isosteviol, dihydrosteviol, steviol and the diterpene-O-tetrahydro-pyran derivatives, such as isosteviol-19-0-β-D-glucuronide, dihydrosteviol-19-0-β-D-glucuronide, steviol-19-0-β-D-glucuronide or stevioside, steviol-O-β-D-Glucopyranosiduronic acid, steviol-O-β-D-Glucopyranoside, steviol-O-α-D-Glucopyranoside, steviol-O-β-D-Glucopyranose,, steviol-O-β-D-gluco-Hexodialdo-1,5-pyranoside, steviol-O-β-D-Glucopyranoside, steviol-O-β-L-Mannopyranose, steviol-O-β-D-Glucopyranose, steviol-O-β-D-Xylopyranoside, steviol-O-β-L-Xylopyranose, steviol-O-β-D-galacto-Heptopyranoside, steviol-O-β-D-Galactopyranoside, rebaudioside A, rebaudioside C, Dulcoside A, steviolbioside, rebaudioside B, rebaudioside D and rebaudioside E and pharmaceutically acceptable salts or esters thereof and/or mixtures thereof for use in a treatment of or the use of such diterpenoic or diterpenoic or diterpenoic tetrahydropyran PPAR agonists for the manufacture of a medicament or a dosage form to improve lipid metabolism and insulin signalling associated with decreased tissue deposition of oxidized LDL.

Furthermore, the present invention concerns diterpenoic compounds such as isosteviol, dihydrosteviol, steviol and the diterpene-O-tetrahydro-pyran derivatives, such as isosteviol-19-0-β-D-glucuronide, dihydrosteviol-19-0-β-D-glucuronide, steviol-19-0-β-D-glucuronide or stevioside, steviol-O-β-D-Glucopyranosiduronic acid, steviol-O-β-D-Glucopyranoside, steviol-O-α-D-Glucopyranoside, steviol-O-β-D-Glucopyranose,, steviol-O-β-D-gluco-Herodialdo-1,5-pyranoside, steviol-O-β-D-Glucopyranoside, steviol-O-β-L-Mannopyranose, steviol-O-β-D-Glucopyranose, steviol-O-β-D-Xylopyranoside, steviol-O-β-L-Xylopyranose, steviol-O-β-D-galacto-Heptopyranoside, steviol-O-β-D-Galactopyranoside, rebaudioside A, rebaudioside C, Dulcoside A, steviolbioside, rebaudioside B, rebaudioside D and rebaudioside E and pharmaceutically acceptable salts or esters thereof and/or mixtures thereof for use in a treatment of or the use of such diterpenoic or diterpenoic or diterpenoic tetrahydropyran PPAR agonists for the manufacture of a medicament or a dosage form to increase β-oxidation of fatty acids such as oleate or palmitate and to decrease fatty acid incorporation into triglycerides.

Furthermore the present invention concerns diterpenoic tetrahydropyrans such as isosteviol, dihydrosteviol, steviol and the diterepene-O-tetrahydro-pyran derivatives, such as isosteviol-19-0-β-D-glucuronide, dihydrosteviol-19-0-β-D-glucuronide, steviol-19-0-β-D-glucuronide or stevioside and pharmaceutically acceptable salts or esters thereof and/or mixtures thereof for use in a treatment of or the use of such diterpenoic tetrahydropyrans PPAR-α agonists for the manufacture of a medicament to induce weight loss or to decrease, retard or reduce increase in adipose tissue formation.

Furthermore the present invention concerns diterpenoic tetrahydropyrans such as isosteviol, dihydrosteviol, steviol and the diterepene-O-tetrahydro-pyran derivatives, such as isosteviol-19-0-β-D-glucuronide, dihydrosteviol-19-0-β-D-glucuronide, steviol-19-0-β-D-glucuronide or stevioside and pharmaceutically acceptable salts or esters thereof and/or mixtures thereof for use in a treatment of or the use of such diterpenoic tetrahydropyrans PPAR-α agonists for the manufacture of a medicament to improve lipid metabolism and insulin signalling associated with decreased tissue deposition of oxidized LDL.

Furthermore the present invention concerns diterpenoic tetrahydropyrans such as isosteviol, dihydrosteviol, steviol and the diterepene-O-tetrahydro-pyran derivatives, such as isosteviol-19-0-β-D-glucuronide, dihydrosteviol-19-0-β-D-glucuronide, steviol-19-0-β-D-glucuronide or stevioside and pharmaceutically acceptable salts or esters thereof and/or mixtures thereof for use in a treatment of or the use of such diterpenoic tetrahydropyrans PPAR-α agonists for the manufacture of a medicament to increase β-oxidation of fatty acids such as oleate or palmitate and to decrease fatty acid incorporation into triglycerides and to decrease lipid accumulation in tissues, for instance in adipose tissue.

Furthermore the present invention demonstrated that treatment with stevioside and thus also its aglycone forms or other glycosides of steviol, isosteviol or dihydroisosteviol or other diterepene-O-tetrahydro-pyran derivatives affect the gene expression in adipose tissue by an increased expression of the insulin receptor (INSR) and an increased expression of catalase and of the superoxide dismutase 3 (SOD3), the predominant SOD in adipose tissues. This indicates an improved antioxidant defence; indeed SOD is known to convert oxygen radicals to hydrogen peroxide that is converted to water by catalase. Furthermore the present invention demonstrated that treatment with stevioside and thus also its aglycone forms or other glycosides of steviol, isosteviol or dihydroisosteviol or other diterepene-O-tetrahydro-pyran derivatives affect the gene expression in adipose tissue by an increased expression of CD36 that plays a role in the clearance of free fatty acids (FFA) and is a scavenger receptor that is important for the clearance of oxidized LDL from the blood, and thus for reducing oxidized LDL levels in the blood. Thus, The diterpenoic and/or diterpenoic tetrahydropyran derivativesof the present invention can be used to manufacture a medicament or a dosage form to reduce the level of oxidized LDL in the blood.

We demonstrated an association between increased INSR expression in the adipose tissue and increased expression of catalase and SOD3, indicative of increased antioxidant defence, and increased expression of CD36 that is known to decrease FFA and oxidized LDL.

Thus, PPAR modulators of present invention, such as those described herein, can be used in the prophylaxis and/or therapeutic treatment of a variety of different disease and conditions, such as weight disorders (e.g. obesity, overweight condition, bulimia, and anorexia nervosa), lipid disorders (e. g. hyperlipidemia, dyslipidemia including associated diabetic dyslipidemia and mixed dyslipidemia, hypoalphalipoproteinemia, hypertriglyceridemia, hypercholesterolemia, and low HDL (high density lipoprotein)),

Each year, approximately 60,000 Americans join the 1.5 million Americans currently afflicted by Parkinson's Disease (PD). This disorder is thought to originate in the part of the brain called the substantia nigra. As affected nerve cells in this portion of the brain die, their production of dopamine likewise vanishes. Without dopamine, smooth and coordinated muscle movement is lost. The PPAR agonist of present invention can protect cellular viability in the substantia nigra while simultaneously attenuating microglial activation The activation of peroxisome proliferator receptor- gamma (PPARgamma) has been associated with increasing dopamine, altering mitochondrial bioenergetics, and reducing oxidative stress, a variety of factors that are altered in PD For instance PPAR-γ activation has been demonstrated of clinically relevance treatment approach to neuroinflammation and PD related neurodegeneration. Agonism of Peroxisome Proliferator Receptor-Gamma as for the PPARgamma agonist, pioglitazone has a therapeutic action on Parkinson's Disease and for the repair of mitochondrial dysfunction and dopaminergic neurodegeneration in the nigrostriatal and to protect against LPS neurotoxicity and against mitochondrial dysfunction and dopaminergic neurodegeneration in the nigrostriatal system (Current Neuropharmacology Volume 5, Number 1, March 2007 , pp. 35-46(12) Authors: Randy L. Hunter, Guoying Bing & Hunter RL et al J Neurochem. 2007 Mar;100(5):1375-86. Epub 2007 Jan 23.). Simarly, the peroxisome proliferator-activated receptor (PPAR)-γ agonist, Rosiglitazone, protects human neuroblastoma SH-SY5Y cells against MPP+ induced cytotoxicity via inhibition of mitochondrial dysfunction and ROS production. (Journal of the Neurological Sciences, Volume 253, Issue 1-2, Pages 53-60. 30 January 2007 T. Jung, et al.) whereby it has been demonstrated that 3.1. Rosiglitazone ameliorated MPP+ induced loss of neuronal cell viability and oxidative stress, rescued MPP+-induced changes in nuclear morphology, affected the expression of Bcl-2 and Bax in MPP+-treated cells by regulation of anti-oxidant enzyme expressions, inhibited MPP+-induced cytochrome c release and Rosiglitazone inhibited MPP+-induced caspase-3 expression.

The compounds of the present invention lower plasma FFA, triglycerides, glucose, insulin, lower total cholesterol (TC) and and decrease low density lipoprotein (LDL), which have a beneficial effect on the protection of coronary heart disease and atherosclerosis.

The compounds of general formula (1) are useful in improving adipocyte differentiation and adipogenesis and for the treatment and/or prophylaxis of related disorders. These compounds are useful for the treatment of hyperlipidemia, hyperglycemia, hypercholesterolemia, lowering of atherogenic lipoproteins, VLDL (very low density lipoprotein) and LDL. The compounds of general formula I and the derivatives compounds of present invention are also useful for the treatment and/or prophylaxis of leptin resistance, impaired glucose tolerance, disorders related to syndrome X such as hypertension, obesity, insulin resistance. The compounds of the present invention are also useful in the treatment and/or prophylaxis of the above said diseases in combination/concomittant with one or more of HMG CoA reductase inhibitor ; cholesterol absorption inhibitor; antiobesity drug; lipoprotein disorder treatment drug ; hypoglycemic agent; insulin; biguanide; sulfonylurea ; thiazolidinedione; dual PPARalpha and agonists or a mixture thereof.

### B. Background of the Invention

Atherosclerosis and other peripheral vascular diseases affect the quality of life of millions of people. Therefore, considerable attention has been directed towards understanding the etiology of hypercholesterolemia and hyperlipidemia and development of effective therapeutic strategies.

Hypercholesterolemia has been defined as plasma cholesterol level that exceeds arbitrarily defined value called"normal"level. Recently, it has been accepted that"ideal" plasma levels of cholesterol are much below the"normal"level of cholesterol in the general population and the risk of coronary artery disease (CAD) increases as cholesterol level rises above the"optimum" (or"ideal") value. There is clearly a definite cause and effect-relationship between hypercholesterolemia and CAD, particularly for individuals with multiple risk factors. Most of the cholesterol is present in the esterified forms with various lipoproteins such as Low density lipoprotein (LDL), Intermediate density lipoprotein (IDL), High density lipoprotein (HDL) and partially as Very low density lipoprotein (VLDL). Studies clearly indicate that there is an inverse correlationship between CAD and atherosclerosis with serum HDL-cholesterol concentrations (Stampfer et al., N. Eng/. J. Med., 325 (1991), 373-381). The risk of CAD increases with increasing levels of LDL and VLDL.

In CAD, generally "fatty streaks"in carotid, coronary and cerebral arteries, are found which are primarily free and esterified cholesterol. Miller et al., (Br. Med. J. , 282 (1981), 1741-1744) have shown that increase in HDL-particles may decrease the number of sites of stenosis in coronary arteries of human, and high level of HDL-cholesterol may protect against the progression of atherosclerosis. Picardo et al., Arteriosclerosis 6 (1986) 434-441 have shown by iii vitro experiment that HDL is capable of removing cholesterol from cells. They suggest that HDL may deplete tissues of excess free cholesterol and transfer it to liver, which is known as reverse cholesterol transport, (Macikinnon et al., J. Biol. cherra. 261 (1986), 2548-2552). Therefore, agents that increase HDL cholesterol would have therapeutic significance for the treatment of hypercholesterolemia and coronary heart diseases (CHD).

Obesity is a disease highly prevalent in affluent societies and in the developing world and is a major cause of morbidity and mortality. It is a state of excess body fat accumulation. The causes of obesity are unclear. It is believed to be of genetic origin or promoted by an interaction between the genotype and environment. Irrespective of the cause, the result is fat deposition due to imbalance between the energy intake versus energy expenditure. Dieting, exercise and appetite suppression have been a part of obesity treatment. There is a need for efficient therapy to fight this disease since it may lead to coronary heart disease, diabetes, stroke, hyperlipidemia, gout, osteoarthritis, reduced fertility and many other psychological and social problems.

Insulin resistance is yet another disease, which severely affects the quality of life of large population in the world, and it generally occurs in the artifical monitoring situation of a critical care unit. Insulin resistance is the diminished ability of insulin to exert its biological action across a broad range of concentrations. In insulin resistance, the body secretes abnormally high amounts of insulin to compensate for this defect; failing which, the plasma glucose concentration inevitably raises and develops into diabetes. Among the developed countries, diabetes mellitus is a common problem and is associated with a variety of abnormalities including obesity, hypertension, hyperlipidemia (J. Clin. Invest., 75 (1985) 809-817 ; N. Engl. J. ll ! I. 317 (1987) 350-357; J. Clin. Endocrinol. Metab., 66 (1988) 580-583; J. Clin. Invest., 68 (1975) 957-969) and other renal complications (patent publication No. WO 95/21608).

Hyperlipidemia is the primary cause for cardiovascular (CVD) and other peripheral vascular diseases. High risk of CVD is related to the higher LDL (Low Density Lipoprotein) and VLDL (Very Low Density Lipoprotein) seen in hyperlipidemia. Patients having glucose intolerance/insulin resistance in addition to hyperlipidemia have higher risk of CVD. Numerous studies in the past have shown that lowering of plasma triglycerides and total cholesterol, in particular LDL and VLDL and increasing HDL cholesterol help in preventing cardiovascular diseases.

Peroxisome Proliferator Activated Receptors (PPARs) are orphan receptors belonging to the steroid/retinoid receptor super family of ligand activated transcription factors. (Wilson T. M. and Wahli W., Curr. Opin. Chem. Biol. , 1997, Vol. 1,235-241). Three mammalian Peroxisome Proliferator Activated Receptors (PPARs) have been isolated and termed PPAR-alpha, PPAR-gamma and PPAR-beta/delta. These PPARs regulate expression of target genes by binding to DNA sequence elements.

Certain compounds that activate or otherwise interact with one or more of the PPARs have been implicated in the regulation of triglyceride and cholesterol levels in animal models (U. S. patents 5,847, 008; 5,859, 051 and PCT publications WO 97/28149; WO 99/04815).

A wealth of information exists on the influence of PPAR-alpha a agonists on the cardiovascular risk profile for example fibrate class of compounds which are weak PPAR-alpha agonists correct atherogenic dyslipoproteinemia. Several angiographic intervention trials have demonstrated a beneficial action of these drugs on atherosclerotic lesion progression and results from primary and secondary prevention trials show a decreased incidence of cardiovascular events. (Ricote M. and Glass C. K. Trends in Pharmacological Sciences; 2001; 22 (9); 441-443.

Despite the fact that fibrates, which are weak PPAR-alpha activators, reduce the plasma triglyceride levels and elevate the levels of HDL-cholesterol simultaneously, they are not the drugs of choice, because of low efficacy requiring high doses, incidence of Myositis and contra-indicated in patients with impaired renal and hepatic function and in pregnant and nursing women.

However, there has been rapid progress in the understanding of the role of PPARalpha in different pathophysiological conditions in addition to the well-documented favourable effects on lipid profile. The inflammatory activation of aortic smooth muscle cells, which is the hallmark of atherosclerosis, seems to be inhibited by the enhanced PPARalpha activity (Vamecq J. and Latruffe N ; Lancet; 1999; 354; 141-148).

Recent evidence suggests the role of PPARalpha receptors in improving insulin sensitivity. It has been demonstrated that by lowering circulatory and muscle lipids in insulin-resistant rodent models such as obese Zucker rats, high fat-fed mice and sucrose- lard fed rats PPAR-alpha ligands improve insulin sensitivity and obesity. Further, the lipid lowering activity of the statins has been linked to a cross talk with PPAR-alpha receptor in addition to limited cholesterol availability. Some clinical trials have shown improvement in insulin sensitivity indices, wherein fibrates were employed. (Guerre-Millo M, Rounalt C. and Poulain P; Diabetes; 2001; 50; 2809-2814, Muoio D. M. , Way J. M. and Tanner C. J. ; Diabetes; 2002; 51; 901-909, Ye J, Doyle P. J. and Iglesias M. A.; Diabetes; 2001; 50; 411-417, and Roglans N, Sanguino E. and Peris C; JPET; 2002; 302; 232-239).

Thus, there is an interesting evidence for PPAR-alpha agonists to be used for lipid control and as per recent evidence even for insulin resistance. Limitations of the currently available medications coupled with the fact that lipid abnormalities are on the rise worldover necessitate the discovery of more potent and safer PPAR-alpha agonists (U. S. Patents 5,885, 997; 6,054, 453; 6,265, 401: PCT application PCT/IB02/04275).

A method for treating a subject suffering from or at risk of a disease or condition for which PPAR modulation provides a therapeutic benefit wherein said disease or condition is a PPAR-mediated disease or condition , comprising: administering to said subject an effective amount of a diterpenoic or diterpenoic tetrahydropyran PPAR modulator of the group of compounds diterpenoic compounds such as isosteviol, dihydrosteviol, steviol and the diterpene-O-tetrahydro-pyran derivatives, such as isosteviol-19-0-β-D-glucuronide, dihydrosteviol-19-0-β-D-glucuronide, steviol-19-0-β-D-glucuronide or stevioside, steviol-O-β-D-Glucopyranosiduronic acid, steviol-O-β-D-Glucopyranoside, steviol-O-α-D-Glucopyranoside, steviol-O-β-D-Glucopyranose,, steviol-O-β-D-gluco-Hexodialdo-1,5-pyranoside, steviol-O-β-D-Glucopyranoside, steviol-O-β-L-Mannopyranose, steviol-O-β-D-Glucopyranose, steviol-O-β-D-Xylopyranoside, steviol-O-β-L-Xylopyranose, steviol-O-β-D-galacto-Heptopyranoside, steviol-O-β-D-Galactopyranoside, rebaudioside A, rebaudioside C, Dulcoside A, steviolbioside, rebaudioside B, rebaudioside D and rebaudioside E and pharmaceutically acceptable salts or esters thereof and/or mixtures thereof or having the chemical structure according to the following general formula (I): 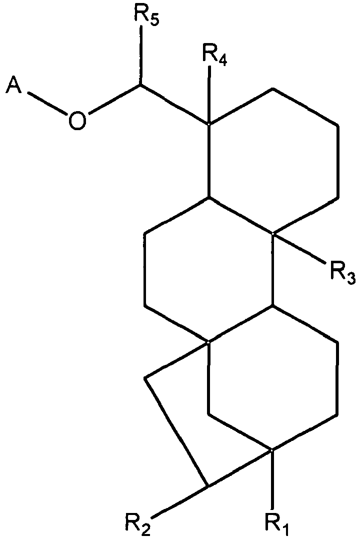 wherein
R1 is methyl, hydroxyl, hydrogen or a or a tetrahydro-pyran compound for instance represented by the following general formula 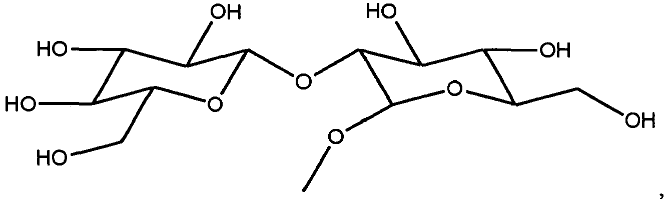 or R1 is a a, tetrahydro-pyran compound represented by the following general formula (II) 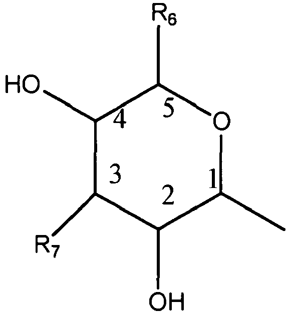
R6 is hydroxyl, ethyl, methylene, oxymethyl, nitrite, carboxyl, aldehyde, amino, or oxyamino
or R1 is a -O-beta- Glc-beta- Glc (2-1)
R2 is =O, methyl, oxymethyl, hydroxyl, or forms an ethene, ethanol, 2-methoxy-propene, formaldehyde or 1-methoxy-ethanol bound or hydrogen,
R3 is methyl, ethyl, or is hydrogen,
R4 is methyl, ethyl, or is hydrogen,
R5 is =O or hydroxyl or is hydrogen,

A is hydrogen ortetrahydro-pyran compound represented by the following general formula (II) 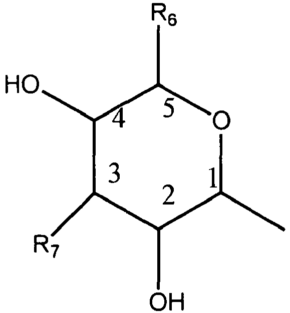
R6 is hydroxyl, ethyl, methylene, oxymethyl, nitrite, carboxyl, aldehyde, amino, or oxyamino.
R7 is hydroxyl or carboxyl

The invention further relates to these compounds for use in such treatment or it concerns the use of these compounds to manufacture a medicament or a dosage form for the above mentioned treatments.

A method for treating a subject suffering from or at risk of a disease or condition for which PPAR modulation provides a therapeutic benefit wherein said disease or condition is a PPAR-mediated disease or condition selected from the group of lipid disorders consisting of hyperlipidemia, dyslipidemia including associated diabetic dyslipidemia and hypoalphalipoproteinemia, hypertriglyceridemia, hypercholesterolemia, and low HDL (high density lipoprotein)), comprising: administering to said subject an effective amount of a diterpenoic or diterpenoic tetrahydropyran PPAR modulator of the group of compounds diterpenoic compounds such as isosteviol, dihydrosteviol, steviol and the diterpene-O-tetrahydro-pyran derivatives, such as isosteviol-19-0-β-D-glucuronide, dihydrosteviol-19-0-β-D-glucuronide, steviol-19-0-β-D-glucuronide or stevioside, steviol-O-β-D-Glucopyranosiduronic acid, steviol-O-β-D-Glucopyranoside, steviol-O-α-D-Glucopyranoside, steviol-O-β-D-Glucopyranose,, steviol-O-β-D-gluco-Hexodialdo-1,5-pyranoside, steviol-O-β-D-Glucopyranoside, steviol-O-β-L-Mannopyranose, steviol-O-β-D-Glucopyranose, steviol-O-β-D-Xylopyranoside, steviol-O-β-L-Xylopyranose, steviol-O-β-D-galacto-Heptopyranoside, steviol-O-β-D-Galactopyranoside, rebaudioside A, rebaudioside C, Dulcoside A, steviolbioside, rebaudioside B, rebaudioside D and rebaudioside E and pharmaceutically acceptable salts or esters thereof and/or mixtures thereof or having the chemical structure according to the following general formula (I): 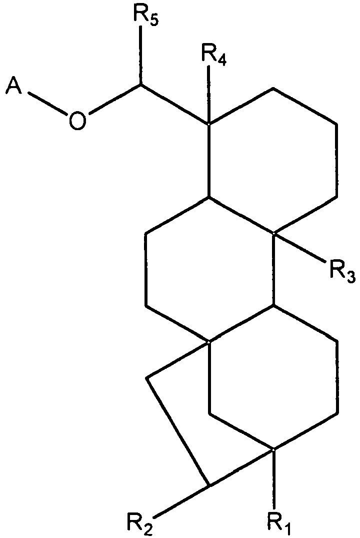 wherein
R1 is methyl or hydroxyl,
R2 is oxygen, methyl, oxymethyl, hydroxyl, or forms an ethene, ethanol, 2-methoxy-propene, formaldehyde or 1-methoxy-ethanol bound.
R3 is methyl, ethyl,
R4 is methyl, ethyl,
R5 is oxygen or hydroxyl

A is hydrogen or a tetrahydro-pyran compound represented by the following general formula (II) 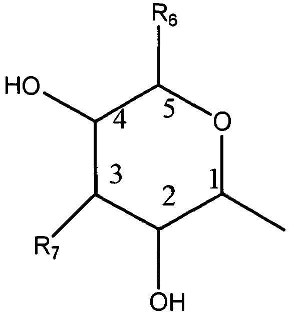
R6 is hydroxyl, ethyl, methylene, oxymethyl, nitrite, carboxyl, aldehyde, amino, or oxyamino.
R7 is hydroxyl or carboxyl

The invention further relates to these compounds for use in such treatment or it concerns the use of these compounds to manufacture a medicament or a dosage form for the above mentioned treatments.

The invention further relates to these compounds for use in such treatment or it concerns the use of these compounds to manufacture a medicament or a dosage form for the above-mentioned treatments.

A method for treating a subject to induce weight loss, to counteract weight gain due to overeating or to induce a calorie-burning effect, for instance a calorie-burning effect similar to vigorous exercise for which PPAR modulation provides a therapeutic benefit comprising: administering to said subject an effective amount of a diterpenoic or diterpenoic tetrahydropyran PPAR modulator of the group of compounds diterpenoic compounds such as isosteviol, dihydrosteviol, steviol and the diterpene-O-tetrahydro-pyran derivatives, such as isosteviol-19-0-β-D-glucuronide, dihydrosteviol-19-0-β-D-glucuronide, steviol-19-0-β-D-glucuronide or stevioside, steviol-O-β-D-Glucopyranosiduronic acid, steviol-O-β-D-Glucopyranoside, steviol-O-α-D-Glucopyranoside, steviol-O-β-D-Glucopyranose,, steviol-O-β-D-gluco-Hexodialdo-1,5-pyranoside, steviol-O-β-D-Glucopyranoside, steviol-O-β-L-Mannopyranose, steviol-O-β-D-Glucopyranose, steviol-O-β-D-Xylopyranoside, steviol-O-β-L-Xylopyranose, steviol-O-β-D-galacto-Heptopyranoside, steviol-O-β-D-Galactopyranoside, rebaudioside A, rebaudioside C, Dulcoside A, steviolbioside, rebaudioside B, rebaudioside D and rebaudioside E and pharmaceutically acceptable salts or esters thereof and/or mixtures thereof or having the chemical structure according to the following general formula (I): 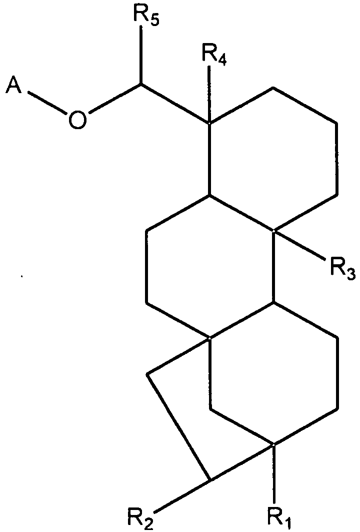 wherein
R1 is methyl or hydroxyl,
R2 is oxygen, methyl, oxymethyl, hydroxyl, or forms an ethene, ethanol, 2-methoxy-propene, formaldehyde or 1-methoxy-ethanol bound.
R3 is methyl or ethyl,
R4 is methyl or ethyl,
R5 is oxygen or hydroxyl

A is hydrogen or a tetrahydro-pyran compound represented by the following general formula (II) 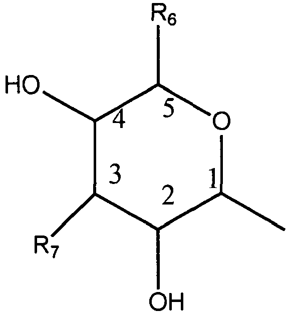
R6 is hydroxyl, ethyl, methylene, oxymethyl, nitrite, carboxyl, aldehyde, amino, or oxyamino.
R7 is hydroxyl or carboxyl

The invention further relates to these compounds for use as weight-loss drug in a treatment to protect against weight gain on high-fat and high-caloric diets or it concerns the use of these compounds to manufacture a medicament or a dosage form for the above mentioned treatments.

Other suitable compounds for the method of treatment of present invention, for use in a method to treat or to manufacture a medicament to or to increase cellular PPAR activity and treat a disease or condition which is a PPAR-mediated disease or a disease that can be treated through PPAR-dependent mechanisms are compounds of the group consisting of 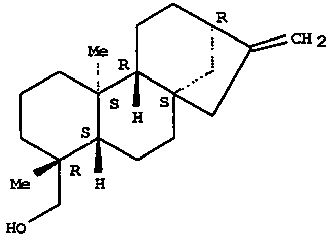 Kaur-16-en-18-ol, (4a)- 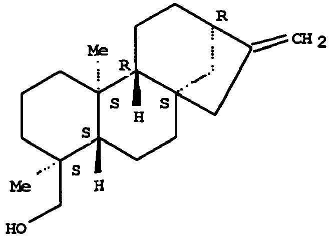 Kaur-16-en-18-ol, (4**β**)- (9Cl) 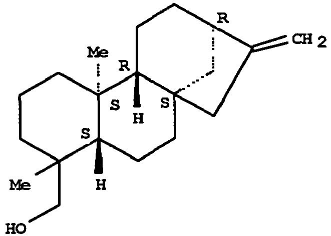 Kaur-16-en-18-ol (7Cl,9Cl) 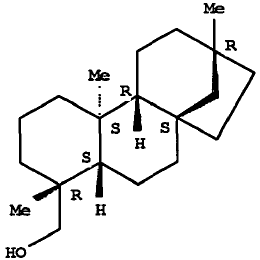 17-Norkauran-18-ol, 13-methyl-, (4**α**,8**β**,13**β**)- (9Cl) 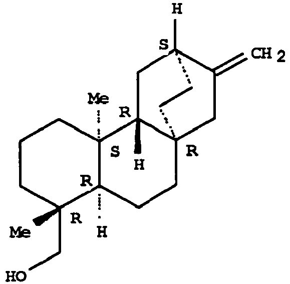 Atis-16-en-18-ol, (4**α**,5**β**,8**α**,9**β**,10**α**,12**α**)- (9Cl) 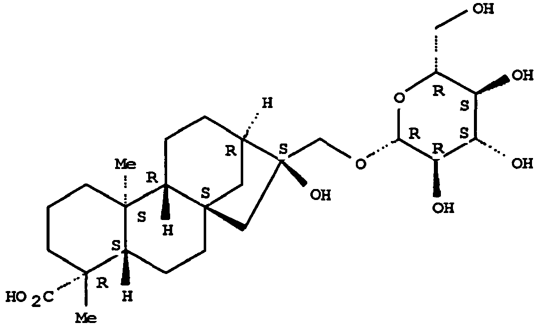 Kauran-18-oic acid, 17-(β-D-glucopyranosyloxy)-16-hydroxy-, (4α,16α)- (9Cl) or -2,10a-Ethanophenanthrene, kauran-18-oic acid deriv. ; Sagittarioside B 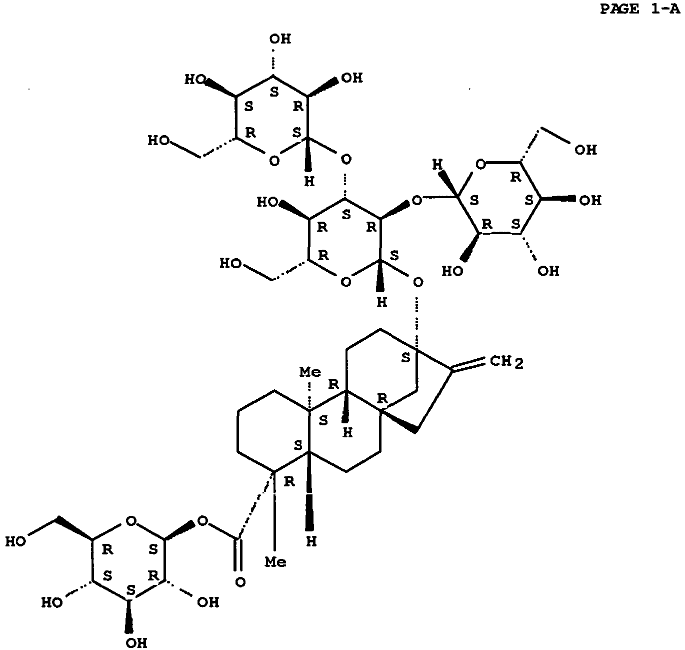 REBAUDIOSIDE A: Kaur-16-en-18-oic acid, 13-[(O-b-D-glucopyranosyl-(1®2)-O-[b-D-glucopyranosyl-(1®3)]-b-D-glucopyranosyl)oxy]-, b-D-glucopyranosyl ester, (4a)- 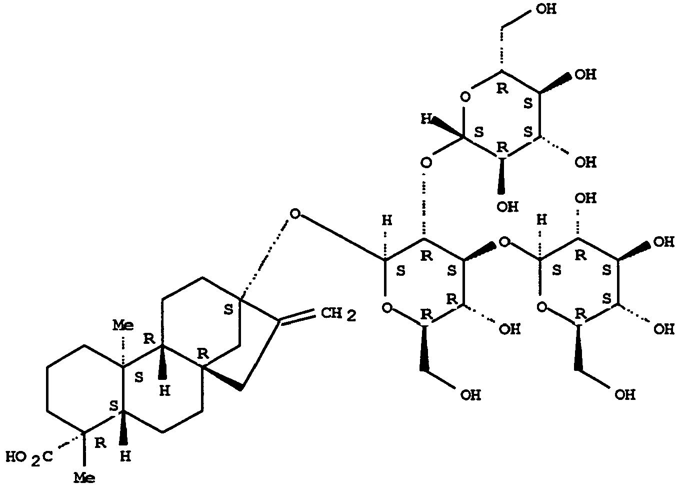 REBAUDIOSIDE B(P) or also named Kaur-16-en-18-oic acid, 13-[(O-β-D-glucopyranosyl-(1→2)-O-[β-D-glucopyranosyl-(1→3)]-β-D-glucopyranosyl)oxy]-, (4α)-

Other suitable compounds for the method of treatment of present invention, for use in a method to treat or to manufacture a medicament to or to increase cellular PPAR activity and treat a disease or condition which is a PPAR-mediated disease or a disease that can be treated through PPAR-dependent mechanisms are compounds with the basic structure hereunder of which R1, R2, R3, R4 and R5 are as described hereunder (Table III) .

**Table III**

| | | | | | |
|---|---|---|---|---|---|
| 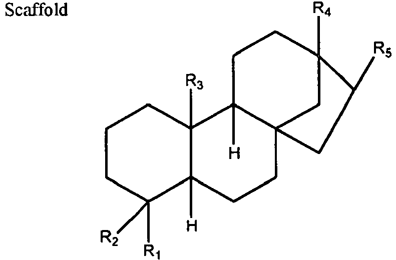 | | | | | |

| Group | R1 | R2 | R3 | R4 | R5 |
|---|---|---|---|---|---|
| 1 | -CH3 | -COOH | -CH3 | -OH | =CH2 |
| 2 | -COOH | -CH3 | -CH3 | -OH | =O |
| 6 | 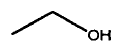 | -CH3 | -CH3 | -H | =CH2 |
| 11 | -CH3 | -COO2 | -CH3 | -CH3 | =O |
| 15 | -COOH | -CH3 | -CH3 | -OH | -CH3 |
| 16 | 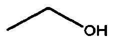 | -CH3 | -CH3 | -CH3 | -H |

Other suitable compounds for the method of treatment of present invention, for use in a method to treat or to manufacture a medicament to or to increase cellular PPAR activity and treat a disease or condition which is a PPAR-mediated disease or a disease that can be treated through PPAR-dependent mechanisms are compounds the basic structure hereunder of which R1, R2, R3, R4, R5 and R6 are as described hereunder (Table V)

**Table V.**

| | | | | | | |
|---|---|---|---|---|---|---|
| 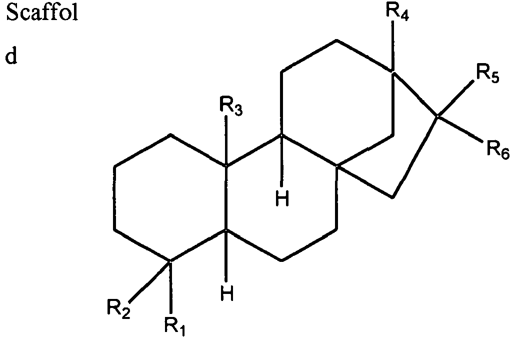 | | | | | | |

| Group | R1 | R2 | R3 | R4 | R5 | R6 |
|---|---|---|---|---|---|---|
| 27 | -CH3 | -COOH | -CH3 | -OH | -H | -OH |
| 28 | -COOH | -CH3 | -CH3 | -OH | -H | -OH |
| 32 | 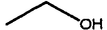 | -CH3 | -CH3 | -H | -H | -OH |
| 37 | -CH3 | -COO2 | -CH3 | -CH3 | -H | -OH |
| 41 | -COOH | -CH3 | -CH3 | -OH | -H | -OH |
| 42 | 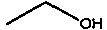 | -CH3 | -CH3 | -CH3 | -H | -OH |
| 51 | -COOH | -CH3 | -CH3 | -CH3 | -H | -OH |

Other suitable compounds for the method of treatment of present invention, for use in a method to treat or to manufacture a medicament to or to increase cellular PPAR activity and treat a disease or condition which is a PPAR-mediated disease or a disease that can be treated through PPAR-dependent mechanisms are compounds of the group consisting of 7a,15a-Methano-8H-naphth[2',1':5,6]azuleno[2,1-d]phenanthrene-4,7-dione, 1,2,2a,3,5,5a,9,9a,10,11,12,13,13a,13b,14,15-hexadecahydro-1,16-dihydroxy-17-methoxy-3,3,10,10,13a-pentamethyl-18-(1-methylethyl)-, (1R,2aR,5aS,13aR)-rel-(-)- (9Cl), Kauran-16-ol, 13-methyl-, (8**β**,13**β**)- (9Cl), Kaurane-16,18-diol, 13-methyl-, (4**α**,8**β**,13**β**)- (9Cl), Kauran-18-oic acid, 16-hydroxy-13-methyl-, (4a,8b,13b)- (9Cl) or ent-16b-Hydroxy-16a-methylbeyeran-19-oic acid, Kauran-18-oic acid, 11,13,16,17-tetrahydroxy-, (4a,11a)-, Kaur-16-en-18-oic acid, 7,11,13-trihydroxy-, (4**α**,7**β**,11**α**)-, Kauran-18-oic acid, 16,17-epoxy-11,13-dihydroxy-, (4a,11a)-, Kauran-18-oic acid, 17-(acetyloxy)-13,16-dihydroxy-, (4**α**)-, Kaur-16-en-18-oic acid, 13-hydroxy-7-oxo-, (4**α**)-, 1H-2,10a-Ethanophenanthrene-8-carboxylic acid, dodecahydro-10-hydroxy-2-(hydroxymethyl)-4b,8-dimethyl-12-oxo-(2S,4aS,4bS,8R,8aS,10R,10aS)- , 17-Norkauran-18-oic acid, 16-hydroxy-13-methyl-, methyl ester, (4**α**,8**β**,13**β**,16**α**)-, Kaur-16-ene-3,18-diol, 13-methyl-, (3**α**,4**α**,8**β**,13**β**)-(±)- (9Cl), 17-Norkauran-18-oic acid, 13-methyl-16-(2-oxopropylidene)-, (4**α**,8**β**,13**β**)-(±)- (9Cl), Kaur-16-en-18-oic acid, 13-methyl-3-oxo-, ethyl ester, (4**β**,8**β**,13**β**)-(±)- (9Cl), Kaur-16-en-18-oic acid, 3-hydroxy-13-methyl-, ethyl ester, (3**α**,4**α**,8**β**,13**β**)-(±)- (9Cl), and Kaur-16-en-18-oic acid, 13-methyl-3-oxo-, ethyl ester, (4**α**,8**β**,13**β**)-(±)- (9Cl) or functional derivatives thereof.

Particularly suitable compounds for the method of treatment of present invention, for use in a method to treat or to manufacture a medicament to or to increase cellular PPAR activity and treat a disease or condition which is a PPAR-mediated or a disease that can be treated through PPAR-dependent mechanisms are compounds of the group consisting of 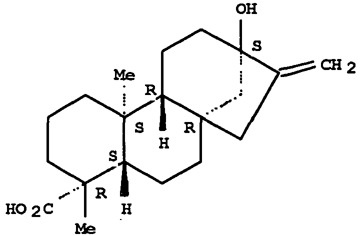 steviol 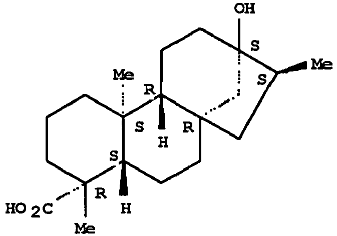 Kauran-18-oic acid, 13-hydroxy-, (4a)- (9Cl) or Steviol, dihydro- (6Cl,7Cl); 1H-2,10a-Ethanophenanthrene, kauran-18-oic acid deriv.; Dihydrosteviol 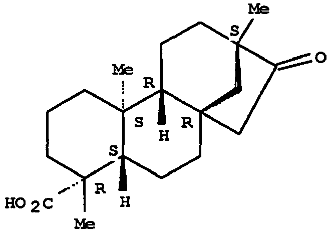 17-Norkauran-18-oic acid, 13-methyl-16-oxo-, (4a,8b,13b)- or 17-Nor-8b,13b-kauran-18-oic acid, 13-methyl-16-oxo- (7Cl,8Cl); Isosteviol (6Cl); 1H-2,10a-Ethanophenanthrene, 17-norkauran-18-oic acid deriv.; (-)-Isosteviol; 1H-2,10a-Ethanophenanthrene-8-carboxylic acid, dodecahydro-2,4b,8-trimethyl-12-oxo-, (2S,4aR,4bS,8R,8aS,10aR)-; Ketoisostevic acid; NSC 231875; iso-Steviol 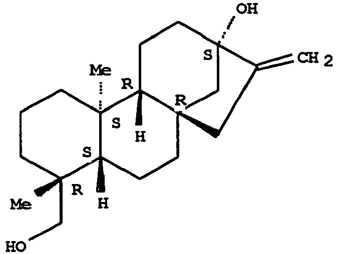 Kaur-16-ene-13,18-diol, (4a)- (9Cl) or 1H-2,10a-Ethanophenanthrene, kaur-16-ene-13,18-diol deriv.; (-)-ent-Kaur-16-en-13,19-diol; ent-Kaur-16-ene-13,19-diol 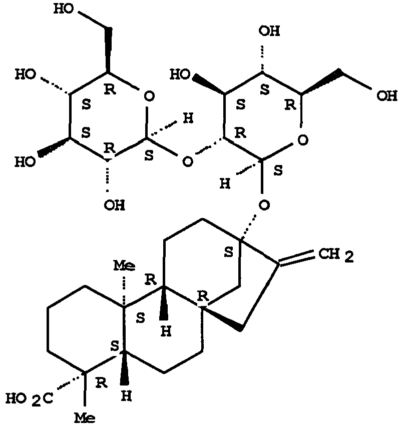 Kaur-16-en-18-oic acid, 13-[(2-O-b-D-glucopyranosyl-b-D-glucopyranosyl)oxy]-, (4a)- or Steviobioside; Steviolbioside. 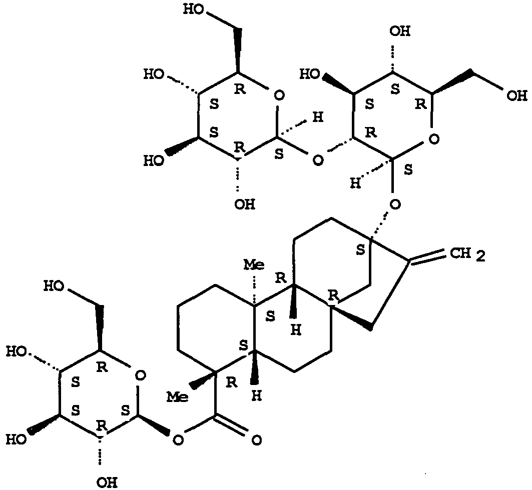 Kaur-16-en-18-oic acid, 13-[(2-O-b-D-glucopyranosyl-b-D-glucopyranosyl)oxy]-, b-D-glucopyranosyl ester, (4a)- or Stevioside (6Cl,7Cl); 1H-2,10a-Ethanophenanthrene, kaur-16-en-18-oic acid deriv.; a-G-Sweet; Steviosin. 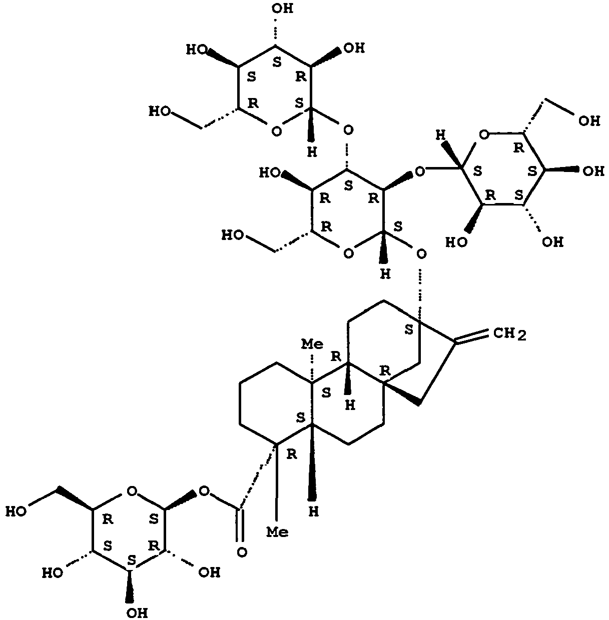 Kaur-16-en-18-oic acid, 13-[(O-b-D-glucopyranosyl-(1→2)-O-[b-D-glucopyranosyl-(1→3)]-b-D-glucopyranosyl)oxy]-, b-D-glucopyranosyl ester, (4a)- or 4G-S; Glycoside A3, from Stevia rebaudiana; Glycoside X; Rebaudioside A; Stevioside a3; Sweetener 4G-S. 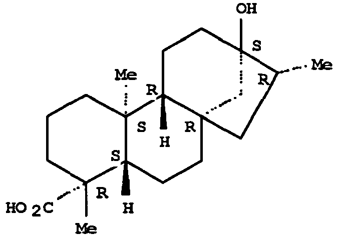 Kauran-18-oic acid, 13-hydroxy-, (4a,16a)- (9Cl) or Dihydrosteviol B 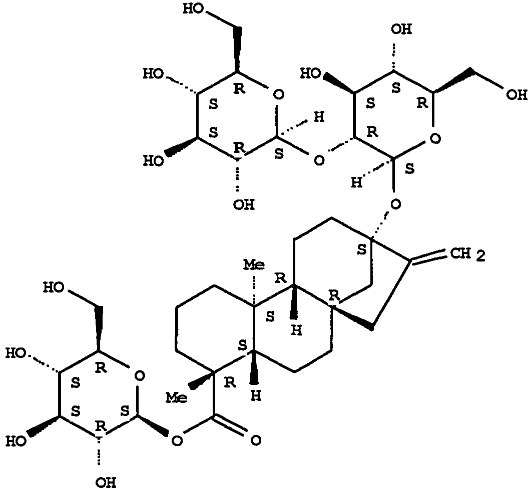 stevioside

Particularly suitable compounds for the method of treatment of present invention, for use in a method to treat or to manufacture a medicament to or to increase cellular PPAR activity and treat a disease or condition which is a PPAR-mediated or a disease that can be treated through PPAR-dependent mechanisms are compounds of the group consisting of 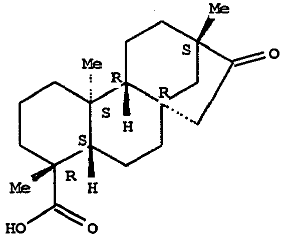 1H-2,10a-Ethanophenanthrene-8-carboxylic acid, dodecahydro-2,4b,8-trimethyl-12-oxo-, (2S,4aR,4bS,8R,8aS,10aR)- 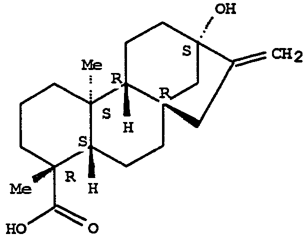 Kaur-16-en-18-oic acid, 13-hydroxy-, (4a)- 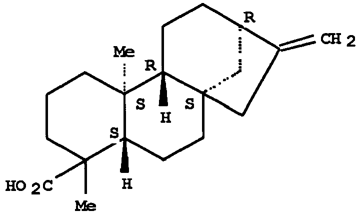 Kaur-16-en-18-oic acid (9Cl) 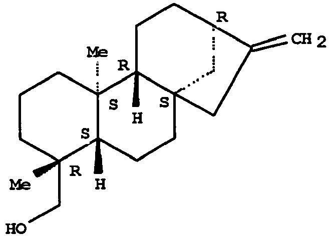 Kaur-16-en-18-ol, (4α)- or (-)-Kaur-16-en-18-ol; (-)-Kauren-19-ol; Kaurenol; ent-Kaurenol and 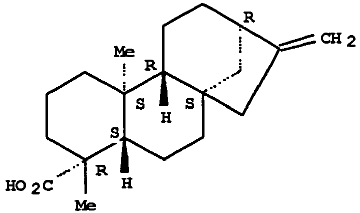 Kaur-16-en-18-oic acid, (4α)- or (-)-Kaur-16-en-19-oic acid; (-)-Kauren-19-oic acid; 4α-Kaur-16-en-18-oic acid; Cunabic acid; Kaurenic acid; Kaurenoic acid; NSC 339145; ent-Kaur-16(17)-en-19-oic acid; ent-Kaur-16-en-19-oic acid; ent-Kaurenoic acid

Suitable dosages of such above described PPAR agonist(s) to treat, prevent or reduce the disorders of present inventions are in the range of 100 µg to 500 mg/kg body weight, more preferably in the range of 250 µg to 100 mg / kg body weight, yet more preferably 500 µg g to 50 mg per kg body weight, and most preferably 1 mg to 25 mg / kg body weight.

The invention further relates to these compounds for use in such treatment or it concerns the use of these compounds to manufacture a medicament for the above-mentioned treatments.

### Detailed Description

### DETAILED DESCRIPTION OF EMBODIMENTS OF THE INVENTION

The following detailed description of the invention refers to the accompanying drawings. In addition, the following detailed description does not limit the invention. Instead, the scope of the invention is defined by the appended claims and equivalents thereof.

It will be apparent to those skilled in the art that various modifications and variations can be made in the compounds, processes of production of the same, the use of the same and using disclosure of the present invention and in construction of the system and method without departing from the scope or spirit of the invention. Examples of such modifications have been previously provided.

Other embodiments of the invention will be apparent to those skilled in the art from consideration of the specification and practice of the invention disclosed herein. It is intended that the specification and examples be considered as exemplary only, with a true scope and spirit of the invention being indicated by the following claims.

Macrophages are cells within the tissues that originate from specific white blood cells called monocytes. Monocytes and macrophages are phagocytes, acting in nonspecific defence or innate immunity, as well as specific defence or cell-mediated immunity of vertebrate animals. Their role is to phagocytise (engulf and then digest) cellular debris and pathogens either as stationary or mobile cells, and to stimulate lymphocytes and other immune cells to respond to the pathogen. Oxidized LDL is the result of oxidative modification of LDL in the circulation and/or the adiopose tissue and/or the vessel wall, by cellular and/or a-cellular mechanisms, which do or do not involve enzymes of which the activity is dependent or independent of metal ions. The uptake of oxidized LDL by macrophages results in the generation of foam cells, a primary event in the development of atherosclerosis.

The meaning of pleiotropic in present invention is 1. producing many effects or 2. multiple effects from a single gene. For example, the Marfan gene is pleiotropic with widespread effects and can cause long fingers and toes (arachnodactyly), dislocation of the lens of the eye, and dissecting aneurysm of the aorta. For example, the pleiotropic effects of statins include improvement of endothelial dysfunction, increased nitric oxide bioavailability, antioxidant properties, inhibition of inflammatory responses, and stabilization of atherosclerotic plaques independent of their cholesterol lowering effect. Related to this invention, an effect of a compound on PPAR expression in the adipose tissues may lead to changes in insulin signalling in the vessel wall where it affects the expression of antioxidant enzymes which inhibit the accumulation of oxidized LDL in the vessel wall, and thereby atherosclerosis.

As used herein, a "subject" includes mammals, e.g., humans, companion animals (e.g., dogs, cats, birds and the like), farm animals (e.g., cows, sheep, pigs, horses, fowl and the like) and laboratory animals (e.g., rats, mice, guinea pigs and the like). In a preferred embodiment of the disclosed methods, the subject is human.

HOMA (Homeostatic model assessment) = insulin resistance by HOMA. HOMA index is one of the methods used for insulin resistance (IR) measurement, mainly in epidemiological studies.

Most obese individuals have elevated plasma levels of FFA which are known to cause peripheral (muscle) insulin resistance. They do this by inhibiting insulin-stimulated glucose uptake and glycogen synthesis. The mechanism involves intramyocellular accumulation of diacylglycerol and activation of protein kinase C. FFAs also cause hepatic insulin resistance. They do this by inhibiting insulin-mediated suppression of glycogenolysis. On the other hand, FFAs support between 30 and 50 % of basal insulin secretion and potentate glucose-stimulated insulin secretion.

Dyslipemia is a metabolic dysfunction of lipids typically diagnosed by high triglyceride levels and/or low levels of HDL cholesterol, and/or the occurrence of small dense LDL which are prone to oxidation and thus can be associated with high levels of circulating oxidized LDL.

The following terms are similar, yet distinct, in both spelling and meaning, and can be easily confused: arteriosclerosis, arteriolosclerosis and atherosclerosis.

Arteriosclerosis also called hardening of the arteries chronic disease is characterized by abnormal thickening and hardening of the walls of arteries, with a resulting loss of elasticity. The major form of arteriosclerosis is atherosclerosis, in which plaques of consisting of macrophages, fatty deposits in foam cells, or atheromas, form on the inner walls of the arteries. These fatty acids are largely due to the uptake of oxidized LDL by macrophages. Arteriosclerosis is a general term describing any hardening (and loss of elasticity) of medium or large arteries (in Greek, "Arterio" meaning artery and "sclerosis" meaning hardening); arteriolosclerosis is arteriosclerosis mainly affecting the arterioles (small arteries); atherosclerosis is a hardening of an artery specifically due to an atheromatous plaque. Therefore, atherosclerosis is a form of arteriosclerosis. Arteriosclerosis ("hardening of the artery") results from a deposition of tough, rigid collagen inside the vessel wall and around the atheroma. This increases the stiffness, decreases the elasticity of the artery wall. Arteriolosclerosis (hardening of small arteries, the arterioles) is the result of collagen deposition, but also muscle wall thickening and deposition of protein ("hyaline"). Calcification, sometimes even ossification (formation of complete bone tissue) occurs within the deepest and oldest layers of the sclerosed vessel wall.

Atherosclerosis causes two main problems. First, the atheromatous plaques, though long compensated for by artery enlargement, eventually lead to plaque ruptures and stenosis (narrowing) of the artery and, therefore, an insufficient blood supply to the organ it feeds. If the compensating artery enlargement process is excessive, a net aneurysm results. Atherosclerosis chronic disease is caused by the deposition of fats, cholesterol, calcium, and other substances in the innermost layer (endothelium) of the large and medium-sized arteries. Atherosclerosis is a disease affecting the arterial blood vessel. It is commonly referred to as a "hardening" or "furring" of the arteries. It is caused by the formation of multiple plaques within the arteries.

These complications are chronic, slowly progressing and cumulative. Most commonly, soft plaque suddenly ruptures cause the formation of a thrombus that will rapidly slow or stop blood flow, e.g. 5 minutes, leading to death of the tissues fed by the artery. This catastrophic event is caled an infarction. For instance, the also called vulnerable plaque is such atheromatous plaque, which is particularly prone to produce sudden major problems, such as a heart attack or stroke. One of the most common recognized scenarios is caled coronary thrombosis of a coronary artery causing myocardial infarction (a heart attack). Another common scenario in very advanced disease is claudication from insufficient blood supply to the legs, typically due to a combination of both stenosis and aneurismal segments narrowed with clots. Since atherosclerosis is a body wide process, similar events also occur in the arteries to the brain, intestines, kidneys, legs, etc.

Pathologically, the atheromatous plaque is divided into three distinct components: the nodular accumulation of a soft, flaky, yellowish material at the centre of large plaques composed of macrophages nearest the lumen of the artery; sometimes with underlying areas of cholesterol crystals; and possibly also calcification at the outer base of older/more advanced lesions.

Hypercholesterolemia is an excess of cholesterol in the blood.

Thrombogenicity refers to the tendency of a material in contact with the blood to produce a thrombus, or clot. It not only refers to fixed thrombi but also to emboli i.e. thrombi which have become detached and travel through the bloodstream. Thrombogenicity can also encompass events such as the activation of immune pathways and the complement system. All materials are considered to be thrombogenic with the exception of the endothelial cells which line the vasculature. Certain medical implants appear non-thrombogenic due to high flow rates of blood past the implant, but in reality, all are thrombogenic to a degree. A thrombogenic implant will eventually be covered by a fibrous cap, the thickness of this capsule can be considered one measure of thrombogenicity, and if extreme can lead to the failure of the implant.

In cell biology, peroxisome proliferator activated receptors (PPARs) are a group of nuclear receptor isoforms that exist across biology. Originally identified in Xenopus frogs as receptors that induce the proliferation of peroxisomes in cells, PPARs are intimately connected to cellular metabolism (carbohydrate, lipid and protein) and cell differentiation. They are transcription factors.

Three types of PPARs have been identified: alpha, gamma and delta (beta).
α (alpha) - expressed in liver, kidney, heart, muscle, adipose tissue, and others.
γ (gamma) - although transcribed by the same gene, this PPAR exists in three forms:
γ1 - expressed in virtually all tissues, including heart, muscle, colon, kidney, pancreas and spleen.
y2 - expressed mainly in adipose tissue (30 amino acids longer)
y3 - expressed in macrophages, large intestine, white adipose tissue.
B (beta) or δ (delta) - expressed in many tissues but markedly in brain, adipose tissue and skin.

The term "pharmaceutically acceptable " is used adjectivally herein to mean that the compounds are appropriate for use in a pharmaceutical product.

As used herein, the phrase "pharmaceutically acceptable salts" or "nutriceutically acceptable salts" refers to salts prepared from pharmaceutically acceptable, preferably nontoxic, acids and bases, including inorganic and organic acids and bases, including but not limited to, sulfuric, citric, maleic, acetic, oxalic, hydrochloride, hydro bromide, hydro iodide, nitrate, sulfate, bisulfite, phosphate, acid phosphate, isonicotinate, acetate, lactate, salicylate, citrate, acid citrate, tartrate, oleate, tannate, pantothenate, bitartrate, ascorbate, succinate, maleate, gentisinate, fumarate, gluconate, glucaronate, saccharate, fornate, benzoate, glutamate, methanesulfonate, ethanesulfonate, benzenesulfonate, p-toluenesulfonate and pamoate (i.e., 1,1'-methylene-bis-(2-hydroxy-3-naphthoate)) salts. Pharmaceutically acceptable salts include those formed with free amino groups such as, but not limited to, those derived from hydrochloric, phosphoric, acetic, oxalic, and tartaric acids. Pharmaceutically acceptable salts also include those formed with free carboxyl groups such as, but not limited to, those derived from sodium, potassium, ammonium, sodium lithium, calcium, magnesium, ferric hydroxides, isopropylamine, triethylamine, 2-ethylamino ethanol, histidine, and procaine.

As used herein, the term "carrier" refers to a diluent, adjuvant, excipient, or vehicle. Such carriers can be sterile liquids, such as saline solutions in water, or oils, including those of petroleum, animal, vegetable or synthetic origin, such as peanut oil, soybean oil, mineral oil, sesame oil and the like. A saline solution is a preferred carrier when the pharmaceutical composition is administered intravenously. Saline solutions and aqueous dextrose and glycerol solutions can also be employed as liquid carriers, particularly for injectable solutions.

As used herein, the tern "mineral" refers to a substance, preferably a natural substance that contains calcium, magnesium or phosphorus. Illustrative nutrients and minerals include beef bone, fish bone, calcium phosphate, egg shells, sea shells, oyster shells, calcium carbonate, calcium chloride, calcium lactate, calcium gluconate and calcium citrate.

As used herein, the term "biological sample" is broadly defined to include any cell, tissue, organ or multicellular organism. A biological sample can be derived, for example, from cells or tissue cultures in vitro. Alternatively, a biological sample can be derived from a living organism or from a population of single cell organisms. Preferably, the biological sample is live tissue. More preferably, the biological sample is live bone or adipose tissue.

The term "treatment" refers to any process, action, application, therapy, or the like, wherein a mammal, including a human being, is subject to medical aid with the object of improving the mammal's condition, directly or indirectly.

The term 'a compound that increases the expression' refers here to gene expression and thus to the increase of gene transcription and/or translation of a gene transcript (mRNA) such as for example the PPAR gene or PPAR mRNA. Preferably said increase is at least 10%, 20%, 30%, 40%, 50%, 60%, 70%, 80%, 90% or even higher. The term 'a compound that increases the activity' refers here to the receptor that is produced such as the PPAR receptor or to the receptor activity.

The present invention relates to the diterpenoic tetrahydropyran, steviol-19-glucuronide (a natural metabolite of stevioside) and other diterpenoic tetrahydropyran derivatives. Stevioside (250 mg capsules) given thrice daily to 10 healthy subjects according to the present invention does not provide significant differences at different time points 3 days after the administration of stevioside between the control and the stevioside condition for blood pressure and blood biochemical parameters. The 24-hour urinary volume and urinary excretion of electrolytes are not significantly different. Likewise, no significant difference can be found for mean blood glucose and insulin between control and stevioside condition. However, oral administration of stevioside results in the surprising finding that it increases the expression and activity of PPARs, and that it exerts system biological effects to normalise lipid metabolism .

To date, three PPAR subtypes have been identified: α (Accession numbers L02932, NM_001001928, MGI:104740, NM_005036, MGC2237; MGC2452, Sher,T., et al. Biochemistry 32 (21), 5598-5604 (1993)), β or δ (HPRD:02679; MIM:600409, Schmidt,A., et al. Mol. Endocrinol. 6 (10), 1634-1641 (1992)), and γ (HGNC:9236 , HPRD:03288; MIM:601487, Lambe,K.G. and Tugwood,J.D.Eur. J. Biochem. 239 (1), 1-7 (1996)). They perform different physiological functions based on their divergent patterns of tissue expression, ligand-binding specificities, and physiological functions when activated. PPARalpha was originally cloned from a mouse liver cDNA library (Issemann I, et al. Nature. 1990;347:645-650) and subsequently cloned from frogs,Dreyer C, et al. Cell. 1992;68:879-887guinea pigs, Göttlicher M, et al. Proc Natl Acad Sci USA. 1992;89:4653-4657 rats, and humans (Sher T, et al. Biochemistry. 1993;32:5598-5604 and Mukherjee R, et al. J Steroid Biochem Mol Biol. 1994;51:157-166). PPARalpha is considered a therapeutic target for dyslipidemia with a promising future given the existing literature and clinical experience with fibrates throughout several decades of clinic use. Fibrates have been used to treat dyslipidemia because of their triglyceride lowering and high-density lipoprotein-elevating effects. More recent research demonstrates the anti-inflammatory properties of PPARalpha agonists at the vessel wall, which can contribute to the reduction of atherosclerosis observed in animal models and humans (Li AC, et al. J Clin Invest. 2004;114:1564-1576 and Rubins HB, et al. N Engl J Med. 1999;341:410-418). The PPARalpha or PPARalpha gene and its expression has well characterised and described in various publication [Sher,T., et al. cDNA cloning, chromosomal mapping, and functional characterization of the human peroxisome proliferator activated receptor Biochemistry 32 (21), 5598-5604 (1993), Waxman,J.S. and Yelon,D. Dev. Dyn. 236 (2), 587-595 (2007), Plager,D.A., et al. Exp. Dermatol. 16 (1), 28-36 (2007), Ziouzenkova,O., et al. Mol. Endocrinol. 21 (1), 77-88 (2007), Jedidi,I., et al.Biochem. Biophys. Res. Commun. 351 (3), 733-738 (2006) Reynolds,W.F., et al. Biochem. Biophys. Res. Commun. 349 (2), 846-854 (2006), Tugwood,J.D., et al. Ann. N. Y. Acad. Sci. 804, 252-265 (1996), Chu,R., et al. J. Biol. Chem. 271 (44), 27670-27676 (1996), Miyata,K.S., et al; J. Biol. Chem. 271 (16), 9189-9192 (1996), Mukherjee,R, et al. J. Steroid Biochem. Mol. Biol. 51 (3-4), 157-166 (1994)] and is thus truly available to the man skilled in the art. Recombinant PPARalpha protein is obtainable from an E coli strain that carries the coding sequence of the human PPARalpha under the control of a T7 promoter and is available from ActiveMotif.

Atherosclerosis, the underlying cause of heart attacks, stroke and peripheral vascular disease, is responsible for over 50% of all deaths in developed countries. The process is believed to be triggered by damage to the arterial endothelial cells leading to dramatic changes in their properties [C.K. Glass and J.L. Witztum , Atherosclerosis: the road ahead. Cell 104 (2001), pp. 503-516]. This causes an infiltration of both T lymphocytes and monocytes to the site of damage. The monocytes then differentiate into macrophages, internalise lipoproteins, and transform into lipid-loaded foam cells to form the fatty streak seen in early lesions [C.K. Glass and J.L. Witztum , Atherosclerosis: the road ahead. Cell 104 (2001), pp. 503-516]. This transformation of macrophages into foam cells represents a critical initial event in the pathogenesis of atherosclerosis. Differential inhibition of macrophage foam-cell formation and atherosclerosis is controlled by PPARs (PPAR α, β/δ, and γ) [Li AC, et al. J Clin Invest. 2004;114:1564-1576] The recruitment of circulating monocytes into the vascular intima and their subsequent transformation into macrophage/foam cells are key elements of the initiation of atherosclerosis [Holvoet et al. Arteriosclerosis, Thrombosis, and Vascular Biology. 2006;26:1558.]. Present invention demonstrated that treatment with a diterpenoic compounds such as isosteviol, dihydrosteviol, steviol and the diterpene-O-tetrahydro-pyran derivatives, such as isosteviol-19-0-β-D-glucuronide, dihydrosteviol-19-0-β-D-glucuronide, steviol-19-0-β-D-glucuronide, rebaudioside A or stevioside; can decrease plaque formation and can decrease the infiltration of such macrophages in plaques. Moreover present invention demonstrated that treatment with a diterpenoic compounds such as isosteviol, dihydrosteviol, steviol and the diterpene-O-tetrahydro-pyran derivatives, such as isosteviol-19-0-β-D-glucuronide, dihydrosteviol-19-0-β-D-glucuronide, steviol-19-0-β-D-glucuronide or stevioside; induces increased expression of PPARalpha in adipose tissue. It is known in the art that PPARalpha activation leads to increases in the levels of lipoprotein lipase (Per Sauerberg, et al. J. Med. Chem.; 2002; 45(4) pp 789 - 804 and Panadero M, et al. J Physiol Biochem. 2006 Sep;62(3):189-98.). Lipoprotein lipase (EC 3.1.1.34) is an enzyme that hydrolyzes lipids in lipoproteins, like those found in chylomicrons and very low density lipoproteins (VLDL), into three fatty acids and one glycerol molecule. The very-low-density-lipoprotein receptor (VLDLR) is a lipoprotein receptor that shows considerable similarity to the low-density-lipoprotein receptor. This receptor has been suggested to be important for the metabolism of apoprotein-E-containing triacylglycerol-rich lipoproteins, such as very-low-density-lipoprotein (VLDL), beta-migrating VLDL and intermediate-density lipoprotein. Very low density lipoproteins and the VLDLR has been demonstrated a role in the risk of developing atherosclerotic cardiovascular disease (CVD) and age-related macular degeneration (AMD) [Haines JL, et al Invest. Ophthalmol. Vis. Sci. (2006] and has been also proposed to play a possible risk factor for Alzheimer disease (AD) [Neurosci. Lett. (1996Pritchard ML et al. and Nature Genetics 11, 207 - 209 (1995) Kaoru Okuizumi et al]. An increased concentration of atherogenic plasma lipoproteins is one of the most important risk factors for the development of atherosclerosis. Factors that promote the increase of atherogenic lipoproteins in plasma will therefore also promote the development of atherosclerosis followed by major increases in coronary heart diseases. One of the more important of these factors is an overproduction of large, triglyceride-rich very low density lipoproteins (VLDL). It has been earlier demonstrated that treatment of primary hepatocytes with PPARalpha agonists increases the production of apolipoprotein B (apoB100), the primary apolipoprotein in VLDL and LDL, while it decreases the biosynthesis of triglycerides. PPARalpha agonists thus induce a shift of apoB100, from VLDL to denser particles. The shift of apoB from large VLDL to smaller, less triglyceride rich VLDL results in the formation of less atherogenic LDL, which turn over more rapidly than LDL, formed from large VLDL. Thus, PPARalpha agonist can regulate the amounts of small and large VLDL that are being formed. This molecular mechanism for the increased secretion of apoB 100 has been investigated earlier and found to be a decrease in the intracellular degradation of apoB100. The degradation that was inhibited occurred after the translation of the proteins. Thus, PPARalpha appears to influence post-ER pre-secretory proteolysis rather than proteasomal degradation (Stillemark Pet al. J Biol Chem 2000; 275:10506-13; Asp L, et al. J Biol Chem 2000; 275:26285-92 and Olofsson SO, et al. Trends Cardiovasc Med 2000; 10:338-45).

Lipoprotein lipase is specifically found in endothelial cells lining the capillaries. It has been demonstrated that PPARalpha agonists lower plasma triglycerides, FFA and cholesterol (Per Sauerberg, et al. J. Med. Chem.; 2002; 45(4) pp 789 - 804). It has been demonstrated that PPARs play an important role in controlling cellular and whole-body sterol homeostasis, including fatty acid, triglyceride, and lipoprotein metabolism and reverse cholesterol transport. Binding of fatty acids, eicosanoids, and drug ligands to PPARalpha leads to activation of numerous genes involved in the uptake and beta-oxidation of fatty acids in the heart, kidney, and muscle. Increased diversion of fatty acids into beta-oxidation decreases the availability of fatty-acyl-coenzyme A substrates for triglyceride synthesis and, therefore, reduces secretion of very-low-density lipoprotein by the liver. PPAR agonists may also decrease triglyceride levels by increasing the expression of lipoprotein lipase in the liver (PPARalpha), in adipocytes (PPARalpha and PPARgamma), in skeletal muscle (PPARgamma), and in macrophages (PPARalpha and PPARgamma).

Moreover, PPARalpha can increase the expression and activity of the desaturases, Delta6 and Delta5 desaturases and stearoylcoenzyme A desaturase-1 but also can increase the expression of palmitoylcoenzyme A elongase, evidencing a dual regulation in the fatty acid biosynthesis at the level of desaturases and elongases, independently of insulinemia. (Montanaro MA et al. Lipids. 2007 Apr;42(3):197-210. Epub 2007 Jan 20). PAR-α agonists also decrease intracellular fatty acyl CoA and malonyl CoA, and increase fatty acid oxidation (Young ME et al. Am J Physiol Endocrinol Metab 2001;280:E471-479 and Furuhashi M, et al. J Endocrinol 2002;174:321-329). PPARalpha agonist treatment in human myocytes increases β-oxidation of oleate and decreases oleate incorporation into triglycerides. For instance, the PPARalpha agonist fenofibrate can increase palmitate oxidation and decrease intracellular lipids through increasing mitochondrial fatty acid beta oxidation (Nutrition & Metabolism 2007, 4:9 23 April 2007

Furthermore, PPARalpha agonist treatment increases palmitate beta oxidation and decreases PKC activity (protein kinase C-θ (PKC-θ) and protein kinase C-β (PKC-β)). PKC-θ has been reported to cause reduced insulin sensitivity in acute trauma (Shulman GI: 2004;19:183-190). PKC has been associated with insulin resistance in many settings. PKCs are thought to be inhibitory to glucose transporter translocation by causing serine phosphorylation of the insulin receptor and IRS-1 (Shulman GI: 2004;19:183-190). In animals overfed with either fat or glucose, increased levels of activated PKC and insulin resistance have been found (Laybutt DR, et al. Am J Physiol 1999;277:E1070-1076 and Schmitz-Peiffer C, et al. Diabetes 1997;46:169-178). Increased levels of activated PKC have also been found in diabetic patients (Itani SI, et al. Metabolism 2001;50:553-557). Thus, the reduction in PKC activity induced by the diterpenoic or diterpenoic or diterpenoic tetrahydropyran PPARalpha agonist of present invention is associated with improved insulin sensitivity, as been found and described in the examples and figures.

In the adipose tissue, the free fatty acid (FFA) binding protein 4 (FABP4) and glucose transporter (GLUT-4) are known regulators of fatty acid homeostasis and glucose transport which are under the transcriptional control of PPARs. Reducing the availability of FFAs from the adipose tissue to liver and muscles and heart is a pivotal component of the insulin-sensitizing mechanism of PPAR agonists in the adipose tissue. Improved insulin sensitivity can thus result from a decrease in circulating FFAs by increased uptake in the AT, supported by increased expression of FABP-4. This increase in FABP-4 was associated with an increase in PPARα expression, supporting its role in regulating FFA uptake in the AT (Verreth W, et al. Arteriosclerosis, Thrombosis, and Vascular Biology 2006;26:922-928). Increased insulin signalling due to increased expression of the insulin receptor (INSR) and of the major insulin receptor substrates IRS1 and IRS2 leads to improved glucose homeostasis by increased expression of the glucose transporter 4 (GLUT-4) (Thirone A, et al. Trends in Endocrinology and Metabolism 2006; 17:72-78). Impaired FA metabolism can also be due to CD36 deficiency. Decreased FA metabolism by non-hepatic peripheral tissues such as adipose and vascular tissues and on the large scale observed in CD36 null mice undoubtedly had an impact on glucose and amino acid homeostasis and might predispose deficient individuals to the development of metabolic disorders such as obesity and insulin resistance. CD36 deficiency has recently been linked to defective FA metabolism in spontaneously hypertensive rats and may contribute to the insulin resistance observed in this rodent model of type II diabetes (Aitman TJ, et al. Nature Genetics 1999; 21:76-83). In line with this, CD36 null mice (Abumrad NA, et al., Journal Biological Chemistry 1984; 259:8945-8953) and transgenic mice overexpressing CD36 (Ibrahimi A, et al. Proc. Natl. Acad. Sci. U.S.A. 1996; 93:2646-2651) exhibit altered levels of glucose and insulin .

Peroxisome proliferator-activated receptors (PPARs) are promising targets for the development of new drugs for the treatment of metabolic disorders such as diabetes, dyslipidemia and atherosclerosis. In clinical practice, PPARalpha agonists, such as the already available fibrates, improve dyslipidemia, while PPARgamma agonists, such as thiazolidinediones, improve insulin resistance and diabetes.

Peroxisome proliferator activating receptors (PPAR) are nuclear receptors that, when stimulated by endogenous lipids, activate specific genes involved in fat metabolism. The peroxisome proliferator activated receptors PPAR, PPARalpha, PPARgamma, and PPARdelta are ligand-activated transcription factors that play a key role in lipid homeostasis. J. Med. Chem., 50 (4), 685 -695, 2007. Michael L. Sierra, et al. PPARalpha agonists are used in cholesterol disorders (generally as an adjunctive to statins) and disorders that feature high triglycerides. They are widely prescribed as hypolipidemic agents to reduce triglycerides while increasing plasma HDL-cholesterol [Elisaf, Effects of fibrates on serum metabolic parameters, Curr. Med. Res. Opin. 18 (2002), pp. 269-276.]. Moreover, they reduce thrombogenicity [O. Barbier, I.P. Torra, Y. Duguay, C. Blanquart, J.C. Fruchart, C. Glineur and B. Staels, Pleiotropic actions of peroxisome proliferator-activated receptors in lipid metabolism and atherosclerosis, Arterioscler. Thromb. Vasc. Biol. 22 (2002), pp. 717-726].

Induction of PPARalpha or PPARgamma in adipose tissue, heart, or aortic arch is a key mechanism for reducing atherosclerosis and improving cardiovascular function resulting from weight loss. An improved lipid metabolism and insulin signalling, which can be induced by PPARs is associated with decreased tissue deposition of oxidized LDL that increases cardiovascular risk in persons with the metabolic syndrome (Verreth et al. Circulation. 2004;110:3259-3269). In particular, PPARgamma or PPARalpha expression is inversely related to plaque volume and to oxidized LDL content in the plaques.

Further scope of applicability of the present invention will become apparent from the detailed description given hereinafter. However, it should be understood that the detailed description and specific examples, while indicating preferred embodiments of the invention, are given by way of illustration only, since various changes and modifications within the spirit and scope of the invention will become apparent to those skilled in the art from this detailed description. It is to be understood that both the foregoing general description and the following detailed description are exemplary and explanatory only and are not restrictive of the invention, as claimed.

Present invention provides methods to synthesise steviol-19-glucuronide or to purify it of body excretion of a warm-blooded animal, such as a human, for instance from the urine of the animal.

An embodiment of present invention is essentially pure or an isolated tetrahydro-pyran-diterpene compound represented by the following general formula (I): 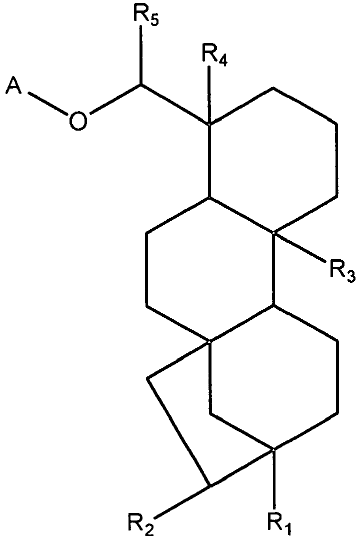 wherein
R1 is methyl or hydroxyl,
R2 is oxygen, methyl, oxymethyl, hydroxyl, or forms an ethene, ethanol, 2-methoxy-propene, formaldehyde or 1-methoxy-ethanol bound.
R3 is methyl, ethyl,
R4 is methyl, ethyl,
R5 is oxygen or hydroxyl

A is hydrogen or tetrahydro-pyran compound represented by the following general formula (II) 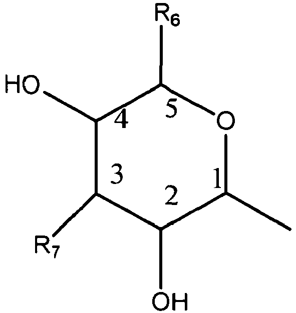
R6 is hydroxyl, ethyl, methylene, oxymethyl, nitrite, carboxyl, aldehyde, amino, or oxyamino.
R7 is hydroxyl or carboxyl
or a pharmaceutically acceptable salt of such a compound.

The essentially pure or an isolated compound can be a diterpenoic compound whereof an hydrogen has been replaced by a group selected of D-Galactopyranosiduronic acid, D-Glucopyranosiduronic acid, D-Glucopyranoside, D-Glucopyranose, D-gluco-Hexodialdo-1,5-pyranoside, L-Mannopyranose, D-Xylopyranoside, L-Xylopyranose, D-galacto-Heptopyranoside and D-Galactopyranoside

In yet another embodiment essentially pure or an isolated compound is represented by the following general formula (III): 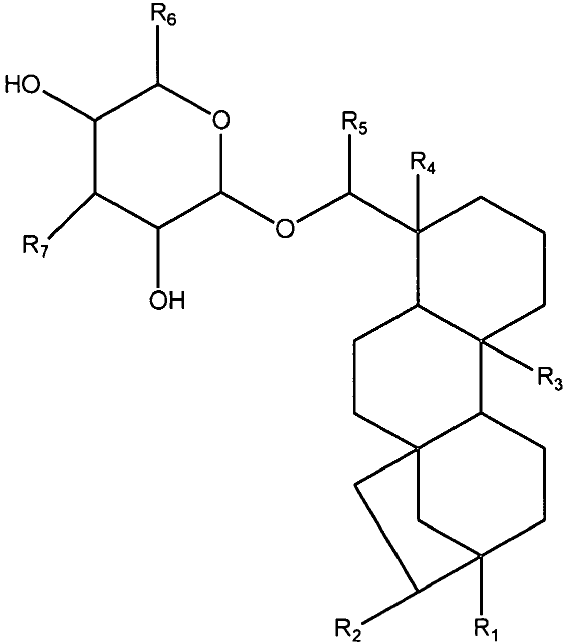 wherein
R1 is methyl or hydroxyl,
R2 is oxygen, methyl, oxymethyl, hydroxyl, or forms an ethene, ethanol, 2-Methoxy-propene, formaldehyde or 1-methoxy-ethanol bound.
R3 is methyl, ethyl,
R4 is methyl, ethyl,
R5 is oxygen or hydroxyl
R6 is hydroxyl, ethyl, methylene, oxymethyl, nitrite, carboxyl, aldehyde, amino, or oxyamino.
R7 is hydroxyl or carboxyl
or a pharmaceutically acceptable salt of such a compound. The compound can for instance be tetrahydro-pyran-diterpene, characterised in that it is represented by the following general formula (IV): 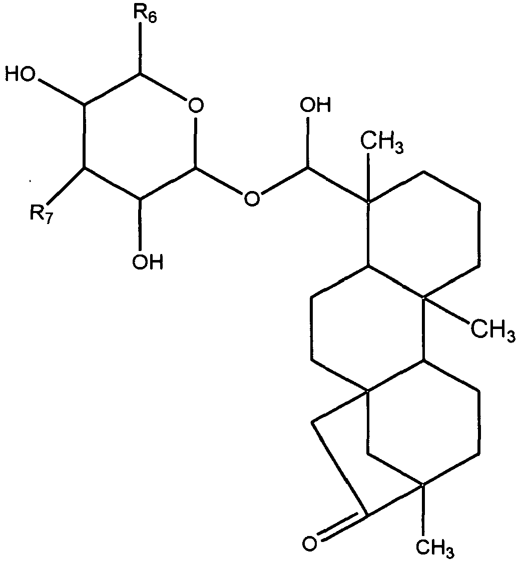 wherein R6 is hydroxyl, ethyl, methylene, oxymethyl, methylene, nitrite, carboxyl, aldehyde, amino, or oxyamino and R7 is hydroxyl or carboxyl. It can be a tetrahydro-pyran-diterpene, characterised in that it is represented by the following general formula (V): 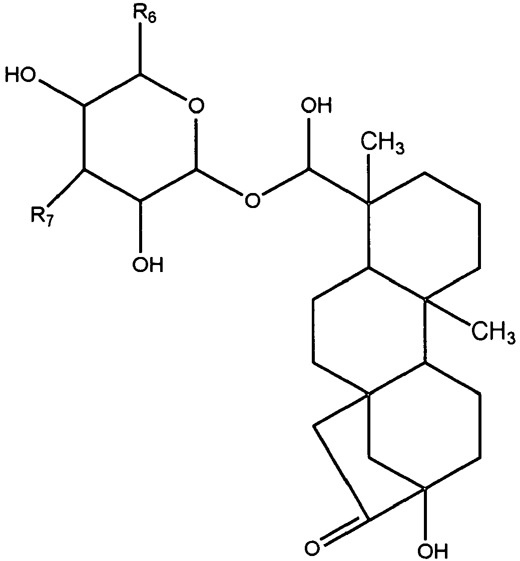 wherein R6 is hydroxyl, ethyl, methylene, oxymethyl, methylene, nitrite, carboxyl, aldehyde, amino, or oxyamino and R7 is hydroxyl or carboxyl; or it can be a tetrahydro-pyran-diterpene, characterised in that it is represented by the following general formula (VI): 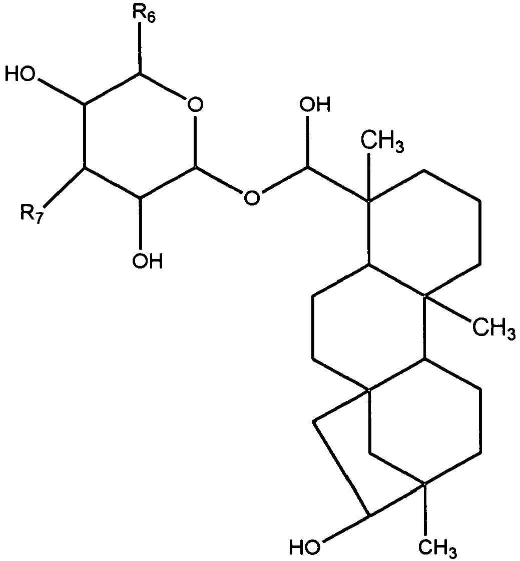 wherein R6 is hydroxyl, ethyl, methylene, oxymethyl, methylene, nitrite, carboxyl, aldehyde, amino, or oxyamino and R7 is hydroxyl or carboxyl; or it can be a tetrahydro-pyran-diterpene, characterised in that it is represented by the following general formula (VII): 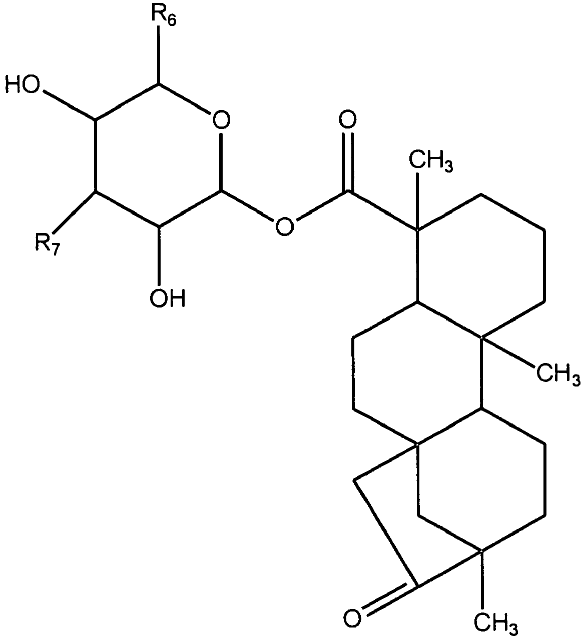 wherein R6 is hydroxyl, ethyl, methylene, oxymethyl, methylene, nitrite, carboxyl, aldehyde, amino, or oxyamino and R7 is hydroxyl or carboxyl; or it can be a tetrahydro-pyran-diterpene, characterised in that it is represented by the following general formula (IIX): 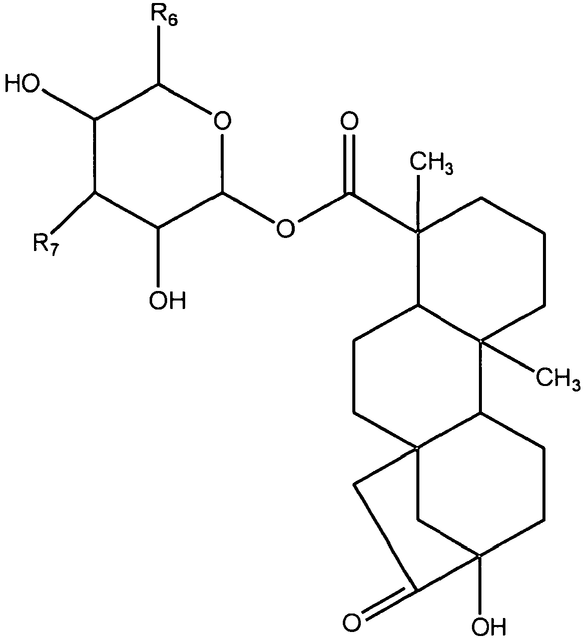 wherein R6 is hydroxyl, ethyl, methylene, oxymethyl, methylene, nitrite, carboxyl, aldehyde, amino, or oxyamino and R7 is hydroxyl or carboxyl; or it can be a, characterised in that it is represented by the following general formula (IX): 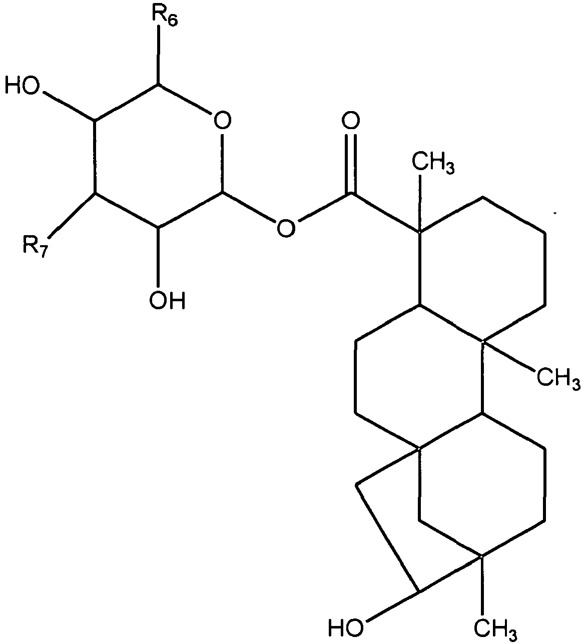 wherein R6 is hydroxyl, ethyl, methylene, oxymethyl, methylene, nitrite, carboxyl, aldehyde, amino, or oxyamino and R7 is hydroxyl or carboxyl.

PPAR gamma agonists inhibit the formation of fibrosis. Fibrosis is the formation or development of excess fibrous connective tissue in an organ or tissue as a reparative or reactive process. Types of fibrosis are cystic fibrosis of the pancreas and lungs, endomyocardial fibrosis, idiopathic pulmonary fibrosis of the lung, mediastinal fibrosis, myleofibrosis , retroperitoneal fibrosis, progressive massive fibrosis and nephrogenic systemic fibrosis.

Yet another embodiment of present invention is the use of a tetrahydro-pyran-diterpene compound represented by the following general formula (I): 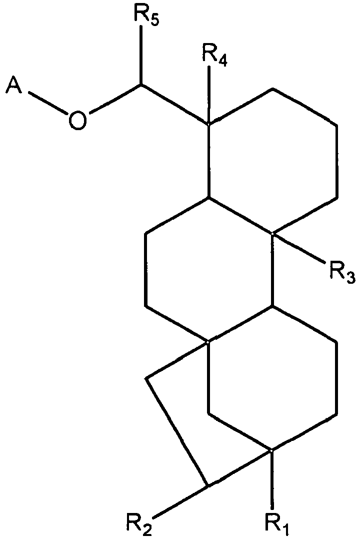 wherein
R1 is methyl or hydroxyl, or a tetrahydro-pyran compound represented by the following general formula 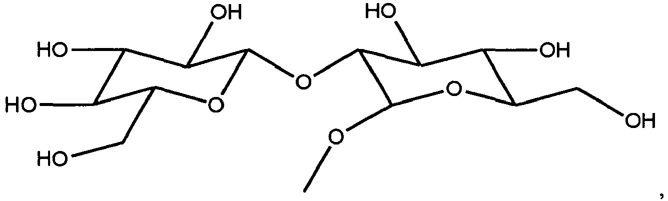
R2 is oxygen, methyl, oxymethyl, hydroxyl, or forms an ethene, ethanol, 2-methoxy-propene, formaldehyde or 1-methoxy-ethanol bound.
R3 is hydrogen, methyl, or ethyl,
R4 is hydrogen, methyl, or ethyl,
R5 is oxygen or hydroxyl

A is hydrogen or a tetrahydro-pyran compound represented by the following general formula (II) 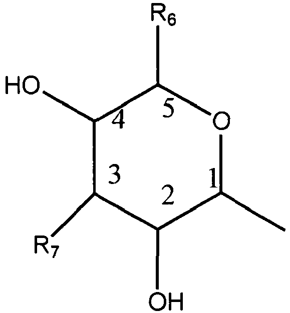
R6 is hydroxyl, ethyl, methylene, oxymethyl, nitrite, carboxyl, aldehyde, amino, or oxyamino.
R7 is hydroxyl or carboxyl
or a pharmaceutically acceptable salt of such a compound to manufacture a medicament or medicated dosage form to treat, prevent or reduce a fibrosis disorder

Moreover the invention relates to diterpenoic compounds such as isosteviol, dihydrosteviol, steviol and the diterpene-O-tetrahydro-pyran derivatives, such as isosteviol-19-0-β-D-glucuronide, dihydrosteviol-19-0-β-D-glucuronide, steviol-19-0-β-D-glucuronide or stevioside and pharmaceutically acceptable salts or esters thereof and/or mixtures thereof for use in a treatment of PPAR mediated disorders or to the use of these diterpenoic or diterpenoic tetrahydropyran PPAR agonists or PPAR activating diterpenoic compounds or diterpenoic tetrahydropyrans to manufacture a medicament for treating PPAR mediated disorders.

Moreover the invention relates to diterpenoic compounds such as isosteviol, dihydrosteviol, steviol and the diterpene-O-tetrahydro-pyran derivatives, such as isosteviol-19-0-β-D-glucuronide, dihydrosteviol-19-0-β-D-glucuronide, steviol-19-0-β-D-glucuronide or stevioside and pharmaceutically acceptable salts or esters thereof and/or mixtures thereof for use in a treatment of PPAR mediated lipid disorders or to the use of these diterpenoic or diterpenoic tetrahydropyran PPAR agonists or PPAR activating diterpenoic tetrahydropyran to manufacture a medicament for treating PPAR mediated lipid disorders. Such lipid disorder can be hypercholesterolemia, dyslipidemia or cholesterol disorders.

Moreover the invention relates to diterpenoic compounds such as isosteviol, dihydrosteviol, steviol and the diterpene-O-tetrahydro-pyran derivatives, such as isosteviol-19-0-β-D-glucuronide, dihydrosteviol-19-0-β-D-glucuronide, steviol-19-0-β-D-glucuronide or stevioside and pharmaceutically acceptable salts or esters thereof and/or mixtures thereof for use in a treatment to or to the use of these diterpenoic or diterpenoic tetrahydropyran PPAR agonists or PPAR activating diterpenoic tetrahydropyran to manufacture a medicament to lower FFA, triglycerides and/or (total) cholesterol in plasma or blood circulation, to increases in the levels of lipoprotein lipase in adipose tissue, to induce hydrolyzes of the lipids very low density lipoproteins (VLDL) into three fatty acids and one glycerol molecule, to increase the production of apoB100 while it deceases the biosynthesis of triglycerides, to induce a shift of apoB100 from VLDL to denser particles or to smaller, less triglyceride rich VLDL, to induce the formation of less atherogenic LDL, to lower plasma triglycerides, FFA and cholesterol to modulate the whole-body sterol homeostasis, including fatty acid, triglyceride, and lipoprotein metabolism and reverse cholesterol transport, to increased diversion of fatty acids into beta-oxidation, to decreases the availability of fatty-acyl-coenzyme A substrates for triglyceride synthesis, to reduces secretion of very-low-density lipoprotein by the liver, to decrease triglyceride levels by increasing the expression of lipoprotein lipase in the liver and in macrophages, to increase plasma HDL-cholesterol, to improve lipid metabolism and insulin signalling associated with decreased tissue deposition of oxidized LDL, to normalise the lipid homeostasis to decrease levels of intracellular triglycerides, diacylglycerol and increase fat Beta-oxidation, to increase the oxidation rate of fatty acids, to increase mitochondrial fatty acid oxidation in patients in need thereof for instance paediatric burn trauma patients.

Moreover the invention relates to diterpenoic compounds such as isosteviol, dihydrosteviol, steviol and the diterpene-O-tetrahydro-pyran derivatives, such as isosteviol-19-0-β-D-glucuronide, dihydrosteviol-19-0-β-D-glucuronide, steviol-19-0-β-D-glucuronide or stevioside, steviol-O-β-D-Glucopyranosiduronic acid, steviol-O-β-D-Glucopyranoside, steviol-O-α-D-Glucopyranoside, steviol-O-β-D-Glucopyranose,, steviol-O-β-D-gluco-Hexodialdo-1,5-pyranoside, steviol-O-β-D-Glucopyranoside, steviol-O-β-L-Mannopyranose, steviol-O-β-D-Glucopyranose, steviol-O-β-D-Xylopyranoside, steviol-O-β-L-Xylopyranose, steviol-O-β-O-galacto-Heptopyranoside, steviol-O-β-D-Galactopyranoside, rebaudioside A, rebaudioside C, Dulcoside A, steviolbioside, rebaudioside B, rebaudioside D and rebaudioside E and pharmaceutically acceptable salts or esters thereof and/or mixtures thereof for use in a treatment as hypolipidemic agents to reduce triglycerides or triglyceride deposition or to the use of these diterpenoic or diterpenoic tetrahydropyran PPAR agonists or PPAR activating diterpenoic tetrahydropyran as hypolipidemic agents to manufacture a medicament to lower free to reduce triglycerides or triglyceride deposition.

Moreover the invention concerns the use of diterpenoic compounds such as isosteviol, dihydrosteviol, steviol and the diterpene-O-tetrahydro-pyran derivatives, such as isosteviol-19-0-β-D-glucuronide, dihydrosteviol-19-0-β-D-glucuronide, steviol-19-0-β-D-glucuronide or stevioside and pharmaceutically acceptable salts or esters thereof and/or mixtures thereof for the preparation of a medicament for treating a mammal suffering from or susceptible to a condition which can be improved or prevented by lowering FFA, triglycerides and/or (total) cholesterol in plasma or blood circulation, increasing in the levels of lipoprotein lipase in adipose tissue, to induce hydrolyzes of the lipids very low density lipoproteins (VLDL) into three fatty acids and one glycerol molecule, increasing the production of apoB 100 while it deceases the biosynthesis of triglycerides, inducing a shift of apoB100 from VLDL to denser particles or to smaller, less triglyceride rich VLDL, inducing the formation of less atherogenic LDL, lowering plasma triglycerides, FFA and cholesterol to modulate the whole-body sterol homeostasis, including fatty acid, triglyceride, and lipoprotein metabolism and reverse cholesterol transport, increasing diversion of fatty acids into beta-oxidation, to decreases the availability of fatty-acyl-coenzyme A substrates for triglyceride synthesis, reducing the secretion of very-low-density lipoprotein by the liver, decreasing triglyceride levels by increasing the expression of lipoprotein lipase in the liver and in macrophages, increasing plasma HDL-cholesterol, improving lipid metabolism and insulin signalling associated with decreased tissue deposition of oxidized LDL, normalising the lipid homeostasis, increasing levels of intracellular triglycerides, diacylglycerol and increasing fat beta-oxidation, increasing the oxidation rate of fatty acids, increasing mitochondrial fatty acid oxidation.

The compounds of present invention can in particular been used for normalising the lipid homeostasis, increasing levels of intracellular triglycerides, diacylglycerol and increasing fat beta-oxidation, increasing the oxidation rate of fatty acids, increasing mitochondrial fatty acid oxidation in patients in need thereof for instance paediatric burn trauma patients.

Moreover the invention relates to diterpenoic compounds such as isosteviol, dihydrosteviol, steviol and the diterpene-O-tetrahydro-pyran derivatives, such as isosteviol-19-0-β-D-glucuronide, dihydrosteviol-19-0-β-D-glucuronide, steviol-19-0-β-D-glucuronide or stevioside, steviol-O-β-D-Glucopyranosiduronic acid, steviol-O-β-D-Glucopyranoside, steviol-O-α-D-Glucopyranoside, steviol-O-β-D-Glucopyranose,, steviol-O-β-D-gluco-Hexodialdo-1,5-pyranoside, steviol-O-β-D-Glucopyranoside, steviol-O-β-L-Mannopyranose, steviol-O-β-D-Glucopyranose, steviol-O-β-D-Xylopyranoside, steviol-O-β-L-Xylopyranose, steviol-O-β-D-galacto-Heptopyranoside, steviol-O-β-D-Galactopyranoside, rebaudioside A, rebaudioside C, Dulcoside A, steviolbioside, rebaudioside B, rebaudioside D and rebaudioside E and pharmaceutically acceptable salts or esters thereof and/or mixtures thereof for use in a treatment to or to the use of these diterpenoic or diterpenoic tetrahydropyran PPAR agonists or PPAR activating diterpenoic tetrahydropyran as a weight control agent or as a weight-loss drug to manufacture a medicament to induce weight loss, to counteract weight gain due to overeating, to induce a calorie-burning effect, for instance a calorie-burning effect similar to vigorous exercise or to protect against weight gain on high-fat and high-caloric diets.

Moreover the invention relates to diterpenoic compounds such as isosteviol, dihydrosteviol, steviol and the diterpene-O-tetrahydro-pyran derivatives, such as isosteviol-19-0-β-D-glucuronide, dihydrosteviol-19-0-β-D-glucuronide, steviol-19-0-β-D-glucuronide or stevioside, steviol-O-β-D-Glucopyranosiduronic acid, steviol-O-β-D-Glucopyranoside, steviol-O-α-D-Glucopyranoside, steviol-O-β-D-Glucopyranose,, steviol-O-β-D-gluco-Hexodialdo-1,5-pyranoside, steviol-O-β-D-Glucopyranoside, steviol-O-β-L-Mannopyranose, steviol-O-β-D-Glucopyranose, steviol-O-β-D-Xylopyranoside, steviol-O-β-L-Xylopymnose, steviol-O-β-D-galacto-Heptopyranoside, steviol-O-β-D-Galactopyranoside, rebaudioside A, rebaudioside C, Dulcoside A, steviolbioside, rebaudioside B, rebaudioside D and rebaudioside E and pharmaceutically acceptable salts or esters thereof and/or mixtures thereof for use in a treatment to or to the use of these diterpenoic or diterpenoic tetrahydropyran PPAR agonists or PPAR activating diterpenoic tetrahydropyran to manufacture a medicament for modifying the differentiation or function of a cell for instance an antigen presenting cell (APC), a macrophage and adipose cell or a monocyte by increasing the expression or activity of an endogenous PPAR, preferably the PPARgamma, PPARalpha or PPARbeta/delta receptor. The invention also concerns the use of diterpenoic compounds such as isosteviol, dihydrosteviol, steviol and the diterpene-O-tetrahydro-pyran derivatives, such as isosteviol-19-0-β-D-glucuronide, dihydrosteviol-19-0-β-D-glucuronide, steviol-19-0-β-D-glucuronide or stevioside, steviol-O-β-D-Glucopyranosiduronic acid, steviol-O-β-D-Glucopyranoside, steviol-O-α-D-Glucopyranoside, steviol-O-β-D-Glucopyranose,, steviol-O-β-D-gluco-Hexodialdo-1,5-pyranoside, steviol-O-β-D-Glucopyranoside, steviol-O-β-L-Mannopyranose, steviol-O-β-D-Glucopyranose, steviol-O-β-D-Xytopyranoside, steviol-O-β-L-Xylopyranose, steviol-O-β-D-galacto-Heptopyranoside, steviol-O-β-D-Galactopyranoside, rebaudioside A, rebaudioside C, Dulcoside A, steviolbioside, rebaudioside B, rebaudioside D and rebaudioside E and pharmaceutically acceptable salts or esters thereof for the preparation of a medicament for treating a mammal suffering from or susceptible to a condition which can be improved or prevented by increasing the expression or activity of an endogenous PPAR, preferably the PPARgamma, PPARalpha or PPARbeta/delta receptor in an antigen presenting cell (APC), a macrophage and adipose cell or a monocyte.

One embodiment of present invention involves an isolated or essentially pure compound of the group consisting of steviol-glucuronide, isosteviol-glucuronide or dihydroisosteviol-glucuronide or pharmaceutically acceptable acid addition or cationic salt thereof. The present invention, can involve an isolated or essentially pure compound of the group consisting of steviol-galacturonide, isosteviol- galacturonide or dihydroisosteviol-galacturonide or pharmaceutically acceptable acid- addition or cationic salt thereof.

Yet another embodiment of present invention is an isolated or essentially pure compound of the group consisting of steviol-O-β-D-gtucuronide, dihydroisosteviol-O-β-D-glucuronide and isosteviol-O-β-D-glucuronide or of group consisting of steviol-O-β-D-galacturonide, dihydroisosteviol-O-β-D galacturonide and isosteviol-O-β-D-galacturonide.

The compound of present invention also can be selected of the group consisting of dihydroisosteviol-O-α-D-glucuronide, steviol-O-α-D-glucuronide, isosteviol-O-α-D-glucuronide, dihydroisosteviol-O-α-D-glucuronide, steviol-O-α-D-galacturonide, dihydroisosteviol-O-α-D-galacturonide and isosteviol-O-α-D-galacturonide, it can be a compound selected from the group consisting of dihydroisosteviol-O-β-L-glucuronide, steviol-O-β-L-glucuronide, isosteviol-O-β-L-glucuronide, dihydroisosteviol-O-β-L-glucuronide, steviol-O-β-L-galacturonide, dihydroisosteviol-O-β-L-galacturonide and isosteviol-O-β-L-galacturonide; or a compound selected from the group consisting of dihydroisosteviol-O-α-L-glucuronide, steviol-O-α-L-glucuronide, isosteviol-O-α-L-glucuronide, dihydroisosteviol-O-α-L-glucuronide, steviol-O-α-L-galacturonide, dihydroisosteviol-O-α-L-galacturonide and isosteviol-O-α-L-galacturonide; or a compound selected from the group consisting of steviol-O-β-D-glucuronide, steviol- octan-O-β-D-galacturonide, isosteviol-O-β-D-glucuronide, isosteviol-O-β-D-galacturonide, dihydroisosteviol-O-β-D-glucuronide, dihydroisosteviol-O-β-D-galacturonide; or a compound from the group consisting of steviol-O-β-D-Glucopyranosiduronic acid, steviol-O-β-D-Glucopyranoside, steviol-O-α-D-Glucopyranoside, steviol-O-β-D-Glucopyranose,, steviol-O-β-D-gluco-Hexodialdo-1,5-pyranoside, steviol-O-β-D-Glucopyranoside, steviol-O-β-L-Mannopyranose, steviol-O-β-D-Glucopyranose, steviol-O-β-D-Xylopyranoside, steviol-O-β-L-xylopyranose, steviol-O-β-D-galacto-Heptopyranoside, steviol-O-β-D-Galactopyranoside.

In a particular embodiment of present invention the isolated or essentially pure compound is steviol-19-O-β-D-glucuronide.

Another embodiment of present invention is the use of one of the compounds selected of present invention to manufacture a medicament.

Yet another embodiment of present invention is a diterpenoic tetrahydropyran, in particular steviol-19-glucorinide, or other selected compounds of present invention for treating conditions of hypertension, hyperglycaemia or metabolic syndrome, said composition comprising a pharmaceutically-acceptable inert carrier and, as the active component thereof, an effective amount of the compounds of any of the previous claims or pharmaceutically-acceptable acid-addition or cationic salt thereof. Such composition may comprise as active compound steviol-O-glucuronide, isosteviol-O-glucuronide, dihydroisosteviol-O-glucuronide, steviol-O- galacturonid, isosteviol-O-galacturonide or dihydroisosteviol-O- galacturonide.

In a preferred embodiment tetrahydro-pyran-diterpene compounds, their hydrochloride or any pharmaceutically acceptable salt or derivatives there of may be administered orally in a regime of 10 to 2000 mg/patient/day, more preferably 100 to 1500 mg/patient/day, and yet more preferably 150 to 1000 mg/patient/day and most preferably 250 to 750 mg/patient/day. A possible daily dose can for instance be 5 to 15 mg/kg body weight. The active compound may be delivered as a solid medicine in pill or tablet form and alternatively as liquid, semi-solid. The parenteral administration form may be an isotonic injection solution. The daily dose can be 100 µg to 50 mg kg body weight, preferably 250 µg / kg body weight to 30 mg / kg body weight, more preferably 500 µg to 25 mg/kg body weight and most preferably 1 to 15 mg/kg body weight.

The present invention demonstrates that a cellular contact with a or direct action on cellular level of the glucuronide free scaffolds of the compound of the group consisting of stevioside, rebaudioside A, rebaudioside C, Dulcoside A, steviolbioside, rebaudioside B, rebaudioside D and rebaudioside E such as is steviol, isosteviol, steviol-glucuronide, isosteviol -glucuronide or dihydroisosteviol-glucuronide improves adipocyte differentiation (differentiation of pre-adipocytes to adipocytes).

The adipocytes play a critical role in energy balance. Adipose tissue growth involves an increase in adipocyte size and the formation of new adipocytes from precursor cells. When preadipocytes differentiate, it is known that committed preadipocytes undergo growth arrest and subsequent terminal differentiation into adipocytes. Such is accompanied by a dramatic increase in expression of adipocyte genes including adipocyte fatty acid binding protein and lipid-metabolizing enzymes. The present invention thus provides a new class of compounds for the therapy or for use in a treatment of the pathophysiological mechanisms underlying excess of adipose tissue which is for instance for an obesity therapy. Moreover, this demonstrates that the compounds of present invention can be used in a treatment to prevent or reduce excessive body weight gain or to improve glucose tolerance. Since the glycosides are know to be removed in the gastrointestinal tract by enzymatic or bacterial flora activity the glycosides of steviol, isosteviol, or the other glycoside PPAR agonist compounds of present invention can be used as well for improving adipocyte differentiation in a mammalian subject or patient by oral, peroral or gastrointestinal delivery of these compound to such subject. Particularly suitable for use in a treatment of improving adipocyte are steviol-glucuronide, isosteviol -glucuronide or dihydroisosteviol-glucuronide. Adipocyte differentiation is a step that controls adipogenesis. Adipogenesis is a multistep organogenenic process that begins in the prenatal period, but unlike osteogenesis and myogenesis, the adipogenesis process never ends. In this process, mesenchymal cells can proliferate in clonal expansion, and at some point, some of these cells can differentiate into preadipocytes or cells committed to fill with lipid (i.e., fat) and then become adipocytes. When preadipocytes undergo a differentiation step and begin to fill with lipid, lipid first accumulates within the cell in small droplets (multilocular cells) and eventually the droplets fuse into one large droplet (unilocular cells). The adipocyte can continue to enlarge by accumulating additional lipid. A typical mesenchymal cell is 10 to 20 µm in diameter, but adipocytes can easily reach 100 µm (and in some cases 200 µm) in diameter. The volume of the cell can increase as much as a thousand fold largely because of lipid accumulation.

The present invention thus concerns also PPAR agonists such as steviol, isosteviol, steviol-glucuronide, isosteviol -glucuronide or dihydroisosteviol-glucuronide or the glycoside compounds such as rebaudioside C, Dulcoside A, steviolbioside, rebaudioside B, rebaudioside D and rebaudioside E such for use in a treatment to improve adipogenesis or lipid accumulation, or adipose tissue growth in a subject or patient in need there of

Moreover, present invention thus also concerns a pharmaceutical composition for administration in mammals comprising one or more pharmaceutically acceptable carriers and one or more compounds of the group consisting of steviol, isosteviol, steviol-glucuronide, isosteviol ―glucuronide, dihydroisosteviol-glucuronide or of their glycoside precursors such as glycoside compounds such as rebaudioside C, Dulcoside A, steviolbioside, rebaudioside B, rebaudioside D or rebaudioside E. in an amount effective for decreasing or inhibiting the lipid accumulation and improving adipocyte differentiation or present invention also concerns the method of decreasing or inhibiting the lipid accumulation or improving adipocyte differentiation comprising administering to a mammal, patient or subject diagnosed as needing of such treatment the pharmaceutical composition described above.

Yet another particular embodiment can be also the use of the compounds of present invention to support stem cell therapy in tissue repair.

**Embodiments of present invention are described in the following points:**
1) A diterpene or diterpene glycoside compound represented by the following general formula (I): 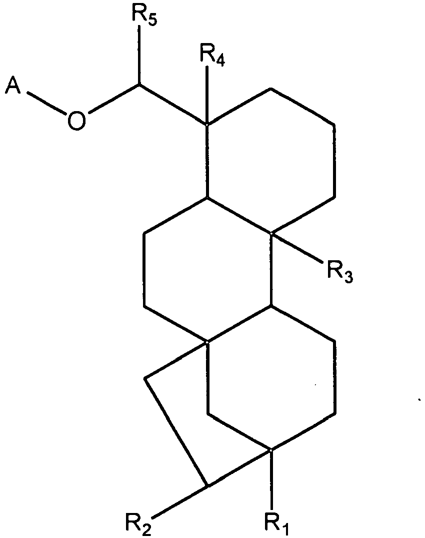 wherein
   R1 is methyl, hydroxyl, hydrogen, or a ―O-glycosyl,
   R2 is =O, methyl, oxymethyl, hydroxyl, or forms an ethene, ethanol, 2-methoxy-propene, formaldehyde or 1-methoxy-ethanol bound or hydrogen,
   R3 is methyl, ethyl, or hydrogen,
   R4 is methyl, ethyl, or hydrogen,
   R5 is =O, hydroxyl or hydrogen,
   A is a glycosyl, a hydrogen or a tetrahydro-pyran group represented by the following general formula (II) 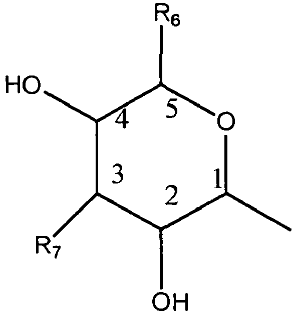
   R6 is hydroxyl, ethyl, methylene, hydrogen, oxymethyl, methylene, nitrite, carboxyl, aldehyde, amino, or oxyamino.
   R7 is hydroxyl, carboxyl or hydrogen
   or a pharmaceutically acceptable salt of such a compound for use in a treatment to induce adipocyte differentiation (differentiation of pre-adipocytes to adipocytes) for reducing the size of the adopocytes and for prevention of adipose mass increase by a direct action of said compound on on adipocytes.
2) Point 1 49, whereby the treatment further comprises simultaneous or separate administration of a statin
3) Any of the previous points, characterised in compound is represented by the following general formula (I): 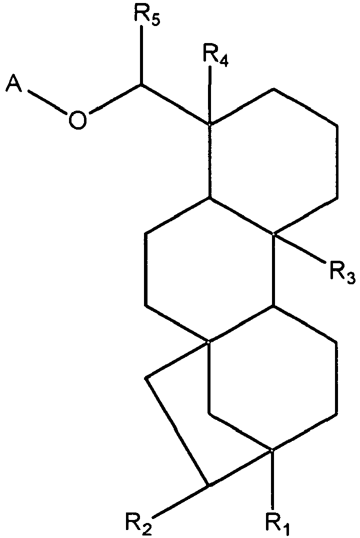 wherein
   R1 is methyl, hydroxyl, hydrogen, a tetrahydro-pyran compound represented by the following general formula 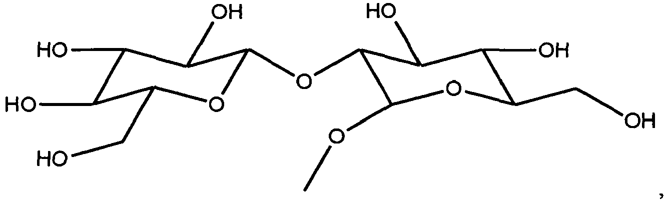
   or R1 is a, tetrahydro-pyran compound represented by the following general formula (II) 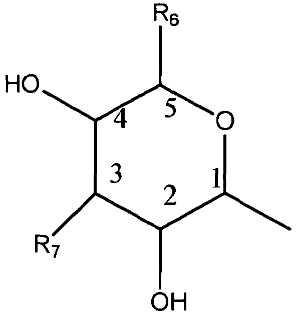
   wherein R6 is hydroxyl, ethyl, hyfrogen, methylene, oxymethyl, methylene, nitrite, carboxyl, aldehyde, amino, or oxyamino
   or R1 is a ―O-beta- Glc-beta- Glc (2-1) or has the following formula 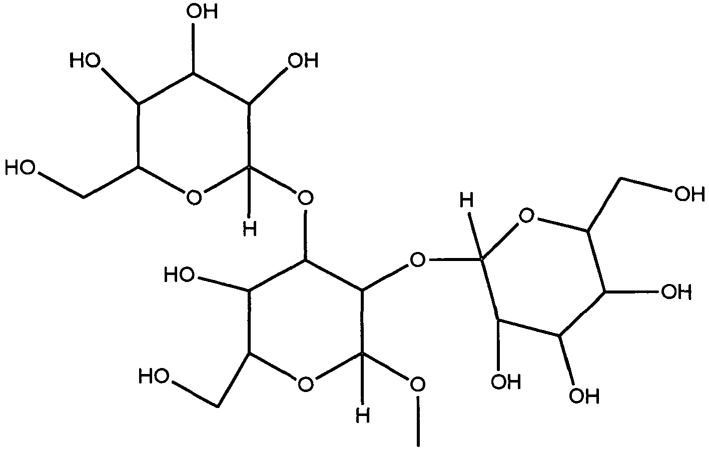
   R2 is =O, methyl, oxymethyl, hydroxyl, or forms an ethene, ethanol, 2-methoxy-propene, formaldehyde or 1-methoxy-ethanol bound or hydrogen,
   R3 is methyl, ethyl, or hydrogen,
   R4 is methyl, ethyl, or hydrogen,
   R5 is =O, hydroxyl or hydrogen,
      and
   A is hydrogen or a tetrahydro-pyran compound represented by the following general formula (II) 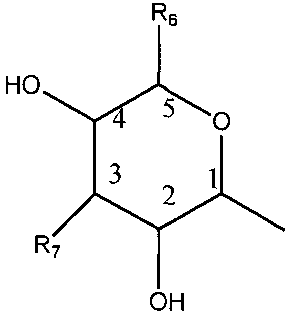
   R6 is hydroxyl, ethyl, methylene, oxymethyl, methylene, nitrite, carboxyl, aldehyde, amino, or oxyamino.
   R7 is hydroxyl or carboxyl
4) Point 1, characterised in that A is a group selected from D-Galactopyranosiduronic acid, D-Glucopyranosiduronic acid, D-Glucopyranoside, D-Glucopyranose, D-gluco-Hexodialdo-1,5-pyranoside, L-Mannopyranose, D-Xylopyranoside, L-Xylopyranose, D-galacto-Heptopyranoside and D-Galactopyranoside.
5) Any of the previous points, whereby the compound of point 1, represented by the following general formula (III): 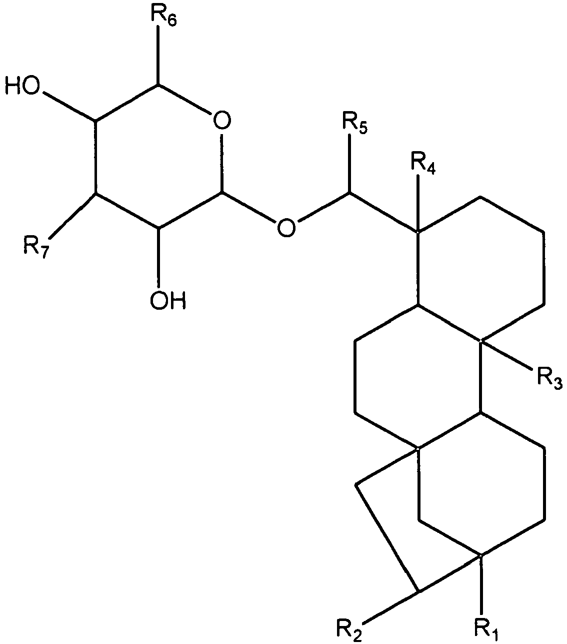 wherein
   R1 is methyl, hydrogen, or hydroxyl,
   R2 is =O, methyl, oxymethyl, hydroxyl, or forms an ethene, ethanol, 2-Methoxy-propene, formaldehyde or 1-methoxy-ethanol bound.
   R3 is methyl, ethyl, or hydrogen,
   R4 is methyl, ethyl, or hydrogen,
   R5 is =O, hydrogen, or hydroxyl
   R6 is hydroxyl, ethyl, hydrogen, methylene, oxymethyl, nitrite, carboxyl, aldehyde, amino, or oxyamino.
   R7 is hydroxyl, hydrogen, carboxyl
   or a pharmaceutically acceptable salt of such a compound.
6a ) Any of the previous points,, characterised in that the compound is represented by the following general formula (IV): 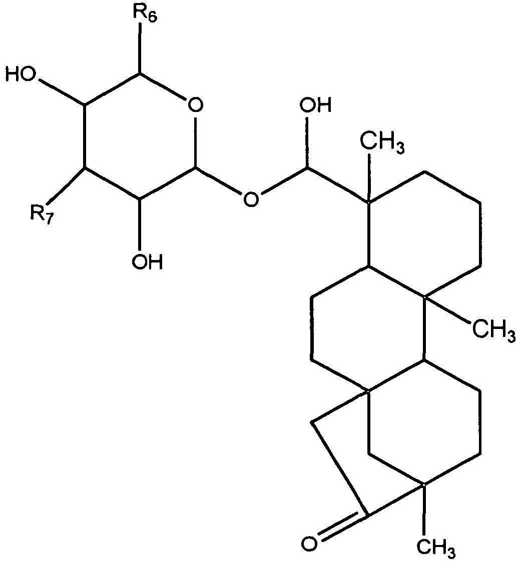 wherein R6 is hydroxyl, ethyl, hydrogen, methylene, oxymethyl, nitrite, carboxyl, aldehyde, amino, or oxyamino and R7 is hydroxyl or carboxyl
6b ) Any of the previous points, characterised in that the compound is represented by the following general formula (V): 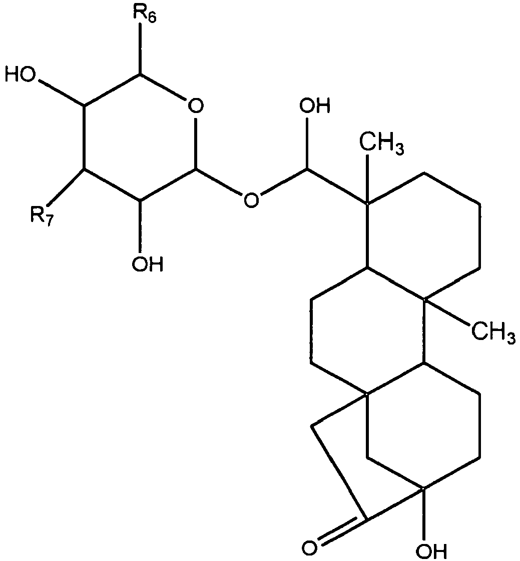 wherein R6 is hydroxyl, ethyl, hydrogen, methylene, oxymethyl, nitrite, carboxyl, aldehyde, amino, or oxyamino and R7 is hydroxyl or carboxyl
6c ) Any of the previous points, characterised in that the compound is represented by the following general formula (VI): 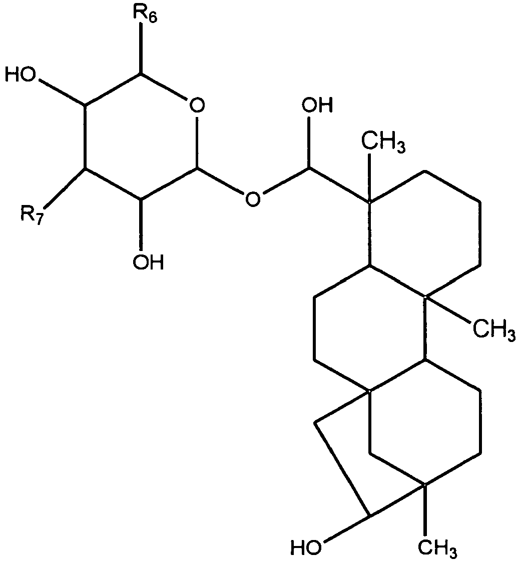 wherein R6 is hydroxyl, hydrogen ethyl, methylene, oxymethyl, nitrite, carboxyl, aldehyde, amino, or oxyamino and R7 is hydroxyl or carboxyl
6d ) Any of the previous points, characterised in that the compound is represented by the following general formula (VII): 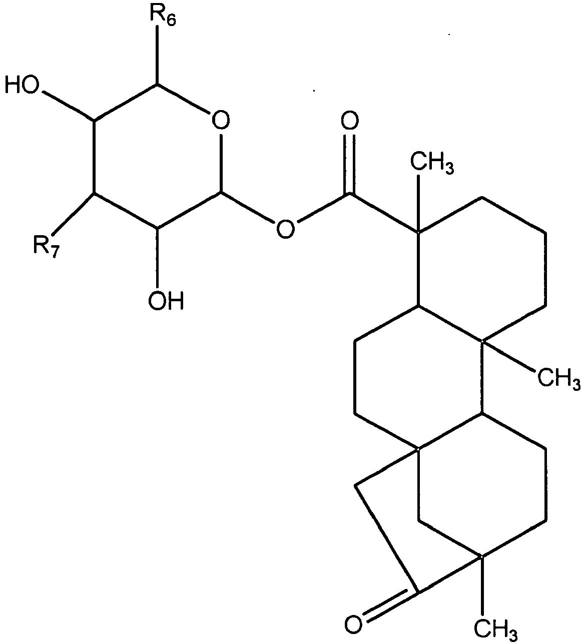 wherein R6 is hydroxyl, ethyl, methylene, hydrogen, oxymethyl, nitrite, carboxyl, aldehyde, amino, or oxyamino and R7 is hydroxyl or carboxyl
6e ) Any of the previous points, characterised in that the compound is represented by the following general formula (IIX): 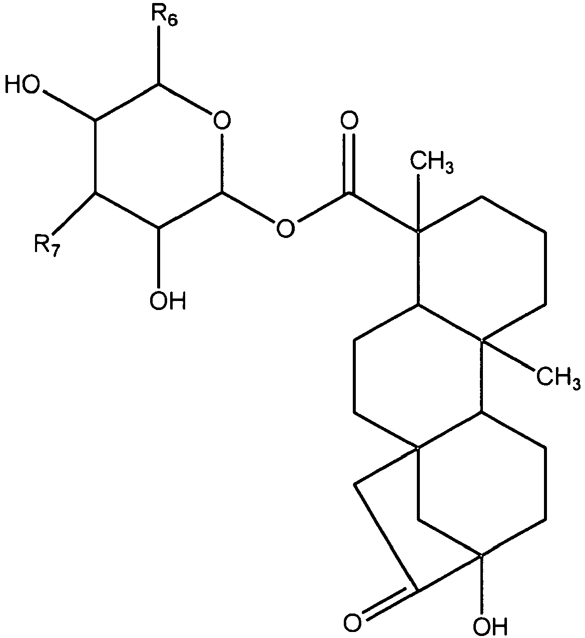 wherein R6 is hydroxyl, ethyl, methylene, hydrogen oxymethyl, nitrite, carboxyl, aldehyde, amino, or oxyamino and R7 is hydroxyl, hydrogen, carboxyl
6f ) Any of the previous points, characterised in that the compound is by the following general formula (IX): 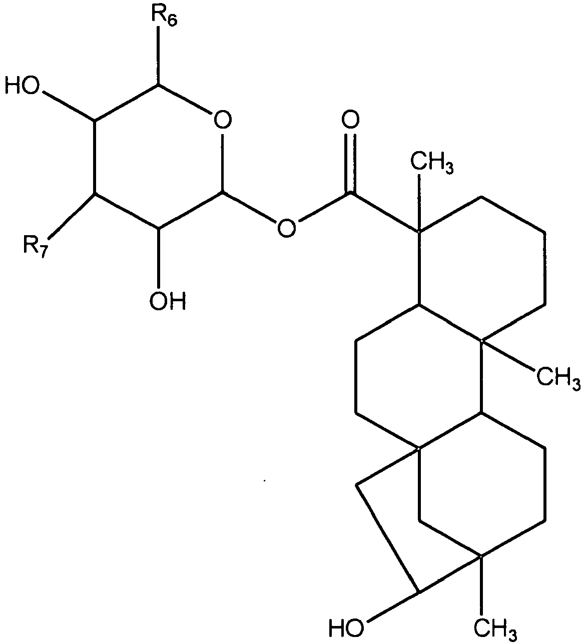 wherein R6 is hydroxyl, ethyl, hydrogen, methylene, oxymethyl, nitrite, carboxyl, aldehyde, amino, or oxyamino and R7 is hydroxyl or carboxyl
7) Any of the previous points, characterised in that the compound is by the following general formula (X): 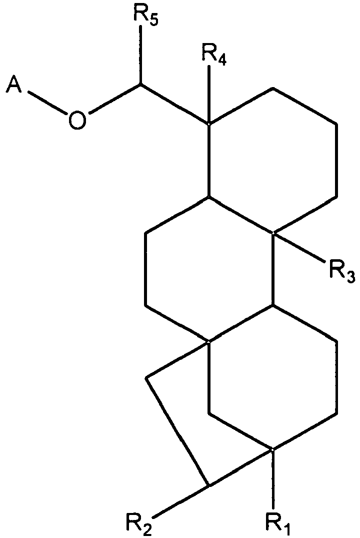 wherein
   R1 is a tetrahydro-pyran compound represented by the following general formula 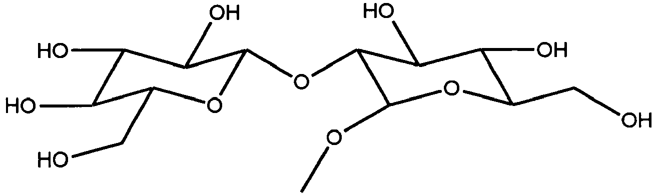
   R2 is =O, methyl, hydrogen, oxymethyl, hydroxyl, or forms an ethene, ethanol, 2-methoxy-propene, formaldehyde or 1-methoxy-ethanol bound.
   R3 is methyl, ethyl, or hydrogen,
   R4 is methyl, ethyl, or hydrogen,
   R5 is =O, hydrogen, or hydroxyl
   A is hydrogen
   R6 is hydroxyl, ethyl, hydrogen, methylene, oxymethyl, nitrite, carboxyl, aldehyde, amino, or oxyamino.
   R7 is hydroxyl, hydrogen, or carboxyl
   or a pharmaceutically acceptable salt of such a compound.
8a ) Any of the previous points, characterised in that the compound is steviol, isosteviol, steviol-glucuronide, isosteviol -glucuronide or dihydroisosteviol-glucuronide or pharmaceutically-acceptable acid- addition or cationic salt thereof.
8b ) Any of the previous points, characterised in that the compound is steviol-galacturonide, isosteviol - galacturonide or dihydroisosteviol- galacturonide or pharmaceutically-acceptable acid- addition or cationic salt thereof.
9a ) Any of the previous points, characterised in that the compound is selected from the group consisting of steviol-O-β-D-glucuronide, dihydroisosteviol-O-β-D-glucuronide and isosteviol-O-β-D-glucuronide.
9b ) Any of the previous points, characterised in that the compound is selected from the group consisting of steviol-O-β-D-galacturonide, dihydroisosteviol-O-β-D-galacturonide and isosteviol-O-β-D-galacturonide.
9c ) Any of the previous points, characterised in that the compound is selected from the group consisting of dihydroisosteviol-O-α-D-glucuronide, steviol-O- α-D-glucuronide, isosteviol-O-α-D-glucuronide, dihydroisosteviol-O-α-D-glucuronide, steviol-O-α-D-galacturonide, dihydroisosteviol-O-α-D-galacturonide and isosteviol-O-α-D-galacturonide.
9d ) Any of the previous points, characterised in that the compound is selected from the group consisting of dilhydroisosteviol-O-β-L-glucuronide, steviol-O-β-L-glucuronide, isosteviol-O-β-L-glucuronide, dihydroisosteviol-O-β-L-glucuronide, steviol-O-β-L-galacturonide, dihydroisosteviol-O-β-L-galacturonide and isosteviol-O-β-L-galacturonide.
9e ) Any of the previous points, characterised in that the compound is selected from the group consisting of dihydroisosteviol-O-α-L-glucuronide, steviol-O- α-L-glucuronide, isosteviol-O-α-L-glucuronide, dihydroisosteviol-O-α-L-glucuronide, steviol-O-α-L-galacturonide, dihydroisosteviol-O-α-L-galacturonide and isosteviol-O-α-L-galacturonide.
9f ) Any of the previous points, characterised in that the compound is selected from the group consisting of steviol-O-β-D-glucuronide, steviol- octan-O-β-D-galacturonide, isosteviol-O-β-D-glucuronide, isosteviol-O-β-D-galacturonide, dihydroisosteviol-O-β-D-glucuronide, dihydroisosteviol-O-β-D-galacturonide.
10) Any of the previous points, characterised in that the compound is selected from the group consisting of steviol-O-β-D-Glucopyranosiduronic acid, steviol-O-β-D-Glucopyranoside, steviol-O-α-D-Glucopyranoside, steviol-O-β-D-Glucopyranose,, steviol-O-β-D-gluco-Hexodialdo-1,5-pyranoside, steviol-O-β-L-Mannopyranose, steviol-O-β-D-Xylopyranoside, steviol-O-β-L-Xylopyranose, steviol-O-β-D-galacto-Heptopyranoside, steviol-O-β-D-Galactopyranoside
11) Any of the previous points, characterised in that the compound is Steviol-19-O-β-D-glucuronide
12) Any of the previous points, characterised in that the compound is the group consisting of 7a,15a-Methano-8H-naphth[2',1':5,6]azuleno[2,1-d]phenanthrene-4,7-dione, 1,2,2a,3,5,5a,9,9a,10,11,12,13,13a,13b,14,15-hexadecahydro-1,16-dihydroxy-17-methoxy-3,3,10,10,13a-pentamethyl-18-(1-methylethyl)-, (1R,2aR,5aS,13aR)-rel-(-)-(9Cl), Kauran-16-ol, 13-methyl-, (8**β**,13**β**)-(9Cl), Kaurane-16,18-diol, 13-methyl-, (4**α**,8**β**,13**β**)-(9Cl), Kauran-18-oic acid, 16-hydroxy-13-methyl-, (4a,8b,13b)- (9Cl) or ent-16b-Hydroxy-16a-methylbeyeran-19-oic acid, Kauran-18-oic acid, 11,13,16,17-tetrahydroxy-, (4**a**,11**a**)-, Kaur-16-en-18-oic acid, 7,11,13-trihydroxy-, (4**α**,7**β**,11**α**)-, Kauran-18-oic acid, 16,17-epoxy-11,13-dihydroxy-, (4**a**,11**a**)-, Kauran-18-oic acid, 17-(acetyloxy)-13,16-dihydroxy-, (4**a**)-, Kaur-16-en-18-oic acid, 13-hydroxy-7-oxo-, (4**a**)-, 1H-2,10a-Ethanophenanthrene-8-carboxylic
   acid, dodecahydro-10-hydroxy-2-(hydroxymethyl)-4b,8-dimethyl-12-oxo-(2S,4aS,4bS,8R,8aS,10R,10aS)-, 17-Norkauran-18-oic acid, 16-hydroxy-13-methyl-, methyl ester, (4**α**,8**β**,13**β**,16**α**)-, Kaur-16-ene-3,18-diol, 13-methyl-, (3**α**,4**α**,8**β**,13**β**)-(±)-(9Cl), 17-Norkauran-18-oic acid, 13-methyl-16-(2-oxopropylidene)-, (4**α**,8**β**,13**β**)-(±)-(9Cl), Kaur-16-en-18-oic acid, 13-methyl-3-oxo-, ethyl ester, (4**β**,8**β**,13**β**)-(±)-(9Cl), Kaur-16-en-18-oic acid, 3-hydroxy-13-methyl-, ethyl ester, (3**α**,4**α**,8**β**,13**β**)-(±)-(9Cl), and Kaur-16-en-18-oic acid, 13-methyl-3-oxo-, ethyl ester, (**4**α,8**β**,13**β**)-(±)-(9Cl) or functional derivatives thereof
13) Any of the previous points, characterised in that the compound is rabaudioside A (Kaur-16-en-18-oic acid or 13-[(O-**β**-D-glucopyranosyl-(1**→**2)-O-[**β**-D-glucopyranosyl-(1**→**3)]-**β**-D-glucopyranosyl)oxy]-, **β**-D-glucopyranosyl ester, (4**α**)-**)** or stevioside (Kaur-16-en-18-oic acid or 13-[(2-O-**β**-D-glucopyranosyl-**β**-D-glucopyranosyl)oxy]-, **β**-D-glucopyranosyl ester, (4**α**)- )
14) Any of the previous points, characterised in that the compound is of the group consisting of stevioside, rebaudioside A, rebaudioside C, Dulcoside A, steviolbioside, rebaudioside B, rebaudioside D, rebaudioside E, steviol, isosteviol, steviol-glucuronide, isosteviol -glucuronide or dihydroisosteviol-glucuronide

The PPAR agonists or PPAR activators of present invention can be delivered in an oral dosage forms are preferred for those therapeutic agents that are orally active, and include tablets, capsules, caplets, solutions, suspensions and/or syrups, and may also comprise a plurality of granules, beads, powders or pellets that may or may not be encapsulated. Such dosage forms are prepared using conventional methods known to those in the field of pharmaceutical formulation and described in the pertinent texts, e.g., in Remington: The Science and Practice of Pharmacy, 20th Edition, Gennaro, A.R., Ed. (Lippincott, Williams and Wilkins, 2000). Tablets and capsules represent the most convenient oral dosage forms, in which case solid pharmaceutical carriers are employed. It is clear that the dose can vary to the moleculer weight and the presence or abasence of glycoside groups on the PPA agonists or PPAR activators of present invention but daily dose can be in the range of 100 µg to 500 mg/kg body weight, more preferably in the range of 250 µg to 100 mg / kg body weight, yet more preferably 500 µg g to 50 mg per kg body weight, and most preferably 1 mg to 25 mg / kg body weight

Tablets may be manufactured using standard tablet processing procedures and equipment. One method for forming tablets is by direct compression of a powdered, crystalline or granular composition containing the active agent(s), alone or in combination with one or more carriers, additives, or the like. As an alternative to direct compression, tablets can be prepared using wet-granulation or dry-granulation processes. Tablets may also be moulded rather than compressed, starting with a moist or otherwise tractable material; however, compression and granulation techniques are preferred.

In addition to the active agent(s), then, tablets prepared for oral administration using the method of the invention will generally contain other materials such as binders, diluents, lubricants, desintegrants, fillers, stabilisers, surfactants, colouring agents, and the like. Binders are used to impart cohesive qualities to a tablet, and thus ensure that the tablet remains intact after compression. Suitable binder materials include, but are not limited to, starch (including corn starch and pregelatinised starch), gelatine, sugars (including sucrose, glucose, dextrose and lactose), polyethylene glycol, waxes, and natural and synthetic gums, e.g., acacia sodium alginate, polyvinylpyrrolidone, cellulosic polymers (including hydroxypropyl cellulose, hydroxypropyl methylcellulose, methyl cellulose, ethyl cellulose, hydroxyethyl cellulose, and the like), and Veegum. Diluents are typically necessary to increase bulk so that a practical size tablet is ultimately provided.

Suitable diluents include lactose, cellulose, kaolin, mannitol, sodium chloride, dry starch and powdered sugar. Lubricants are used to facilitate tablet manufacture; examples of suitable lubricants include, for example, magnesium stearate and stearic acid. Stearates, if present, preferably represent at no more than approximately 2 wt. % of the drug-containing core.

Disintegrants are used to facilitate disintegration of the tablet, and are generally starches, clays, celluloses, algins, gums or crosslinked polymers. Fillers include, for example, materials such as silicon dioxide, titanium dioxide, alumina, talc, kaolin, powdered cellulose and microcrystalline cellulose, as well as soluble materials such as mannitol, urea, sucrose, lactose, dextrose, sodium chloride and sorbitol. Stabilisers are used to inhibit or retard drug decomposition reactions that include, by way of example, oxidative reactions. Surfactants may be anionic, cationic, amphoteric or non-ionic surface active agents.

The dosage form may also be a capsule, in which case the active agent-containing composition may be encapsulated in the form of a liquid or solid (including particulates such as granules, beads, powders or pellets). Suitable capsules may be either hard or soft, and are generally made of gelatine, starch, or a cellulosic material, with gelatin capsules preferred. Two-piece hard gelatine capsules are preferably sealed, such as with gelatine bands or the like. See, for example, Remington: The Science and Practice of Pharmacy, which describes materials and methods for preparing encapsulated pharmaceuticals. If the active agent-containing composition is present within the capsule in liquid form, a liquid carrier is necessary to dissolve the active agent(s). The carrier must be compatible with the capsule material and all components of the pharmaceutical composition, and must be suitable for ingestion.

Solid dosage forms, whether tablets, capsules, caplets, or particulates, may, if desired, be coated so as to provide for delayed release. Dosage forms with delayed release coatings may be manufactured using standard coating procedures and equipment. Such procedures are known to those skilled in the art and described in the pertinent texts, e.g., in Remington, supra. Generally, after preparation of the solid dosage form, a delayed release coating composition is aplied using a coating pan, an airless spray technique, fluidised bed coating equipment, or the like. Delayed release coating compositions comprise a polymeric material, e.g., cellulose butyrate phthalate, cellulose hydrogen phthalate, cellulose proprionate phthalate, polyvinyl acetate phthalate, cellulose acetate phthalate, cellulose acetate trimellitate, hydroxypropyl methylcellulose phthalate, hydroxypropyl methylcellulose acetate, dioxypropyl methylcellulose succinate, carboxymethyl ethylcellulose, hydroxypropyl methylcellulose acetate succinate, polymers and copolymers formed from acrylic acid, methacrylic acid, and/or esters thereof.

Sustained release dosage forms provide for drug release over an extended time period, for instance relatively short period up to 1 hour wherein at least 80 % of the compound of present invention will be released, or for instance a medium term release period of 1 to 8 hours wherein up to 80% of the compound of present invention will be released or for instance a long term release period of more than 8 hours wherein up to 80% of the compound of present invention will be released. Generally, as will be appreciated by those of ordinary skill in the art, sustained release dosage forms are formulated by dispersing a drug within a matrix of a gradually bioerodible (hydrolysable) material such as an, insoluble plastic, a hydrophilic polymer, or a fatty compound, or by coating a solid, drug containing dosage form with such a material. Insoluble plastic matrices may be comprised of, for example, polyvinyl chloride or polyethylene. Hydrophilic polymers useful for providing a sustained release coating or matrix cellulosic polymers include, without limitation: cellulosic polymers such as hydroxypropyl cellulose, hydroxyethyl cellulose, hydroxypropyl methyl cellulose, methyl cellulose, ethyl cellulose, cellulose acetate, cellulose acetate phthalate, cellulose acetate trimellitate, hydroxypropylmethyl cellulose phthalate, hydroxypropylcellulose phthalate, cellulose hexahydrophthalate, cellulose acetate hexahydrophthalate, and carboxymethylcellulose sodium; acrylic acid polymers and copolymers, preferably formed from acrylic acid, methacrylic acid, acrylic acid alkyl esters, methacrylic acid alkyl esters, and the like, e.g. copolymers of acrylic acid, methacrylic acid, methyl acrylate, ethyl acrylate, methyl methacrylate and/or ethyl methacrylate, with a terpolymer of ethyl acrylate, methyl methacrylate and trimethylammonioethyl methacrylate chloride (sold under the tradename Eudragit RS) preferred; vinyl polymers and copolymers such as polyvinyl pyrrolidone, polyvinyl acetate, polyvinylacetate phthalate, vinylacetate crotonic acid copolymer, and ethylenevinyl acetate copolymers; zein; and shellac, ammoniated shellac, shellacacetyl alcohol, and shellac n-butyl stearate. Fatty compounds for use as a sustained release matrix material include, but are not limited to, waxes generally (e.g., camauba wax) and glyceryl tristearate.

Techques are are available to render sparingly water soluble PPAR agonists such as steviol methyl esters more soluble. This can for instance be done by the dosage form and the preparation thereof. For instance by loading such in porous silica material which can have a composition consisting of pure silica. But as an alternative, it can be a mixture of silica with one or more of aluminum (Al), titanium (Ti), magnesium (Mg), zirconium (Zr), gallium (Ga), beryllium (Be), yttrium (Y), lanthanum (La), tin (Sn), lead (Pb), vanadium (V), boron (B) and the like. Such materials preferably are characterized in that the porous silica material has an average pore diameter in a range of from 1 to 20 nm, pores having diameters within ±40% of the average pore size account for at least 60% of a total pore volume of the porous silica material, and in X-ray diffractometry, the porous silica material has at least one peak at a position of diffraction angle (2θ) corresponding to a d value of at least 1 nm. The average pore diameter of the instant porous silica material can be determined by the gas adsorption method. Its measurement can be conducted, for example, by an automated specific surface area/pore distribution analyzer, "TRISTAR 3000" (manufactured by Micrometrics Instrument Corporation), or the like. Such systems have been described in the art and also methods to load said drug in said microporous material. Examples of the porous silica material for use in the present invention include porous silica materials each having a skeleton of a polymerized metal oxide, typically porous silica materials each having a silicate skeleton. In the instant porous silica material, such metal-oxygen bonds are formed in a network structure, and as a whole, make up a porous material. Also included as examples are porous silica materials each of which has a skeleton containing, in place of a fraction of the silicon atoms in the silicate skeleton, other metal atoms such as aluminium, zirconium, tantalum, niobium, tin, hafnium, magnesium, molybdenum, cobalt, nickel, gallium, beryllium, yttrium, lanthanum, lead and/or vanadium atoms. Also usable are porous silica materials each of which has a skeleton containing such other metal atoms or silicon atoms in a silicate skeleton or a skeleton containing bonds of such other metal atoms and oxygen atoms. It is to be noted that various metal atoms, organic functional groups and/or inorganic functional groups may be added to or as side chains bonded to atoms making up such a basic skeleton. Those containing, for example, thiol groups, carboxyl groups, lower alkyl groups such as methyl groups or ethyl groups, phenyl groups, amino groups, vinyl groups and or the like are preferred. Commercial examples of such porous silica materials include "FSM-C8", "FSM-C10", "FSM-C12", "FSM-C14", "FSM-C16", "FSM-C18" and "FSM-C22" (all, products of Toyota Central R&D Labs. Inc.), and "MCM-41" (mesoporous molecular sieve, product of Mobil Chemical Corp.), with "FSM-C16" and "FSM-C12" being particularly preferred. The average pore diameter of the porous silica material for use in the present invention can be preferably from 1 to 20 nm, more preferably from 1.5to 10 nm, especially preferably from 2 to 3 nm. The specific surface area of the porous silica material for use in the present invention can be preferably from 100 to 2,000 m 2/g, more preferably from 600 to 1,800 m2/g, especially preferably from 800 to 1,500 m2/g. It is to be noted that the specific surface area can be measured by the adsorption method. Such porous materials can be loaded up with the sparingly soluble PPAR agonists of present invention. Particularly suitable is treating the porous silica material and the sparingly soluble PPAR agonists of present invention with the supercritical fluid or subcritical fluid of carbon dioxide in the present invention, various components authorized as drug additives can be added as desired insofar as they do not impair the advantageous effects Such components can include, for example, solvents, polymer compounds, and surfactants. The mixing weight ratio of the porous silica material to the sparingly water-soluble drug to be used in the present invention can be preferably from 0.1:1 to 1,000:1, more preferably from 0.5:1 to 100:1, especially preferably from 1:1 to 50:1.

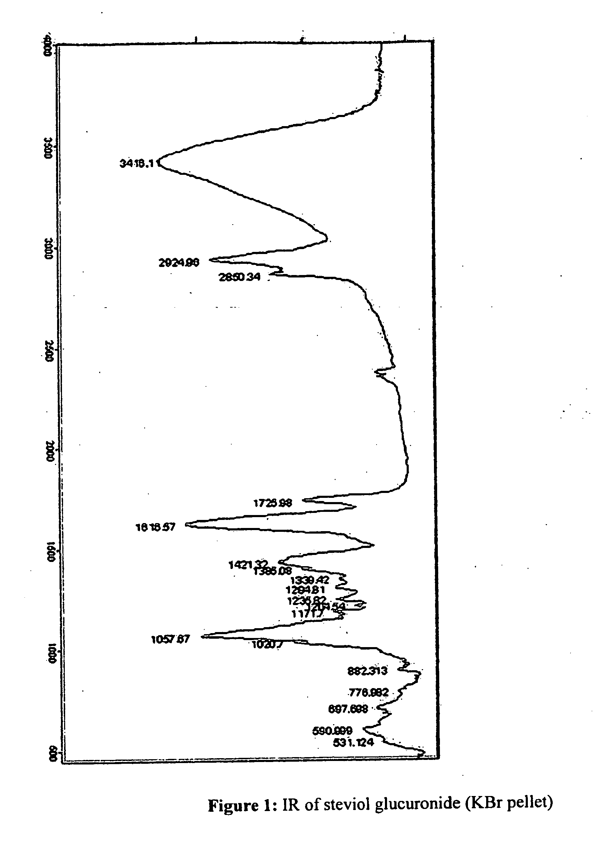

Examples of carbon dioxide for use in the present invention include liquid carbon dioxide, gaseous carbon dioxide, and dry ice. A "supercritical state" means a state in which the pressure and the temperature both exceed the critical points (in the case of carbon dioxide, pressure: about 7.38 MPa, temperature: about 31.0° C.), while the term "subcritical state" as used herein means a state in which only one of the pressure and the temperature exceeds the corresponding critical point. The term "critical points" has a meaning, for example, as described in detail by J. W. Tom and P. G. Debenedetti in FIG. 1 of "Particle Formation with Superctirical Fluids--A Review", J. Aerosol Sci., 22(5), 555-584 (1991). The weight ratio of the sparingly or poorly water-soluble PPAR agonist of the present invention to the supercritical fluid or subcritical fluid of carbon dioxide in the present invention can be preferably from 1:1 to 1:1,000,000, more preferably from 1:10 to 1:100,000, especially preferably from 1:50 to 1:50,000. The time of the treatment with the supercritical fluid or subcritical fluid of carbon dioxide in the present invention can be preferably from 1 minute to 24 hours, more preferably from 0.5 to 12 hours, especially preferably from 1 to 8 hours.
More details are for instance provided in WO04096281. Also other techniques are present to load the PPAR agonist of present invention in such porous materials.

Another possibility to increase the solubility of the PPAR agonists of present invention is to disperse it in a highly soluble hydrophilic matrix to make a solid dispersion (solid solutions or eutectic dispersions) as a means to enhance the dissolution rate of the poorly or sparingly water soluble PPAR agonists of present invention. Techniques are present in the art to make such in hydrophilic polymers or inert fillers. Suitably, the solid dispersions of this invention may contain up to about 10% inert fillers that do not materially affect the properties of the end product. Examples of such fillers include, hydroxypropylmethylcellulose phthalate 22084 (HP50), hydroxypropylmethylcellulose phthalate 220731 (HP55), hydroxypropylmethyl-cellulose acetate succinate (AQOAT), carboxymethylethylcellulose (CMEC), cellulose acetate phthalate (CAP), methacrylic copolymer LD (L30 D55), methacrylic copolymers S (S-100), aminoalkyl methacrylate copolymer E (gastric coating base), poly (vinyl acetal) diethylaminoacetate (AEA), polyvinylpyrrolidone (K-25, 30,90; PVP), ethylcellulose (EC), methacrylic copolymer RS (RS 30D), polyvinyl alcohol (PVA), methylcellulose (MC), hydroxypropylcellulose (HPC), hydroxypropylmethylcellulose 2208 (Metolose 90SH), hydroxypropyl- methylcellulose 2906 (Metolose 65SH), hydroxypropylmethylcellulose 2910 (Metolose 60SH), carboxymethylcellulose sodium (sodium cellulose glycolate), dextrin, pullulan, Acacia, tragacanth, sodium alginate, propylene glycol alginate, agar powder, gelatin, starch, processed starch, phospholipids (lecithin),glucomannan and the like.

Such solid dispersions can be by hydrophilic polymer systems and surfactants. Physical blends of the poorly soluble PPAR agonists of present invention can be with hydrophilic polymers such as PVP-K30, Plasdone-S630, HPMC-E5, HPMCAS, and Eudragit L100 and surfactants such as Tween-80, Docusate sodium, Myrj-52, Pluronic-F68, SLS or other pharmaceutical or food grade surfactants and solid dispersions can be prepared with for instance a hot-melt extrusion process.

Characterized by differential scanning calorimetry, X-ray diffraction, Raman spectroscopy, and polarized microscopy can be used in a quality control to demonstrated that the glass transition temperature of the carrier polymers decreased as direct result of the surfactants in the extrudate, due to an increase in the chain mobility of polymers and by the release profiles of active ingredient of the extruded solid dispersions. Also systems are available to reduce the particle size to the nanoscale and increase this way the solubility, their hydrochloride or any pharmaceutically acceptable salt or derivatives thereof may be administered topically on the respective nerve entrapment areas. The transdermal administration of tetrahydro-pyran-diterpene compound, their hydrochloride or any pharmaceutically acceptable salt or derivatives thereof can be transdermal electromotive administration, the transdermal absorption being accelerated by use of an electrode-drug receptacle attached to the patients. For such topical treatment the pharmaceutical product can be used as liquid, semi-solid or solid medicine. Liquid medicines are solutions, suspensions, emulsions or dispersions of the above-cited active ingredients or combinations of active ingredients as drops, tinctures and sprays. As semi-solid medicines, for example, gels, ointments, creams and foams are used while, for example, powders, toilet powders, granulates, pellets and microcapsules are used as solid medicines.

If the pharmaceutical product containing as active ingredient tetrahydro-pyran-diterpene compound, its hydrochloride or any pharmaceutically acceptable salt or derivatives thereof, is used as a liquid, it is recommended to use as far as possible irritation-free diluting agents, as for example water, monovalent alcohols, especially ethanol, polyvalent alcohols, especially glycerine and/or propanediol, polyglycols, especially polyethylene glycols and/or miglyols, glycerine formal, dimethylisosorbide, natural and synthetic oils and/or esters.

For the production of semi-solid products, as for example gels, ointments, creams and foams, in addition to the above-cited diluting agents basic materials, as for example bentonite, veegum, guar flour and/or cellulose derivatives, especially methylcellulose and/or caboxymethylcellulose, are suitable. The tetrahydro-pyran-diterpene compound hydrochloride, maleate and/or alkali and/or alkaline earth salts may be in the form of a physico-chemical complex with a phospholipid selected from the group consisting of lecithin, cephalin, phosphatidylserine, phosphoinositide, and phosphatidic acid, or mixtures thereof in the form of a cream, an ointment, a pomade, a gel, or an emulsion to the area to be treated. The process of manufacture of such complexes has been described by Bertini Curri in US5,280,020.

Furthermore, instead of the above-cited basic materials or in addition to these materials polymers of vinylalcohol and vinylpyrrolidone, alginates, pectines, polyacrylates, solid and/or liquid polyethylenglycols, paraffins, fatty alcohols, vaseline and/or waxes, fatty acids and/or fatty acid esters are used. It is possible to use the above-cited active ingredients without filler for the production of solid products, as for example powders, toilet powder, granules, pellets and microcapsules. The pharmaceutical product described here is especially suited for the attention of such of the above-described diseases which are in a much progressed stage so that at first an increased concentration of active ingredients is necessary. With less serious disease conditions or with progressive healing of the disease such embodiments of the solid pharmaceutical product are used which contain fillers, as for example colloidal silicic acid, powdered soapstone, milk sugar, starch powder, sugar, cellulose derivatives, gelatine, metal oxides and/or metal salts, wherein the concentration of the active ingredient or of the combination of active ingredients varies between 0.001% by weight and 50% by weight.

A suitable kind of pharmaceutical form may be a topical deliver form of the above-described active ingredient, which is made by the application of the solid, liquid or semi-solid pharmaceutical product onto a gauze strip, a compress or a plaster so that such a gauze strip, such a compress or such a plaster then is only locally applied onto the spot which is to be treated. The pharmaceutical product can be filled into the known receptacles, as for example bottles, tubes, toilet powder boxes and baby powder boxes as well as seal edge bags, which are possibly provided with metering means, as for example droplet forming means, metering valves or metering chambers.

For the manufacture of the pharmaceutical product of present examples the tetrahydro-pyran-diterpene compound indicated above are used. Instead of the tetrahydro-pyran-diterpene compound or in addition to it salts, especially the corresponding hydrochloride, maleate and/or alkali and/or alkaline earth salts of tetrahydro-pyran-diterpene compound, which can be sulphated and/or sulfonated on the aromatic moiety and/or the pyrrolidine moiety, can be used for the manufacture of the pharmaceutical product.

The pharmaceutical diterpene compounds and tetrahydro-pyran-diterpene compounds may be administered as the hydrochloride, hydrobromide, sulphate, maleate and/or alkali and/or alkaline earth salts. It may be bound to a cation-exchange resin for instance a tetrahydro-pyran-diterpene compound resinate complex. Tetrahydro-pyran-diterpene hydrochloride compound can for instance be bound to cation-exchange resins by soaking the resin in an aqueous solution of the drug, which renders it particularly suitable for oral administration. Process for making a taste-masked resinate are described by R. A. Honeysett et al. in EP0501763.

Generally, the PPAR agonist of present invention may be in a food, beverage, pharmaceutical, tobacco, nutraceutical, oral hygienic, or cosmetic.

The compounds of present invention for use of the medcial or nutriceutical treatment of present invention can also be in an orally ingestible compositions, eventually an orally ingestible sweetening composition for those compounds that have the terahydro-pyrans groups of which our examples have demonstrated that these terahydro-pyrans groups are important for the sweeting effect and the PPAR effect can also be induced for steviol like structure without terahydor-pyrans groups

There are no restrictions on the type of orally ingestible composition or dosage forms encompassed by embodiments of this invention as long as they are safe for human or animal consumption when used in a generally acceptable range. These compositions include food, beverage, pharmaceutical, tobacco, nutraceutical, oral hygienic/cosmetic products, and the like. Non-limiting examples of these products include non-carbonated and carbonated beverages such as colas, ginger ales, root beers, ciders, fruit-flavored soft drinks (e.g., citrus-flavored soft drinks such as lemon-lime or orange), powdered soft drinks (e.g., cola, juice, tea, water, coffee), and the like; fruit juices originating in fruits or vegetables, fruit juices including squeezed juices or the like, fruit juices containing fruit particles, fruit beverages, fruit juice beverages, beverages containing fruit juices, beverages with fruit flavorings, vegetable juices, juices containing vegetables, and mixed juices containing fruits and vegetables; sport drinks, energy drinks, near water and the like drinks (e.g., water with natural or synthetic flavorants); tea type or favorite type beverages such as coffee, cocoa, black tea, green tea, oolong tea and the like; beverages containing milk components such as milk beverages, coffee containing milk components, café au lait, milk tea, fruit milk beverages, drinkable yogurt, lactic acid bacteria beverages or the like; dairy products; bakery products; desserts such as yogurt, jellies, drinkable jellies, puddings, Bavarian cream, blancmange, cakes, ready to eat cereals, brownies, mousse and the like, sweetened food products eaten at tea time or following meals; frozen foods; cold confections, e.g. types of ice cream such as ice cream, ice milk, lacto-ice and the like (food products in which sweeteners and various other types of raw materials are added to milk products, and the resulting mixture is agitated and frozen), and ice confections such as sherbets, dessert ices and the like (food products in which various other types of raw materials are added to a sugary liquid, and the resulting mixture is agitated and frozen); ice cream; ready to eat cereals, general confections, e.g., baked confections or steamed confections such as cakes, crackers, biscuits, buns with bean-jam filling and the like; rice cakes and snacks; table top products; general sugar confections such as chewing gum (e.g., including compositions which comprise a substantially water-insoluble, chewable gum base, such as chicle or substitutes thereof, including jetulong, guttakay rubber or certain comestible natural synthetic resins or waxes), hard candy, soft candy, mints, nougat candy, jelly beans and the like; sauces including fruit flavored sauces, chocolate sauces and the like; edible gels; crèmes including butter crèmes, flour pastes, whipped cream and the like; jams including strawberry jam, marmalade and the like; breads including sweet breads and the like or other starch products; spice; general condiments including seasoned soy sauce used on roasted meats, roast fowl, barbecued meat and the like, as well as tomato catsup, sauces, noodle broth and the like; processed agricultural products, livestock products or seafood; processed meat products such as sausage and the like; retort food products, pickles, preserves boiled in soy sauce, delicacies, side dishes; snacks such as potato chips, cookies, or the like; cereal products; drugs or quasi-drugs that are administered orally or used in the oral cavity (e.g., vitamins, cough syrups, cough drops, chewable medicine tablets, amino acids, bitter-tasting agents, acidulants or the like), wherein the drug may be in solid, liquid, gel, or gas form such as a pill, tablet, spray, capsule, syrup, drop, troche agent, powder, and the like; personal care products such as other oral compositions used in the oral cavity such as mouth freshening agents, gargling agents, mouth rinsing agents, toothpaste, tooth polish, dentrifices, mouth sprays, teeth-whitening agent and the like; dietary supplements; tobacco products including smoke and smokeless tobacco products such as snuff, cigarette, pipe and cigar tobacco, and all forms of tobacco such as shredded filler, leaf, stem, stalk, homogenized leaf cured, reconstituted binders and reconstituted tobacco from tobacco dust, fines or ether sources in sheet, pellet or other forms, tobacco substitutes formulated from non-tobacco materials, dip or chewing tobacco; animal feed; nutraceutical products, which includes any food or part of a food that may provide medicinal or health benefits, including the prevention and treatment of disease (e.g., cardiovascular disease and high cholesterol, diabetes, osteoporosis, inflammation, or autoimmune disorders), non-limiting, examples of nutraceuticals include naturally nutrient-rich or medicinally active food, such as garlic, soybeans, antioxidants, fibers, phytosterols and phytostanols and their esters, glucosamine, chondroitin sulfate, stenol, stanol, ginseng, ginko, echinacea, or the like; other nutrients that provide health benefits, such as amino acids, vitamins, minerals, carotenoids, dietary fiber, fatty acids such as omega-3 or omega-6 fatty acids, DHA, EPA, or ALA which can be derived from plant or animal sources (e.g., salmon and other cold-water fish or algae), flavonoids, phenols, polyols, polyphenols (e.g., catechins, proanthocyanidins, procyanidins, anthocyanins, quercetin, resveratrol, isoflavones, curcumin, punicalagin, ellagitannin, citrus flavonoids such as hesperidin and naringin, and chlorogenic acid), prebiotics/probiotics, phytoestrogens, sulfides/thiols, policosanol, saponin, rubisco peptide, appetite suppressants, hydration agents, autoimmune agents, C-reactive protein reducing agents, or anti-inflammatory agents; or any other functional ingredient that is beneficial to the treatment of specific diseases or conditions, such as diabetes, osteoporosis, inflammation, or high cholesterol levels in the blood.

The PPAR agonist of present invention may be combined with fatty acids (including long chain polyunsaturated fatty acids), and especially with omega-3 fatty acids and omega-6 fatty acids which are nutrients required in the human diet. Such are are found in natural sources such as the oil of certain plants and animals, including fishes, walnuts, lingonberries, hemp, flax, chia, perilla, purslane, and algae. Since essential fatty acids such as omega-3 fatty acids and omega-6 fatty acids are not synthesized by the body, they, and their health benefits, must be obtained through food or dietary supplement. Therefore, omega-3 fatty acids have been used as beneficial functional ingredients for various compositions, including beverages. In addition, long chain omega-3 fatty acids (e.g., docosahexaenoic acid (DHLA) and eicosapentaenoic acid (EPA)) are known to enhance cognitive function and maintain cardiovascular health, among other health benefits (See, e.g., von Schacky, C , "Omega-3 Fatty Acids and Cardiovascular Disease," Current Opinion in Clinical Nutrition and Metabolic Care 7, no. 2 (March 2004): 131-6. and Simopoulos, A.P., "Essential Fatty Acids in Health and Chronic Disease," American Journal of Clinical Nutrition 79, no. 3 (March 2004): 523-4.).

The PPAR agonist of present invention may be as functional ingredients in the dosage form in a purity greater than about 50 % PPAR agonist by weight on a dry basis, in a purity greater than about 70 % PPAR agonist by weight on a dry basis, in a purity greater than about 80 % PPAR agonist by weight on a dry basis, in a purity greater than about 90 % PPAR agonist by weight on a dry basis, in a purity greater than about 97 % PPAR agonist by weight on a dry basis, in a purity greater than about 98 % PPAR agonist by weight on a dry basis.or in a purity greater than about 99 % PPAR agonist by weight on a dry basis.

In accordance with an embodiment of present invention the PPAR agonist(s) of present invention for the treatmen or prevention of the disorder of present may be in a dosafe form as a confection. Such confection may be in the form of any food that is typically perceived to be rich in sugar or is typically sweet. According to particular embodiments of the present invention, the confections may be bakery products such as pastries; desserts such as yogurt, jellies, drinkable jellies, puddings, Bavarian cream, blancmange, cakes, brownies, mousse and the like, sweetened food products eaten at tea time or following meals; frozen foods; cold confections, e. g. types of ice cream such as ice cream, ice milk, lacto-ice and the like (food products in which sweeteners and various other types of raw materials are added to milk products, and the resulting mixture is agitated and frozen), and ice confections such as sherbets, dessert ices and the like (food products in which various other types of raw materials are added to a sugary liquid, and the resulting mixture is agitated and frozen); ready to eat cereals, general confections, e. g., baked confections or steamed confections such as crackers, biscuits, buns with bean-jam filling, halvah, alfajor, and the like; rice cakes and snacks; table top products; general sugar confections such as chewing gum (e.g. including compositions which comprise a substantially water- insoluble, chewable gum base, such as chicle or substitutes thereof, including jetulong, guttakay rubber or certain comestible natural synthetic resins or waxes), hard candy, soft candy, mints, nougat candy, jelly beans, fudge, toffee, taffy, Swiss milk tablet, licorice candy, chocolates, gelatin candies, marshmallow, marzipan, divinity, cotton candy, and the like; sauces including fruit flavored sauces, chocolate sauces and the like; edible gels; cremes including butter cremes, flour pastes, whipped cream and the like; jams including strawberry jam, marmalade and the like; and breads including sweet breads and the like or other starch products, and combinations thereof. Ans such confection may comprise additional functional or non functional sweeteners such as synthetic high-potency sweetener selected from the group consisting of sucralose, acesulfame potassium and other salts, aspartame, alitame, saccharin, neohesperidin dihydrochalcone, cyclamate, neotame, N-[N-[3-(3-hydroxy-4-methoxyphenyl)propyl]-L-α-aspartyl]-L-phenylalanine 1-methyl ester, N-[N-[3-(3-hydroxy-4-methoxyphenyl)-3-methylbutyl]-L-α-aspartyl]-L-phenylalanine 1-methyl ester, N-[N-[3-(3-methoxy-4-hydroxyphenyl)propyl]-L-α-aspartyl]-L-phenylalanine 1-methyl ester, salts thereof, and combinations thereof or as mogroside IV, mogroside V, Luo Han Guo sweetener, siamenoside, monatin and its salts (monatin SS, RR, RS, SR), curculin, glycyrrhizic acid and its salts, thaumatin, monellin, mabinlin, brazzein, hernandulcin, phyllodulcin, glycyphyllin, phloridzin, trilobatin, baiyunoside, osladin, polypodoside A, pterocaryoside A, pterocaryoside B, mukurozioside, phlomisoside I, periandrin I, abrusoside A, cyclocarioside I, and combinations thereof.

As referred to herein, "confection" can mean a sweet, a lollie, a confectionery, or similar term. As referred to herein, "base composition" means any composition which can be a food item and provides a matrix for carrying the natural and/or synthetic high-potency sweetener and perhaps other flavors.

Suitable base compositions for embodiments of this invention may include flour, yeast, water, salt, butter, eggs, milk, milk powder, liquor, gelatin, nuts, chocolate, citric acid, tartaric acid, fumaric acid, natural flavors, artificial flavors, colorings, polyols, sorbitol, isomalt, maltitol, lactitol, malic acid, magnesium stearate, lecithin, hydrogenated glucose syrup, glycerine, natural or synthetic gum, starch, and the like, and combinations thereof. Such components generally are recognized as safe (GRAS) and/or are U.S. Food and Drug Administration (FDA)-approved. According to particular embodiments of the invention, the base composition is present in the confection in an amount ranging from about 0.1 to about 99 weight percent of the confection. Generally, the base composition is present in the confection in an amount, in combination with the at least one natural and/or synthetic high-potency sweetener and the at least one sweet taste improving composition, to provide a food product.

The base composition of the confection may optionally include other artificial or natural sweeteners, bulk sweeteners, or combinations thereof. BuUc sweeteners include both caloric and non-caloric compounds. In a particular embodiment, the sweet taste improving composition functions as the bulk sweetener. Non-limiting examples of bulk sweeteners include sucrose, dextrose, maltose, dextrin, dried invert sugar, fructose, high fructose corn syrup, levulose, galactose, corn syrup solids, tagatose, polyols (e.g., sorbitol, mannitol, xylitol, lactitol, erythritol, and maltitol), hydrogenated starch hydrolysates, isomalt, trehalose, and mixtures thereof. Generally, the amount of bulk sweetener present in the confection ranges widely depending on the particular embodiment of the confection and the desired degree of sweeteness. Those of ordinary skill in the art will readily ascertain the appropriate amount of bulk sweetener.

In a particular embodiment, a confection comprises at least one natural and/or synthetic high-potency sweetener in combination with at least one sweet taste improving composition and a base composition. Generally, the amount of natural and/or synthetic high-potency sweetener present in the confection ranges widely depending on the particular embodiment of the confection and the desired degree of sweeteness. Those of ordinary skill in the art will readily ascertain the appropriate amount of sweetener. In a particular embodiment, the at least one natural and/or synthetic high-potency sweetener is present in the confection in an amount in the range of about 30 ppm to about 6000 ppm of the confection. In another embodiment, the at least one natural and/or synthetic high- potency sweetener is present in the confection in an amount in the range of about 50 ppm to about 3000 ppm of the confection. In embodiments where the confection comprises hard candy, the at least one natural or synthetic high-potency sweetener is present in an amount in the range of about 150 ppm to about 2250 ppm of the hard candy.

In accordance with an embodiment of present invention the PPAR agonist of present invention may in a dosage form with synthetic sweetener sweetened compositions. In accordance with desirable embodiments of this invention, synthetic sweetener sweetened compositions such as those described hereinabove comprise a sweetenable orally ingestible composition, at least one synthetic sweetener, and at least one sweet taste improving composition selected from the group consisting of carbohydrates, polyols, amino acids, other sweet taste improving additives, and combinations thereof. For example, according to a particular embodiment of this invention, a synthetic sweetener sweetened beverage comprises an orally ingestible beverage composition, such as an aqueous beverage composition or the like, and a synthetic sweetener composition with a more sugar-like temporal profile and/or flavor profile, as disclosed herein. In addition, according to a particular embodiment of this invention, a synthetic sweetener sweetened food comprises an orally ingestible food composition and a synthetic sweetener composition with a more sugar-like temporal profile and/or flavor profile, as disclosed herein. In addition, according to a particular embodiment of this invention, a synthetic sweetener sweetened pharmaceutical comprises a pharmaceutically active composition and/or pharmaceutically acceptable salts thereof, and a synthetic sweetener composition with a more sugar-like temporal profile and/or flavor profile, as disclosed herein. Alternatively, in addition, according to a particular embodiment of this invention, a synthetic sweetener sweetened pharmaceutical comprises a pharmaceutically active composition and/or pharmaceutically acceptable salts thereof and a coating comprising an orally ingestible composition and a synthetic sweetener composition with a more sugar-like temporal profile and/or flavor profile, as disclosed herein. In addition, according to a particular embodiment of this invention, a synthetic sweetener sweetened tobacco product comprises a tobacco and a synthetic sweetener composition with a more sugar-like temporal profile and/or flavor profile, as disclosed herein. In addition, according to a particular embodiment of this invention, a synthetic sweetener sweetened nutraceutical product comprises an orally ingestible nutraceutical composition and a synthetic sweetener composition with a more sugar-like temporal profile and/or flavor profile, as disclosed herein. In addition, according to a particular embodiment of this invention, a synthetic sweetener sweetened oral hygenic product comprises an orally ingestible oral hygenic composition and a synthetic sweetener composition with a more sugar-like temporal profile and/or flavor profile, as disclosed herein. In addition, according to a particular embodiment of this invention, a synthetic sweetener sweetened cosmetic product comprises an orally ingestible cosmetic composition and a synthetic sweetener composition with a more sugar-like temporal profile and/or flavor profile, as disclosed herein.

Furthermore the compounds of present invention may further be incorporated in or combined with a sweet taste improving composition

As used herein, the phrase "sweet taste improving composition" includes any composition which imparts a more sugar-like temporal profile, sugar-like flavor profile, or both to a synthetic sweetener. Examples of sweet taste improving compositions include, but are not limited to, carbohydrates, polyols, amino acids, and other sweet taste improving taste additives imparting such sugar-like characteristics.

The term "carbohydrate" generally refers to aldehyde or ketone compounds substituted with multiple hydroxyl groups, of the general formula (CH2O)n, wherein n is 3-30, as well as their oligomers and polymers. The carbohydrates of the present invention can, in addition, be substituted or deoxygenated at one or more positions. Carbohydrates as used herein encompasses unmodified carbohydrates, carbohydrate derivatives, substituted carbohydrates, and modified carbohydrates. As used herein the phrases "carbohydrate derivatives", "substituted carbohydrate", and "modified carbohydrates" are synonymous. Modified carbohydrate means any carbohydrate wherein at least one atom has been added, removed, substituted, or combinations thereof. Thus, carbohydrate derivatives or substituted carbohydrates include substituted and unsubstituted monosaccharides, disaccharides, oligosaccharides, and polysaccharides. The carbohydrate derivatives or substituted carbohydrates optionally can be deoxygenated at any corresponding C-position, and/or substituted with one or more moieties such as hydrogen, halogen, haloalkyl, carboxyl, acyl, acyloxy, amino, amido, carboxyl derivatives, alkylamino, dialkylamino, arylamino, alkoxy, aryloxy, nitro, cyano, sulfo, mercapto, imino, sulfonyl, sulfenyl, sulfinyl, sulfamoyl, carboalkoxy, carboxamido, phosphonyl, phosphinyl, phosphoryl, phosphino, thioester, thioether, oximino, hydrazino, carbamyl, phosphor phosphonato, or any other viable functional group provided the carbohydrate derivative or substituted carbohydrate functions to improve the sweet taste of a synthetic sweetener.

Non-limiting examples of carbohydrates in embodiments of this invention include tagatose, trehalose, galactose, rhamnose, cyclodextrin (e.g., α-cyclodextrin, β-cyclodextrin, and γ-cyclodextrin), maltodextrin (including resistant maltodextrins such as Fibersol-2®), dextran, sucrose, glucose, ribulose, fructose, threose, arabinose, xylose, lyxose, allose, altrose, mannose, idose, lactose, maltose, invert sugar, isotrehalose, neotrehalose, palatinose or isomaltulose, erythrose, deoxyribose, gulose, idose, talose, erythrulose, xylulose, psicose, turanose, cellobiose, amylopectin, glucosamine, mannosamine, fucose, glucuronic acid, gluconic acid, glucono-lactone, abequose, galactosamine, beet oligosaccharides, isomalto-oligosaccharides (isomaltose, isomaltotriose, panose and the like), xylo-oligosaccharides (xylotriose, xylobiose and the like), gentio-oligoscaccharides (gentiobiose, gentiotriose, gentiotetraose and the like), sorbose, nigero-oligosaccharides, palatinose oligosaccharides, fucose, fructooligosaccharides (kestose, nystose and the like), maltotetraol, maltotriol, malto-oligosaccharides (maltotriose, maltotetraose, maltopentaose, maltohexaose, maltoheptaose and the like), lactulose, melibiose, raffinose, rhamnose, ribose, isomerized liquid sugars such as high fructose corn/starch syrup (e.g., HFCS55, HFCS42, or HFCS90), coupling sugars, soybean oligosaccharides, and glucose syrup. Additionally, the carbohydrates as used herein may be in either the D-or L-configuration.

The term "alkyl", as used herein, unless otherwise specified, refers to a saturated straight, branched, or cyclic, primary, secondary, or tertiary hydrocarbon, typically of C1 to C18, and specifically includes methyl, ethyl, propyl, isopropyl, butyl, isobutyl, t-butyl, pentyl, cyclopentyl, isopentyl, neopentyl, hexyl, isohexyl, cyclohexyl, cyclohexylmethyl, 3-methylpentyl, 2,2-dimethylbutyl, 2,3-dimethylbutyl, and 3,3-dimethylbutyl. The alkyl group optionally can be substituted with one or more moieties selected from the group consisting of hydroxyl, carboxy, carboxamido, carboalkoxy, acyl, amino, alkylamino, arylamino, alkoxy, aryloxy, nitro, cyano, sulfo, sulfato, phospho, phosphato, or phosphonato.

The term "alkenyl", as referred to herein, and unless otherwise specified, refers to a straight, branched, or cyclic hydrocarbon of C2 to C10 with at least one double bond. The alkenyl groups optionally can be substituted in the same manner as described above for the alkyl groups and also optionally can be substituted with a substituted or unsubstituted alkyl group.

The term "alkynyl", as referred to herein, and unless otherwise specified, refers to a C2 to C10 straight or branched hydrocarbon with at least one triple bond. The alkynyl groups optionally can be substituted in the same manner as described above for the alkyl groups and also optionally can be substituted with a substituted or unsubstituted alkyl group.

The term "aryl", as used herein, and unless otherwise specified, refers to phenyl, biphenyl, or naphthyl, and preferably phenyl. The aryl group optionally can be substituted with one or more moieties selected from the group consisting of hydroxyl, acyl, amino, halo, carboxy, carboxamido, carboalkoxy, alkylamino, alkoxy, aryloxy, nitro, cyano, sulfo, sulfato, phospho, phosphate, or phosphonato.

The term "heteroaryl" or "heteroaromatic", as used herein, refers to an aromatic or unsaturated cyclic moiety that includes at least one sulfur, oxygen, nitrogen, or phosphorus in the aromatic ring. Non-limiting examples are furyl, pyridyl, pyrimidyl, thienyl, isothiazolyl, imidazolyl, tetrazolyl, pyrazinyl, benzofuranyl, benzothiophenyl, quinolyl, isoquinolyl, benzothienyl, isobenzofuryl, pyrazolyl, indolyl, isoindolyl, benzimidazolyl, purinyl, carbazolyl, oxazolyl, thiazolyl, isothiazolyl, 1,2,4-thiadiazolyl, isooxazolyl, pyrrolyl, quinazolinyl, pyridazinyl, pyrazinyl, cinnolinyl, phthalazinyl, quinoxalinyl, xanthinyl, hypoxanthinyl, and pteridinyl. The heteroaryl or heteroaromatic group optionally can be substituted with one or more moieties selected from the group consisting of hydroxyl, acyl, amino, halo, alkylamino, alkoxy, aryloxy, nitro, cyano, sulfo, sulfato, phospho, phosphate, or phosphonato.

The term "heterocyclic" refers to a saturated nonaromatic cyclic group which may be substituted, and wherein there is at least one heteroatom, such as oxygen, sulfur, nitrogen, or phosphorus in the ring. The heterocyclic group optionally can be substituted in the same manner as described above for the heteroaryl group.

The term "aralkyl", as used herein, and unless otherwise specified, refers to an aryl group as defined above linked to the molecule through an alkyl group as defined above. The term alkaryl, as used herein, and unless otherwise specified, refers to an alkyl group as defined above linked to the molecule through an aryl group as defined above. The aralkyl or alkaryl group optionally can be substituted with one or more moieties selected from the group consisting of hydroxyl, carboxy, carboxamido, carboalkoxy, acyl, amino, halo, alkylamino, alkoxy, aryloxy, nitro, cyano, sulfo, sulfato, phospho, phosphate, or phosphonato.

The term "halo", as used herein, specifically includes chloro, bromo, iodo, and fluoro.

The term "alkoxy", as used herein, and unless otherwise specified, refers to a moiety of the structure--O-alkyl, wherein alkyl is as defined above.

The term "acyl", as used herein, refers to a group of the formula C(O)R', wherein R' is an alkyl, aryl, alkaryl or aralkyl group, or substituted alkyl, aryl, aralkyl or alkaryl, wherein these groups are as defined above.

The term "polyol", as used herein, refers to a molecule that contains more than one hydroxyl group. A polyol may be a diol, triol, or a tetraol which contains 2, 3, and 4 hydroxyl groups respectively. A polyol also may contain more than four hydroxyl groups, such as a pentaol, hexaol, heptaol, or the like, which contain, 5, 6, or 7 hydroxyl groups, respectively. Additionally, a polyol also may be a sugar alcohol, polyhydric alcohol, or polyalcohol which is a reduced form of carbohydrate, wherein the carbonyl group (aldehyde or ketone, reducing sugar) has been reduced to a primary or secondary hydroxyl group.

Non-limiting examples of polyols in embodiments of this invention include erythritol, maltitol, mannitol, sorbitol, lactitol, xylitol, isomalt, propylene glycol, glycerol (glycerine), threitol, galactitol, palatinose, reduced isomalto-oligosaccharides, reduced xylo-oligosaccharides, reduced gentio-oligosaccharides, reduced maltose syrup, reduced glucose syrup, and sugar alcohols or any other carbohydrates capable of being reduced which do not adversely affect the taste of the synthetic sweetener or the orally ingestible composition.

As used herein, the phrase "sweet taste improving additive" means any material that imparts a more sugar-like temporal profile or sugar-like flavor profile or both to a synthetic sweetener. Suitable sweet taste improving additives useful in embodiments of this invention include amino acids and their salts, polyamino acids and their salts, peptides, sugar acids and their salts, nucleotides and their salts, organic acids, inorganic acids, organic salts including organic acid salts and organic base salts, inorganic acid salts (e.g., sodium chloride, potassium chloride, magnesium chloride), bitter compounds, flavorants and flavoring ingredients, astringent compounds, polymers, proteins or protein hydrolysates, surfactants, emulsifiers, flavonoids, alcohols, and natural high-potency sweeteners.

Suitable sweet taste improving amino acid additives for use in embodiments of this invention include, but are not limited to, aspartic acid, arginine, glycine, glutamic acid, proline, threonine, theanine, cysteine, cystine, alanine, valine, tyrosine, leucine, isoleucine, asparagine, serine, lysine, histidine, ornithine, methionine, carnitine, aminobutyric acid (alpha-, beta-, or gamma-isomers), glutamine, hydroxyproline, taurine, norvaline, sarcosine, and their salt forms such as sodium or potassium salts or acid salts. The sweet taste improving amino acid additives also may be in the D-or L-configuration and in the mono-, di-, or tri-form of the same or different amino acids. Additionally, the amino acids may be α-, β-, γ-, δ-, and ε-isomers if appropriate. Combinations of the foregoing amino acids and their corresponding salts (e.g., sodium, potassium, calcium, magnesium salts or other alkali or alkaline earth metal salts thereof, or acid salts) also are suitable sweet taste improving additives in embodiments of this invention. The amino acids may be natural or synthetic. The amino acids also may be modified. Modified amino acids refers to any amino acid wherein at least one atom has been added, removed, substituted, or combinations thereof (e.g., N-alkyl amino acid, N-acyl amino acid, or N-methyl amino acid). Non-limiting examples of modified amino acids include amino acid derivatives such as trimethyl glycine, N-methyl-glycine, and N-methyl-alanine. As used herein, modified amino acids encompass both modified and unmodified amino acids. As used herein, amino acids also encompass both peptides and polypeptides (e.g., dipeptides, tripeptides, tetrapeptides, and pentapeptides) such as glutathione and L-alanyl-L-glutamine. Suitable sweet taste improving polyamino acid additives include poly-L-aspartic acid, poly-L-lysine (e.g., poly-L-α-lysine or poly-L-ε-lysine), poly-L-ornithine (e.g., poly-L-α-ornithine or poly-L-ε-ornithine), poly-L-arginine, other polymeric forms of amino acids, and salt forms thereof (e.g., calcium, potassium, sodium, or magnesium salts such as L-glutamic acid mono sodium salt). The sweet taste improving polyamino acid additives also may be in the D-or L-configuration. Additionally, the polyamino acids may be α-, β-, γ-, δ-, and ε-isomers if appropriate. Combinations of the foregoing polyamino acids and their corresponding salts (e.g., sodium, potassium, calcium, magnesium salts or other alkali or alkaline earth metal salts thereof or acid salts) also are suitable sweet taste improving additives in embodiments of this invention. The polyamino acids described herein also may comprise co-polymers of different amino acids. The polyamino acids may be natural or synthetic. The polyamino acids also may be modified, such that at least one atom has been added, removed, substituted, or combinations thereof (e.g., N-alkyl polyamino acid or N-acyl polyamino acid). As used herein, polyamino acids encompass both modified and unmodified polyamino acids. In accordance with particular embodiments of this invention, modified polyamino acids include, but are not limited to polyamino acids of various molecular weights (MW), such as poly-L-α-lysine with a MW of 1,500, MW of 6,000, MW of 25,200, MW of 63,000, MW of 83,000, or MW of 300,000.

Suitable sweet taste improving sugar acid additives for use in embodiments of this invention include but are not limited to aldonic, uronic, aldaric, alginic, gluconic, glucuronic, glucaric, galactaric, galacturonic, and their salts (e.g., sodium, potassium, calcium, magnesium salts or other physiologically acceptable salts), and combinations thereof.

Suitable sweet taste improving nucleotide additives for use in embodiments of this invention include but are not limited to inosine monophosphate ("IMP"), guanosine monophosphate ("GMP"), adenosine monophosphate ("AMP"), cytosine monophosphate (CMP), uracil monophosphate (UMP), inosine diphosphate, guanosine diphosphate, adenosine diphosphate, cytosine diphosphate, uracil diphosphate, inosine triphosphate, guanosine triphosphate, adenosine triphosphate, cytosine triphosphate, uracil triphosphate, and their alkali or alkaline earth metal salts, and combinations thereof. The nucleotides described herein also may comprise nucleotide-related additives, such as nucleosides or nucleic acid bases (e.g., guanine, cytosine, adenine, thymine, uracil).

Suitable sweet taste improving organic acid additives include any compound which comprises a--COOH moiety. Suitable sweet taste improving organic acid additives for use in embodiments of this invention include but are not limited to C2-C30 carboxylic acids, substituted hydroxyl C2-C30 carboxylic acids, benzoic acid, substituted benzoic acids (e.g., 2,4-dihydroxybenzoic acid), substituted cinnamic acids, hydroxyacids, substituted hydroxybenzoic acids, substituted cyclohexyl carboxylic acids, tannic acid, lactic acid, tartaric acid, citric acid, gluconic acid, glucoheptonic acids, adipic acid, hydroxycitric acid, malic acid, fruitaric acid (a blend of malic, fumaric, and tartaric acids), fumaric acid, maleic acid, succinic acid, chlorogenic acid, salicylic acid, creatine, caffeic acid, bile acids, acetic acid, ascorbic acid, alginic acid, erythorbic acid, polyglutamic acid, glucono delta lactone, and their alkali or alkaline earth metal salt derivatives thereof. In addition, the organic acid additives also may be in either the D-or L-configuration.

Suitable sweet taste improving organic acid additive salts include, but are not limited to, sodium, calcium, potassium, and magnesium salts of all organic acids, such as salts of citric acid, malic acid, tartaric acid, fumaric acid, lactic acid (e.g., sodium lactate), alginic acid (e.g., sodium alginate), ascorbic acid (e.g., sodium ascorbate), benzoic acid (e.g., sodium benzoate or potassium benzoate), and adipic acid. The examples of the sweet taste improving organic acid additives described optionally may be substituted with one or more of the following moiety selected from the group consisting of hydrogen, alkyl, alkenyl, alkynyl, halo, haloalkyl, carboxyl, acyl, acyloxy, amino, amido, carboxyl derivatives, alkylamino, dialkylamino, arylamino, alkoxy, aryloxy, nitro, cyano, sulfo, thiol, imine, sulfonyl, sulfenyl, sulfinyl, sulfamyl, carboxalkoxy, carboxamido, phosphonyl, phosphinyl, phosphoryl, phosphino, thioester, thioether, anhydride, oximino, hydrazino, carbamyl, phospho, phosphonato, or any other viable functional group provided the substituted organic acid additives function to improve the sweet taste of a synthetic sweetener.

Suitable sweet taste improving inorganic acid additives for use in embodiments of this invention include but are not limited to phosphoric acid, phosphorous acid, polyphosphoric acid, hydrochloric acid, sulfuric acid, carbonic acid, sodium dihydrogen phosphate, and their corresponding alkali or alkaline earth metal salts thereof (e.g., inositol hexaphosphate Mg/Ca).

Suitable sweet taste improving bitter compound additives for use in embodiments of this invention include but are not limited to caffeine, quinine, urea, bitter orange oil, naringin, quassia, and salts thereof.

Suitable sweet taste improving flavorant and flavoring ingredient additives for use in embodiments of this invention include but are not limited to vanillin, vanilla extract, mango extract, cinnamon, citrus, coconut, ginger, viridiflorol, almond, menthol (including menthol without mint), grape skin extract, and grape seed extract. "Flavorant" and "flavoring ingredient" are synonymous and can include natural or synthetic substances or combinations thereof. Flavorants also include any other substance which imparts flavor and may include natural or non-natural (synthetic) substances which are safe for human or animals when used in a generally accepted range. Non-limiting examples of proprietary flavorants include Doehler® Natural Flavoring Sweetness Enhancer K14323 (Doehler®, Darmstadt, Germany), Symrise® Natural Flavor Mask for Sweeteners 161453 and 164126 (Symrise®, Holzminden, Germany), Natural Advantage® Bitterness Blockers 1, 2, 9 and 10 (Natural Advantage®, Freehold, N.J., U.S.A.), and Sucramask® (Creative Research Management, Stockton, Calif., U.S.A.).

Systemic sclerosis (SSc) is a systemic connective tissue disease. Characteristics of SSc include essential vasomotor disturbances; fibrosis; subsequent atrophy of the skin, subcutaneous tissue, muscles, and internal organs (eg, alimentary tract, lungs, heart, kidney, CNS); and immunologic disturbances accompany these findings.

Essentially pure for the meaning of this application is in a purity greater than about 90 % of the concerned compound by weight on a dry basis, more preferably in a purity greater than about 97 % by weight on a dry basis, and more preferably in a purity greater than about 98 % by weight on a dry basis.or most preferably in a purity greater than about 99 % by weight on a dry basis.

Suitable sweet taste improving polymer additives for use in embodiments of this invention include, but are not limited to, chitosan, pectin, pectic, pectinic, polyuronic, polygalacturonic acid, starch, food hydrocolloid or crude extracts thereof (e.g., gum acacia senegal (Fibergum®), gum acacia seyal, carageenan), poly-L-lysine (e.g., poly-L-α-lysine or poly-L-ε-lysine), poly-L-ornithine (e.g., poly-L-α-ornithine or poly-L-α-omithine), polypropylene glycol, polyethylene glycol, poly(ethylene glycol methyl ether), polyarginine, polyaspartic acid, polyglutamic acid, polyethyleneimine, alginic acid, sodium alginate, propylene glycol alginate, sodium polyethyleneglycolalginate, sodium hexametaphosphate or its salts, and other cationic polymers and anionic polymers.

Suitable sweet taste improving protein or protein hydrolysate additives for use in embodiments of this invention include, but are not limited to, bovine serum albumin (BSA), whey protein (including fractions or concentrates thereof such as 90% instant whey protein isolate, 34% whey protein, 50% hydrolyzed whey protein, and 80% whey protein concentrate), soluble rice protein, soy protein, protein isolates, protein hydrolysates, reaction products of protein hydrolysates, glycoproteins, and/or proteoglycans containing amino acids (e.g., glycine, alanine, serine, threonine, asparagine, glutamine, arginine, valine, isoleucine, leucine, norvaline, methionine, proline, tyrosine, hydroxyproline, and the like), collagen (e.g., gelatin), partially hydrolyzed collagen (e.g., hydrolyzed fish collagen), and collagen hydrolysates (e.g., porcine collagen hydrolysate).

Suitable sweet taste improving surfactant additives for use in embodiments of this invention include but are not limited to polysorbates (e.g., polyoxyethylene sorbitan monooleate (polysorbate 80), polysorbate 20, polysorbate 60), sodium dodecylbenzenesulfonate, dioctyl sulfosuccinate or dioctyl sulfosuccinate sodium, sodium dodecyl sulfate, cetylpyridinium chloride (hexadecylpyridinium chloride), hexadecyltrimethylammonium bromide, sodium cholate, carbamoyl, choline chloride, sodium glycocholate, sodium taurodeoxycholate, lauric arginate, sodium stearoyl lactylate, sodium taurocholate, lecithins, sucrose oleate esters, sucrose stearate esters, sucrose palmitate esters, sucrose laurate esters, and other emulsifiers, and the like.

Suitable sweet taste improving flavonoid additives for use in embodiments of this invention generally are classified as flavonols, flavones, flavanones, flavan-3-ols, isoflavones, or anthocyanidins. Non-limiting examples of flavonoid additives include catechins (e.g., green tea extracts such as Polyphenon® 60, Polyphenon® 30, and Polyphenon® 25 (Mitsui Norin Co., Ltd., Japan), polyphenols, rutins (e.g., enzyme modified rutin Sanmelin® AO (San-fi Gen F.F.I., Inc., Osaka, Japan)), neohesperidin, naringin, neohesperidin dihydrochalcone, and the like.

Suitable sweet taste improving alcohol additives for use in embodiments of this invention include, but are not limited to, ethanol.

Suitable sweet taste improving astringent compound additives include, but are not limited to, tannic acid, europium chloride (EuCl3), gadolinium chloride (GdCl3), terbium chloride (TbCl3), alum, tannic acid, and polyphenols (e.g., tea polyphenol).

Suitable sweet taste improving vitamins include nicotinamide (Vitamin B3) and pyridoxal hydrochloride (Vitamin B6).

Suitable sweet taste improving natural high-potency sweetener additives for use in embodiments of this invention include, but are not limited to, rebaudioside A, rebaudioside B, rebaudioside C, rebaudioside D, rebaudioside E, rebaudioside F, dulcoside A, dulcoside B, rubusoside, stevia, stevioside, mogroside IV, mogroside V, Luo Han Guo sweetener, siamenoside, monatin and its salts (monatin SS, RR, RS, SR), curculin, glycyrrhizic acid and its salts, thaumatin, monellin, mabinlin, brazzein, hernandulcin, phyllodulcin, glycyphyllin, phloridzin, trilobtain, baiyunoside, osladin, polypodoside A, pterocaryoside A, pterocaryoside B, mukurozioside, phlomisoside I, periandrin I, abrusoside A, and cyclocarioside I. As used herein, the phrase "natural high-potency sweetener" or "NHPS" means any sweetener found in nature which may be in raw, extracted, purified, or any other form, singularly or in combination thereof and characteristically have a sweetness potency greater than sucrose, fructose, or glucose, yet have less calories. NHPS may be further modified. Modified NHPSs includes NHPSs which have been altered naturally or synthetically. For example, a modified NHPS includes, but is not limited to, NHPSs which have been fermented, contacted with enzyme, or derivatized, or the product of any process wherein at least one atom has been added to, removed from, or substituted on the NHPS. In one embodiment, extracts of NHPS may be used in any purity percentage. In another embodiment, when a NHPS is used as a non-extract, the purity of the NHPS may range for example from about 25% to about 100%. In another example, the purity of the NHPS may range from about 70% to about 100%; from about 80% to about 90%; from about 90% to about 100%; from about 95% to about 100%; from about 96% to about 99%; from about 97% to about 98%; from about 98% to about 99%; and from about 99% to about 100%. Specific embodiments of NHPS compositions in combination with sweet taste improving compositions are disclosed in U.S. Provisional Application No. 60/739,302, entitled "Natural High-Potency Sweetener Compositions with Improved Temporal Profile and/or Flavor Profile, Methods for Their Formulation, and Uses," filed on Nov. 23, 2005, by DuBois, et al., the disclosure of which is incorporated herein by reference in its entirety.

The sweet taste improving compositions also may be in salt form which may be obtained using standard procedures well known in the art. The term "salt" also refers to complexes that retain the desired chemical activity of the sweet taste improving compositions of the present invention and are safe for human or animal consumption in a generally acceptable range. Alkali metal (for example, sodium or potassium) or alkaline earth metal (for example, calcium or magnesium) salts also can be made. Salts also may include combinations of alkali and alkaline earth metals. Non-limiting examples of such salts are (a) acid addition salts formed with inorganic acids and salts formed with organic acids; (b) base addition salts formed with metal cations such as calcium, bismuth, barium, magnesium, aluminum, copper, cobalt, nickel, cadmium, sodium, potassium, and the like, or with a cation formed from ammonia, N,N-dibenzylethylenediamine, D-glucosamine, tetraethylammonium, or ethylenediamine; or (c) combinations of (a) and (b). Thus, any salt forms which may be derived from the sweet taste improving compositions may be used with the embodiments of the present invention as long as the salts of the sweet taste improving additives do not adversely affect the taste of synthetic sweeteners or the orally ingestible compositions or dosage forms which comprises at least one synthetic sweetener. The salt forms of the additives can be added to the synthetic sweetener composition in the same amounts as their acid or base forms.

In particular embodiments, suitable sweet taste improving inorganic salts useful as sweet taste improving additives include sodium chloride, potassium chloride, sodium sulfate, magnesium phosphate, potassium citrate, europium chloride (EuCl3), gadolinium chloride (GdCl3), terbium chloride (TbCl3), magnesium sulfate, alum, magnesium chloride, mono-, di-, tri-basic sodium or potassium salts of phosphoric acid (e.g., inorganic phosphates), salts of hydrochloric acid (e.g., inorganic chlorides), sodium carbonate, sodium bisulfate, and sodium bicarbonate. Furthermore, in particular embodiments, suitable organic salts useful as sweet taste improving additives include, but are not limited to, alginic acid sodium salt (sodium alginate), glucoheptonic acid sodium salt, choline chloride, gluconic acid sodium salt (sodium gluconate), gluconic acid potassium salt potassium gluconate), guanidine HCl, glucosamine HCl, monosodium glutamate (MSG), amiloride HCl, adenosine monophosphate salt, magnesium gluconate, potassium tartrate (monohydrate), and sodium tartrate (dihydrate).

Embodiments of the sweet taste improving compositions of this invention can impart a more sharp and clean sensation to the taste of synthetic sweeteners. Furthermore, embodiments of the sweet taste improving compositions of the present invention have a superior effect in improving the temporal profile and/or flavor profile of synthetic sweeteners while at the same time providing a sweetener composition with a low-caloric or non-caloric content, imparting more sugar-like characteristics.

In yet an embodiment of present invention the compounds of present invention can for use of present invention be combined with or incorporated in According a synthetic sweetener composition. A synthetic sweetener composition comprises at least one sweet taste improving composition present in the synthetic sweetener composition in an amount effective for the synthetic sweetener composition to impart an osmolarity of at least 10 mOsmoleslL to an aqueous solution of the synthetic sweetener composition wherein the synthetic sweetener is present in the aqueous solution in an amount sufficient to impart a maximum sweetness intensity equivalent to that of a 10% aqueous solution of sucrose by weight. As used herein, "mOsmoles/L" refers to milliosmoles per liter. According to another embodiment, a synthetic sweetener composition comprises at least one sweet taste improving composition in an amount effective for the synthetic sweetener composition to impart an osmolarity of 10 to 500 mOsmoles/L, preferably 25 to 500 mOsmoles/L preferably, 100 to 500 mOsmoles/L, more preferably 200 to 500 mOsmoles/L, and still more preferably 300 to 500 mOsmoles/L to an aqueous solution of the synthetic sweetener composition wherein the synthetic sweetener is present in the aqueous solution in an amount sufficient to impart a maximum sweetness intensity equivalent to that of a 10% aqueous solution of sucrose by weight. In particular embodiments, a plurality of sweet taste improving compositions may be combined with a synthetic sweetener and in that case, the osmolarity impacted is that of the total combination of the plurality of sweet taste improving compositions.

Osmolarity refers to the measure of osmoles of solute per liter of solution, wherein osmole is equal to the number of moles of osmotically active particles in an ideal solution (e.g., a mole of glucose is one osmole), whereas a mole of sodium chloride is two osmoles (one mole of sodium and one mole of chloride). Thus, in order to improve in the quality of taste of synthetic sweeteners, the osmotically active compounds or the compounds which impart osmolarity must not introduce significant off taste to the formulation.

In one embodiment, suitable sweet taste improving compositions which improves the temporal profile of the synthetic sweetener or sweetenable composition to be more sugar-like include carbohydrates, polyols, amino acids, other sweet taste improving additives (e.g., sugar acids and their salts, nucleotides, organic acids, inorganic acids, organic salts including organic acid salts and/organic base salts, inorganic salts, bitter compounds, astringent compounds, proteins or protein hydrolysates, surfactants, emulsifiers, flavonoids, alcohols, and natural high-potency sweeteners).

In more preferred embodiments, non-limiting examples of suitable compounds which impart osmolarity include sweet taste improving carbohydrate additives, sweet taste improving polyol additives, sweet taste improving alcohol additives, sweet taste improving inorganic acid additives, sweet taste improving organic acid additives, sweet taste improving inorganic salt additives, sweet taste improving organic salt additives, sweet taste improving organic base salt additives, sweet taste improving amino acid additives, sweet taste improving amino acid salt additives, sweet taste improving bitter additives, and sweet taste improving astringent additives.

In one embodiment, suitable compounds which impart osmolarity include, but are not limited to, sucrose, fructose, glucose, maltose, lactose, mannose, galactose, tagatose, erythritol, glycerol, propylene glycol, ethanol, phosphoric acid (including corresponding sodium, potassium, calcium, and magnesium salts thereof), citric acid, malic acid, tartaric acid, fumaric acid, gluconic acid, adipic acid, glucosamine and glucosamine salt, choline salt, guanidine salt, protein or protein hydrolysate, glycine, alanine, serine, threonine, theanine, caffeine, quinine, urea, naringin, tannic acid, AlNa(SO4)2, AlK(SO4)2 and other forms of alum, and combinations thereof.

In one embodiment, suitable sweet taste improving carbohydrate additives for the present invention have a molecular weight less than or equal to 500 and desirably have a molecular weight from 50 to 500. In particular embodiments, suitable carbohydrates with a molecular weight less than or equal to 500 include, but are not limited to, sucrose, fructose, glucose, maltose, lactose, mannose, galactose, and tagatose. Generally, in accordance with desirable embodiments of this invention, a carbohydrate is present in the synthetic sweetener compositions in an amount from about 1,000 to about 100,000 ppm. (Throughout this specification, the term ppm means parts per million by weight or volume. For example, 500 ppm means 500 mg in a liter.) In accordance with other desirable embodiments of this invention, a carbohydrate is present in the synthetic sweetener sweetened compositions in an amount from about 2,500 to about 10,000 ppm. In another embodiment, suitable sweet taste improving carbohydrate additives for imparting osmolarities ranging from about 10 mOsmoles/L to about 500 mOsmoles/L to a sweetenable composition include, but are not limited to, sweet taste improving carbohydrate additives with a molecular weight ranging from about 50 to about 500.

In one embodiment, suitable polyol additives have a molecular weight less than or equal to 500 and desirably have a molecular weight from 76 to 500. In particular embodiments, suitable polyols with a molecular weight less than or equal to 500 include, but are not limited to, erythritol, glycerol, and propylene glycol. Generally, in accordance with desirable embodiments of this invention, a polyol is present in the synthetic sweetener compositions in an amount from about 400 ppm to about 80,000 ppm. In other embodiments of this invention, a polyol is present in the synthetic sweetener compositions in an amount from about 5,000 to about 40,000 ppm of the composition, more particularly from about 10,000 to about 35,000 ppm of the composition. In accordance with other desirable embodiments of this invention, a polyol is present in the synthetic sweetener sweetened compositions in an amount from about 400 to about 80,000 ppm. In a sub-embodiment, suitable sweet taste improving polyol additives for imparting osmolarities ranging from about 10 mOsmoles/L to about 500 mOsmoles/L to a sweetenable composition include, but are not limited to sweet taste improving polyol additives with a molecular weight ranging from about 76 to about 500.

Generally, in accordance with another embodiment of this invention, a suitable sweet taste improving alcohol is present in the synthetic sweetener compositions in an amount from about 625 to about 10,000 ppm. In another embodiment, suitable sweet taste improving alcohol additives for imparting osmolarities ranging from about 10 mOsmoles/L to about 500 mOsmoles/L to a sweetenable composition include, but are not limited to sweet taste improving alcohol additives with a molecular weight ranging from about 46 to about 500. A non-limiting example of a sweet taste improving alcohol additive with a molecular weight ranging from about 46 to about 500 includes ethanol.

In one embodiment, suitable sweet taste improving amino acid additives have a molecular weight of less than or equal to 250 and desirably have a molecular weight from 75 to 250. In particular embodiments, suitable amino acids with a molecular weight less than or equal to 250 include, but are not limited to, glycine, alanine, serine, valine, leucine, isoleucine, proline, theanine, and threonine. Preferred amino acids include those which are sweet tasting at high concentrations, but are desirably present in embodiments of this invention at amounts below or above their sweetness taste detection threshold. Even more preferred are mixtures of amino acids at amounts below or above their sweetness taste detection threshold. Generally, in accordance with desirable embodiments of this invention, an amino acid is present in the synthetic sweetener compositions in an amount from about 100 ppm to about 25,000 ppm, more particularly from about 1,000 to about 10,000 ppm, and still more particularly from about 2,500 to about 5,000 ppm. In accordance with other desirable embodiments of this invention, an amino acid is present in the synthetic sweetener sweetened compositions in an amount from about 250 ppm to about 7,500 ppm. In a sub-embodiment, suitable sweet taste improving amino acid additives for imparting osmolarities ranging from about 10 mOsmoles/L to about 500 mOsmoles/L to a sweetenable composition include, but are not limited to sweet taste improving amino acid additives with a molecular weight ranging from about 75 to about 250.

Generally, in accordance with yet another embodiment of this invention, a suitable sweet taste improving amino acid salt additive is present in the synthetic sweetener compositions in an amount from about 25 to about 10,000 ppm more particularly from about 1,000 to about 7,500 ppm, and still more particularly from about 2,500 to about 5,000 ppm. In another embodiment, suitable sweet taste improving amino acid salt additives for imparting osmolarities ranging from about 10 mOsmoles/L to about 500 mOsmoles/L to a sweetenable composition include, but are not limited to, sweet taste improving amino acid additives with a molecular weight ranging from about 75 to about 300. Non-limiting examples of sweet taste improving amino acid salt additives with a molecular weight ranging from about 75 to about 300 include salts of glycine, alanine, serine, theanine, and threonine.

Generally, in accordance with still another embodiment of this invention, a suitable sweet taste improving protein or protein hydroyslate additive is present in the synthetic sweetener compositions in an amount from about 200 to about 50,000 ppm. In another embodiment, suitable sweet taste improving protein or protein hydrolysate additives for imparting osmolarities ranging from about 10 mOsmoles/L to about 500 mOsmoles/L to a sweetenable composition include, but are not limited to, sweet taste improving protein hydrolysate additives with a molecular weight ranging from about 75 to about 300. Non-limiting examples of sweet taste improving protein or protein hydrolysate additives with a molecular weight ranging from about 75 to about 300 include proteins or protein hydrolysates containing glycine, alanine, serine, and threonine.

Generally, in accordance with another embodiment of this invention, a suitable sweet taste improving inorganic acid additive is present in the synthetic sweetener compositions in an amount from about 25 to about 5,000 ppm. In another embodiment, suitable sweet taste improving inorganic acid additives for imparting osmolarities ranging from about 10 mOsmoles/L to about 500 mOsmoles/L to a sweetenable composition include, but are not limited to, phosphoric acid, HCl, and H2SO4 and any other inorganic acid additives which are safe for human or animal consumption when used in a generally acceptable range. In a sub-embodiment, suitable sweet taste improving inorganic acid additives for imparting osmolarities ranging from about 10 mOsmolesAL to about 500 mOsmoles/L to a sweetenable composition include, but are not limited to, sweet taste improving inorganic acid additives with a molecular weight range from about 36 to about 98.

Generally, in accordance with still another embodiment of this invention, a suitable sweet taste improving inorganic acid salt additive is present in the synthetic sweetener compositions in an amount from about 25 to about 5,000 ppm. In another embodiment, suitable sweet taste improving inorganic acid salt additives for imparting osmolarities ranging from about 10 mOsmoles/L to about 500 mOsmolesAL to a sweetenable composition include, but are not limited to, salts of inorganic acid, for example sodium, potassium, calcium, and magnesium salts of phosphoric acid (e.g., inorganic phosphates), and any other alkali or alkaline earth metal salts of other inorganic acid additives (e.g., sodium bisulfate) which are safe for human or animal consumption when used in a generally acceptable range. In a particular embodiment, suitable sweet taste improving inorganic acid salt additives include magnesium chloride, magnesium sulfate, sodium chloride, or combinations thereof.

In a sub-embodiment, suitable sweet taste improving inorganic acid salt additives for imparting osmolarities ranging from about 10 mOsmoles/L to about 500 mOsmoles/L to a sweetenable composition include, but are not limited to, sweet taste improving inorganic acid salt additives with a molecular weight range from about 58 to about 120.

Generally, in accordance with still another embodiment of this invention, a suitable sweet taste improving organic acid additive is present in the synthetic sweetener compositions in an amount from about 10 to about 5,000 ppm. In another embodiment, suitable sweet taste improving organic acid additives for imparting osmolarities ranging from about 10 mOsmoles/L to about 500 mOsmoles/L to a sweetenable composition include, but are not limited to, creatine, citric acid, malic acid, succinic acid, hydroxycitric acid, tartaric acid, fumaric acid, gluconic acid, glutaric acid, adipic acid, and any other sweet taste improving organic acid additives which are safe for human or animal consumption when used in a generally acceptable range. In one embodiment, the sweet taste improving organic acid additive comprises a molecular weight range from about 60 to about 208.

Generally, in accordance with still another embodiment of this invention, a suitable sweet taste improving organic acid salt additive is present in the synthetic sweetener compositions in an amount from about 20 to about 10,000 ppm. In another embodiment, suitable sweet taste improving organic acid salt additives for imparting osmolarities ranging from about 10 mOsmoles/L to about 500 mOsmoles/L to a sweetenable composition include, but are not limited to, salts the sweet taste improving organic acid additives such as sodium, potassium, calcium, magnesium, and other alkali or alkaline metal salts of citric acid, malic acid, tartaric acid, fumaric acid, gluconic acid, adipic acid, hydroxycitric acid, succinic acid, glutaric acid, and salts of any other sweet taste improving organic acid additives which are safe for human or animal consumption when used in a generally acceptable range. In one embodiment, the sweet taste improving organic acid salt additive comprises a molecular weight range from about 140 to about 208.

Generally, in accordance with yet another embodiment of this invention, a suitable sweet taste improving organic base salt additive is present in the synthetic sweetener compositions in an amount from about 10 to about 5,000 ppm. In another embodiment, suitable sweet taste improving organic base salt additives for imparting osmolarities ranging from about 10 mOsmoles/L to about 500 mOsmoles/L to a sweetenable composition include, but are not limited to, inorganic and/organic acid salts of organic bases such as glucosamine salts, choline salts, and guanidine salts.

Generally, in accordance with yet another embodiment of this invention, a suitable sweet taste improving astringent additive is present in the synthetic sweetener compositions in an amount from about 25 to about 1,000 ppm. In another embodiment, suitable sweet taste improving astringent additives for imparting osmolarities ranging from about 10 mOsmoles/L to about 500 mOsmoles/L to a sweetenable composition include, but are not limited to, tannic acid, tea pholyphenols, catechins, aluminum sulfate, AlNa(SO4)2, AlK(SO4)2 and other forms of alum.

Generally, in accordance with yet another embodiment of this invention, a suitable sweet taste improving nucleotide additive is present in the synthetic sweetener compositions in an amount from about 5 to about 1,000 ppm. In another embodiment, suitable sweet taste improving nucleotide additives for imparting osmolarities ranging from about 10 mOsmoles/L to about 500 mOsmoles/L to a sweetenable composition include, but are not limited to, adenosine monophosphate.

Generally, in accordance with yet another embodiment of this invention, a suitable sweet taste improving polyamino acid additive is present in the synthetic sweetener compositions in an amount from about 30 to about 2,000 ppm. In another embodiment, suitable sweet taste improving polyamino acid additives for imparting osmolarities ranging from about 10 mOsmoles/L to about 500 mOsmoles/L to a sweetenable composition include, but are not limited to, poly-L-lysine (e.g., poly-L-α-lysine or poly-L-ε-lysine), poly-L-ornithine (e.g., poly-L-α-ornithine or poly-ε-ornithine), and poly-L-arginine.

Generally, in accordance with yet another embodiment of this invention, a suitable sweet taste improving polymer additive is present in the synthetic sweetener compositions in an amount from about 30 to about 2,000 ppm. In another embodiment, suitable sweet taste improving polymer additives for imparting osmolarities ranging from about 10 mOsmoles/L to about 500 mOsmoles/L to a sweetenable composition include, but are not limited to, chitosan, pectin, hydrocolloids such as gum acacia senegal, propylene glycol, polyethylene glycol, and poly(ethylene glycol methyl ether).

Generally, in accordance with yet another embodiment of this invention, a suitable sweet taste improving surfactant additive is present in the synthetic sweetener compositions in an amount from about 1 to about 5,000 ppm. In another embodiment, suitable sweet taste improving surfactant additives for imparting osmolarities ranging from about 10 mOsmoles/L to about 500 mOsmoles/L to a sweetenable composition include, but are not limited to, polysorbates, choline chloride, sodium taurocholate, lecithins, sucrose oleate esters, sucrose stearate esters, sucrose palmitate esters, and sucrose laurate esters.

Generally, in accordance with yet another embodiment of this invention, a suitable sweet taste improving flavonoid additive is present in the synthetic sweetener compositions in an amount from about 0.1 to about 1,000 ppm. In another embodiment, suitable sweet taste improving flavonoid additives for imparting osmolarities ranging from about 10 mOsmoles/L to about 500 mOsmoles/L to a sweetenable composition include, but are not limited to, naringin, catechins, rutins, neohesperidin, and neoheperidin dihydrochalcone.

Furthermore the compounds of present invention can for use of present invention be combined with a suitable sweet taste improving compositions which is a flavour profile modulator or improves the favour profile. The flavor profile imparts sugar-like characteristic to synthetic sweeteners. Any sweet taste improving composition that imparts a sugar-like flavor profile to synthetic sweeteners will be effective by this mechanism. In particular, any sweet taste improving composition that imparts a more sugar-like osmotic taste will be effective by this mechanism. In one embodiment, suitable sweet taste improving compositions which improves the flavor profile, including the osmotic taste, of the synthetic sweetener or sweetenable composition to be more sugar-like include carbohydrates, polyols, amino acids, and other sweet taste improving additives (e.g., polyamino acids, peptides, sugar acids and their salts, nucleotides, organic acids, inorganic acids, organic salts including organic acid salts and organic base salts, inorganic salts, bitter compounds, flavorants and flavoring ingredients, astringent compounds, proteins or protein hydrolysates, surfactants, emulsifiers, flavonoids, alcohols, and natural high-potency sweeteners).

In a preferred embodiment, non-limiting examples of sweet taste improving compositions enhancing the synthetic sweetener's osmotic taste to be more sugar-like include sweet taste improving carbohydrate additives, sweet taste improving alcohol additives, sweet taste improving polyol additives, sweet taste improving amino acid additives, sweet taste improving amino acid salt additives, sweet taste improving inorganic acid salt additives, sweet taste improving polymer additives, and sweet taste improving protein or protein hydrolysate additives.

In another embodiment, suitable sweet improving amino acid additives for improving the osmotic taste of the synthetic sweeteners to be more sugar-like include, but are not limited to, amino acid additives comprising a molecular weight less than or equal to 250. In one example, suitable sweet taste improving amino acids include, but are not limited to, low molecular weight amino acids such as glycine, leucine, valine, isoleucine, proline, hydroxyproline, alanine, serine, theanine, and threonine.

In another embodiment, suitable sweet taste improving carbohydrate additives for improving the osmotic taste of the synthetic sweeteners to be more sugar-like include, but are not limited to, sweet taste improving carbohydrate additives with a molecular weight ranging from about 50 to about 500. Non-limiting examples of sweet taste improving carbohydrate additives with a molecular weight ranging from about 50 to about 500 include sucrose, fructose, glucose, maltose, lactose, mannose, galactose, ribose, rhamnose, trehalose, and tagatose.

In another embodiment, suitable sweet taste improving polyol additives for improving the osmotic taste of the synthetic sweeteners to be more sugar-like include, but are not limited to, sweet taste improving polyol additives with a molecular weight ranging from about 76 to about 500. Non-limiting examples of sweet taste improving polyol additives with a molecular weight ranging from about 76 to about 500 include erythritol, glycerol, and propylene glycol. In a sub-embodiment, other suitable sweet taste improving polyol additives include sugar alcohols.

In another embodiment, suitable sweet taste improving alcohol additives for improving the osmotic taste of the synthetic sweeteners to be more sugar-like include, but are not limited to, sweet taste improving alcohol additives with a molecular weight ranging from about 46 to about 500. A non-limiting example of sweet taste improving alcohol additive with a molecular weight ranging from about 46 to about 500 includes ethanol.

In another embodiment, suitable sweet taste improving amino acid additives for improving the osmotic taste of the synthetic sweeteners to be more sugar-like include, but are not limited to, sweet taste improving amino acid additives with a molecular weight ranging from about 75 to about 250. Non-limiting examples of sweet taste improving amino acid additives with a molecular weight ranging from about 75 to about 250 include glycine, alanine, serine, leucine, valine, isoleucine, proline, hydroxyproline, glutamine, theanine, and threonine.

In another embodiment, suitable sweet taste improving amino acid salt additives for improving the osmotic taste of the synthetic sweeteners to be more sugar-like include, but are not limited to, sweet taste improving amino acid salt additives with a molecular weight ranging from about 75 to about 300. Non-limiting examples of sweet taste improving amino acid salt additives with a molecular weight ranging from about 75 to about 300 include salts of glycine, alanine, serine, leucine, valine, isoleucine, proline, hydroxyproline, glutamine, theanine, and threonine.

In another embodiment, suitable sweet taste improving protein or protein hydrolysate additives for improving the osmotic taste of the synthetic sweeteners to be more sugar-like include, but are not limited to, sweet taste improving protein or protein hydrolysate additives with a molecular weight ranging from about 75 to about 300. Non-limiting examples of sweet taste improving protein or protein hydrolysate additives with a molecular weight ranging from about 75 to about 300 include proteins or protein hydrolysates containing glycine, alanine, serine, leucine, valine, isoleucine, proline, hydroxyproline, glutamine, and threonine.

In another embodiment, suitable sweet taste improving inorganic acid salt additives for improving the osmotic taste of synthetic sweeteners to be more sugar-like include, but are not limited to, sodium chloride, potassium chloride, magnesium chloride, KH2PO4 and NaH2PO4. Suitable sweet taste improving inorganic acid salt additives for improving the osmotic taste may comprise a molecular weight from about 58 to about 120.

In another embodiment, suitable sweet taste improving bitter additives for improving the osmotic taste of the synthetic sweetener to be more sugar-like include, but are not limited to, caffeine, quinine, urea, quassia, tannic acid, and naringin.

In a further embodiment, the sweet taste improving compositions improve the synthetic sweeteners to be similar to that of sucrose through at least one mechanism selected from temporal profile (e.g., sweetness onset or sweetness linger), maximal response, flavor profile (e.g., osmotic taste), adaptation behavior, and flavor profile. In a sub-embodiment, the sweet taste compositions improve the synthetic sweeteners to be similar to that of sucrose through at least one mechanism selected from temporal profile, maximal response, flavor profile, adaptation behavior, and flavor profile, and optionally impart a masking effect to suppress, reduce, or eliminate the undesirable taste of the synthetic sweetener and/or impart sugar-like characteristics to the synthetic sweeteners.

The PPAR agonist compounds of present invention for use in present invention can also be incorporated in a composition or dosage form which is a beverage. Such beverage may be of the group of a non-carbonated beverage or a carbonated beverage.

The PPAR agonist compounds of present invention for use in present invention can also be incorporated in a composition or dosage form which is a beverage which is of the group of cola, fruit- flavored beverage, citrus- flavored beverage (for instance lemon-lime flavored beverage or a orange-flavored beverage), root beer, fruit juice, fruit- flavored, fruit-containing beverage, vegetable juice or vegetable containing beverage, tea, coffee, beverage comprising a dairy component, sports drink, energy drink, flavored water.

A suitable dosage form for the PPAR agonist of present invention is for instance composition comprising water in an amount between 97-99% by volume of the total composition; citric acid in an amount between 0.18 and 0.22% by volume of the total composition; juice concentrate in an amount between 3.0-7.0% by volume of the total composition; ascorbic acid in an amount between 0.046-0.054 g/L by weight of the total composition; natural sweeteners in an amount between 0.65-0.79% by volume of the total composition; Vitamin E in amount between 0.015-0.019 g/L by weight of the total composition; green tea extract in an amount between 0.050-0.062 g/L by weight of the total composition; grape seed extract in an amount between 0.050-0.062 g/L by weight of the total composition; artificial sweeteners in an amount between 0.47-0.58 g/L by weight of the total composition; and carbonation in an amount between 2.5 and 3.0 volumes or more preferably such composition comprising water in an amount of 98% by volume of the total composition; citric acid in an amount of 0.20% by volume of the total composition; juice concentrate in an amount of 5.3% by volume of the total composition; ascorbic acid in an amount 0.05 g/L by weight of the total composition; natural sweeteners in an amount between 0.65-0.79% by volume of the total composition; Vitamin E in amount of 0.017 g/L by weight of the total composition; green tea extract in an amount of 0.056 g/L by weight of the total composition; grape seed extract in an amount of 0.056 g/L by weight of the total composition sucralose in an amount between 0.45-0.55 g/L by weight of the total composition; acesulfame potassium in an amount between 0.020-0.024 g/L by weight of the total composition; and carbonation in an amount of 2.5 volumes.

The PPAR agonist compounds of present invention for use in present invention can also be incorporated in a composition or dosage form which is a beverage or a drink such as a fruit juice, beer, lemonade for instance a sparkling fruit juice antioxidant beverage or a sparkling vegetable juice antioxidant beverage.

Preferred ingredients of such sparkling fruit juice antioxidant beverage or such of such sparkling vegetable juice antioxidant beverage suitable for comprising the PPAR agonist compounds of present invention are in the ranges in which they may be present according to the invention, the preferred ranges in which they may be present, and the most preferred amount in which they may be present as follows: water (, critic acid, asciobic acid, HFCS-55, (Brix: 77.0), Fruit Juice concentrate(s) or vegetable juice concentrates, FD&Color(s), Potassium, sorbate, sodium benzoate, potassium, citrate, EDTA/ calcium disodium sucralose liquid concentrate, acesulfame potassium, Flavors/flavorings, vitamin E, grape seed extract, greent tea extract, carbonation. These can be in ranges of water More Preferred Relative Amount 97-99% v/v SSJ or Most Preferred Relative Amount 98% v/v SSJ), citric acid (More Preferred Relative Amount 0.18-0.22% v/v SSJ or Most Preferred Relative Amount 0.20% v/v SSJ), ascorbic acid, HFCS-55 (Brix: 77.0) (More Preferred Relative Amount 0.65-0.79% v/v SSJ or Most Preferred Relative Amount 0.72% v/v SSJ), Fruit Juice concentrate(s) or vegetable juice concentrates (More Preferred Relative Amount 3.0-7.0% v/v SSJ or Most Preferred Relative Amount 5.3% % v/v SSJ) , FD&Color(s) (More Preferred Relative Amount 0.0364-0.0444 g/L or Most Preferred Relative Amount 0.0404 g/L), Potassium sorbate (More Preferred Relative Amount 0.440-0.538 g/L or Most Preferred Relative Amount 0.489 g/L), sodium benzoate (More Preferred Relative Amount 0.196-0.240 g/L or Most Preferred Relative Amount 0.218 g/L), potassium, citrate (More Preferred Relative Amount 0.18-0.22 g/L or Most Preferred Relative Amount 0.200 g/L), EDTA/ calcium disodium (More Preferred Relative Amount 0.022-0.275 g/L or Most Preferred Relative Amount 0.025 g/L), sucralose liquid concentrate (More Preferred Relative Amount 0.45-0.55 g/L or Most Preferred Relative Amount 0.495 g/L), acesulfame potassium (More Preferred Relative Amount 0.020-0.024 g/L orMost Preferred Relative Amount 0.022 g/L), Flavors/flavorings (More Preferred Relative Amount 0.99-12.1 g/L or Most Preferred Relative Amount 1.1 g/L), vitamin E (More Preferred Relative Amount 0.015-0.019 g/L or Most Preferred Relative Amount 0.017 g/L.), grape seed extract (0.050-0.062 g/L or Most Preferred Relative Amount 0.056 g/L), green tea extract (More Preferred Relative Amount 0.050-0.062 g/L or Most Preferred Relative Amount 0.056 g/L), carbonation (More Preferred Relative Amount 2.5-3.0 volumes or Most Preferred Relative Amount 2.5 volumes). The above mentioned lists of juice concentrates, FD&C colors, and flavors/flavorings among the components of the sparkling juice beverages of the invention. The particular juice concentrates, FD&C colors and flavorings may be selected from a large variety of known juices, colors and flavors according to taste and aesthetic factors. The vitamin E listed above be liquid or encapsulated powder form. The artificially sweetened sparkling juice beverage compositions formulated according to the ranges above offer the antioxidant benefits of vitamin C and E as well as green tea and grape seed extracts.

The PPAR agonist of present invention may be further combined in a functional composition or dosage form with suitable C-reactive protein reducing substances which include, but are not limited to, phytosterols, 3-hydroxy-3-methylglutaryl coenzyme A reductase inhibitors (i.e., statins), peroxisome proliferators-activated receptor-α agonists (i.e., fibrates), peroxisome proliferators-activated receptor-α agonists (i.e., glitazones), aspirin, RRR-α-tocopherol, policosanol, leukotriene inhibitors, antihistamines, corticosteroids, 2-aryl-3-aroylbenzo[b]thiophenes, similar type substances, and combinations thereof. For example, suitable phytosterols for use hi embodiments of the present invention include, but are not limited to, sitosterol, campesterol, stigmasterol, spinosterol, taraxasterol, brassicasterol, demosterol, chalinosterol, poriferasterol, clionasterol, ergosterol, sitostanol, campestanol, stigmastanol, spinostanol, taraxastanol, brassicastanol, desmostanol, chalinostanol, poriferastanol, clionastanol, ergostanol, and similar type substances, and combinations thereof. Suitable phytosterols for embodiments of the present invention may also be derived from, for example, rice bran, corn bran, corn germ, wheat germ oil, corn oil, safflower oil, oat oil, olive oil, cotton seed oil, soybean oil, peanut oil, black tea, green tea, colocsia, kale, broccoli, seasame seeds, shea oils, grapeseed oil, rapeseed oil, linseed oil, canola oil, tall oil, other oils obtained from wood pulp, and similar type sources. As used herein, "phytosterols" refers to plant sterols and plant stanols in their free and esterified forms. In other embodiments, suitable C-reactive protein reducing substances comprise a policosanol selected from the group consisting of 1-tetracosanol, 1-hexacosanol, 1-heptacosanol, 1-octacosanol, 1-triacontanol, 1- dotriacontanol, 1-tetracontanol, any other high molecular weight straight chain primary alcohol selected from 20 to 36 carbon atoms, and similar type materials, and combinations thereof. Within the human body, C-reactive protein is an acute-phase protein produced by the liver. C-reactive protein is considered an acute-phase protein because it is released into the body in response to acute injury, infection, or other inflammatory stimuli. These C-reactive protein has been used as a marker of inflammation. In addition, C-reactive protein has been useful in monitoring the activity of rheumatoid arthritis (i.e., rheumatology) and as a risk marker for cardiovascular disease (e.g., atherogenesis). More recently, it has been suggested the C-reactive protein is not only a marker for cardiovascular disease, but may also play a role in the causing artherogenisis. For example, C-reactive protein may play a role in the expression of different adhesion molecules on endothelial cells and may be able to activate human complement within plaque. Thus, C-reactive protein reducing substances can desirably be used to decrease, block, or inhibit C-reactive protein or its production in the human body. As used herein, "C-reactive protein reducing substance" refers to any substance effective in causing a biological response of a tissue, system, or patient which may include decreasing, modulating, blocking, or inhibiting C-reactive protein, its production, or its detrimental effects.

For its use in a treatment of the health disorders according to present invention the PPAR agonist(s) of present invention may be incorporated in an edible gel mix. Such edible gel mix can comprise at least one natural and/or synthetic high-potency sweetener, at least one sweet taste improving composition, and at least one gelling ingredient. In other embodiments, an edible gel composition is provided that comprises at least one natural and/or synthetic high-potency sweetener, at least one sweet taste improving composition, at least one gelling ingredient, and at least one fluid. As used herein the phrase "the at least one natural and/or synthetic high-potency sweetener and at least one sweet taste improving composition" is synonymous with the phrase "the sweetener composition." I. Edible Gel Mixes and Edible Gel Compositions A gel is a colloidal system in which a network of particles spans the volume of a liquid medium. Although gels mainly are composed of liquids, and thus exhibit densities similar to liquids, gels have the structural coherence of solids due to the network of particles that spans the liquid medium. For this reason, gels generally appear to be solid, jelly-like materials. Gels can be used in a number of applications. For example, gels can be used in foods, paints, and adhesives. Gels that can be eaten are refered to herein as "edible gel compositions." Edible gel compositions typically are eaten as snacks, as desserts, as a part of staple foods, or along with staple foods. Non-limiting examples of edible gel compositions for use in particular embodiments include gel desserts, puddings, jellies, pastes, trifles, aspics, marshmallows, gummy candies, or the like. Edible gel mixes generally are powdered or granular solids to which a fluid may be added to form an edible gel composition. Non-limiting examples of fluids for use in particular embodiments include water, dairy fluids, dairy analogue fluids, juices, alcohol, alcoholic beverages, and combinations thereof. Non-limiting examples of dairy fluids which may be used in particular embodiments include milk, cultured milk, cream, fluid whey, and mixtures thereof. Non-limiting examples of dairy analogue fluids which may be used in particular embodiments include, for example, soy milk and non-dairy coffee whitener. As used herein, the term "gelling ingredient" denotes any material that can form a colloidal system within a liquid medium. Non-limiting examples of gelling ingredients for use in particular embodiments include gelatin, alginate, carageenan, gum, pectin, konjac, agar, food acid, rennet, starch, starch derivatives, and combinations thereof. It is well known to those having ordinary skill in the art that the amount of gelling ingredient used in an edible gel mix or an edible gel composition varies considerably depending on a number of factors, such as the particular gelling ingredient used, the particular fluid base used, and the desired properties of the gel. In a particular embodiment, the gelling ingredient is present in the edible gel mix in an amount from about 0.5% to about 80% by weight of the edible gel mix. In a particularly desirable embodiment, the gelling ingredient present in the edible gel mix is gelatin. Desirably, the gelatin is present in the edible gel mix in an amount from about 25% to about 80% by weight of the edible gel mix, and more desirably in an amount from about 38% to about 50% by weight of the edible gel mix. In another embodiment, the gelling ingredient is present in the edible gel composition in an amount from about 0.05% to about 10% by weight of the edible gel composition. In a particularly desirable embodiment, the gelling ingredient present in the edible gel composition is gelatin. Desirably, the gelatin is present in the edible gel composition in an amount from about 0.8% to about 4% by weight of the edible gel composition, and more desirably in an amount from about 1.2% to about 1.6% by weight of the edible gel composition. Edible gel compositions generally appeal to consumers because of their sweet taste. Because edible gel products found in the marketplace typically are sweetened with sucrose, it is desirable to sweeten edible gels with an alternative sweetener in order provide a low-calorie or non-calorie alternative. Accordingly, in a particular embodiment, the edible gel mix comprises at least one natural and/or synthetic high-potency sweetener in combination with at least one sweet taste improving composition and at least one gelling ingredient. The sweetener composition may be added to the edible gel mix composition in an amount effective to sweeten the edible gel mix. In other embodiments, an edible gel composition is provided that comprises at least one natural and/or synthetic high-potency sweetener, at least one sweet taste improving composition, at least one gelling ingredient, and at least one fluid. The sweetener composition may be added to the edible gel composition as a coating, as a frosting, as a glaze, or as a matrix blend (i.e. added as an ingredient to the edible gel mix prior to the preparation of the edible gel composition).

In an embodiment of present invention the PPAR agonist(s) of present invention or administered in a orally deliverable dosage form which comprises dietary fibers. Food sources of dietary fiber include, but are not limited to, grains, legumes, fruits, and vegetables. Grains providing dietary fiber include, but are not limited to, oats, rye, barley, wheat,. Legumes providing fiber include, but are not limited to, peas and beans such as soybeans. Fruits and vegetables providing a source of fiber include, but are not limited to, apples, oranges, pears, bananas, berries, tomatoes, green beans, broccoli, cauliflower, carrots, potatoes, celery. Plant foods such as bran, nuts, and seeds (such as flax seeds) are also sources of dietary fiber. Parts of plants providing dietary fiber include, but are not limited to, the stems, roots, leaves, seeds, pulp, and skin.Although dietary fiber generally is derived from plant sources, indigestible animal products such as chitins are also classified as dietary fiber. Chitin is a polysaccharide composed of units of acetylglucosamine joined by β(1-4) linkages, similar to the linkages of cellulose. Sources of dietary fiber often are divided into categories of soluble and insoluble fibre based on their solubility in water. Both soluble and insoluble fibres are found in plant foods to varying degrees depending upon the characteristics of the plant. Although insoluble in water, insoluble fiber has passive hydrophilic properties that help increase bulk, soften stools, and shorten transit time of fecal solids through the intestinal tract.Unlike insoluble fiber, soluble fiber readily dissolves in water. Soluble fiber undergoes active metabolic processing via fermentation in the colon, increasing the colonic microflora and thereby increasing the mass of fecal solids. Fermentation of fibers by colonic bacteria also yields end products with significant health benefits. For example, fermentation of the food masses produces gases and short-chain fatty acids. Acids produced during fermentation include butyric, acetic, propionic, and valeric acids that have various beneficial properties such as stabilizing blood glucose levels by acting on pancreatic insulin release and providing liver control by glycogen breakdown. In addition, fiber fermentation may reduce atherosclerosis by lowering cholesterol synthesis by the liver and reducing blood levels of LDL and triglycerides. The acids produced during fermentation lower colonic pH, thereby protecting the colon lining from cancer polyp formation. The lower colonic pH also increases mineral absorption, improves the barrier properties of the colonic mucosal layer, and inhibits inflammatory and adhesion irritants. Fermentation of fibers also may benefit the immune system by stimulating production of T-helper cells, antibodies, leukocytes, splenocytes, cytokinins and lymphocytes. Dietary fiber has been demonstrated to have assorted health benefits despite not being absorbed by the gastrointestinal tract. Dietary fiber can potentially reduce the incidence of an assortment of chronic diseases, especially those involving the gastrointestinal tract. Consumption of dietary fiber has been demonstrated to alter metabolism of carbohydrates, lipids, and proteins. Medical studies show that diets high in fiber reduce the risk of colon cancer, coronary heart disease, type-2 diabetes, diverticular disease, irritable bowel syndrome, and constipation. High fiber diets also reduce the risk of developing obesity, high blood cholesterol, and inflammatory bowel diseases such as ulcerative colitis and Crohn's disease. Accordingly, it will be desirable to supplement foods and beverages with dietary fiber. It is well known to those of ordinary skill in the art that phytonutrients, plant extracts, and herbal compositions may be used in their natural and/or modified form. Modified phytonutrients, plant extracts, and herbal compositions include phytonutrients, plant extracts, and herbal compositions which have been altered naturally. For example, a modified phytonutrient includes, but is not limited to, phytonutrients which have been fermented, contacted with enzyme, or derivatized or substituted on the phytonutrient. i one embodiment, modified phytonutrients may be used individually or in combination with unmodified phytonutrients. For the sake of brevity, however, in the description of embodiments of this invention, a modified phytonutrient is not described expressly as an alternative to an unmodified phytonutrient, but it should be understood that modified phytonutrients can be substituted for or combined with phytonutrients in any embodiment disclosed herein. The same embodiments will be applicable to plant extracts and other herbal compositions. Plant extracts include extracts from foliage, stems, bark, fruit, seed, and any other plant matter. As used herein, the at least one dietary fiber source may comprise a single dietary fiber source or a plurality of dietary fiber sources as a functional ingredient for the sweetener compositions provided herein. Generally, according to particular embodiments of this invention, the at least one dietary fiber source is present in the sweetener composition or sweetened orally ingestible composition in an amount sufficient to promote health and wellness. In a preferred embodiment, dietary fiber is provided in the dietary fiber composition in an amount from about 0.5 to about 6.0 g per single serving. In a more preferred embodiment, dietary fiber is provided in the dietary fiber composition in an amount from about 2.0 to about 3.0 grams per single serving.

Suitable polymer additives for the delivering the PPAR agonis(s) of present invention in the form of a chewable gum are but not limited to, chitosan, pectin, pectic, pectinic, polyuronic, polygalacturonic acid, starch, food hydrocolloid or crude extracts thereof (e.g., gum acacia Senegal (Fibergum™), gum acacia seyal, carageenan), poly-L-lysine (e.g., poly-L-α-lysine or poly-L-ε-lysine), poly-L-ornithine (e.g., poly-L-α-ornithine or poly-L-ε-ornithine), polyarginine, polypropylene glycol, polyethylene glycol, poly(ethylene glycol methyl ether), polyaspartic acid, polyglutamic acid, polyethyleneimine, alginic acid, sodium alginate, propylene glycol alginate, sodium hexametaphosphate (SHMP) and its salts, and sodium polyethyleneglycolalginate and other cationic and anionic polymers. Suitable sweet taste improving protein or protein hydrolysate additives for use in the embodiments of such chewable gum include, but are not limited to, bovine serum albumin (BSA), whey protein (including fractions or concentrates thereof such as 90% instant whey protein isolate, 34% whey protein, 50% hydrolyzed whey protein, and 80% whey protein concentrate), soluble rice protein, soy protein, protein isolates, protein hydrolysates, reaction products of protein hydrolysates, glycoproteins, and/or proteoglycans containing amino acids (e.g., glycine, alanine, serine, threonine, asparagine, glutamine, arginine, valine, isoleucine, leucine, norvaline, methionine, proline, tyrosine, hydroxyproline, and the like), collagen (e.g., gelatin), partially hydrolyzed collagen (e.g., hydrolyzed fish collagen), and collagen hydrolysates (e.g., porcine collagen hydrolysate). Suitable sweet taste improving surfactant additives for use in embodiments of this invention include, but are not limited to, polysorbates (e.g., polyoxyethylene sorbitan monooleate (polysorbate 80), polysorbate 20, polysorbate 60), sodium dodecylbenzenesulfonate, dioctyl sulfosuccinate or dioctyl sulfosuccinate sodium, sodium dodecyl sulfate, cetylpyridinium chloride (hexadecylpyridinium chloride), hexadecyltrimethylammonium bromide, sodium cholate, carbamoyl, choline chloride, sodium glycocholate, sodium taurodeoxycholate, lauric arginate, sodium stearoyl lactylate, sodium taurocholate, lecithins, sucrose oleate esters, sucrose stearate esters, sucrose palmitate esters, sucrose laurate esters, and other emulsifiers, and the like.

According to particular embodiments of this invention, the sweetener compositions for comprising the PPAR agonists of present invention provided herein further may comprise at least one functional ingredient different than the dietary fiber sources described above. According to particular embodiments of this invention, non-limiting examples of such functional ingredients include naturally nutrient- rich or medicinally active food, such as garlic, soybeans, antioxidants, fibers, glucosamine, chondroitin sulfate, ginseng, ginko, Echinacea, or the like; other nutrients that provide health benefits, such as amino acids, vitamins, minerals, carotenoids, fatty acids such as omega-3 or omega-6 fatty acids, DHA, EPA, or ALA which can be derived from plant or animal sources (e.g., salmon and other cold-water fish or algae), flavonoids, phenols, polyols, prebiotics/probiotics, phytosterols and phytostanols and their esters, phytoestrogens, sulfides/thiols, policosanol, saponin, rubisco peptide, appetite suppressants, hydration agents, autoimmune agents, C-reactive protein reducing agents, or anti-inflammatory agents; or any other functional ingredient that is beneficial to the treatment of specific diseases or conditions, such as diabetes, osteoporosis, inflammation, or cholesterol.

Another dosage for comprising the functional PPAR agonist of present invention in order to delivery them to the subject to treat or prevent a PPAR related disorder is a baked good. Baked goods, as used herein, include ready to eat and all ready to bake products, flours, and mixes requiring preparation before serving. Non-limiting examples of baked goods include cakes, crackers, cookies, brownies, muffins, rolls, bagels, donuts, strudels, pastries, croissants, biscuits, bread, bread products, and buns. Preferred baked goods in accordance with embodiments of this invention can be classified into three groups: bread-type doughs (e.g., white breads, variety breads, soft buns, hard rolls, bagels, pizza dough, and flour tortillas), sweet doughs (e.g., danishes, croissants, crackers, puff pastry, pie crust, biscuits, and cookies), and batters (e.g., cakes such as sponge, pound, devil's food, cheesecake, and layer cake, donuts or other yeast raised cakes, brownies, and muffins). Doughs generally are characterized as being flour-based, whereas batters are more water-based. Baked goods in accordance with particular embodiments of this invention generally comprise a combination of sweetener, water, and fat. Baked goods made in accordance with many embodiments of this invention also contain flour in order to make a dough or a batter. The term "dough" as used herein is a mixture of flour and other ingredients stiff enough to knead or roll. The term "batter" as used herein consists of flour, liquids such as milk or water, and other ingredients, and is thin enough to pour or drop from a spoon. Desirably, in accordance with particular embodiments of the invention, the flour is present in the baked goods in an amount in the range of about 15 to about 60% on a dry weight basis, more desirably from about 23 to about 48% on a dry weight basis. The type of flour may be selected based on the desired product. Generally, the flour comprises an edible non-toxic flour that is conventionally utilized in baked goods.

According to particular embodiments, the flour may be a bleached bake flour, general purpose flour, or unbleached flour. In other particular embodiments, flours also may be used that have been treated in other manners. For example, in particular embodiments flour may be enriched with additional vitamins, minerals, or proteins. Non-limiting examples of flours suitable for use in particular embodiments of the invention include wheat, corn meal, whole grain, fractions of whole grains (wheat, bran, and oatmeal), and combinations thereof. Starches or farinaceous material also may be used as the flour in particular embodiments. Common food starches generally are derived from potato, corn, wheat, barley, oat, tapioca, arrow root, and sago. Modified starches and pregelatinized starches also may be used in particular embodiments of the invention. The type of fat or oil used in particular embodiments of the invention may comprise any edible fat, oil, or combination thereof that is suitable for baking. Non-limiting examples of fats suitable for use in particular embodiments of the invention include vegetable oils, tallow, lard, marine oils, and combinations thereof. According to particular embodiments, the fats may be fractionated, partially hydrogenated, and/or interesterified. In another particular embodiment, the fat desirably comprises reduced, low calorie, or non-digestible fats, fat substitutes, or synthetic fats. In yet another particular embodiment, shortenings, fats, or mixtures of hard and soft fats also may be used. In particular embodiments, shortenings may be derived principally from triglycerides derived from vegetable sources (e.g., cotton seed oil, soybean oil, peanut oil, linseed oil, sesame oil, palm oil, palm kernel oil, rapeseed oil, safflower oil, coconut oil, corn oil, sunflower seed oil, and mixtures thereof). Synthetic or natural triglycerides of fatty acids having chain lengths from 8 to 24 carbon atoms also may be used in particular embodiments. Desirably, in accordance with particular embodiments of this invention, the fat is present in the baked good in an amount in the range of about 2 to about 35% by weight on a dry basis, more desirably from about 3 to about 29% by weight on a dry basis. Baked goods in accordance with particular embodiments of this invention also comprise water in amounts sufficient to provide the desired consistency, enabling proper forming, machining and cutting of the baked good prior or subsequent to cooking. The total moisture content of the baked good includes any water added directly to the baked good as well as water present in separately added ingredients (e.g., flour, which generally includes about 12 to about 14% by weight moisture). Desirably, in accordance with particular embodiments of this invention, the water is present in the baked good in an amount up to about 25% by weight of the baked good. Baked goods in accordance with particular embodiments of this invention also may comprise a number of additional conventional ingredients such as leavening agents, flavors, colors, milk, milk by-products, egg, egg by-products, cocoa, vanilla or other flavoring, as well as inclusions such as nuts, raisins, cherries, apples, apricots, peaches, other fruits, citrus peel, preservative, coconuts, flavored chips such a chocolate chips, butterscotch chips, and caramel chips, and combinations thereof. In particular embodiments, the baked goods may also comprise emulsifiers, such as lecithin and monoglycerides. In a particular embodiment, the at least one sweet taste improving composition comprises at least one of the additional conventional ingredients described above. According to particular embodiments of this invention, leavening agents may comprise chemical leavening agents or yeast leavening agents. Non-limiting examples of chemical leavening agents suitable for use in particular embodiments of this invention include baking soda (e.g., sodium, potassium, or aluminum bicarbonate), baking acid (e.g., sodium aluminum phosphate, monocalcium phosphate, or dicalcium phosphate), and combinations thereof. In accordance with another particular embodiment of this invention, cocoa may comprise natural or "Dutched" chocolate from which a substantial portion of the fat or cocoa butter has been expressed or removed by solvent extraction, pressing, or other means. In a particular embodiment, it may be necessary to reduce the amount of fat in a baked good comprising chocolate because of the additional fat present in cocoa butter. In particular embodiments, it may be necessary to add larger amounts of chocolate as compared to cocoa in order to provide an equivalent amount of flavoring and coloring. Baked goods generally also comprise caloric sweeteners, such as sucrose, high fructose corn syrup, erythritol, molasses, honey, or brown sugar. In exemplary embodiments of the baked goods provided herein, the sweetener comprises at least one natural and/or synthetic high-potency sweetener and at least one sweet taste improving composition. Accordingly, a baked good in accordance with a particularly desirable embodiment comprises at least one natural and/or synthetic high-potency sweetener in combination with at least one sweet taste improving composition, a fat, water, and optionally flour. In a particular embodiment, the baked good optionally may include other natural and/or synthetic high-potency sweeteners and/or bulk sweeteners. As described hereinabove, the baked goods comprise at least one natural and/or synthetic high-potency sweetener and at least one sweet taste improving composition. The combination of the at least one natural and/or synthetic high-potency sweetener and at least one sweet taste improving composition, as used herein, comprises the "sweetener composition." In addition, the combination of the sweetener composition in a baked good comprises a "sweetened composition."

Accordingly, in a particular embodiment, the at least one natural and/or synthetic high-potency sweetener in combination with at least one sweet taste improving composition is present in the edible gel mix in an amount of at least about 0.3% by weight of the edible gel mix. More desirably, the sweetener composition is present in the edible gel mix in an amount from about 0.5% to about 30% by weight of the edible gel mix, even more desirably from about 1% to about 10% by weight of the edible gel mix, and yet even more desirably from about 1.8% to about 3.6% by weight of the edible gel mix.

In another particular embodiment the at least one natural and/or synthetic high-potency sweetener in combination with at least one sweet taste improving composition is present in the edible gel composition in an amount of at least about 0.006% by weight of the edible gel composition. More desirably, the sweetener composition is present in the edible gel mix in an amount from to about 0.01% to about 1.5% by weight of the edible gel composition, even more desirably from about .025% to about 0.5% by weight of the edible gel composition, and yet even more desirably from about 0.045% to about 0.09% by weight of the edible gel composition.

It is well known to those having ordinary skill in the art that the edible gel mixes and edible gel compositions of this invention may be prepared using other ingredients in addition to the sweetener composition and the gelling agent. Non-limiting examples of other ingredients for use in particular embodiments include a food acid, a salt of a food acid, a buffering system, a bulking agent, a sequestrant, a cross-linking agent, one or more flavors, one or more colors, and combinations thereof. Non-limiting examples of food acids for use in particular embodiments include citric acid, adipic acid, fumaric acid, lactic acid, malic acid, and combinations thereof. Non-limiting examples of salts of food acids for use in particular embodiments include sodium salts of food acids, potassium salts of food acids, and combinations thereof. Non-limiting examples of bulking agents for use in particular embodiments include raftilose, isomalt, sorbitol, polydextrose, maltodextrin, and combinations thereof. Non-limiting examples of sequestrants for use in particular embodiments include calcium disodium ethylene tetra-acetate, glucono delta-lactone, sodium gluconate, potassium gluconate, ethylenediaminetetraacetic acid (EDTA), and combinations thereof. Non-limiting examples of cross-linking agents for use in particular embodiments include calcium ions, magnesium ions, sodium ions, and combinations thereof.

One of ordinary skill in the art, with the teachings of the present invention, may arrive at all the possible combinations of natural and/or synthetic high-potency sweeteners and sweet taste improving compositions in a dosage form of the PPAR agonist of present invention. For example, non-limiting combinations of the natural and/or synthetic high-potency sweetener and sweet taste improving compositions include: 1. at least one natural and/or synthetic high-potency sweetener and at least one carbohydrate; 2. at least one natural and/or synthetic high-potency sweetener and at least one polyol; 3. at least one natural and/or synthetic high-potency sweetener and at least one amino acid; 4. at least one natural and/or synthetic high-potency sweetener and at least one other sweet taste improving additive; 5. at least one natural and/or synthetic high-potency sweetener, at least one carbohydrate, at least one polyol, at least one amino acid, and at least one other sweet taste improving additive; 6. at least one natural and/or synthetic high-potency sweetener, at least one carbohydrate, and at least one polyol; 7. at least one natural and/or synthetic high-potency sweetener, at least one carbohydrate, and at least one amino acid; 8. at least one natural and/or synthetic high-potency sweetener, at least one carbohydrate, and at least one other sweet taste improving additive; 9. at least one natural and/or synthetic high-potency sweetener, at least one polyol, and at least one amino acid; 10. at least one natural and/or synthetic high-potency sweetener, at least one polyol, and at least one other sweet taste improving additive; 11. at least one natural and/or synthetic high-potency sweetener, at least one amino acid, and at least one other sweet taste improving additive; 12. at least one natural and/or synthetic high-potency sweetener, at least one carbohydrate, at least one polyol, and at least one amino acid; 13. at least one natural and/or synthetic high-potency sweetener, at least one carbohydrate, at least one polyol, and at least one other sweet taste improving additive; 14. at least one natural and/or synthetic high-potency sweetener, at least one polyol, at least one amino acid, and at least one other sweet taste improving additive; and 15. at least one natural and/or synthetic high-potency sweetener, at least one carbohydrate, at least one amino acid, and at least one other sweet taste improving additive.

These fifteen major combinations further may be broken down into further combinations in order to improve the overall taste of the natural and/or synthetic high-potency sweetener or the sweetened compositions comprising the natural and/or synthetic high-potency sweetener.

As explained above, the sweet taste improving composition is selected from the group consisting of polyols, carbohydrates, amino acids, other sweet taste improving additives, and combinations thereof. The other sweet taste improving additives useful in embodiments of this invention are described hereinabove. In one embodiment, a single sweet taste improving composition may be used with a single natural or synthetic high- potency sweetener and a gelling ingredient. In another embodiment of the present invention, a single sweet taste improving composition may be used with one or more natural and/or synthetic high-potency sweeteners and a gelling ingredient, hi yet another embodiment, one or more sweet taste improving compositions may be used with a single natural or synthetic high-potency sweetener and a gelling ingredient. In a further embodiment, there may be a plurality of sweet taste improving compositions used in combination with one or more natural and/or synthetic high-potency sweeteners and a gelling ingredient. Thus, non-limiting examples of sweet taste improving composition combinations for embodiments of this invention include: i. at least one polyol, at least one carbohydrate, at least one amino acid, and at least one other sweet taste improving additive; ii. at least one polyol, at least one carbohydrate, and at least one other sweet taste improving additive; iii. at least one polyol and at least one other sweet taste improving additive; iv. at least one polyol and at least one carbohydrate; v. at least one carbohydrate and at least one other sweet taste improving additive; vi. at least one polyol and at least one amino acid; vii. at least one carbohydrate and at least one amino acid; viii. at least one amino acid and at least one other sweet taste improving additive.

Other sweet taste improving composition combinations that in accordance with embodiments of this invention can be in a dosage for for the PPA agonist(s) of present invention include: 1. at least one polyol, at least one carbohydrate, and at least one amino acid; 2. at least one polyol, at least one carbohydrate, and at least one polyamino acid; 3. at least one polyol, at least one carbohydrate, and at least one sugar acid; 4. at least one polyol, at least one carbohydrate, and at least one nucleotide; 5. at least one polyol, at least one carbohydrate, and at least one organic acid; 6. at least one polyol, at least one carbohydrate, and at least one inorganic acid; 7. at least one polyol, at least one carbohydrate, and at least one bitter compound; 8. at least one polyol, at least one carbohydrate, and at least one flavorant or flavoring ingredient; 9. at least one polyol, at least one carbohydrate, and at least one polymer; 10. at least one polyol, at least one carbohydrate, and at least one protein or protein hydrolysate or protein or protein hydrolysate with low molecular weight amino acid; 11. at least one polyol, at least one carbohydrate, and at least one surfactant; 12. at least one polyol, at least one carbohydrate, and at least one flavonoid; 13. at least one polyol, at least one carbohydrate, and at least one alcohol; 14. at least one polyol, at least one carbohydrate, and at least one emulsifier; 15. at least one polyol, at least one carbohydrate, and at least one inorganic salt, 16. at least one polyol, at least one carbohydrate, and at least one organic salt, 17. at least one polyol, at least one carbohydrate, and at least one amino acid, and at least one other sweet taste improving additive; 18. at least one polyol, at least one carbohydrate, and at least one polyamino acid, and at least one other sweet taste improving additive; 19. at least one polyol, at least one carbohydrate, and at least one sugar acid, and at least one other sweet taste improving additive; 20. at least one polyol, at least one carbohydrate, and at least one nucleotide, and at least one other sweet taste improving additive; 21. at least one polyol, at least one carbohydrate, and at least one organic acid, and at least one other sweet taste improving additive; 22. at least one polyol, at least one carbohydrate, and at least one inorganic acid, and at least one other sweet taste improving additive; 23. at least one polyol, at least one carbohydrate, and at least one bitter compound, and at least one other sweet taste improving additive; 24. at least one polyol, at least one carbohydrate, and at least one flavorant or flavoring ingredient, and at least one other sweet taste improving additive; 25. at least one polyol, at least one carbohydrate, and at least one polymer, and at least one other sweet taste improving additive; 26. at least one polyol, at least one carbohydrate, and at least one protein or protein hydrolysate, and at least one other sweet taste improving additive; 27. at least one polyol, at least one carbohydrate, and at least one surfactant, and at least one other sweet taste improving additive; 28. at least one polyol, at least one carbohydrate, and at least one flavonoid, and at least one other sweet taste improving additive; 29. at least one polyol, at least one carbohydrate, and at least one alcohol, and at least one other sweet taste improving additive; 30. at least one polyol, at least one carbohydrate, at least one amino acid, and at least one polyamino acid; 31. at least one polyol, at least one carbohydrate, at least one amino acid, at least one polyamino acid, and at least one sugar acid; 32. at least one polyol, at least one carbohydrate, at least one amino acid, at least one polyamino acid, at least one sugar acid, and at least one nucleotide; 33. at least one polyol, at least one carbohydrate, at least one amino acid, at least one polyamino acid, at least one sugar acid, at least one nucleotide, and at least one organic acid; 34. at least one polyol, at least one carbohydrate, at least one amino acid, at least one polyamino acid, at least one sugar acid, at least one nucleotide, at least one organic acid, and at least one inorganic acid; 35. at least one polyol, at least one carbohydrate, at least one amino acid, at least one polyamino acid, at least one sugar acid, at least one nucleotide, at least one organic acid, at least one inorganic acid, and at least one bitter compound; 36. at least one polyol, at least one carbohydrate, at least one amino acid, at least one polyamino acid, at least one sugar acid, at least one nucleotide, at least one organic acid, at least one inorganic acid, at least one bitter compound, and at least one polymer; 37. at least one polyol, at least one carbohydrate, at least one amino acid, at least one polyamino acid, at least one sugar acid, at least one nucleotide, at least one organic acid, at least one inorganic acid, at least one bitter compound, at least one polymer, and at least one protein or protein hydrolysate; 38. at least one polyol, at least one carbohydrate, at least one amino acid, at least one polyamino acid, at least one sugar acid, at least one nucleotide, at least one organic acid, at least one inorganic acid, at least one bitter compound, at least one polymer, at least one protein or protein hydrolysate, and at least one surfactant; 39. at least one polyol, at least one carbohydrate, at least one amino acid, at least one polyamino acid, at least one sugar acid, at least one nucleotide, at least one organic acid, at least one inorganic acid, at least one bitter compound, at least one polymer, at least one protein or protein hydrolysate, at least one surfactant, and at least one flavonoid; 40. at least one polyol, at least one carbohydrate, at least one amino acid, at least one polyamino acid, at least one sugar acid, at least one nucleotide, at least one organic acid, at least one inorganic acid, at least one bitter compound, at least one polymer, at least one protein or protein hydrolysate, at least one surfactant, at least one flavonoid, and at least one alcohol; 41. at least one polyol, at least one carbohydrate, at least one amino acid, and at least one sugar acid; 42. at least one polyol, at least one carbohydrate, at least one amino acid, and at least one nucleotide; 43. at least one polyol, at least one carbohydrate, at least one amino acid, and at least one organic acid; 44. at least one polyol, at least one carbohydrate, at least one amino acid, and at least one inorganic acid; 45. at least one polyol, at least one carbohydrate, at least one amino acid, and at least one bitter compound; 46. at least one polyol, at least one carbohydrate, at least one amino acid, and at least one polymer; 47. at least one polyol, at least one carbohydrate, at least one amino acid, and at least one protein or protein hydrolysate; 48. at least one polyol, at least one carbohydrate, at least one amino acid, and at least one surfactant; 49. at least one polyol, at least one carbohydrate, at least one amino acid, and at least one flavonoid; 50. at least one polyol, at least one carbohydrate, at least one amino acid, and at least one alcohol; 51. at least one polyol, at least one carbohydrate, at least one polyamino acid, and at least one sugar acid; 52. at least one polyol, at least one carbohydrate, at least one polyamino acid, and at least one nucleotide; 53. at least one polyol, at least one carbohydrate, at least one polyamino acid, and at least one organic acid; 54. at least one polyol, at least one carbohydrate, at least one polyamino acid, and at least one inorganic acid; 55. at least one polyol, at least one carbohydrate, at least one polyamino acid, and at least one bitter compound; 56. at least one polyol, at least one carbohydrate, at least one polyamino acid, and at least one polymer; 57. at least one polyol, at least one carbohydrate, at least one polyamino acid, and at least one protein or protein hydrolysate; 58. at least one polyol, at least one carbohydrate, at least one polyamino acid, and at least one surfactant; 59. at least one polyol, at least one carbohydrate, at least one polyamino acid, and at least one flavonoid; 60. at least one polyol, at least one carbohydrate, at least one polyamino acid, and at least one alcohol; 61. at least one polyol, at least one carbohydrate, at least one sugar acid, and at least one nucleotide; 62. at least one polyol, at least one carbohydrate, at least one sugar acid, and at least one organic acid; 63. at least one polyol, at least one carbohydrate, at least one sugar acid, and at least one inorganic acid; 64. at least one polyol, at least one carbohydrate, at least one sugar acid, and at least one bitter compound; 65. at least one polyol, at least one carbohydrate, at least one sugar acid, and at least one polymer; 66. at least one polyol, at least one carbohydrate, at least one sugar acid, and at least one protein or protein hydrolysate; 67. at least one polyol, at least one carbohydrate, at least one sugar acid, and at least one surfactant; 68. at least one polyol, at least one carbohydrate, at least one sugar acid, and at least one flavonoid; 69. at least one polyol, at least one carbohydrate, at least one sugar acid, and at least one alcohol; 70. at least one polyol, at least one carbohydrate, at least one nucleotide, and at least one organic acid; 71. at least one polyol, at least one carbohydrate, at least one nucleotide, and at least one inorganic acid; 72. at least one polyol, at least one carbohydrate, at least one nucleotide, and at least one bitter compound; 73. at least one polyol, at least one carbohydrate, at least one nucleotide, and at least one polymer; 74. at least one polyol, at least one carbohydrate, at least one nucleotide, and at least one protein or protein hydrolysate; 75. at least one polyol, at least one carbohydrate, at least one nucleotide, and at least one surfactant; 76. at least one polyol, at least one carbohydrate, at least one nucleotide, and at least one flavonoid; 77. at least one polyol, at least one carbohydrate, at least one nucleotide, and at least one alcohol; 78. at least one polyol, at least one carbohydrate, at least one organic acid, and at least one inorganic acid; 79. at least one polyol, at least one carbohydrate, at least one organic acid, and at least one bitter compound; 80. at least one polyol, at least one carbohydrate, at least one organic acid, and at least one polymer; 81. at least one polyol, at least one carbohydrate, at least one organic acid, and at least one protein or protein hydrolysate; 82. at least one polyol, at least one carbohydrate, at least one organic acid, and at least one surfactant; 83. at least one polyol, at least one carbohydrate, at least one organic acid, and at least one flavonoid; 84. at least one polyol, at least one carbohydrate, at least one organic acid, and at least one alcohol; 85. at least one polyol, at least one carbohydrate, at least one inorganic acid, and at least one bitter compound; 86. at least one polyol, at least one carbohydrate, at least one inorganic acid, and at least one polymer; 87. at least one polyol, at least one carbohydrate, at least one inorganic acid, and at least one protein or protein hydrolysate; 88. at least one polyol, at least one carbohydrate, at least one inorganic acid, and at least one surfactant; 89. at least one polyol, at least one carbohydrate, at least one inorganic acid, and at least one flavonoid; 90. at least one polyol, at least one carbohydrate, at least one inorganic acid, and at least one alcohol; 91. at least one polyol, at least one carbohydrate, at least one bitter compound, and at least one polymer; 92. at least one polyol, at least one carbohydrate, at least one bitter compound, and at least one protein or protein hydrolysate; 93. at least one polyol, at least one carbohydrate, at least one bitter compound, and at least one surfactant; 94. at least one polyol, at least one carbohydrate, at least one bitter compound, and at least one flavonoid; 95. at least one polyol, at least one carbohydrate, at least one bitter compound, and at least one alcohol; 96. at least one polyol, at least one carbohydrate, at least one polymer, and at least one protein or protein hydrolysate; 97. at least one polyol, at least one carbohydrate, at least one polymer, and at least one surfactant; 98. at least one polyol, at least one carbohydrate, at least one polymer, and at least one flavonoid; 99. at least one polyol, at least one carbohydrate, at least one polymer, and at least one alcohol; 100. at least one polyol, at least one carbohydrate, at least one protein or protein hydrolysate, and at least one surfactant; 101. at least one polyol, at least one carbohydrate, at least one protein or protein hydrolysate, and at least one flavonoid; 102. at least one polyol, at least one carbohydrate, at least one surfactant, and at least one flavonoid; 103. at least one polyol, at least one carbohydrate, at least one surfactant, and at least one alcohol; and 104. at least one polyol, at least one carbohydrate, at least one flavonoid, and at least one alcohol.

Other sweet taste improving composition combinations in accordance with embodiments of this invention include: 1. at least one polyol and at least one amino acid; 2. at least one polyol and at least one polyamino acid; 3. at least one polyol and at least one sugar acid; 4. at least one polyol and at least one nucleotide; 5. at least one polyol and at least one organic acid; 6. at least one polyol and at least one inorganic acid; 7. at least one polyol and at least one bitter compound; 8. at least one polyol and at least one flavorant or flavoring ingredient; 9. at least one polyol and at least one polymer; 10. at least one polyol and at least one protein or protein hydrolysate; 11. at least one polyol and at least one surfactant; 12. at least one polyol and at least one flavonoid; 13. at least one polyol and at least one alcohol; 14. at least one polyol and at least one emulsifier; 15. at least one polyol and at least one inorganic salt; 16. at least one polyol and at least one organic salt; 17. at least one polyol and at least one protein or protein hydrolysate or mixture of low molecular weight amino acids; 18. at least one polyol, at least one amino acid, and at least one other sweet taste improving additive; 19. at least one polyol, at least one polyamino acid, and at least one other sweet taste improving additive; 20. at least one polyol, at least one sugar acid, and at least one other sweet taste improving additive; 21. at least one polyol, at least one nucleotide, and at least one other sweet taste improving additive; 22. at least one polyol, at least one organic acid, and at least one other sweet taste improving additive; 23. at least one polyol, at least one inorganic acid, and at least one other sweet taste improving additive; 24. at least one polyol, at least one bitter compound, and at least one other sweet taste improving additive; 25. at least one polyol, at least one flavorant or flavoring ingredient, and at least one other sweet taste improving additive; 26. at least one polyol, at least one polymer, and at least one other sweet taste improving additive; 27. at least one polyol, at least one protein or protein hydrolysate, and at least one other sweet taste improving additive; 28. at least one polyol, at least one surfactant, and at least one other sweet taste improving additive; 29. at least one polyol, at least one flavonoid, and at least one other sweet taste improving additive; 30. at least one polyol, at least one alcohol, and at least one other sweet taste improving additive; 31. at least one polyol, at least one amino acid, and'at least one polyamino acid; 32. at least one polyol, at least one amino acid, at least one polyamino acid, and at least one sugar acid; 33. at least one polyol, at least one amino acid, at least one polyamino acid, at least one sugar acid, and at least one nucleotide; 34. at least one polyol, at least one amino acid, at least one polyamino acid, at least one sugar acid, at least one nucleotide, and at least one organic acid; 35. at least one polyol, at least one amino acid, at least one polyamino acid, at least one sugar acid, at least one nucleotide, at least one organic acid, and at least one inorganic acid; 36. at least one polyol, at least one amino acid, at least one polyamino acid, at least one sugar acid, at least one nucleotide, at least one organic acid, at least one inorganic acid, and at least one bitter compound; 37. at least one polyol, at least one amino acid, at least one polyamino acid, at least one sugar acid, at least one nucleotide, at least one organic acid, at least one inorganic acid, at least one bitter compound, and at least one polymer; 38. at least one polyol, at least one amino acid, at least one polyamino acid, at least one sugar acid, at least one nucleotide, at least one organic acid, at least one inorganic acid, at least one bitter compound, at least one polymer, and at least one protein or protein hydrolysate; 39. at least one polyol, at least one amino acid, at least one polyamino acid, at least one sugar acid, at least one nucleotide, at least one organic acid, at least one inorganic acid, at least one bitter compound, at least one polymer, at least one protein or protein hydrolysate, and at least one surfactant; 40. at least one polyol, at least one amino acid, at least one polyamino acid, at least one sugar acid, at least one nucleotide, at least one organic acid, at least one inorganic acid, at least one bitter compound, at least one polymer, at least one protein or protein hydrolysate, at least one surfactant, and at least one flavonoid; 41. at least one polyol, at least one amino acid, at least one polyamino acid, at least one sugar acid, at least one nucleotide, at least one organic acid, at least one inorganic acid, at least one bitter compound, at least one polymer, at least one protein or protein hydrolysate, at least one surfactant, at least one flavonoid, and at least one alcohol; 42. at least one polyol, at least one amino acid, and at least one sugar acid; 43. at least one polyol, at least one amino acid, and at least one nucleotide; 44. at least one polyol, at least one amino acid, and at least one organic acid; 45. at least one polyol, at least one amino acid, and at least one inorganic acid; 46. at least one polyol, at least one amino acid, and at least one bitter compound; 47. at least one polyol, at least one amino acid, and at least one polymer; 48. at least one polyol, at least one amino acid, and at least one protein or protein hydrolysate; 49. at least one polyol, at least one amino acid, and at least one surfactant; 50. at least one polyol, at least one amino acid, and at least one flavonoid; 51. at least one polyol, at least one amino acid, and at least one alcohol; 52. at least one polyol, at least one polyamino acid, and at least one sugar acid; 53. at least one polyol, at least one polyamino acid, and at least one nucleotide; 54. at least one polyol, at least one polyamino acid, and at least one organic acid; , 55. at least one polyol, at least one polyamino acid, and at least one organic salt; 56. at least one polyol, at least one polyamino acid, and at least one inorganic acid; 57. at least one polyol, at least one polyamino acid, and at least one inorganic salt; 58. at least one polyol, at least one polyamino acid, and at least one bitter compound; 59. at least one polyol, at least one polyamino acid, and at least one polymer; 60. at least one polyol, at least one polyamino acid, and at least one protein or protein hydrolysate; 61. at least one polyol, at least one polyamino acid, and at least one surfactant; 62. at least one polyol, at least one polyamino acid, and at least one flavonoid; 63. at least one polyol, at least one polyamino acid, and at least one alcohol; 64. at least one polyol, at least one sugar acid, and at least one nucleotide; 65. at least one polyol, at least one sugar acid, and at least one organic acid; 66. at least one polyol, at least one sugar acid, and at least one inorganic acid; 67. at least one polyol, at least one sugar acid, and at least one bitter compound; 68. at least one polyol, at least one sugar acid, and at least one polymer; 69. at least one polyol, at least one sugar acid, and at least one protein or protein hydrolysate; 70. at least one polyol, at least one sugar acid, and at least one surfactant; 71. at least one polyol, at least one sugar acid, and at least one flavonoid; 72. at least one polyol, at least one sugar acid, and at least one alcohol; 73. at least one polyol, at least one nucleotide, and at least one organic acid; 74. at least one polyol, at least one nucleotide, and at least one inorganic acid; 75. at least one polyol, at least one nucleotide, and at least one bitter compound; 76. at least one polyol, at least one nucleotide, and at least one polymer; 77. at least one polyol, at least one nucleotide, and at least one protein or protein hydrolysate; 78. at least one polyol, at least one nucleotide, and at least one surfactant; 79. at least one polyol, at least one nucleotide, and at least one flavonoid; 80. at least one polyol, at least one nucleotide, and at least one alcohol; 81. at least one polyol, at least one organic acid, and at least one inorganic acid; 82. at least one polyol, at least one organic acid, and at least one bitter compound; 83. at least one polyol, at least one organic acid, and at least one polymer; 84. at least one polyol, at least one organic acid, and at least one protein or protein hydrolysate; 85. at least one polyol, at least one organic acid, and at least one surfactant; 86. at least one polyol, at least one organic acid, and at least one flavonoid; 87. at least one polyol, at least one organic acid, and at least one alcohol; 88. at least one polyol, at least one inorganic acid, and at least one bitter compound; 89. at least one polyol, at least one inorganic acid, and at least one polymer; 90. at least one polyol, at least one inorganic acid, and at least one protein or protein hydrolysate; 91. at least one polyol, at least one inorganic acid, and at least one surfactant; 92. at least one polyol, at least one inorganic acid, and at least one flavonoid; 93. at least one polyol, at least one inorganic acid, and at least one alcohol; 94. at least one polyol, at least one bitter compound, and at least one polymer; 95. at least one polyol, at least one bitter compound, and at least one protein or protein hydrolysate; 96. at least one polyol, at least one bitter compound, and at least one surfactant; 97. at least one polyol, at least one bitter compound, and at least one flavonoid; 98. at least one polyol, at least one bitter compound, and at least one alcohol; 99. at least one polyol, at least one polymer, and at least one protein or protein hydrolysate; 100. at least one polyol, at least one polymer, and at least one surfactant; 101 . at least one polyol, at least one polymer, and at least one flavonoid; 102. at least one polyol, at least one polymer, and at least one alcohol; 103. at least one polyol, at least one protein or protein hydrolysate, and at least one surfactant; 104. at least one polyol, at least one protein or protein hydrolysate, and at least one flavonoid; 105. at least one polyol, at least one surfactant, and at least one flavonoid; 106. at least one polyol, at least one surfactant, and at least one alcohol; 107. at least one polyol, at least one flavonoid, and at least one alcohol; 108. at least one sweet taste improving additive and erythritol; 109. at least one sweet taste improving additive and maltitol; 110. at least one sweet taste improving additive and mannitol; 111. at least one sweet taste improving additive and sorbitol; 112. at least one sweet taste improving additive and lactitol; 113. at least one sweet taste improving additive and xylitol; 114. at least one sweet taste improving additive and isomalt; 115. at least one sweet taste improving additive and propylene glycol; 116. at least one sweet taste improving additive and glycerol; 117. at least one sweet taste improving additive and palatinose; 118. at least one sweet taste improving additive and reduced isomalto-oligosaccharides; 119. at least one sweet taste improving additive and reduced xylo-oligosaccharides; 120. at least one sweet taste improving additive and reduced gentio-oligosaccharides; 121. at least one sweet taste improving additive and reduced maltose syrup; 122. at least one sweet taste improving additive and reduced glucose syrup; 123. at least one sweet taste improving additive, erythritol, and at least one other polyol; 124. at least one sweet taste improving additive, maltitol, and at least one other polyol; 125. at least one sweet taste improving additive, mannitol, and at least one other polyol; 126. at least one sweet taste improving additive, sorbitol, and at least one other polyol; 127. at least one sweet taste improving additive, lactitol, and at least one other polyol; 128. at least one sweet taste improving additive, xylitol, and at least one other polyol; 129. at least one sweet taste improving additive, isomalt, and at least one other polyol; 130. at least one sweet taste improving additive, propylene glycol, and at least one other polyol; 131. at least one sweet taste improving additive, glycerol, and at least one other polyol; 132. at least one sweet taste improving additive, palatinose, and at least one other polyol; 133. at least one sweet taste improving additive, reduced isomalto-oligosaccharides, and at least one other polyol; 134. at least one sweet taste improving additive, reduced xylo-oligosaccharides, and at least one other polyol; 135. at least one sweet taste improving additive, reduced gentio-oligosaccharides, and at least one other polyol; 136. at least one sweet taste improving additive, reduced maltose syrup, and at least one other polyol; and 137. at least one sweet taste improving additive, reduced glucose syrup, and at least one other polyol.

Other sweet taste improving composition combinations in accordance with embodiments of this invention include: 1. at least one polyol and tagatose; 2. at least one polyol and trehalose; 3. at least one polyol and galactose; 4. at least one polyol and rhamnose; 5. at least one polyol and dextrin; 6. at least one polyol and cyclodextrin; 7. at least one polyol and α-cyclodextrin, β-cyclodextrin, or γ-cyclodextrin; 8. at least one polyol and maltodextrin; 9. at least one polyol and dextran; 10. at least one polyol and sucrose; 11. at least one polyol and glucose; 12. at least one polyol and fructose; 13. at least one polyol and threose; 14. at least one polyol and arabinose; 15. at least one polyol and xylose; 16. at least one polyol and lyxose; 17. at least one polyol and allose; 18. at least one polyol and altrose; 19. at least one polyol and mannose; 20. at least one polyol and idose; 21. at least one polyol and talose; 22. at least one polyol and lactose; 23. at least one polyol and maltose; 24. at least one polyol and invert sugar; 25. at least one polyol and trehalose; 26. at least one polyol and isotrehalose; 27. at least one polyol and neotrehalose; 28. at least one polyol and palatinose; 29. at least one polyol and galactose; 30. at least one polyol and beet oligosaccharides; 31. at least one polyol and isomalto-oligosaccharides; 32. at least one polyol and isomaltose; 33. at least one polyol and isomaltotriose; 34. at least one polyol and panose; 35. at least one polyol and xylo-oligosaccharides; 36. at least one polyol and xylotriose; 37. at least one polyol and xylobiose; 38. at least one polyol and gentio-oligoscaccharides; 39. at least one polyol and gentiobiose; 40. at least one polyol and gentiotriose; 41. at least one polyol and gentiotetraose; 42. at least one polyol, and sorbose; 43. at least one polyol and nigero-oligosaccharides; 44. at least one polyol and palatinose oligosaccharides; 45. at least one polyol and fucose; 46. at least one polyol and fructooligosaccharides; 47. at least one polyol and kestose; 48. at least one polyol and nystose; 49. at least one polyol and maltotetraol; 50. at least one polyol and maltotriol; 51. at least one polyol and malto-oligosaccharides; 52. at least one polyol and maltotriose; 53. at least one polyol and maltotetraose; 54. at least one polyol and maltopentaose; 55. at least one polyol and maltohexaose; 56. at least one polyol and maltoheptaose; 57. at least one polyol and lactulose; 58. at least one polyol and melibiose; 59. at least one polyol and raffinose; 60. at least one polyol and rhamnose; 61. at least one polyol and ribose; 62. at least one polyol and isomerized liquid sugars; 63. at least one polyol and high fructose corn syrup (e.g. HFCS55, HFCS42, or HFCS90) or starch syrup; 64. at least one polyol and coupling sugars; 65. at least one polyol and soybean oligosaccharides; 66. at least one polyol and glucose syrup; 67. at least one polyol, tagatose, and at least one other carbohydrate; 68. at least one polyol, trehalose, and at least one other carbohydrate; 69. at least one polyol, galactose, and at least one other carbohydrate; 70. at least one polyol, rhamnose, and at least one other carbohydrate; 71. at least one polyol, dextrin, and at least one other carbohydrate; 72. at least one polyol, cyclodextrin, and at least one other carbohydrate; 73. at least one polyol, β-cyclodextrin, and at least one other carbohydrate; 74. at least one polyol, maltodextrin, and at least one other carbohydrate; 75. at least one polyol, dextran, and at least one other carbohydrate; 76. at least one polyol, sucrose, and at least one other carbohydrate; 77. at least one polyol, glucose, and at least one other carbohydrate; 78. at least one polyol, fructose, and at least one other carbohydrate; 79. at least one polyol, threose, and at least one other carbohydrate; 80. at least one polyol, arabinose, and at least one other carbohydrate; 81. at least one polyol, xylose, and at least one other carbohydrate; 82. at least one polyol, lyxose, and at least one other carbohydrate; 83. at least one polyol, allose, and at least one other carbohydrate; 84. at least one polyol, altrose, and at least one other carbohydrate; 85. at least one polyol, mannose, and at least one other carbohydrate; 86. at least one polyol, idose, and at least one other carbohydrate; 87. at least one polyol, talose, and at least one other carbohydrate; 88. at least one polyol, lactose, and at least one other carbohydrate; 89. at least one polyol, maltose, and at least one other carbohydrate; 90. at least one polyol, invert sugar, and at least one other carbohydrate; 91. at least one polyol, trehalose, and at least one other carbohydrate; 92. at least one polyol, isotrehalose, and at least one other carbohydrate; 93. at least one polyol, neotrehalose, and at least one other carbohydrate; 94. at least one polyol, palatinose, and at least one other carbohydrate; 95. at least one polyol, galactose, and at least one other carbohydrate; 96. at least one polyol, beet oligosaccharides, and at least one other carbohydrate; 97. at least one polyol, isomalto-oligosaccharides, and at least one other carbohydrate; 98. at least one polyol, isomaltose, and at least one other carbohydrate; 99. at least one polyol, isomaltotriose, and at least one other carbohydrate; 100. at least one polyol, panose, and at least one other carbohydrate; 101. at least one polyol, xylo-oligosaccharides, and at least one other carbohydrate; 102. at least one polyol, xylotriose, and at least one other carbohydrate; 103. at least one polyol, xylobiose, and at least one other carbohydrate; 104. at least one polyol, gentio-oligoscaccharides, and at least one other carbohydrate; 105. at least one polyol, gentiobiose, and at least one other carbohydrate; 106. at least one polyol, gentiotriose, and at least one other carbohydrate; 107. at least one polyol, gentiotetraose, and at least one other carbohydrate; 108. at least one polyol, sorbose, and at least one other carbohydrate; 109. at least one polyol, nigero-oligosaccharides, and at least one other carbohydrate; 110. at least one polyol, palatinose oligosaccharides, and at least one other carbohydrate; 111. at least one polyol, fucose, and at least one other carbohydrate; 112. at least one polyol, fructooligosaccharides, and at least one other carbohydrate; 113. at least one polyol, kestose, and at least one other carbohydrate; 114. at least one polyol, nystose, and at least one other carbohydrate; 115. at least one polyol, maltotetraol, and at least one other carbohydrate; 116. at least one polyol, maltotriol, and at least one other carbohydrate; 117. at least one polyol, malto-oligosaccharides, and at least one other carbohydrate; 118. at least one polyol, maltotriose, and at least one other carbohydrate; 119. at least one polyol, maltotetraose, and at least one other carbohydrate; 120. at least one polyol, maltopentaose, and at least one other carbohydrate; 121 . at least one polyol, maltohexaose, and at least one other carbohydrate; 122. at least one polyol, maltoheptaose, and at least one other carbohydrate; 123. at least one polyol, lactulose, and at least one other carbohydrate; 124. at least one polyol, melibiose, and at least one other carbohydrate; 125. at least one polyol, raffinose, and at least one other carbohydrate; 126. at least one polyol, rhamnose, and at least one other carbohydrate; 127. at least one polyol, ribose, and at least one other carbohydrate; 128. at least one polyol, isomerized liquid sugars, and at least one other carbohydrate; 129. at least one polyol, high fructose corn syrup (e.g. HFCS55, HFCS42, or HFCS90) or starch syrup, and at least one other carbohydrate; 130. at least one polyol, coupling sugars, and at least one other carbohydrate; 131. at least one polyol, soybean oligosaccharides, and at least one other carbohydrate; 132. at least one polyol, glucose syrup, and at least one other carbohydrate; 133. at least one carbohydrate and erythritol; 134. at least one carbohydrate and maltitol; 135. at least one carbohydrate and mannitol; 136. at least one carbohydrate and sorbitol; 137. at least one carbohydrate and lactitol; 138. at least one carbohydrate and xylitol; 139. at least one carbohydrate and isomalt; 140. at least one carbohydrate and propylene glycol; 141. at least one carbohydrate and glycerol; 142. at least one carbohydrate and palatinose; 143. at least one carbohydrate and reduced isomalto-oligosaccharides; 144. at least one carbohydrate and reduced xylo-oligosaccharides; 145. at least one carbohydrate and reduced gentio-oligosaccharides; 146. at least one carbohydrate and reduced maltose syrup; 147. at least one carbohydrate and reduced glucose syrup; 148. at least one carbohydrate, erythritol, and at least one other polyol; 149. at least one carbohydrate, maltitol, and at least one other polyol; 150. at least one carbohydrate, mannitol, and at least one other polyol; 151. at least one carbohydrate, sorbitol, and at least one other polyol; 152. at least one carbohydrate, lactitol, and at least one other polyol; 153. at least one carbohydrate, xylitol, and at least one other polyol; 154. at least one carbohydrate, isomalt, and at least one other polyol; 155. at least one carbohydrate, propylene glycol, and at least one other polyol; 156. at least one carbohydrate, glycerol, and at least one other polyol; 157. at least one carbohydrate, palatinose, and at least one other polyol; 158. at least one carbohydrate, reduced isomalto-oligosaccharides, and at least one other polyol; 159. at least one carbohydrate, reduced xylo-oligosaccharides, and at least one other polyol; 160. at least one carbohydrate, reduced gentio-oligosaccharides, and at least one other polyol; 161. at least one carbohydrate, reduced maltose syrup, and at least one other polyol; and 162. at least one carbohydrate, reduced glucose syrup, and at least one other polyol.

Other sweet taste improving composition combinations in accordance with embodiments of this invention include: 1. at least one carbohydrate and at least one amino acid; 2. at least one carbohydrate and at least one polyamino acid; 3. at least one carbohydrate and at least one sugar acid; 4. at least one carbohydrate and at least one nucleotide; 5. at least one carbohydrate and at least one organic acid; 6. at least one carbohydrate and at least one inorganic acid; 7. at least one carbohydrate and at least one bitter compound; 8. at least one carbohydrate and at least one flavorant or flavoring ingredient; 9. at least one carbohydrate and at least one polymer; 10. at least one carbohydrate and at least one protein or protein hydrolysate; 11. at least one carbohydrate and at least one surfactant; 12. at least one carbohydrate and at least one flavonoid; 13. at least one carbohydrate and at least one alcohol; 14. at least one carbohydrate and at least one protein or protein hydrolysate or mixture of low molecular weight amino acids; 15. at least one carbohydrate and at least one emulsifier; 16. at least one carbohydrate and at least one inorganic salt; 17. at least one carbohydrate, at least one amino acid, and at least one other sweet taste improving additive; 18. at least one carbohydrate, at least one polyamino acid, and at least one other sweet taste improving additive; 19. at least one carbohydrate, at least one sugar acid, and at least one other sweet taste improving additive; 20. at least one carbohydrate, at least one nucleotide, and at least one other sweet taste improving additive; 21. at least one carbohydrate, at least one organic acid, and at least one other sweet taste improving additive; 22. at least one carbohydrate, at least one inorganic acid, and at least one other sweet taste improving additive; 23. at least one carbohydrate, at least one bitter compound, and at least one other sweet taste improving additive; 24. at least one carbohydrate, at least one flavorant or flavoring ingredient, and at least one other sweet taste improving additive; 25. at least one carbohydrate, at least one polymer, and at least one other sweet taste improving additive; 26. at least one carbohydrate, at least one protein or protein hydrolysate, and at least one other sweet taste improving additive; 27. at least one carbohydrate, at least one surfactant, and at least one other sweet taste improving additive; 28. at least one carbohydrate, at least one flavonoid, and at least one other sweet taste improving additive; 29. at least one carbohydrate, at least one alcohol, and at least one other sweet taste improving additive; 30. at least one carbohydrate, at least one amino acid, and at least one polyamino acid; 31. at least one carbohydrate, at least one amino acid, at least one polyamino acid, and at least one sugar acid; 32. at least one carbohydrate, at least one amino acid, at least one polyamino acid, at least one sugar acid, and at least one nucleotide; 33. at least one carbohydrate, at least one amino acid, at least one polyamino acid, at least one sugar acid, at least one nucleotide, and at least one organic acid; 34. at least one carbohydrate, at least one amino acid, at least one polyamino acid, at least one sugar acid, at least one nucleotide, at least one organic acid, and at least one inorganic acid; 35. at least one carbohydrate, at least one amino acid, at least one polyamino acid, at least one sugar acid, at least one nucleotide, at least one organic acid, at least one inorganic acid, and at least one bitter compound; 36. at least one carbohydrate, at least one amino acid, at least one polyamino acid, at least one sugar acid, at least one nucleotide, at least one organic acid, at least one inorganic acid, at least one bitter compound, and at least one polymer; 37. at least one carbohydrate, at least one amino acid, at least one polyamino acid, at least one sugar acid, at least one nucleotide, at least one organic acid, at least one inorganic acid, at least one bitter compound, at least one polymer, and at least one protein or protein hydrolysate; 38. at least one carbohydrate, at least one amino acid, at least one polyamino acid, at least one sugar acid, at least one nucleotide, at least one organic acid, at least one inorganic acid, at least one bitter compound, at least one polymer, at least one protein or protein hydrolysate, and at least one surfactant; 39. at least one carbohydrate, at least one amino acid, at least one polyamino acid, at least one sugar acid, at least one nucleotide, at least one organic acid, at least one inorganic acid, at least one bitter compound, at least one polymer, at least one protein or protein hydrolysate, at least one surfactant, and at least one flavonoid; 40. at least one carbohydrate, at least one amino acid, at least one polyamino acid, at least one sugar acid, at least one nucleotide, at least one organic acid, at least one inorganic acid, at least one bitter compound, at least one polymer, at least one protein or protein hydrolysate, at least one surfactant, at least one flavonoid, and at least one alcohol; 41. at least one carbohydrate, at least one amino acid, and at least one sugar acid; 42. at least one carbohydrate, at least one amino acid, and at least one nucleotide; 43. at least one carbohydrate, at least one amino acid, and at least one organic acid; 44. at least one carbohydrate, at least one amino acid, and at least one inorganic acid; 45. at least one carbohydrate, at least one amino acid, and at least one bitter compound; 46. at least one carbohydrate, at least one amino acid, and at least one polymer; 47. at least one carbohydrate, at least one amino acid, and at least one protein or protein hydrolysate; 48. at least one carbohydrate, at least one amino acid, and at least one surfactant; 49. at least one carbohydrate, at least one amino acid, and at least one flavonoid; 50. at least one carbohydrate, at least one amino acid, and at least one alcohol; 51. at least one carbohydrate, at least one polyamino acid, and at least one sugar acid; 52. at least one carbohydrate, at least one polyamino acid, and at least one nucleotide; 53. at least one carbohydrate, at least one polyamino acid, and at least one organic acid; 54. at least one carbohydrate, at least one polyamino acid, and at least one inorganic acid; 55. at least one carbohydrate, at least one polyamino acid, and at least one bitter compound; 56. at least one carbohydrate, at least one polyamino acid, and at least one polymer; 57. at least one carbohydrate, at least one polyamino acid, and at least one protein or protein hydrolysate; 58. at least one carbohydrate, at least one polyamino acid, and at least one surfactant; 59. at least one carbohydrate, at least one polyamino acid, and at least one flavonoid; 60. at least one carbohydrate, at least one polyamino acid, and at least one alcohol; 61. at least one carbohydrate, at least one sugar acid, and at least one nucleotide; 62. at least one carbohydrate, at least one sugar acid, and at least one organic acid; 63. at least one carbohydrate, at least one sugar acid, and at least one inorganic acid; 64. at least one carbohydrate, at least one sugar acid, and at least one bitter compound; 65. at least one carbohydrate, at least one sugar acid, and at least one polymer, 66. at least one carbohydrate, at least one sugar acid, and at least one protein or protein hydrolysate; 67. at least one carbohydrate, at least one sugar acid, and at least one surfactant; 68. at least one carbohydrate, at least one sugar acid, and at least one flavonoid; 69. at least one carbohydrate, at least one sugar acid, and at least one alcohol; 70. at least one carbohydrate, at least one nucleotide, and at least one organic acid; 71. at least one carbohydrate, at least one nucleotide, and at least one inorganic acid; 72. at least one carbohydrate, at least one nucleotide, and at least one bitter compound; 73. at least one carbohydrate, at least one nucleotide, and at least one polymer; 74. at least one carbohydrate, at least one nucleotide, and at least one protein or protein hydrolysate; 75. at least one carbohydrate, at least one nucleotide, and at least one surfactant; 76. at least one carbohydrate, at least one nucleotide, and at least one flavonoid; 77. at least one carbohydrate, at least one nucleotide, and at least one alcohol; 78. at least one carbohydrate, at least one organic acid, and at least one inorganic acid; 79. at least one carbohydrate, at least one organic acid, and at least one bitter compound; 80. at least one carbohydrate, at least one organic acid, and at least one polymer; 81. at least one carbohydrate, at least one organic acid, and at least one protein or protein hydrolysate; 82. at least one carbohydrate, at least one organic acid, and at least one surfactant; 83. at least one carbohydrate, at least one organic acid, and at least one flavonoid; 84. at least one carbohydrate, at least one organic acid, and at least one alcohol; 85. at least one carbohydrate, at least one inorganic acid, and at least one bitter compound; 86. at least one carbohydrate, at least one inorganic acid, and at least one polymer; 87. at least one carbohydrate, at least one inorganic acid, and at least one protein or protein hydrolysate; 88. at least one carbohydrate, at least one inorganic acid, and at least one surfactant; 89. at least one carbohydrate, at least one inorganic acid, and at least one flavonoid; 90. at least one carbohydrate, at least one inorganic acid, and at least one alcohol; 91. at least one carbohydrate, at least one bitter compound, and at least one polymer; 92. at least one carbohydrate, at least one bitter compound, and at least one protein or protein hydrolysate; 93. at least one carbohydrate, at least one bitter compound, and at least one surfactant; 94. at least one carbohydrate, at least one bitter compound, and at least one flavonoid; 95. at least one carbohydrate, at least one bitter compound, and at least one alcohol; 96. at least one carbohydrate, at least one polymer, and at least one protein or protein hydrolysate; 97. at least one carbohydrate, at least one polymer, and at least one surfactant; 98. at least one carbohydrate, at least one polymer, and at least one flavonoid; 99. at least one carbohydrate, at least, one polymer, and at least one alcohol; 100. at least one carbohydrate, at least one protein or protein hydrolysate, and at least one surfactant; 101. at least one carbohydrate, at least one protein or protein hydrolysate, and at least one flavonoid; 102. at least one carbohydrate, at least one surfactant, and at least one flavonoid; 103. at least one carbohydrate, at least one surfactant, and at least one alcohol; 104. at least one carbohydrate, at least one flavonoid, and at least one alcohol; 105. at least one sweet taste improving additive and D-tagatose; 106. at least one sweet taste improving additive and trehalose; 107. at least one sweet taste improving additive and D-galactose; 108. at least one sweet taste improving additive and rhamnose; 109. at least one sweet taste improving additive and dextrin; 110. at least one sweet taste improving additive and cyclodextrin; 111. at least one sweet taste improving additive and β-cyclodextrin; 112. at least one sweet taste improving additive and maltodextrin; 113. at least one sweet taste improving additive and dextran; 114. at least one sweet taste improving additive and sucrose; 115. at least one sweet taste improving additive and glucose; 116. at least one sweet taste improving additive and fructose; 117. at least one sweet taste improving additive and threose; 118. at least one sweet taste improving additive and arabinose; 119. at least one sweet taste improving additive and xylose; 120. at least one sweet taste improving additive and lyxose; 121. at least one sweet taste improving additive and allose; 122. at least one sweet taste improving additive and altrose; 123. at least one sweet taste improving additive and mannose; 124. at least one sweet taste improving additive and idose; 125. at least one sweet taste improving additive and talose; 126. at least one sweet taste improving additive and lactose; 127. at least one sweet taste improving additive and maltose; 128. at least one sweet taste improving additive and invert sugar; 129. at least one sweet taste improving additive and trehalose; 130. at least one sweet taste improving additive and isotrehalose; 131. at least one sweet taste improving additive and neotrehalose; 132. at least one sweet taste improving additive and palatinose; 133. at least one sweet taste improving additive and galactose; 134. at least one sweet taste improving additive and beet oligosaccharides; 135. at least one sweet taste improving additive and isomalto-oligosaccharides; 136. at least one sweet taste improving additive and isomaltose; 137. at least one sweet taste improving additive and isomaltotriose; 138. at least one sweet taste improving additive and panose; 139. at least one sweet taste improving additive and xylo-oligosaccharides; 140. at least one sweet taste improving additive and xylotriose; 141. at least one sweet taste improving additive and xylobiose; 142. at least one sweet taste improving additive and gentio-oligoscaccharides; 143. at least one sweet taste improving additive and gentiobiose; 144. at least one sweet taste improving additive and gentiotriose; 145. at least one sweet taste improving additive and gentiotetraose; 146. at least one sweet taste improving additive and sorbose; 147. at least one sweet taste improving additive and nigero-oligosaccharides; 148. at least one sweet taste improving additive and palatinose oligosaccharides; 149. at least one sweet taste improving additive and fucose; 150. at least one sweet taste improving additive and fructooligosaccharides; 151. at least one sweet taste improving additive and kestose; 152. at least one sweet taste improving additive and nystose; 153. at least one sweet taste improving additive and maltotetraol; 154. at least one sweet taste improving additive and maltotriol; 155. at least one sweet taste improving additive and malto-oligosaccharides; 156. at least one sweet taste improving additive and maltotriose; 157. at least one sweet taste improving additive and maltotetraose; 158. at least one sweet taste improving additive and maltopentaose; 159. at least one sweet taste improving additive and maltohexaose; 160. at least one sweet taste improving additive and maltoheptaose; 161. at least one sweet taste improving additive and lactulose; 162. at least one sweet taste improving additive and melibiose; 163. at least one sweet taste improving additive and raffinose; 164. at least one sweet taste improving additive and rhamnose; 165. at least one sweet taste improving additive and ribose; 166. at least one sweet taste improving additive and isomerized liquid sugars; 167. at least one sweet taste improving additive and high fructose corn syrup (e.g., HFCS55, HFCS42, or HFCS90) or starch syrup; 168. at least one sweet taste improving additive and coupling sugars; 169. at least one sweet taste improving additive and soybean oligosaccharides; 170. at least one sweet taste improving additive and glucose syrup; 171. at least one sweet taste improving additive, D-tagatose, and at least one other carbohydrate; 172. at least one sweet taste improving additive, trehalose, and at least one other carbohydrate; 173. at least one sweet taste improving additive, D-galactose, and at least one other carbohydrate; 174. at least one sweet taste improving additive, rhamnose, and at least one other carbohydrate; 175. at least one sweet taste improving additive, dextrin, and at least one other carbohydrate; 176. at least one sweet taste improving additive, cyclodextrin, and at least one other carbohydrate; 177. at least one sweet taste improving additive, β-cyclodextrin, and at least one other carbohydrate; 178. at least one sweet taste improving additive, maltodextrin, and at least one other carbohydrate; 179. at least one sweet taste improving additive, dextran, and at least one other carbohydrate; 180. at least one sweet taste improving additive, sucrose, and at least one other carbohydrate; 181. at least one sweet taste improving additive, glucose, and at least one other carbohydrate; 182. at least one sweet taste improving additive, fructose, and at least one other carbohydrate; 183. at least one sweet taste improving additive, threose, and at least one other carbohydrate; 184. at least one sweet taste improving additive, arabinose, and at least one other carbohydrate; 185. at least one sweet taste improving additive, xylose, and at least one other carbohydrate; 186. at least one sweet taste improving additive, lyxose, and at least one other carbohydrate; 187. at least one sweet taste improving additive, allose, and at least one other carbohydrate; 188. at least one sweet taste improving additive, altrose, and at least one other carbohydrate; 189. at least one sweet taste improving additive, mannose, and at least one other carbohydrate; 190. at least one sweet taste improving additive, idose, and at least one other carbohydrate; 191. at least one sweet taste improving additive, talose, and at least one other carbohydrate; 192. at least one sweet taste improving additive, lactose, and at least one other carbohydrate; 193. at least one sweet taste improving additive, maltose, and at least one other carbohydrate; 194. at least one sweet taste improving additive, invert sugar, and at least one other carbohydrate; 195. at least one sweet taste improving additive, trehalose, and at least one other carbohydrate; 196. at least one sweet taste improving additive, isotrehalose, and at least one other carbohydrate; 197. at least one sweet taste improving additive, neotrehalose, and at least one other carbohydrate; 198. at least one sweet taste improving additive, palatinose, and at least one other carbohydrate; 199. at least one sweet taste improving additive, galactose, and at least one other carbohydrate; 200. at least one sweet taste improving additive, beet oligosaccharides, and at least one other carbohydrate; 201. at least one sweet taste improving additive, isomalto-oligosaccharides, and at least one other carbohydrate; 202. at least one sweet taste improving additive, isomaltose, and at least one other carbohydrate; 203. at least one sweet taste improving additive, isomaltotriose, and at least one other carbohydrate; 204. at least one sweet taste improving additive, panose, and at least one other carbohydrate; 205. at least one sweet taste improving additive, xylo-oligosaccharides, and at least one other carbohydrate; 206. at least one sweet taste improving additive, xylotriose, and at least one other carbohydrate; 207. at least one sweet taste improving additive, xylobiose, and at least one other carbohydrate; 208. at least one sweet taste improving additive, gentio-oligoscaccharides, and at least one other carbohydrate; 209. at least one sweet taste improving additive, gentiobiose, and at least one other carbohydrate; 210. at least one sweet taste improving additive, gentiotriose, and at least one other carbohydrate; 211. at least one sweet taste improving additive, gentiotetraose, and at least one other carbohydrate; 212. at least one sweet taste improving additive, sorbose, and at least one other carbohydrate; 213. at least one sweet taste improving additive, nigero-oligosaccharides, and at least one other carbohydrate; 214. at least one sweet taste improving additive, palatinose oligosaccharides, and at least one other carbohydrate; 215. at least one sweet taste improving additive, fucose, and at least one other carbohydrate; 216. at least one sweet taste improving additive, fructooligosaccharides, and at least one other carbohydrate; 217. at least one sweet taste improving additive, kestose, and at least one other carbohydrate; 218. at least one sweet taste improving additive, nystose, and at least one other carbohydrate; 219. at least one sweet taste improving additive, maltotetraol, and at least one other carbohydrate; 220. at least one sweet taste improving additive, maltotriol, and at least one other carbohydrate; 221. at least one sweet taste improving additive, malto-oligosaccharides, and at least one other carbohydrate; 222. at least one sweet taste improving additive, maltotriose, and at least one other carbohydrate; 223. at least one sweet taste improving additive, maltotetraose, and at least one other carbohydrate; 224. at least one sweet taste improving additive, maltopentaose, and at least one other carbohydrate; 225. at least one sweet taste improving additive, maltohexaose, and at least one other carbohydrate; 226. at least one sweet taste improving additive, maltoheptaose, and at least one other carbohydrate; 227. at least one sweet taste improving additive, lactulose, and at least one other carbohydrate; 228. at least one sweet taste improving additive, melibiose, and at least one other carbohydrate; 229. at least one sweet taste improving additive, raffinose, and at least one other carbohydrate; 230. at least one sweet taste improving additive, rhamnose, and at least one other carbohydrate; 231. at least one sweet taste improving additive, ribose, and at least one other carbohydrate; 232. at least one sweet taste improving additive, isomerized liquid sugars, and at least one other carbohydrate; 233. at least one sweet taste improving additive, high fructose corn syrup (e.g.

HFCS55, HFCS42, or HFCS90) or starch syrup, and at least one other carbohydrate; 234. at least one sweet taste improving additive, coupling sugars, and at least one other carbohydrate; 235. at least one sweet taste improving additive, soybean oligosaccharides, and at least one other carbohydrate; and 236. at least one sweet taste improving additive, glucose syrup, and at least one other carbohydrate.

Another functional ingredient that may be combined with the PPAR agonist of present invention is at least one mineral. Minerals, in accordance with the teachings of this invention, comprise inorganic chemical elements required by living organisms. Minerals are comprised of a broad range of compositions (e.g., elements, simple salts, and complex silicates) and also vary broadly in crystalline structure. They may naturally occur in foods and beverages, may be added as a supplement, or may be consumed or administered separately from foods or beverages. Minerals may be categorized as either bulk minerals, which are required in relatively large amounts, or trace minerals, which are required in relatively small amounts. Bulk minerals generally are required in amounts greater than or equal to about 100 mg per day and trace minerals are those that are required in amounts less than about 100 mg per day. In particular embodiments of this invention, the at least one mineral comprises bulk minerals, trace minerals, or combinations thereof. Non-limiting examples of bulk minerals include calcium, chlorine, magnesium, phosphorous, potassium, sodium, and sulfur. Non-limiting examples of trace minerals include chromium, cobalt, copper, fluorine, iron, manganese, molybdenum, selenium, zinc, and iodine. Although iodine generally is classified as a trace mineral, it is required in larger quantities than other trace minerals and often is categorized as a bulk mineral in other particular embodiments of this invention, the at least one mineral may comprise other trace minerals that are believed to be necessary for human nutrition, non-limiting examples of which include bismuth, boron, lithium, nickel, rubidium, silicon, strontium, tellurium, tin, titanium, tungsten, and vanadium. The minerals embodied herein may be in any form known to those of ordinary skill in the art. For example, in a particular embodiment the minerals may be in their ionic form, having either a positive or negative charge. In another particular embodiment the minerals may be in their molecular form. For example, sulfur and phosphorous often are found naturally as sulfates, sulfides, and phosphates. Appropriate intake of dietary minerals is necessary to maintain health. Sodium, potassium, and chlorine largely regulate the fluid balance in the body. Sodium also is involved in the absorption of other nutrients, such as glucose and amino acids. In addition, sodium and potassium act as cofactors for some enzymes. Other minerals, such as calcium, magnesium, and phosphorous, are essential for the proper development of the skeletal system and serve important structural functions in the body. These minerals also are important for maintaining connective tissue and cell membranes. Mineral deficiencies contribute to a number of health problems. For example, a sodium or potassium deficiency may cause abnormal nerve activity, cardiac arrhythmias or even cardiac arrest. Deficiencies of iodine, which is used by the body in synthesis of thyroid hormones, may lead to goiter and, in pregnant females, can lead to serious birth defects such as cretinism. Iron is an essential component of hemoglobin, and dietary iron deficiencies can cause anemia, resulting in tiredness and shortness of breath. Calcium deficiencies, which most often are caused by a deficiency in vitamin D, can lead to poor bone structure and osteoporosis. Manganese and zinc deficiencies can result in rashes on the skin of the upper torso, face, groin, hands, and feet. Although mineral deficiencies can cause serious health problems, excessive mineral intake may also lead to illness. For example, potassium or magnesium toxicity in the body can lead to cardiac arrest. Generally, kidneys that function normally can regulate mineral concentrations within the body and excrete excess amounts of minerals; however, in some cases the body is unable to regulate mineral concentrations properly. This can occur if kidney function is abnormal or if mineral intake is highly excessive. In addition, excess mineral intake of one mineral can influence the absorption and metabolism of other minerals. For example, the presence of a large amount of zinc in the diet decreases the absorption of iron and copper and can lead to harmful deficiencies. Because of the importance of obtaining an appropriate intake of minerals, people generally should eat a well-balanced diet. However, for many people, available food supply or dietary patterns can cause mineral imbalances or mineral deficiencies, falling short of the Recommended Dietary Allowances (RDAs) promulgated by the Food and Nutrition Board of the National Academy of Sciences. Accordingly, dietary mineral supplements and mineral fortification of foods and beverages are desirable and generally recommended by the Food and Nutrition Board. RDAs for males and females of commonly recognized dietary minerals are provided in the table below along with maximum safe levels of daily nutrient intake; however, this table should not be construed as limiting the scope of the invention. Mineral intake beyond the upper limits provided by the Food and Nutrition Board may be appropriate if prescribed by a physician. Note also that the RDAs in the table below are provided for adult males and adult females. Generally, lower dietary allowances are appropriate for infants and for children under the age of 18.
Due to their activity, the PPAR agonists and PPAR activators are advantageously useful in human and veterinary medicine. As described above, the compounds of the invention are useful for treating or preventing arteriosclerosis, dyslipemia or hypercholesterolemia in a patient.

When administered to a patient, a PPAR agonist or PPAR activator is preferably administered as a component of a composition that optionally comprises a pharmaceutically acceptable carrier or vehicle. In a preferred embodiment, these compositions are administered orally.

Compositions for oral administration might require an enteric coating to protect the composition(s) from degradation within the gastrointestinal tract. In another example, the composition(s) can be administered in a liposomal formulation to shield the PPAR agonists and PPAR activators disclosed herein from degradative enzymes, facilitate the molecule's transport in the circulatory system, and effect delivery of the molecule across cell membranes to intracellular sites.

PPAR agonists and PPAR activators intended for oral administration can be coated with or admixed with a material (e.g., glyceryl monostearate or glyceryl distearate) that delays disintegration or affects absorption of the PPAR agonist in the gastrointestinal tract. Thus, for example, the sustained release of a PPAR agonist can be achieved over many hours and, if necessary, the PPAR agonist can be protected from being degraded within the gastrointestinal tract. Taking advantage of the various pH and enzymatic conditions along the gastrointestinal tract, pharmaceutical compositions for oral administration can be formulated to facilitate release of a PPAR agonist at a particular gastrointestinal location.

Selectively permeable membranes surrounding an osmotically active driving compound are also suitable for orally administered compositions. Fluid from the environment surrounding the capsule is imbibed by the driving compound, which swells to displace the PPAR agonist through an aperture, can provide an essentially zero order delivery profile instead of the spiked profiles of immediate release formulations. A time delay material such as, but not limited to, glycerol monostearate or glycerol stearate can also be used.

Suitable pharmaceutical carriers also include starch, glucose, lactose, sucrose, gelatin, saline, gum acacia, talc, keratin, urea, malt, rice, flour, chalk, silica gel, sodium stearate, glycerol monostearate, talc, sodium chloride, dried skim milk, glycerol, propylene, glycol, water, and ethanol. If desired, the carrier, can also contain minor amounts of wetting or emulsifying agents, or pH buffering agents. In addition, auxiliary, stabilizing, thickening, lubricating, and coloring agents may be used. The composition can be formulated as a suppository, with traditional binders and carriers such as triglycerides.

A pharmaceutical composition comprising a PPAR agonist or PPAR activator can be administered via one or more routes such as, but not limited to, oral, intravenous infusion, subcutaneous injection, intramuscular, topical, depo injection, implantation, time-release mode, and intracavitary. The pharmaceutical composition is formulated to be compatible with its intended route of administration. Examples of routes of administration include parenteral, e.g., intravenous, intramuscular, intraperitoneal, intracapsular, intraspinal, intrasternal, intratumor, intranasal, epidural, intra-arterial, intraocular, intraorbital, intradermal, subcutaneous, oral (e.g., inhalation), transdermal (topical--particularly to the ears, nose, eyes, or skin), transmucosal (e.g., oral) nasal, rectal, intracerebral, intravaginal, sublingual, submucosal, and transdermal administration.

Administration can be via any route known to be effective by a physician of ordinary skill. Parenteral administration, i.e., not through the alimentary canal, can be performed by subcutaneous, intramuscular, intra-peritoneal, intratumoral, intradermal, intracapsular, intra-adipose, or intravenous injection of a dosage form into the body by means of a sterile syringe, optionally a pen-like syringe, or some other mechanical device such as an infusion pump. A further option is a composition that can be a powder or a liquid for the administration in the form of a nasal or pulmonary spray. As a still further option, the administration can be transdermally, e.g., from a patch. Compositions suitable for oral, buccal, rectal, or vaginal administration can also be provided.

In one embodiment, a pharmaceutical composition of the invention is delivered by a controlled-release system. For example, the pharmaceutical composition can be administered using intravenous infuision, an implantable osmotic pump, a transdermal patch, liposomes, or other modes of administration. In one embodiment, a pump can be used (See e.g., Langer, 1990, Science 249:1527-33; Sefton, 1987, CRC Crit. Ref. Biomed. Eng. 14:201; Buchwald et al., 1980, Surgery 88:507; Saudek et al., 1989, N. Engl. J. Med. 321:574). In another embodiment, the compound can be delivered in a vesicle, in particular a liposome (See e.g., Langer, 1990, Science 249:1527-33; Treat et al., 1989, in Liposomes in the Therapy of Infectious Disease and Cancer, Lopez-Berestein and Fidler (eds.), Liss, New York, pp. 353-65; Lopez-Berestein, ibid., pp. 317-27; International Patent Publication No. WO 91/04014; U.S. Pat. No. 4,704,355). In another embodiment, polymeric materials can be used (See e.g., Medical Applications of Controlled Release, Langer and Wise (eds.), CRC Press: Boca Raton, Fla., 1974; Controlled Drug Bioavailability, Drug Product Design and Performance, Smolen and Ball (eds.), Wiley: New York (1984); Ranger and Peppas, 1953, J. Macromol. Sci. Rev. Macromol. Chem. 23:61; Levy et al., 1985, Science 228:190; During et al., 1989, Ann. Neurol. 25:351; Howard et al., 1989, J. Neurosurg. 71:105).

In yet another embodiment, a controlled release system can be placed in proximity of the target. For example, a micropump can deliver controlled doses directly into bone or adipose tissue, thereby requiring only a fraction of the systemic dose (See e.g., Goodson, 1984, in Medical Applications of Controlled Release, vol. 2, pp. 115-138). In another example, a pharmaceutical composition of the invention can be formulated with a hydrogel (See, e.g., U.S. Pat. Nos. 5,702,717; 6,117,949; 6,201,072).

In one embodiment, it may be desirable to administer the pharmaceutical composition of the invention locally, i.e., to the area in need of treatment. Local administration can be achieved, for example, by local infusion during surgery, topical application (e.g., in conjunction with a wound dressing after surgery), injection, catheter, suppository, or implant. An implant can be of a porous, non-porous, or gelatinous material, including membranes, such as sialastic membranes, or fibers.

In certain embodiments, it may be desirable to introduce the PPAR agonists and PPAR activators into the central nervous system by any suitable route, including intraventricular, intrathecal, and epidural injection. Intraventricular injection may be facilitated by an intraventricular catheter, for example, attached to a reservoir, such as an Ommaya reservoir.

Pulmonary administration can also be employed, e.g., by use of an inhaler or nebulizer, and formulation with an aerosolizing agent, or via perfusion in a fluorocarbon or synthetic pulmonary surfactant.

In one embodiment, the invention provides for the treatment of a patient using implanted cells that have been regenerated or stimulated to proliferate in vitro or in vivo prior to reimplantation or transplantation into a recipient. Conditioning of the cells ex vivo can be achieved simply by growing the cells or tissue to be transplanted in a medium that has been supplemented with a growth-promoting amount of the combinations and is otherwise appropriate for culturing of those cells. The cells can, after an appropriate conditioning period, then be implanted either directly into the patient or can be encapsulated using established cell encapsulation technology, and then implanted.

The skilled artisan can appreciate the specific advantages and disadvantages to be considered in choosing a mode of administration. Multiple modes of administration are encompassed by the invention. For example, a PPAR agonist of the invention can be administered by subcutaneous injection, whereas another therapeutic agent can be administered by intravenous infusion. Moreover, administration of one or more species of PPAR activators , with or without other therapeutic agents, can occur simultaneously (i.e., co-administration) or sequentially. In another embodiment, the periods of administration of a PPAR agonist or PPAR activator, with or without other therapeutic agents can overlap. For example a PPAR agonist or PPAR activator can be administered for 7 days and another therapeutic agent can be introduced beginning on the fifth day of PPAR A activators treatment. Treatment with the other therapeutic agent can continue beyond the 7-day PPAR activators treatment.

A pharmaceutical composition of a PPAR agonist or PPAR activator can be administered before, during, and/or after the administration of one or more therapeutic agents. In one embodiment, a PPAR agonist or PPAR activator can first be administered to stimulate the expression of insulin, which increases sensitivity to subsequent challenge with a therapeutic agent. In another embodiment, a PPAR agonist or PPAR activator can be administered after administration of a therapeutic agent. In yet another embodiment, there can be a period of overlap between the administration of the PPAR agonist or PPAR activator and the administration of one or more therapeutic agents.

A pharmaceutical composition of the invention can be administered in the morning, afternoon, evening, or diurnally. In one embodiment, the pharmaceutical composition is administered at particular phases of the circadian rhythm. In a specific embodiment, the pharmaceutical composition is administered in the morning. In another specific embodiment, the pharmaceutical composition is administered at an artificially induced circadian state.

The present compositions can take the form of solutions, suspensions, emulsion, tablets, pills, pellets, capsules, capsules containing liquids, powders, sustained-release formulations, suppositories, emulsions, aerosols, sprays, suspensions, or any other form suitable for use. In one embodiment, the pharmaceutically acceptable vehicle is a capsule (See e.g., U.S. Pat. No. 5,698,155). Other examples of suitable pharmaceutical carriers are described in Remington 's Pharmaceutical Sciences, Alfonso R. Gennaro ed., Mack Publishing Co. Easton, Pa., 19th ed., 1995, pp. 1447 to 1676, incorporated herein by reference.

Accordingly, the pharmaceutical compositions herein described can be in the form of oral tablets, capsules, elixirs, syrups and the like.

For instance, for oral administration in the form of a tablet or capsule, the active drug component can be combined with an oral, non-toxic, pharmaceutically acceptable, inert carrier such as, but not limited to, lactose, starch, sucrose, glucose, methyl cellulose, magnesium stearate, dicalcium phosphate, calcium sulfate, mannitol, and sorbitol. For oral administration in liquid form, the oral drug components can be combined with any oral, non-toxic, pharmaceutically acceptable carrier such as, but not limited to, ethanol, glycerol, and water. Moreover, suitable binders, lubricants, disintegrating agents and coloring agents can also be incorporated into the mixture. Suitable binders include, but are not limited to, starch, gelatin, natural sugars (e.g., glucose, beta-lactose), corn sweeteners, natural and synthetic gums (e.g., acacia, tragacanth, sodium alginate), carboxymethylcellulose, polyethylene glycol, and waxes. Lubricants useful for an orally administered drug, include, but are not limited to, sodium oleate, sodium stearate, magnesium stearate, sodium benzoate, sodium acetate, and sodium chloride. Disintegrators include, but are not limited to, starch, methyl cellulose, agar, bentonite, and xanthan gum.

Pharmaceutical compositions adapted for oral administration can be provided, for example, as capsules or tablets; as powders or granules; as solutions, syrups or suspensions (in aqueous or non-aqueous liquids); as edible foams or whips; or as emulsions. For oral administration in the form of a tablet or capsule, the active drug component can be combined with an oral, non-toxic, pharmaceutically acceptable, inert carrier such as, but not limited to, lactose, starch, sucrose, glucose, methyl cellulose, magnesium stearate, dicalcium phosphate, magnesium carbonate, stearic acid or salts thereof, calcium sulfate, mannitol, and sorbitol. For oral administration in the form of a soft gelatine capsule, the active drug component can be combined with an oral, non-toxic, pharmaceutically acceptable, inert carrier such as, but not limited to, vegetable oils, waxes, fats, semi-solid, and liquid polyols. For oral administration in liquid form, the oral drug components can be combined with any oral, non-toxic, pharmaceutically acceptable carrier such as, but not limited to, ethanol, glycerol, polyols, and water. Moreover, suitable binders, lubricants, disintegrating agents and coloring agents can also be incorporated into the mixture. Suitable binders include, but are not limited to, starch, gelatin, natural sugars (e.g. glucose, beta-lactose), corn sweeteners, natural and synthetic gums (e.g., acacia, tragacanth, sodium alginate), carboxymethylcellulose, polyethylene glycol, and waxes. Lubricants useful for an orally administered drug, include, but are not limited to, sodium oleate, sodium stearate, magnesium stearate, sodium benzoate, sodium acetate, and sodium chloride. Disintegrators include, but are not limited to, starch, methyl cellulose, agar, bentonite, and xanthan gum.

Orally administered compositions may contain one or more agents, for example, sweetening agents such as, but not limited to, fructose, ASPARTAME and saccharin. Orally administered compositions may also contain flavoring agents such as, but not limited to, peppermint, oil of wintergreen, and cherry. Orally administered compositions may also contain coloring agents and/or preserving agents.

The PPAR agonists and PPAR activators can also be administered in the form of liposome delivery systems, such as small unilamellar vesicles, large unilamellar vesicles and multilamellar vesicles. Liposomes can be formed from a variety of phospholipids, such as cholesterol, stearylamine or phosphatidylcholines. A variety of cationic lipids can be used in accordance with the invention including, but not limited to, N-(1(2,3-dioleyloxy)propyl)-N,N,N-trimethylammonium chloride ("DOTMA") and diolesylphosphotidylethanolamine ("DOPE"). Such compositions suit the mode of administration.

PPAR agonists and PPAR activators can also be delivered by the use of monoclonal antibodies as individual carriers to which the PPAR agonists and PPAR activators can be coupled. The PPAR agonists and PPAR activators can also be coupled with soluble polymers as targetable drug carriers. Such polymers can include polyvinylpyrrolidone, pyran copolymer, polyhydroxypropylmethacrylamide-phenol, polyhydroxyethylaspartamide-phenol, or polyethyleneoxide-polylysine substituted with palmitoyl residues. Furthermore, the PPAR agonists and PPAR activators can be coupled to a class of biodegradable polymers useful in achieving controlled release of a drug, for example, polylactic acid, polyglycolic acid, copolymers of polylactic and polyglycolic acid, polyepsilon caprolactone, polyhydroxy butyric acid, polyorthoesters, polyacetals, polydihydropyrans, polycyanoacrylates and crosslinked or amphipathic block copolymers of hydrogels.

Pharmaceutical compositions adapted for parenteral administration include, but are not limited to, aqueous and non-aqueous sterile injectable solutions or suspensions, which can contain antioxidants, buffers, bacteriostats and solutes that render the pharmaceutical compositions substantially isotonic with the blood of an intended recipient. Other components that can be present in such pharmaceutical compositions include water, alcohols, polyols, glycerine and vegetable oils, for example. Compositions adapted for parenteral administration can be presented in unit-dose or multi-dose containers (e.g., sealed ampules and vials), and can be stored in a freeze-dried (i.e., lyophilized) condition requiring the addition of a sterile liquid carrier (e.g., sterile saline solution for injections) immediately prior to use. Extemporaneous injection solutions and suspensions can be prepared from sterile powders, granules and tablets.

Pharmaceutical compositions adapted for transdermal administration can be provided as discrete patches intended to remain in intimate contact with the epidennis for a prolonged period of time. Pharmaceutical compositions adapted for topical administration can be provided as, for example, ointments, creams, suspensions, lotions, powders, solutions, pastes, gels, sprays, aerosols or oils. A topical ointment or cream is preferably used for topical administration to the skin, mouth, eye or other external tissues. When formulated in an ointment, the active ingredient can be employed with either a paraffinic or a water-miscible ointment base. Alternatively, the active ingredient can be formulated in a cream with an oil-in-water base or a water-in-oil base.

Pharmaceutical compositions adapted for topical administration to the eye include, for example, eye drops or injectable pharmaceutical compositions. In these pharmaceutical compositions, the active ingredient can be dissolved or suspended in a suitable carrier, which includes, for example, an aqueous solvent with or without carboxymethylcellulose. Pharmaceutical compositions adapted for topical administration in the mouth include, for example, lozenges, pastilles and mouthwashes.

Pharmaceutical compositions adapted for nasal administration can comprise solid carriers such as powders (preferably having a particle size in the range of 20 to 500 microns). Powders can be administered in the manner in which snuff is taken, i.e., by rapid inhalation through the nose from a container of powder held close to the nose. Alternatively, pharmaceutical compositions adopted for nasal administration can comprise liquid carriers such as, for example, nasal sprays or nasal drops. These pharmaceutical compositions can comprise aqueous or oil solutions of a PPAR agonist. Compositions for administration by inhalation can be supplied in specially adapted devices including, but not limited to, pressurized aerosols, nebulizers or insufflators, which can be constructed so as to provide predetermined dosages of the PPAR agonist or PPAR activator.

Pharmaceutical compositions adapted for rectal administration can be provided as suppositories or enemas. Pharmaceutical compositions adapted for vaginal administration can be provided, for example, as pessaries, tampons, creams, gels, pastes, foams or spray formulations.

Suppositories generally contain active ingredients in the range of 0.5% to 10% by weight. Oral formulations preferably contain 10% to 95% active ingredient by weight. In a preferred embodiment, the composition is formulated in accordance with routine procedures as a pharmaceutical composition adapted for intratumoral injection, implantation, subcutaneous injection, or intravenous administration to humans.

Typically, pharmaceutical compositions for injection or intravenous administration are solutions in sterile isotonic aqueous buffer. Where necessary, the composition can also include a solubilizing agent and a local anesthetic such as lidocaine to ease pain at the site of the injection. Generally, the ingredients are supplied either separately or mixed together in unit dosage form, for example, as a dry lyophilized powder or water-free concentrate in a hermetically sealed container such as an ampule or sachette indicating the quantity of active agent.

Where the composition is to be administered by infusion, it can be dispensed with an infusion bottle, bag, or other acceptable container, containing sterile pharmaceutical grade water, saline, or other acceptable diluents. Where the composition is administered by injection, an ampule of sterile water for injection or saline can be provided so that the ingredients may be mixed prior to administration.

For a patient who cannot orally ingest a nutrient, it is essential to supply all nutrients such as an amino acid, a saccharide and an electrolyte through a vein. This way is called the total parenteral nutrition therapy, (TPN therapy) which can be provided by a TPN solution. Such TNP solutions are particularly suitable for crictially yill patients for a therapy in the Intensive Care Unit. As a TPN solution employed in the TPN therapy, there has been known (1) a TPN solution containing a saccharide, an amino acid, a fat and an electrolyte (Japanese Unexamined Patent Publications No. 186822/1989, WO08503002 and EP-A-0 399 341), (2) an emulsion for injection comprising an amino acid and a fat (Japanese Unexamined Patent Publication No. 74637/1986), (3) a TPN solution comprising two separate infusions, one of which contains glucose and an electrolyte and the other of which contains an amino acid (Japanese Unexamined Patent Publications No. 52455/1982 and No. 103823/1986) and the like. In the TPN therapy, an infusion containing a high concentration of saccharide is usually administered to a patient. When a saccharide is degraded in glycolytic pathway to be utilized as a source of energy, vitamin B.sub. 1 is consumed as a coenzyme. Therefore, vitamin B.sub. 1 is deficient and deficiency of vitamin B.sub.1 results in production of lactic acid in patients who are subjected to the TPN therapy for a long term. At worst, some of the patients have difficulty in breathing and the like due to the severe lactic acidosis. Accordingly, it has been known that the use of a water-soluble vitamin B, particularly vitamin B. sub.1, with the infusion containing a high concentration of saccharide is essential in the TPN therapy. However, vitamin B.sub.1 is rapidly degraded by a sulfite ion which is contained in an amino acid-infusion and the like as a stabilizer. Thus a practical TPN solution previously containing a water-soluble vitamin B has not been developed.
Such TNP solution can be combined with a PPAR agonist or PPAR activator of present invention to provide a PPAR activator treatment of a critically ill patient or to increase expression of a PPAR or increase the activity of a PPAR in a critically ill patient.

The PPAR agonists and PPAR activators and optionally another therapeutic agent are administered at an effective dose. The dosing and regimen most appropriate for patient treatment will vary with the disease or condition to be treated, and in accordance with the patient's weight and with other parameters.

An effective dosage and treatment protocol can be determined by conventional means, comprising the steps of starting with a low dose in laboratory animals, increasing the dosage while monitoring the effects (e.g., histology, disease activity scores), and systematically varying the dosage regimen. Several factors may be taken into consideration by a clinician when determining an optimal dosage for a given patient.. Additional factors include, but are not limited to, the size of the patient, the age of the patient, the general condition of the patient, the particular disease being treated, the severity of the disease, the presence of other drugs in the patient, and the in vivo activity of the PPAR agonist.

A typical effective human dose of a PPAR agonist or PPAR activator would be from about 10 µg/kg body weight/day to about 100 mg/kg/day, preferably from about 50 µg/kg/day to about 50 mg/kg/day, and most preferably about 100 µg/kg/day to 20 mg/kg/day. As analogs of the PPAR agonists and PPAR activators disclosed herein can be 2 to 100 times more potent than naturally occurring counterparts, a typical effective dose of such an analog can be lower, for example, from about 100 ng/kg body weight/day to 1 mg/kg/day, preferably 10 µg/kg/day to 900 µg/kg/day, and even more preferably 20 µg/kg/day to 250 µg/kg/day.

In another embodiment, the effective dose of a PPAR agonist or a PPAR activator of present is less than 10 µg/kg/day. In yet another embodiment the effective dose of a PPAR agonist or PPAR activator of present is greater than 10 mg/kg/day.

The specific dosage for a particular patient, of course, has to be adjusted to the degree of response, the route of administration, the patients weight, and the patient's general condition, and is finally dependent upon the judgment of the treating physician.

It is understandable that the ideal dosage per serving to have the health effect will have to vary according the body weight of the subject who consumes the oral ingestable dosage form which comprises the PPAR agonist or PPAR activator of present invention and the molecular weight of the compounds that will vary according to the amounts of glycoside groups on said the scaffold of general formula I of present invention. A PPAR effect can be obtained in a subject with about 50 kg body weight by an orally ingestable dosage form comprising between 5 mg and 2,5 gram, preferably 15 mg to 2 gram, more preferably between 25 mg and 1,5 gram, more preferably between 50 mg and 750 mg of the PPAR agonist of PPAR activator of present invention per serving of said food or beverage product (as demonstrated in Table 2)

**Table 2**

| | | | | | | | | | | |
|---|---|---|---|---|---|---|---|---|---|---|
| Possible amount of the PPAR agonist or PPAR activator active ingredient of present invention per serving by a subject | | | | | | | | | | |

| **Dose** | **BW Kg** | **BW Kg** | **BW Kg** | **BW Kg** | **BW Kg** | **BW Kg** | **BW Kg** | **BW Kg** | **BW Kg** | **BW Kg** |
|---|---|---|---|---|---|---|---|---|---|---|
| **mg/kg** | 50 | 60 | 70 | 80 | 90 | 100 | 110 | 120 | 130 | 140 |
| | mg | mg | mg | mg | mg | mg | mg | mg | mg | mg |
| 0,1 | 5 | 6 | 7 | 8 | 9 | 10 | 11 | 12 | 13 | 14 |
| 0,2 | 10 | 12 | 14 | 16 | 18 | 20 | 22 | 24 | 26 | 28 |
| 0,3 | 15 | 18 | 21 | 24 | 27 | 30 | 33 | 36 | 39 | 42 |
| 0,4 | 20 | 24 | 28 | 32 | 36 | 40 | 44 | 48 | 52 | 56 |
| 0,5 | 25 | 30 | 35 | 40 | 45 | 50 | 55 | 60 | 65 | 70 |
| 1 | 50 | 60 | 70 | 80 | 90 | 100 | 110 | 120 | 130 | 140 |
| 5 | 250 | 300 | 350 | 400 | 450 | 500 | 550 | 600 | 650 | 700 |
| 10 | 500 | 600 | 700 | 800 | 900 | 1000 | 1100 | 1200 | 1300 | 1400 |
| 15 | 750 | 900 | 1050 | 1200 | 1350 | 1500 | 1650 | 1800 | 1950 | 2100 |
| 20 | 1000 | 1200 | 1400 | 1600 | 1800 | 2000 | 2200 | 2400 | 2600 | 2800 |
| 25 | 1250 | 1500 | 1750 | 2000 | 2250 | 2500 | 2750 | 3000 | 3250 | 3500 |
| 30 | 1500 | 1800 | 2100 | 2400 | 2700 | 3000 | 3300 | 3600 | 3900 | 4200 |
| 35 | 1750 | 2100 | 2450 | 2800 | 3150 | 3500 | 3850 | 4200 | 4550 | 4900 |
| 40 | 2000 | 2400 | 2800 | 3200 | 3600 | 4000 | 4400 | 4800 | 5200 | 5600 |
| 45 | 2250 | 2700 | 3150 | 3600 | 4050 | 4500 | 4950 | 5400 | 5850 | 6300 |
| 50 | 2500 | 3000 | 3500 | 4000 | 4500 | 5000 | 5500 | 6000 | 6500 | 7000 |

Also contemplated are methods of prevention or treatment involving combination therapies comprising administering an effective amount of a the PPAR agonist or PPAR activator molecule of present invention can be in combination with another therapeutic agent or agents. The other therapeutic agent or agent can be, for example, an anti-osteoporosis agent, a steroid hormones, a non-steroid hormone, growth factor, a selective estrogen receptor modulator, an insulin-releasing agent, an inhibitor of glucagon secretion, a glucagon antagonists, a circadian rhythm regulator, a growth hormone secretagogue, an agent that increase IGF-1 levels, an immunotherapeutic agent, a cytokine, a protease inhibitor, a vitronectin receptor antagonist, a bisphosphonate compound, a kinase inhibitor, an integrin receptor or antagonist thereof, an anti-obesity agent, a lipid-metabolism improving agent, a neuropeptide Y blocker, a kainate/AMPA receptor antagonist, a β-adrenergic receptor agonist, a compound that reduces caloric intake, an anti-diabetes agent, or a dietary nutrient. Examples of therapeutic agents include, but are not limited to, those in Table 4.

Table 4: Other Therapeutics to be administered with the PPAR agonist or PPAR activators of present invention
● anti-osteoporosis agent
   o alendronate sodium
   o calcium L-threonate (e.g., C₈H₁₄O₁₀Ca)
   o clodronate
   o etidronate
   o gallium nitrate
   o mithramycin
   o norethindrone acetate (e.g., that which is commercially available as ACTIVELLA)
   o osteoprotegerin
   o pamidronate
   o risedronate sodium
● steroid hormones
   o androgen (e.g., androstenedione, testosterone, dehydroepiandrosterone, dihydrotestosterone, 7-alpha-methyl-19-nortestosterone, 7-alphamethyl-19-nortestosterone acetate, methandroil, oxymetholone, methanedione, oxymesterone, nordrolone phenylpropionate, noretbandrolone)
   o glucocorticoid
   o estrogenic hormones (e.g., that which is commercially available as PREMARIN)
   o progestin
● non-steroid hormone
   o calcitonin
   o calcitriol
   o growth hormone (e.g., osteoclast-activating factor)
   o melatonin
   o parathyroid hormone
   o prostaglandin
   o thyroid hormone
● growth factor
   o epidermal growth factor
   o fibroblast growth factor
   o insulin-like growth factor 1
   o insulin-like growth factor 2
   o platelet-derived growth factor
   o vascular endothelial growth factor
● selective estrogen receptor modulator
   o BE-25327
   o CP-336156
   o clometherone
   o delmadinone
   o droloxifene
   o idoxifene
   o nafoxidine
   o nitromifene
   o ormeloxifene
   o raloxifene (e.g., that which is commercially available as EVISTA)
   o tamoxifen
   o toremifene
   o trioxifene
   o [2-(4-hydroxyphenyl)-6-hydroxynaphthalen-1-yl][4-[2-(1-piperidinyl)-ethoxy]p henyl]-methane
● insulin-releasing agent
   o GLP-1
   o nateglinide
   o repaglinide (e.g., that which is commercially available as PRANDIN)
   o sulfonylurea (e.g., glyburide, glipizide, glimepiride)
   o vasopressin
● inhibitor of glucagon secretion
   o somatostatin
● glucagon antagonists
   o substituted glucagons having an alanine residue at position 1, 2, 3-5, 9-11, 21, or 29
   o des-His¹-Ala² glucagons
   o des-His¹-[Ala^{2,11}-Glu²¹]glucagon
● circadian rhythm regulator
   o alkylene dioxybenzene agonist
   o melatonin
   o neuropeptide Y
   o tachykinin agonist
   o visible light therapy
● growth hormone secretagogue
   o cycloalkano[b]thien-4-ylurea
   o GHRP-1
   o GHRP-6
   o growth hormone releasing factor
   o hexarelin
   o thiourea
   o B-HT920
   o benzo-fused lactams (e.g., N-biphenyl-3-amido substituted benzolactams)
   o benzo-fused macrocycles (e.g., 2-substituted piperidines, 2-substituted pyrrolidines, 2-substituted hexahydro-1H-azepines, di-substituted piperidines, di-substituted pyrrolidines, di-substituted hexahydro-1H-azepines, tri-substituted piperidines, tri-substituted pyrrolidines, tri-substituted hexahydro-1H-azepines, L-pyroglutamyl-pyridylalanyl-L-prolinamides)
● agents that increase IGF-1 levels
   o L-acetylcamitine
   o L-isovalerylcamitine
   o L-propionylcarnitine
● immunotherapeutic agent
   o antibody
   o immunomodulator
● cytokine
   o endothelial monocyte activating protein
   o granulocyte colony stimulating factor
   o interferon (e.g., IFN-γ)
   o interleukin (e.g., IL-6)
   o lymphokine
   o lymphotoxin-α
   o lymphotoxin-β
   o tumor necrosis factor
   o tumor necrosis-factor-like cytokine
   o macrophage inflammatory protein
   o monocyte colony stimulating factor
   o 4-1BBL
   o CD27 ligand
   o CD30 ligand
   o CD40 ligand
   o CD137 ligand
   o Fas ligand
   o OX40 ligand
● protease inhibitor
   o cysteine protease inhibitor (e.g., vinyl sulfone, peptidylfluoromethyl ketone, cystatin C, cystatin D, E-64)
   o DPP IV antagonist
   o DPP IV inhibitor (e.g., N-(substituted glycyl)-2-cyanopyrrolidines, N-Ala-Pro-O-nitrobenzyl-hydroxylamine, and ε-(4-nitro)benzoxycarbonyl-Lys-Pro)
   o serine-protease inhibitor (e,g., azapeptide, BMS232632, antipain, leupeptin)
● vitronectin receptor antagonist
   o anti-vitronectin receptor antibody (e.g., 23C6)
   o cyclo-S,S-N α-acetyl-cysteinyl-N alpha-methyl-argininyl-glycyl-aspartyl-penicillamine
   o RGD-containing peptide (e.g., echistatin)
● bisphosphonate compound
   o alendronate (e.g., that which is commercially available as FOSAMAX)
   o aminoalkyl bisphosphonate (e.g., alendronate, pamidronate (3-amino-1-hydroxypropylidene)bisphosphonic acid disodium salt, pamidronic acid, risedronate (1-hydroxy-2-(3-pyridinyl)ethylidene)bisphosphonate, YM 175 [(cycloheptylamino)methylene-bisphosphonic acid], piridronate, aminohexanebisphosphonate, tiludronate, BM-210955, CGP-42446, EB-1053)
   o risedronate (e.g., that which is commercially available as ACTONEL)
● kinase inhibitor
   o Rho-kinase inhibitor (e.g., (+)-trans-4-(1-aminoethyl)-1-(4-pyridylcarbamoyl)cyclohexane, trans-N-(1H-pyrrolo[2,3-b]pyridin-4-yl)-4-guanidinomethylcyclohexanecarbox amide, 1-(5-isoquinolinesulfonyl)homopiperazine, 1-(5-isoquinolinesulfonyl)-2-methylpiperazine)
● integrin receptor
   o α subunit (e.g., subtype 1-9, D, M, L, X, V, IIb, IELb)
   o β subunit (e.g., subtype 1-8)
● integrin receptor antagonists
   o ethyl 3(S)-(2,3-dihydro-benzofuran-6-yl)-3-{2-oxo-3-[3-(5,6,7,8-tetrahydro-[1,8] naphthyridin-2-yl)-propyl]-tetrahydro-pyrimidin-1-yl}-propionate;
   o ethyl 3(S)-(3-fluorophenyl)-3-(2-oxo-3(S or R)-[3-(5,6,7,8-tetrahydro-[1,8]naphthyridin-2-yl)-propyl]-piperidin-1-yl)-propio nate;
   o ethyl 3(S)-(3-fluorophenyl)-3-(2-oxo-3® or S)-[3-(5,6,7,8-tetrahydro-[1,8]naphthyridin-2-yl)-propyl]-piperidin-1-yl)-propio nate;
   o 3(S)-(2,3-dihydro-benzofuran-6-yl)-3-{2-oxo-3-[3-(5,6,7, 8-tetrahydro-[1,8]n aphthyridin-2-yl)-propyl]-tetrahydro-pyrimidin-1-yl}-propionic acid;
   o 3(S)-(3-fluorophenyl)-3-(2-oxo-3® or R)-[3-(5,6,7,8-tetrahydro-[1,8]naphthyridin-2-yl)-propyl]-piperidin-1-yl)-propio nic acid;
   o 3(S)-(3-fluorophenyl)-3-(2-oxo-3(S or S)-[3-(5,6,7,8-tetrahydro-[1,8]naphthyridin-2-yl)-propyl]-piperidin-1-yl)-propio nic acid
● anti-obesity agent
   o benzphetamine (e.g. that which is commercially available as DIDREX)
   o benzylisopropylamine (e.g. that which is commercially available as IONAMIN)
   o bupropion
   o dexfenfluramine (e.g. that which is commercially available as REDUX)
   o dextroamphetamine (e.g. that which is commercially available as DEXEDRINE)
   o diethylpropion (e.g. that which is commercially available as TENUATE)
   o dimethylphenethylamine (e.g. that which is commercially available as ADIPEX or DESOXYN)
   o evodamine
   o fenfluramine (e.g. that which is commercially available as PONDIMIN)
   o fluoxetine
   o mazindol (e.g. that which is commercially available as SANOREX or MAZANOR)
   o methamphetamine
   o naltrexone
   o orlistat (e.g. that which is commercially available as XENICAL)
   o phendimetrazine (e.g. that which is commercially available as BONTRIL or PLEGINE)
   o phentermine (e.g. that which is commercially available as FASTIN)
   o sibutramine (e.g. that which is commercially available as MERIDIA)
● a lipid-metabolism improving agent
   o capsaicin
● an neuropeptide Y blocker
   o NGD-95-1
● kainate/AMPA receptor antagonist
● β-adrenergic receptor agonist
● compound that reduces caloric intake
   o fat substitute (e.g., that which is commercially available as OLESTRA)
   o sugar substitute (e.g., that which is commercially available as ASPARTAME)
● anti-diabetes agent
   o insulin glargine (e.g. that which is commercially available as LANTUS)
   o pioglitazone (e.g. that which is commercially available as ACTOS)
   o rosiglitazone maleate (e.g. that which is commercially available as AVANDIA)
● dietary nutrient
   o sugar
   o dietary fatty acid
   o triglyceride
   o oligosaccharides (e.g., fructo-oligosaccharides, raffinose, galacto-oligosaccharides, xylo-oligosaccharides, beet sugar and soybean oligosaccharides)
   o protein
   o vitamin (e.g., vitamin D)
   o mineral (e.g., calcium, magnesium, phosphorus and iron)

The other therapeutic agents can be made and used at doses as disclosed previously. For example, an anti-osteoporosis agent (see e.g., U.S. Pat. Nos. 2,565,115 and 2,720,483), a non-steroid hormone (see, e.g., U.S. Pat. Nos. 6,121,253; 3,927,197; 6,124,314), a glucagon antagonists (see, e.g., U.S. Pat. No. 5,510,459), a growth hormone secretagogue (see, e.g., U.S. Pat. Nos. 3,239,345; 4,036,979; 4,411,890; 5,206,235; 5,283,241; 5,284,841; 5,310,737; 5,317,017; 5,374,721; 5,430,144; 5,434,261; 5,438,136; 5,494,919; 5,494,920; and 5,492,916; European Patent Nos. 144,230 and 513,974; International Patent Publication Nos. WO 89/07110; WO 89/07111; WO 93/04081; WO 94/07486; WO 94/08583; WO 94/11012; WO 94/13696; WO 94/19367; WO 95/03289; WO 95/03290; WO 95/09633; WO 95/11029; WO 95/12598; WO 95/13069; WO 95/14666; WO 95/16675; WO 95/16692; WO 95/17422; WO 95/17423; WO 95/34311; and WO 96/02530), an agent that increase IGF-1 levels (see, e.g., U.S. Pat. No. 6,166,077), a cytokine (see, e.g., U.S. Pat. No. 4,921,697), a vitronectin receptor antagonist (see e.g., U.S. Pat. No. 6,239,138 and Horton et al., 1991, Exp. Cell Res. 195:368), a bisphosphonate compound (see e.g., U.S. Pat. No. 5,409,911), a kinase inhibitor (U.S. Pat. No. 6,218,410), and an integrin receptor or antagonist thereof (see, e.g., U.S. Pat. No. 6,211,191).

### EXAMPLES

### Example 1: Synthesis of Steviol glucuronide.

Steviolglucuronide was prepared by nucleophilic substitution of the α-ethoxyethyl 2,3,4-tri-*O*-(α-ethoxyethyl)-1-*O*-mesyl-D-glucopyranuronate with the tetrabutylammonium salt of steviol. Removal of the acetal protecting groups by mild acid treatment yielded steviolglucuronide. The α-ethoxyethyl 2,3,4-tri-*O*-(α-ethoxyethyl)-1-*O*-mesyl-D-glucopyranuronate was prepared from glucuronolactone via a seven-step reaction procedure.

### α-ethoxyethyl 2,3,4-tri-O-(α-ethoxyethyl)-1-O-mesyl-D-glucopyranuronate synthesis from glucuronolactone via a seven-step reaction procedure

### Step 1

Glucuronolactone (0.1 mole) was added to 100 ml methanol containing 0.15 g of sodium methoxide. After 1h of stirring at room temperature the methanol was removed under reduced pressure. The residue was dissolved in acetic anhydride and 10 ml perchloric acid (3% in acetic anhydride) was added drop wise so that the reaction temperature never exceeded 40°C. After 15h at room temperature, 0.1 ml of perchloric acid was added and the mixture was stored for 15 hours at 4°C after which crystallization of methyl tetra-O-acetyl-β-D-glucopyranuronate occurred.

### Step 1a

Glucuronolactone (100g) was dissolved in 750 ml MeOH containing 0.3 g sodium hydroxide. After 1 hour at room temperature the MeOH was removed under reduced pressure. The residue was placed on ice and pyridine (250ml) and acetic anhydride (375ml) was added. After 15 hours at 0°C, methyl tetra-O-acetyl-b-D-glucopyranuronate crystallized from the mixture.

### Step 2

Methyl tetra-O-acetyl-β-D-glucopyranuronate (50g) was dissolved in 200 ml 30% hydrobromic acid in acetic acid. The solution was kept at 4°C for 15h after which the solvent was removed under reduced pressure. The residue was dissolved in 100ml chloroform, which was extracted with cold saturated aqueous sodium bicarbonate and water. After drying the chloroform layer using sodium sulphate, the solvent was removed under reduced pressure. The residue was dissolved in 150 ml of absolute ethanol. After further cleanup (carbon treatment, filtering and crystallization) Methyl (tri-O-acetyl-β-D-glucopyranosyl Bromide)-uronate was obtained.

### Step 3

To a solution of Methyl (tri-O-acetyl-β-D-glucopyranosyl Bromide)-uronate (8g) in benzyl alcohol (30ml) were added: molecular sieve (5g) and silver carbonate (8g), and the mixture was stirred in the dark for 6h after which dichloromethane (200ml) was added. The solution was filtered and concentrated and benzyl alcohol was removed in vacuum. Further purification of the residue using silica gel elution chromatography and crystallisation yielded methyl (benzyl 2,3,4-tri-O-acetyl-β-D-glucopyranosid)uronate.

### Step 4

A solution of methyl (benzyl 2,3,4-tri-O-acetyl-β-D-glucopyranosid)uronate (500mg) in methanol (30ml), triethylamine (15ml) and water (15ml) was stirred at 0°C until dissolution was complete. After 48h at room temperature the solution was concentrated in vacuum. The residue was dissolved in water and passed on a Dowex-50 (H+) resin, yielding Benzyl β-D-glucopyranosiduronic acid.

### Step 5

To a solution of Benzyl β-D-glucopyranosiduronic acid (0.31g) in dry tetrahydrofuran (30ml) at 0°C were added: vinyl ether (5 ml) and trifluoroacetic acid (0.3ml). The mixture was kept 3 days at 0°C and then 3h at 22°C. Triethylamine (5ml), dichloromethane (50ml) and water (50ml) were added in sequence, and the organic layer was washed with water (4 x 50 ml), dried over molecular sieve, and concentrated to yield α-ethoxyethyl 2,3,4-tri-*O*-(α-ethoxyethyl)-1-*O*-benzyl-αβ-D-glucopyran uronate.

### Step 6

α-ethoxyethyl 2,3,4-tri-*O*-(α-ethoxyethyl)-1-*O*-benzyl-αβ-D-glucopyran uronate was dissolved in ethylacetate (30 ml); triethylamine (0.2 ml) and 10% palladium-on-carbon (500 mg) were added, and the mixture was shaken with hydrogen (2atm) at 22°C for 6 hours. The solution was filtered and concentrated in vacuum yielding α-ethoxyethyl 2,3,4-tri-*O*-(α-ethoxyethyl)-1-*O*-D-glucopyranuronate.

### Step 7

α-ethoxyethyl 2,3,4-tri-*O*-(α-ethoxyethyl)-1-*O*-D-glucopyran uronate (30mg) was dissolved in dichloromethane (5ml) and s-collidine (0.3ml) and cooled to 0°C. After adding methanesulphonyl chloride (50µl) the mixture was stirred for 10 min at 0°C. The organic layer was shaken with a saturated NaHCO₃/Na₂CO3 buffer solution. The dichloromethane layer was washed with water (3 x 5ml) and dried over molecular sieve (0.4 nm pore diameter) for 2 h. The dichloromethane fraction contained α-ethoxyethyl 2,3,4-tri-*O*-(α-ethoxyethyl)-1-*O*-mesyl-D-glucopyran uronate, which was used to convert steviol into steviolglucuronide.

### Steviolglucuronide synthesis by nucleophilic substitution of the α-ethoxyethyl 2,3,4-tri-O-(α-ethoxyethyl)-1-O-mesyl-D-glucopyranuronate with the tetrabutylammonium salt of steviol followed by removal of protecting groups.

Steviol was converted into the anion by the addition of 0.1M tetrabutylammonium hydroxide (0.2ml) and dichloromethane (1 ml). The solution was evaporated to dryness and the residue was dissolved in dichloromethane (5ml) containing α-ethoxyethyl 2,3,4-tri-*O*-(α-ethoxyethyl)-1-*O*-mesyl-D-glucopyranuronate. Molecular sieve was added and after 1 day at 22°C, the reaction mixture was evaporated in vacuum. The residue was hydrolyzed with acetic acid (1ml) and aqueous 0.1M HCl (0.25ml) at 22°C for 30min, followed by evaporation in vacuum. Further purification of the residue using preparative TLC and silica gel elution chromatography yielded steviolglucuronide.

### METHOD II

Glucuronolactone (100g) was dissolved in 750 ml MeOH containing 0.3 g sodium hydroxide. After 1 hour at room temperature, the MeOH was removed under reduced pressure. The residue was placed on ice and pyridine (250ml) and acetic anhydride (375ml) was added. After 15 hours at 0°C, methyl tetra-O-acetyl-β-D-glucopyranuronate crystallized from the mixture.

Methyl tetra-*O*-acetyl-β-D-glucopyran uronate (50g) was dissolved in 200 ml 30% hydrobromic acid in acetic acid. The solution was kept at 4°C for 15h after which the solvent was removed under reduced pressure. The residue was dissolved in 100ml chloroform, which was extracted with cold saturated aqueous sodium bicarbonate and water. After drying the chloroform layer using sodium sulphate, the solvent was removed under reduced pressure. The residue was dissolved in 150 ml of absolute ethanol. After further clean up (active carbon treatment, filtering and crystallization) Methyl (tri-O-acetyl-β-D-glucopyranosyl Bromide)-uronate was obtained.

Methyl (tri-O-acetyl-β-D-glucopyranosyl Bromide)-uronate (1 mmol) and silver carbonate (1.7mmol) and 3,4-dimethoxybenzyl alcohol were mixed for 72 hours at room temperature. Methanol was added to the reaction mixture to obtain Methyl triO-acetyl-1-O-dimethoxybenzyl-D-glucopyran uronate crystals.

Methyl tri-O-acetyl-1-O-dimethoxybenzyl-D-glucopyran uronate (1 mmol) was dissolved in MeOH and MeONa (0.1 mmol) was added. The mixture was placed at room temperature for 3 hours. The solvents were removed under reduced pressure and the residue was dissolved in pyridine. Allyl chloroformate (25 mmol) was added dropwise while avoiding a rise of the reaction temperature never exceeding 25°C. The mixture was placed at room temperature for 60 hours. Solvents were removed under reduced pressure to obtain Methyl tri-O-allylocarbonyl-1-O-dimethoxybenzyl-D-glucopyran uronate.

Methyl tri-O-allylocarbonyl-1-O-dimethoxybenzyl-D-glucopyran uronate (1mmol) was dissolved in tetrahydrofuran: water (1:1) and NaOH (1mmol;0.1N) was added.

The mixture was kept at room temperature for 18 hours. Treatment with 1-chloro-N, N,2-trimethylpropenylamine (1mmol) for 1 hour at room temperature and reaction at room temperature for 24 hours with benzylic alcohol (1.2mmol) and pyridine (1.2mmol) in dichloromethane gave Benzyl tri-O-allylocarbonyl-1-O-dimethoxy benzyl-D-glucopyran uronate. Deprotection of the anomeric centre was accomplished using DDQ (2.6mmol) in dichloromethane: water (10:1) at room temperature for 24 hours to give Benzyl tri-O-allylocarbonyl-β-D-glucopyran uronate.

Benzyl tri-O-allylocarbonyl-β-D-glucopyran uronate (1mmol) was coupled sterioselectively with steviol (1mmol) in the presence of triphenylphosphine (1.5mmol) and diisopropyl azodicarboxylate (1.5mmol) in tetrahydrofuran. Removal of the protecting allylocarbonyl groups was accomplished using Pd(PPh3)4 (0.2 mmol), PPh3 (1.3mmol) and acetylacetone (15 mmol) in tetrahydrofuran . The mixture was kept at room temperature for 6 hours. Benzyl 1-O-steviol-D-glucopyran uronate was obtained by crystallization or preparative HPLC.

Benzyl 1-O-steviol-D-glucopyran uronate was dissolved in AcOEt:MeOH (20:1) and, using hydrogen and Pd on charcoal as catalyst, the cleavage of the benzylic ester gave steviolglucuronide.

### EXAMPLE 2: Preparation of salts of steviol-19-glucuronide

### Preparation of hydrobromide salts of steviol-19-glucuronide (steviol-19-glucuronide.HBr)

Steviol-19-glucuronide.2H20 (5.35 gram) is dissolved in 11.8 ml Methanol and consequently cooled to -15°C. 1.24 ml HBr (48% in water) is diluted in 0.91 Methanol and consequently cooled to -15°C and slowly added to the solution of Steviol-19-glucuronide. This solution is stirred for 5 minutes before 107 ml 2-propanol (-15°C) is added. After product precipitation the slurry is stirred for 3 - 4 hours at -20°C. Later the crystals are filtered off. They are consequently washed with 40.2 ml cold 2-propanol (-20°C) and dried at room temperature in a high vacuum.

### Preparation of steviol-19-glucuronide.H2SO4

Steviol-19-glucuronide.2H2O (5.4 gram) is dissolved in 11.8 ml Methanol and consequently cooled to -15°C. 0.37 ml H2SO4 (96%) is diluted in 0.91 Methanol and consequently cooled to -15°C and slowly added to the solution of Steviol-19-glucuronide. This solution is stirred for 5 minutes before 100 ml 2-propanol (-15°C) is added. After product precipitation the slurry is stirred for 3 - 4 hours at -20°C. Later the crystals are filtered off. They are consequently washed with 40.2 ml cold 2-propanol (-20°C) and dried at room temperature in a high vacuum.

### EXAMPLE 2: Dosage form.

Capsules containing 100 mg of a diterpenoic tetrahydropyran compound of present invention are usually prepared in one of the following composition:

| | |
|---|---|
| Active ingredient | 100 mg |
| Avicel | 200 mg |
| PVPPXL | 15 mg |
| Aerosil | 2 mg |
| Magnesium stearate | 1.5 mg |
| | 318.5 mg |

The capsules are prepared by mixing the components and filing the mixtures into hard gelatin capsules, size 1.

### EXAMPLE 3: Characterisation and Purification of Steviol Glucuronide in Human Urine.

**Chemicals**. A commercial mixture of steviol glycosides was crystallised repeatedly from MeOH affording stevioside (19-O-β-glucopyranosyl-13-O(β-glucopyranosyl(1-2))-β-glucopyranosyl-steviol) in over 97 % purity; impurities were steviolbioside 2.8 % and a trace of rebaudioside A. Steviol was made according to (Ogawa, T.; et al. Tetrahedron 1980, 36, 2641-2648) and repeatedly crystallized from MeOH to a purity of more than 99%. Solvents of HPLC grade were from: Acros (H₂O, acetonitrile, CHCl₃), BDH (MeOH, EtOH, *N,N*-dimethylformamide), Biosolve (acetone). Triethylamine was from Acros and 4-(bromomethyl)-7-methoxycoumarine (IUPAC name: 4-(bromomethyl)-7-methoxy-2*H*-chromen-2-one) was from Fluka. β-glucuronidase/sulfatase type H-2 from *Helix pomatia* digestive juice was from Sigma.

**Urine Fractionation and Derivatisation of steviol containing fractions.** The total 24 h urine fraction (between 1124 and 2494 mL) form human subject subjected to a steviol treatment (three days of oral stevioside administration (750 mg per day)) was run over an Amberlite XAD-2 column at about 30 mL/min. The column bed volume was 200 mL which is sufficient for the adsorption of all amphipathic molecules in 24 h urine. The columns were then rinsed with 1 L of distilled water and then eluted with 400 mL MeOH:Acetone (50:50, v/v). The eluate was divided into 4 equal fractions and the solvent was evaporated at reduced pressure at 50°C.

Fractions containing steviol and/or dihydroisosteviol used as internal standard were completely dried; the residues were taken up in dry acetone and derivatized to form the 7-methoxy-coumarinyl esters as described (Minne, V. et al. J. Agr. Food Chem., 2004, 52, 2445-2449). After derivatisation, the samples were purified by TLC using CHCl₃:MeOH (98:2) as the eluent. The blue fluorescent TLC band (UV 366 nm) corresponding with the ester derivative of steviol was scraped off and eluted with CHCl₃:MeOH (80:20). After evaporation of the solvent, the residue was dissolved in a known amount of MeOH and HPLC was done using a fluorescence detector (λexc.: 321 nm, λem.: 391 nm) (Minne, V. et al. J. Agr. Food Chem., 2004, 52, 2445-2449). The identity of the steviol 7-methoxy coumarinyl ester was checked by MS.

**Analysis of bound steviol.** In preliminary experiments, steviol possibly bound as glucuronide and sulphate conjugates, was obtained after hydrolysis by β-glucuronidase/sulfatase from *Helix pomatia* digestive juice. Urine fractions were dissolved in 10 mL MeOH:Acetone (50:50) and to samples of 250 *µ*L, 200 *µ*g of dihydroisosteviol were added as IS. The samples were then evaporated and the residues dissolved into 500 *µ*L acetate buffer, pH 5. Then 50 *µ*L β-glucuronidase/sulfatase were added (5000 U and 375 U respectively). The mixture was incubated for 6 h at 37°C. After hydrolysis the samples were purified on C 18 cartridges (500 *µ*L bed volume, Alltech Belgium) that were conditioned before use with 3 mL MeOH followed by 3 mL H₂O. After application of the enzyme mixtures, the columns were rinsed with 3 mL water and 3 mL 50 % MeOH. The steviol and IS were eluted with 5 mL MeOH. 250 *µ*L of the latter fraction were evaporated under a stream of nitrogen at 50°C. The completely dried residue was then derivatized as described above.

**Isolation of larger amounts of steviol glucuronide**. A column (35 mL bed volume) was prepared with 50 g of silica gel for column chromatography (MN-Silicagel 60, 0.063-0.2 mm) suspended in ethyl acetate. About 600 mg of urine residue isolated from the Amberlite XAD-2 purification step (see above) was dissolved in 1 mL of MeOH and this solution was adsorbed to 1 g of silicagel by evaporating the solvent under a gentle flow of N₂ and under continuous mixing. The sample bound to the silicagel was applied onto the top of the column, which was eluted with a solvent mixture ethyl acetate:ethanol:water (80:30:20). Fractions of 5 mL were collected. Samples of each fraction were analysed by TLC using ethyl acetate:ethanol:water (80:30:20) as solvent and compared with steviol glucuronide as a reference compound. In addition, each fraction was tested for the occurrence of steviol glucuronide and/or sulphate conjugates. To this end, 20 *µ*L samples of each fraction were evaporated in an Eppendorf tube. Then 150 *µ*L of acetate buffer (pH 5) were added, followed by 20 *µ*L of β-glucuronidase/sulfatase. After enzymatic reaction for 15 h at 37°C, the reaction mixture was freeze-dried and the residue derivatized by reaction with 4-(bromomethyl)-7-methoxycoumarine (see above). All fractions containing enzyme-sensitive steviol conjugates eluted as a single large peak from the silica gel column, indicating the presence of only one steviol conjugate in the urine samples. Therefore, all the fractions containing this steviol conjugate were pooled and the solvent was evaporated. The residue was subjected to preparative TLC, and about 10 mg of white crystalline material was isolated following elution of the TLC band with MeOH.

**Melting point.** The melting point (uncorrected) was measured on a Reichert-Jung Thermovar 9200 apparatus of Electerothermal.

### Spectroscopic techniques.

**IR.** The IR spectrum was recorded in a KBr pellet (Fig. 1)

**MS.** Mass spectral analysis of steviol glucuronide was carried out using the LCQ Advantage ion trap mass spectrometer of Thermo Finnigan (San Jose, CA, USA) in both positive ESI (+) and negative ESI (-) electrospray ionisation modes. MS/MS spectra were generated by CID (collision-induced decomposition) of the [M + 2Na - H]⁺ adduct ion formed by ESI (+) and of the [M - H]⁻ molecular ion obtained by ESI (-).

**NMR.** ¹H and ¹³C NMR spectra of steviol glucuronide were recorded on a Bruker AMX 400 spectrometer equipped with an inverse ¹H-multinuclel probe and operating at 400.13 MHz for ¹H and 100.62 MHz for ¹³C measurements. The spectra were run in CD₃OD as a solvent. The chemical shifts are reported in ppm *versus* TMS (tetramethylsilane) as an internal reference.

In the ¹³C spectra, the septet of the CD₃OD signal was used as internal reference and placed at δ 49 *versus* TMS. Besides the fully decoupled ¹³C spectrum, the DEPT 135 pulse sequence was used to differentiate between C, CH, CH₂, and CH₃ signals.

Following metabolic conversion of ingested stevioside, steviol glucuronide was isolated as the only metabolite detected in the collected urine. The purified, crystalline material had a melting point of 198-199 °C (uncorrected). The UV spectrum showed a maximum absorbance at 208 nm (methylene). The IR spectrum (Fig. 1) displays bands corresponding to the hydroxyl functions at 3416 (broad) and 1057 (broad) cm⁻¹, to the ester carbonyl group at 1725 cm⁻¹, and to the carboxylic acid at 1615 cm⁻¹.

Electrospray ionisation spectra were run in both positive ESI (+) and negative ESI (-) modes. In the ESI (+) spectrum, the disodium adduct ion [M + 2Na - H]⁺ was the main peak observed at *m*/*z* 539. Further MS/MS analysis of this adduct ion revealed cleavage of the glycosidic ester linkage to form both the [C(19)O₂Na₂]⁺ disodium adduct ion of steviol (*m*/*z* 363), and the complementary disodium adduct of the glucuronic acid moiety (*m*/*z* 221).

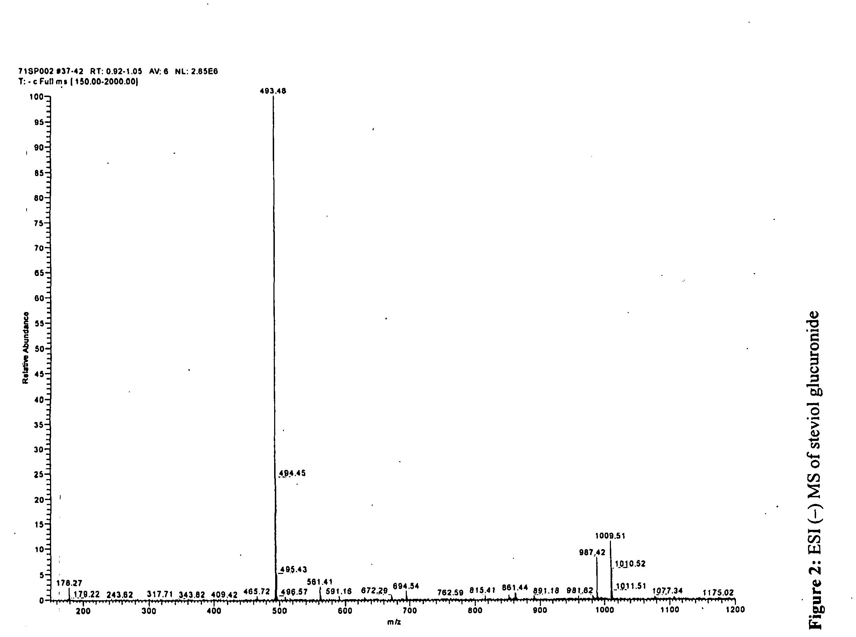

The ESI (-) spectrum (Fig. 2) displayed the molecular ion [M - H]⁻ at *m*/*z* 493 as the main ion species, corresponding to deprotonation of the glucuronic acid moiety. Other interesting ions were due to ion-molecule associations providing the dimer and trimer ion species [2M - H]⁻, [2M + Na - 2H]⁻, and [3M + 2Na - 3H]⁻, observed at *m*/*z* 987, 1009, and 1526 respectively. Separate MS/MS analysis of the [M - H]⁻ ion at *m*/*z* 493 led to cleavage of the glycosidic bond of the ester conjugate to produce two complementary ions at *m*/*z* 317 and *m*/*z* 175, representing the [C(19)O₂]⁻ carboxylate anion of steviol and that of the glucuronic acid part respectively.

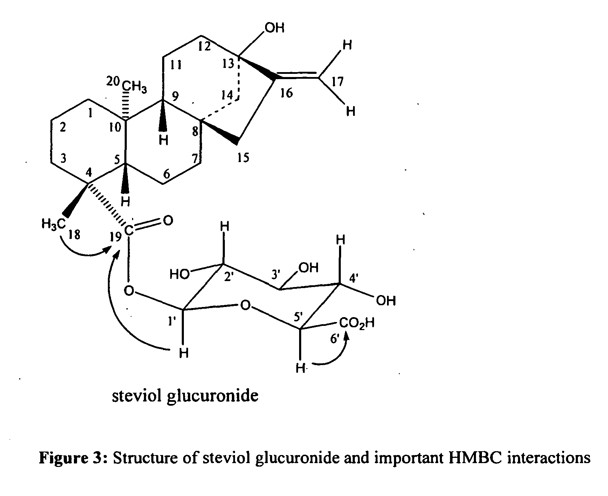

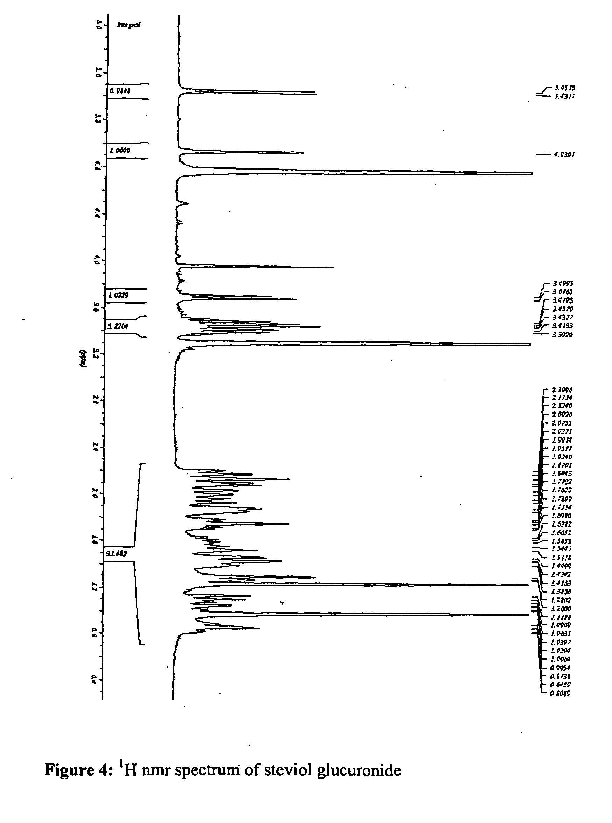

The 1-*O*-acyl ester glycoside structure (Fig. 3) proposed for steviol glucuronide is fully confirmed by the ¹H and ¹³C NMR spectra, in which relevant δ and J values can be discerned for the terpene and glucuronide moieties. The ¹H NMR spectrum (Fig. 4) reveals the two exocyclic vinylidene protons on C17 at δ 4.93 and 4.77 (obscured by the OH of methanol at 25 °C). The methylene and methine protons absorb as overlapping multiplets between δ 2.3 and 0.7. The C18 and C20 methyl groups display two sharp singlets at δ 1.24 and 0.97.

The anomeric proton of the glucuronide appears as a downfield doublet at δ 5.46 (³*J*_{H1'-H2'}= 7.8 Hz); this chemical shift value is consistent with a 1-*O*-acyl glycosidic ester linkage while the vicinal coupling indicates diaxial coupling of H1' and H2'. Proton H5' is observed as a doublet at δ 3.68 (³*J*_{H5'-H4'}= 9 Hz); the signals for H2', H3', H4' appear as a multiplet (3 H) between δ 3.5 and 3.35.

The ¹³C NMR spectrum of steviol glucuronide displays the following absorptions for the terpene moiety: two methyls, nine methylenes, two methines, and four quaternary carbon atoms, two vinylic carbons for the exocyclic C16-C17 double bond and an ester carbonyl carbon for C19. Two dimensional ¹H, ¹³C correlation spectroscopy via one bond ¹J_{CH} coupling (HMQC) or via two and three bonds coupling (HMBC) allowed unequivocal assignment of all C, CH, CH₃ and many of the CH₂ carbon atoms. The other CH₂ signals were assigned by comparison with the values for free steviol reported (Hutchison, M. et al.. J. Chem. Soc. Perkin Trans. 1 1984, 2363-2366). All ¹³C absorptions found for the terpene moiety are fully comparable with those reported for steviol (Table 1).

For the glucuronide part, five absorptions in the ¹³C spectrum are identified as CHO carbon atoms by their correlation with the corresponding protons via the 2D HMQC method. Finally, 2D HMBC correlated spectroscopy was applied to reveal diagnostic ²*J* and ³*J*_{CH} couplings. Thus, the ester glycoside linkage CO(19)→O(1') clearly appears from two ³*J*_{CH} correlations observed between C19 (δ 178) and (i) the C18 methyl group (δ 1.24) and (ii) the anomeric proton (δ 5.46). The free carboxyl group (COOD) of the glucuronide moiety at δ 176.6 is identified by its ²*J*_{CH} correlation with the H5' doublet at δ 3.68.

It has been demonstrated by Geuns et al that oral uptake by human volunteers of dosages of stevioside were around 10-15 mg/kg per day is excreted in urine as steviol glucuronide, a metabolite of stevioside. Due to its molecular size, the uptake of stevioside by the intestinal tract is expected to be extremely low, as suggested by experiments with everted gastrointestinal sacs of rats (Koyama, E. et al. Food and Chemical Toxicology, 2003, 41, 875-883) and Caco-2 cell layers (Geuns, J.M.C. et al. Food Chem. Toxicol., 2003, 41, 1599-1607). Moreover, stevioside is not degraded by the enzymes of the intestinal tract (Hutapea, A.M. et al. J. Clin. Biochem. Nutr. 1997, 23, 177-186, Koyama, E. et al. Food and Chemical Toxicology, 2003, 41, 359-374). However, stevioside is degraded by bacteria of the colon resulting in free steviol that is easily absorbed (Gardana, C. et al. J. Agric. Food Chem., 2003, 51, 6618-6622, Geuns, J.M.C. et al. Food Chem. Toxicol., 2003, 41, 1599-1607 and Koyama, E. et al. Food and Chemical Toxicology, 2003, 41, 875-883).

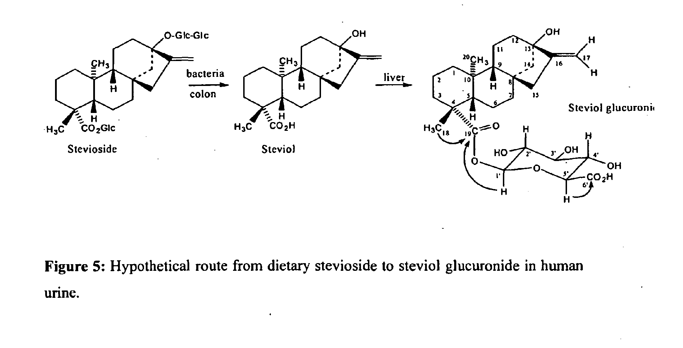

Since no free steviol can be detected in urine and after enzymatic hydrolysis of urine extracts by β-glucuronidase/sulfatase, steviol was found as the only aglycone present, and there was no indication for the occurrence of, e.g., steviol sulphates and from *in vitro* incubations of steviol with human liver microsomes, it was concluded that the transformation of steviol by human microsomes was very low and about 4 times lower than that by rat microsomes (Koyama, E. et al. Food and Chemical Toxicology, 2003, 41, 359-374) and no other metabolites were found, the following excretion route is suggested (Fig. 5): After degradation of stevioside to steviol by bacteria of the colon, part of the steviol is absorbed by the colon and transported to the liver by portal blood. In the liver, the steviol glucuronide is formed, which is released into the blood and filtered out by the kidneys into the urine. The huge amounts of steviol glucuronide in the urine suggest that there is no accumulation of steviol derivatives in the human body.

**Table 1. Assignment of signals in the ¹³C NMR spectra of steviol (18) and steviol glucuronide (this work); δ values all relative to internal SiMe₄**

| Steviol in CDCl₃ (*) | | | Steviol glucuronide in CD₃OD (this work) | |
|---|---|---|---|---|
| | | ¹³C | ¹³C | ¹H correlation |
| CH₃ | C18 | 28.8 | 29.0 | 1.24 (s) |
| | C20 | 15.4 | 16.4 | 0.97 (s) |
| CH₂ | C1 | 40.5 | 41.9 | ¹H of the methylene envelope from δ 2.3 to 0.7 |
| | C2 | 19.0 | 20.2 | |
| | C3 | 37.8 | 39.1 | |
| | C6 | 21.8 | 22.9 | |
| | C7 | 41.2 | 42.8 | |
| | C11 | 20.5 | 21.4 | |
| | C12 | 39.5 | 40.6 | |
| | C14 | 47.4 | 47.3 | |
| | C15 | 47.0 | 48.9 | |
| CH | C5 | 56.9 | 58.7 | 1.12 |
| | C9 | 53.8 | 55.4 | 0.99 |
| C | C4 | 43.6 | 45.1 | |
| | C8 | 41.8 | 42.8 | |
| | C10 | 39.5 | 40.7 | |
| | C13 | 80.4 | 80.8 | |
| sp² C | C16 | 155.7 | 157.1 | |
| | C17 | 103.0 | 103.3 | 4.97 and 4.77 (br. s) |
| | C19 | 183.5 | 178.0 | |
| Glucuronide of part steviol glucuronide | CH1' | | 95.4 | 5.46 (d) |
| | CH5' | | 77.4 | 3.68 (d) |
| | CH2', CH3', CH4' | | 78.6, 73.9, 73.5 | δ 3.5 to 3.35 (m) |
| | CO₂D | | 176.6 (br) | |

| | | | | |
|---|---|---|---|---|
| *** Hutchison, M. Et al. J. Chem. Soc. Perkin Trans. 1 1984, 2363-2366 | | | | |

### EXAMPLE 4: Effects of stevioside in obese, dyslipidemic and diabetic mice.

We measured the effect of stevioside on weight, blood variables (e.g. glucose, insulin, lipids), PPAR expression in adipose tissues, and on atherosclerosis in mice with combined leptin and LDL-receptor deficiency (double knockout [DKO] mice). We selected these mice because they exhibit most of the metabolic syndrome components (obesity, dyslipidemia, diabetes and hypertension) which are associated with increased oxidative stress and inflammation, and accelerated atherosclerosis and impaired heart function ( Mertens A, et al. Circulation 2003; 107(12): 1640-1646.; Verreth W, et al.Circulation 2004; 110(20):3259-3269; Verreth W, et al. Arterioscler Thromb Vasc Biol 2006; 26(4):922-928. 2006; Mackness B, et al. Arterioscler Thromb Vasc Biol 2006; Verreth et al. Br J Pharmacol. 2007 Jun;151(3):347-55. Epub 2007 Mar 26).

### Methods:

### Experimental Protocol

Homozygous LDL-receptor-knockout mice (LDLR^{(-/-)}), heterozygous ob/+, and C57BL6 mice were purchased from Jackson Laboratory, Bar Harbor, Me. LDLR^{(-/-)} mice werebackcrossed into a C57BL6 background to the 10th generation. To obtain DKO mice with combined leptin deficiency (ob/ob) and LDL-receptor deficiency, LDLR^{(-/-)} and ob/+ mice were crossed. All offspring were genotyped by polymerase chain reaction (PCR) techniques. All mice were housed at 22°C on a fixed 12/12-hour light/dark cycle. Experimental procedures in animals were performed in accordance with protocols approved by the Institutional Animal Care and Research Advisory Committee. All mice were fed standard chow containing 4% fat (Pavan Service). Food intake was ≈5.7 g/d. Mice (about 50g) were treated with stevioside (N=14; 10mg/kg), steviol (N=9; 5 mg/kg) or placebo (N=17) for 12 weeks between 12 and 24 weeks of age. Stevioside (Molecular weight: 804) was dissolved in water (1mg/ml) and 500 µl of the solution was administered orally. Steviol (Molecular weight: 318) was dissolved in water containing 6% NaHCO3 (1mg/ml) and 250 µl of the solution was administered orally.

### Real-Time RT-PCR and Microarray Analysis

The level of RNA expression in extracts of white visceral (intra-abdominal) adipose tissue and aortic arch was measured by real-time reverse transcription (RT)-PCR. Total RNA was extracted with the Trizol reagent (InVitrogen) and purified on an RNeasy kit column (Qiagen). First-strand cDNA was generated from total RNA by RT using random primers from Takara and Superscript III reverse transcriptase (InVitrogen). Quantitative real-time PCR was performed using SybrGreen master mix according to the supplier protocols (Applied Biosystems). ). Oligonucleotides (In Vitrogen) used as forward primer (F) and reverse primer (R) were: for Oligonucleotides (In Vitrogen) used as forward primer (F) and reverse primer (R) were: for mouse PPARalpha: *F: 5'-TCAGGGTACCACTACGGAGTTCA-3' ; R: 5 '-CCGAATAGTTCGCCGAAAGA-3';* for mouse PPARgamma: *F*: *5'-GCAGCTACTGCATGTGATCAAGA-3 '; R: 5 '-GTCAGCGGGTGGGACTTTC-3'*; for mouse FABP4: *F:5'-AACTGGGCGTGGAATTCG-3'; R: 5'-CTAGGGTTATGATGCTCTTCACCTT-3';* for mouse iNOS: *F*: *5'-GCCCATGTACCAACCATTGAA-3'; R: 5'-TCCCAAGTACGAGTGGTTCCA-3';* for mouse GLUT-4: *F*: *5'-CACTGCTTCTGGCTCTCACAGTAC-3'; R: 5'-GTTCCGGATGATGTAGAGGTATCTG-3';* for mouse IRS-1: *F: 5'-AGCCCAGTGAGTCTGTCATCTAGTAG-3'; R: 5'- CGCCTCGGGAAGAGACAGT-3';* for mouse IRS-2: *F*: *5 '- CGAAGTACTCGTCCTTGGTGTAGA-3': R: 5'-CGAGAAGAAGTGGAGGAGCAA-3';* for mouse LXRalpha: *F*: *5'-GGAGTGTCGACTTCGCAAATG-3 '; R: 5'-TCAAGCGGATCTGTTCTTCTGA-3';* for mouse SOD1: *F*: *5'-GGGATTGCGCAGTAAACATTC-3*'; *R: 5'-AATGGTTTGAGGGTAGCAGATGA-3*'; for mouse SOD2: *F:* 5'-*TCGGTGGCGTTGAGATTGT; R: 5'-ACACATTAACGCGCAGATCATG-3';* for mouse SOD3: *F: 5'-CATGCAATCTGCAGGGTACAAC-3'; R: 5'-GCTGCCGGAAGAGAACCAA-3';* and for mouse ß-actin (housekeeping gene), *F*: *5'-ACGGCCAGGTCATCACTATTG-3'; R: 5'-CACAGGATTCCATACCCAAGAAG-3'.* The level of mRNA expression was calculated using the threshold cycle (Cₜ) value, i.e., the number of PCR cycles at which the fluorescent signal during the PCR reaches a fixed threshold. For each sample, the Cₜ both for the gene of interest and for the housekeeping gene ß-actin were determined to calculate ΔC_{t,sample} (Cₜ,target gene-C_{t,housekeeping gene}), thus normalizing the data and correcting for differences in amount and/or quality between the different RNA samples. The expression levels were related to an external calibrator consisting of intra-abdominal adipose tissue from C57BL6 control mice. Subsequently, ΔΔCₜ (ΔC_{t,sample}-ΔC_{t,calibrator}) was determined, and the relative expression levels were calculated from 2⁻ΔΔ^{Ct} according to the manufacturer's instructions (Applied Biosystems). RNA expression levels are thus indicated as arbitrary units ±SD

### Biochemical Analyses

Blood of conscious mice was collected by tail bleeding into EDTA tubes after an overnight fast. Plasma was obtained by centrifugation. Plasma cholesterol and triglycerides were measured by means of standard enzymatic colorimetric assays (Boehringer Mannheim), plasma fatty acids (FFA) by means of the FFA-Half Microtest kit (Roche Applied Science), glucose by means of a glucometer (Menarini Diagnostics), plasma insulin and adiponectin by means ELISAs (Mercodia and R&D, respectively). Insulin resistance was calculated by a homeostasis model assessment (HOMA)=fasting serum insulin (mU/L) times fasting blood glucose (mmol/L)/22.5.

### Atherosclerosis

The extent of atherosclerosis was determined by analysis of cross sections from the aortic root. Approximately ten 7-µm frozen sections per staining per animal were used for morphometric and immunohistochemical analysis. Total plaque areas were measured on hematoxylin and eosin stained sections. Lipids were stained with oil-red O, and macrophage areas were stained with an antibody against mouse Mac-3 antigen (Pharmingen), and oxidized LDL with the monoclonal antibody 4E6. Blinded analysis of positive immunostained sections was performed with the Quantimet600 image analyzer (Leica).

### Results:

### Weight

During the treatment period, the weight of stevioside-treated mice increased with 41% compared with 43% in placebo-treated mice.

### Blood analyses

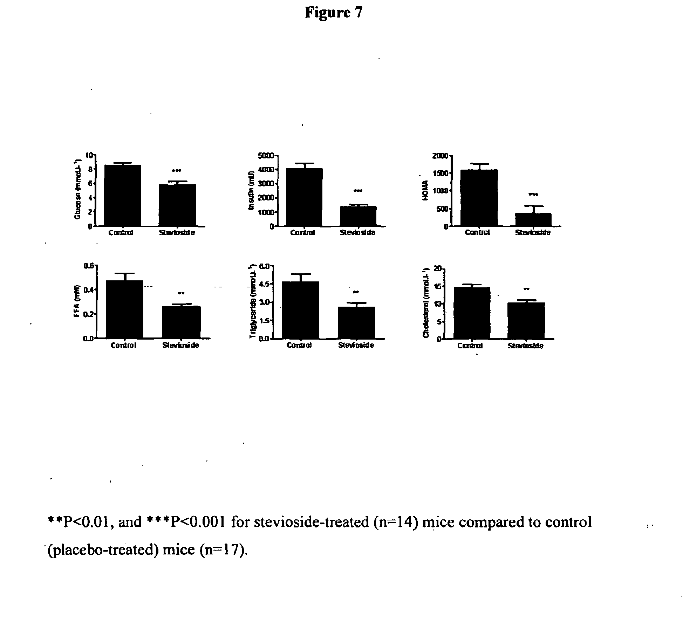

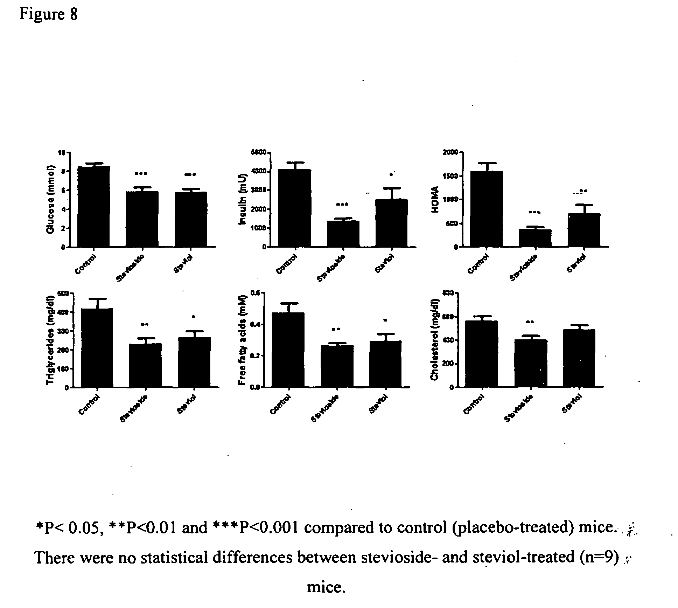

Compared to baseline levels, stevioside reduced glucose, insulin, FFA, triglycerides and cholesterol. The decrease in glucose and insulin resulted in an increase of insulin sensitivity. Figure 7 shows that glucose, insulin, FFA, triglyceride and cholesterol levels and the HOMA values were significantly lower in stevioside-treated than in placebo mice. Figure 8 shows that steviol had similar effects as did stevioside on blood levels of glucose, insulin, FFA, triglyceride and cholesterol levels, and on HOMA values. Interestingly, plasma concentrations of adiponectin increased from 4729±1652 to 6517±1472 ng/ml (P=0.003) indicating improved adipocyte differentiation.

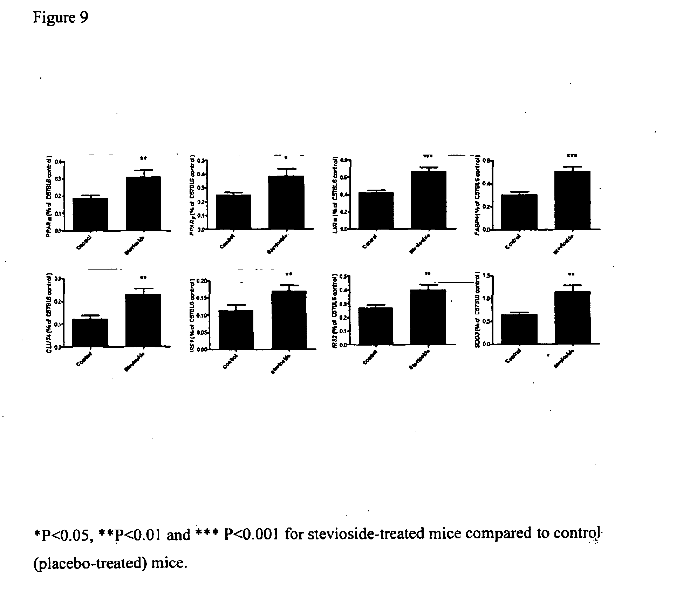

We performed quantitative RT-PCR analysis on extracts of white visceral adipose tissue to determine the underlying molecular mechanisms. We observed increased expression of PPARalpha, PPARgamma and LXRalpha in the adipose tissues of stevioside-treated mice (Figure 9). These changes were associated with increased expression of FABP-4 and GLUT-4 indicating improved adipocyte differentiation that is associated with better fatty acid handling, and glucose uptake. We also have observed an increased expression of the insulin receptor substrates IRS-1 and IRS-2 indicating improved adipocyte differentiation and increased insulin signalling. The latter was associated with increased expression of the antioxidant SOD3 (Figure 9).

### Atherosclerosis

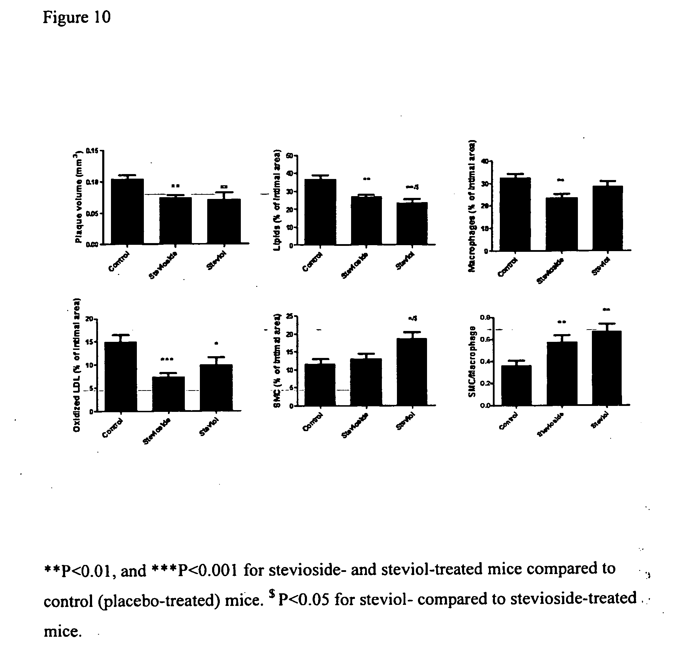

Stevioside and steviol lowered plaque volume by reducing macrophage, lipid and oxLDL deposition (Fig. 10). They also increased the smooth muscle cell (SMC)-to-macrophage ratio that in mice is considered as a proxy for plaque stability (Fig.10). Compared to stevioside, steviol decreased somewhat more lipids and increased somewhat more the SMC content.

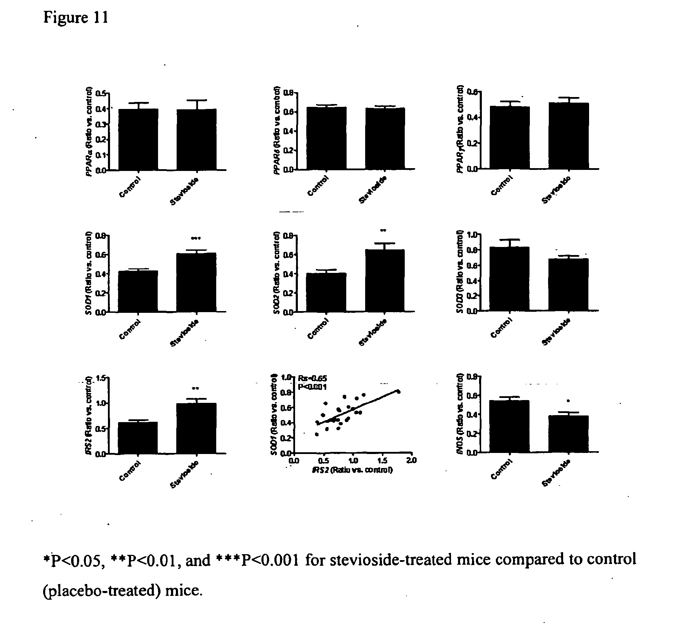

In contrast to observed changes in PPAR expression in adipose tissue, stevioside did not increase the expression of PPARalpha and PPARgamma in the aortic arch. However, the expressions of SOD1 and SOD2 were increased. In addition, the expression of IRS2 was higher (Fig. 11). The association between improved insulin signaling and antioxidant capacity was supported by the significant correlation between IRS2 and SOD1 expression. Interestingly, these changes were also associated with decreased expression of the inflammatory iNOS (Fig. 11).

### Discussion:

In normal physiology, adipose tissue stores energy in the form of triglycerides that can be broken down (lipolysis) to release FFA. In obese individuals, adipose tissue is characterized by hypertrophic adipocytes that are less responsive to insulin and have a higher basal rate of fatty-acid release. This leads to increased fatty acid concentrations in the serum and ectopic fat accumulation in nonadipose tissues such as liver and muscle and heart. Whole-body insulin sensitivity correlates with myocellular lipid content and FFA concentrations. Hence, an increased release of FFA by adipose tissue will lower insulin signalling in other tissues including liver, muscle and heart illustrating the deleterious metabolic consequences of nonadipose lipid storage (Boden G, et al. Journal of Clinical Investigation 1994; 93:2438-2446). In 1994, Spiegelman and colleagues discovered that expression and activation of PPARgamma was sufficient to induce adipogenesis (Tontonoz Pet al. Cell 1994;79:1147-1156). The essential role of PPARgamma in adipogenesis has been clearly demonstrated in functional and genetic knockdown experiments (Barak Y et al. Molec Cell 1999;4:585-595; Rosen ED et al. Molec Cell 1999;4:611-617). In addition to its importance in adipogenesis, PPARgamma plays an important role in regulating lipid metabolism in mature adipocytes. Target genes directly regulated by PPARgamma which are involved in this pathway include lipoprotein lipase (LPL) (Schoonjans K et al. EMBO J 1996;15:5336-5348) and fatty-acid transport protein (FABP) (Frohnert BI et al. Journal Biological Chemistry 1999; 274:3970-3979) which favour adipocyte uptake of circulating FFA. Although increased fat storage would be expected to boost the size of adipocytes, treatment with PPARgamma agonists actually leads to smaller adipocytes (Okuno A et al. Journal of Clinical Investigation 1998; 101:1354-1361). This is partly due to increased adipocyte differentiation induced by PPARgamma, leading to new smaller cells. Together with its coactivator PPARgamma-coactivator 1a (PGC-1α), PPARgamma promotes mitochondrial biogenesis (Wilson-Fritch L et al. Journal of Clinical Investigation 2004; 114: 1281-1289), leading to an increase in fatty acid oxidation that further protects against adipocyte hypertrophy.

As indicated above, pharmacological PPARgamma activation stimulates adipocyte differentiation that is associated with alleviation of insulin resistance, because of decreases in FFA and upregulation of adiponectin. They also improve glucose uptake in insulin-resistant tissues via an increase in the glucose transporter GLUT-4. http://circ.ahajournals.org/cgi/content/full/110/20/3259 - R23-156749#R23-156749 Analysis of gene expression of adipocytes of insulin-resistant patients showed a decreased expression of PPARgamma, GLUT-4, the fatty acid-binding protein (FABP-4), and LPL. http://circ.ahajournals.org/cgi/content/full/110/20/3259 - R25-156749#R25-156749http://circ.ahajournals.org/cgi/content/full/110/20/3259 - R26-156749#R26-156749 In here, we showed PPARgamma expression in adipose tissue is low compared to that in adipose tissue of lean C57BL6 mice and that stevioside increases *in vivo* PPARgamma expression in the adipose tissue. Stevioside-induced PPARgamma activation is associated with increased expression of FABP-4 and GLUT-4 that is associated with a decrease of fatty acids and improved glucose uptake by adipocytes. The GLUT-4 promoter is a direct transcriptional target for the LXR/retinoid X receptor heterodimer and LXR ligands induce GLUT-4 expression (Laffitte BA et al. Proc Natl Acad Sci USA 2003; 29:5419-5424). Thus, the induction of LXRalpha in adipose tissue is in agreement with increased GLUT-4 expression and glucose transport and glucose tolerance. Increased PPAR expression was also associated with increased expression IRS1 and IRS2 in adipose tissue of stevioside-treated mice. The insulin receptor substrates (IRS) are a family of proteins which are phosphorylated by the activation of insulin. Although the four IRS proteins have similar structures, disruption of each individual IRS gene causes distinct phenotypes in mice indicating that the four IRS proteins play different roles in the regulation of pleiotropic effects of insulin. It has been found that IRS-1 KO impairs the differentiation of preadipocytes into adipocytes (Fasshauer M et al. Mol Cell Biol 2001:319-329), whereas IRS-2 KO preadipocytes can differentiate into mature adipocytes but have defects in insulin-induced glucose uptake (Fasshauer M et al. Journal of Biological Chemistry 2000; 275:25494-25501). Thus, the observed increased expression of IRS-1 and IRS-2 in the adipose tissue of stevioside-treated mice is agreement with both improved adipocyte maturation and improved insulin signalling and glucose uptake.

It has been demonstrated that impaired insulin signalling in adipose tissue is associated with obesity-induced oxidative stress associated with increased reactive oxygen species (Park J et al. Diabetes 2006; 55:2939-2949). We have found that increased insulin sensitivity in stevioside-treated mice is associated with increased expression of the antioxidant enzyme SOD3 that is protective against the oxidation of LDL.

In humans, pharmacological PPARalpha activation with fibrates decreases plasma triglycerides by increasing fatty acid uptake and catabolism, resulting in limited triglyceride and VLDL production by the liver. PPARalpha activation is associated with increased expression of lipoprotein lipase (LPL) that catalyzes the hydrolysis of triglycerides and modulates the binding of triglyceride-rich VLDL particles to the VLDL receptor. Conversely, LPL-induced lipolysis of triglyceride-rich lipoproteins generates PPARalpha ligands, providing a link between lipoprotein metabolism and distal PPAR transcriptional effects. Lipid metabolism plays an important role in glucose homeostasis. A reduction in circulating or intracellular lipids by activation of PPARalpha improves insulin sensitivity and the diabetic condition. In here, we showed that PPARalpha expression in adipose tissue of DKO mice is low compared to that in the adipose tissue of lean C57BL6 mice and that stevioside increases *in vivo* PPARalpha expression in the adipose tissue. The increased expression of PPARalpha in the adipose tissue of stevioside-treated mice was, however, not associated with a change in LPL expression. However, this does not mean that the observed increase of PPARalpha in adipose tissue of stevioside-treated mice is not important. Indeed we have found that overexpression of PPARalpha in the absence of overexpression of PPARgamma in adipose tissue of DKO mice lowered FFA levels by increasing FABP-4 and glucose uptake by increasing GLUT-4 (Verreth W et al. Arteriosclerosis Thrombosis and Vascular Biology 2006;29:922-928).

In aggregate, we present a novel effect of stevioside on PPAR expression in adipose tissue associated with improved adipocyte differentiation, fatty acid homeostasis, glucose uptake, insulin signalling and protection against oxidative stress in adipose tissue.

The decrease in blood lipids was associated with a decrease in plaque lipids that was associated with a decrease in macrophages. The decrease in plaque macrophages and plaque lipids resulted in a significant reduction of the overall plaque area. Previously, we have shown a decreased PPARalpha and PPARgamma expression in the aortic arch of DKO mice that was associated with increased oxidative stress, inflammation, macrophage inflammation and atherosclerosis. An increase in PPAR expression was associated with decreased oxidative stress, inflammation, macrophage inflammation and atherosclerosis. In here, we have not found an increase in the expressions of PPARs in the aortic arch of stevioside-treated mice. We cannot exclude, however, that the activity of PPARs was increased. Indeed, as in the adipose tissue, the expression of IRS2, which is regulated by PPARs, was increased indicating improved insulin signalling in the vessel wall. As in the adipose tissue, improved insulin signalling is associated with increased expression of antioxidant enzymes SOD1 and SOD2 that is associated with an inhibition of the accumulation of oxidized LDL, a chemoattractant for monocytes. Therefore, an inhibition of the oxidized LDL deposit in the vessel wall will result in decreased monocyte/macrophage infiltration and lipid accumulation in foam cells, and thereby prevent atherosclerosis. Important is that both in the adipose tissue and in the vessel wall we demonstrated a similar relation between improved insulin signalling and increased antioxidant defence. Previouly, we demonstrated that the expression of SOD depend on PPAR activity (Desjardins F et al. Eur Heart J 2008; 29:128-137). We have especially found a relation between IRS2 and SOD1 expression. There are, however, at least two non-exclusive mechanisms by which stevioside could increase SOD production independent of PPARs. First, we have demonstrated that stevioside treatment was associated with an increase of plasma concentrations of adiponectin that result from improved adipocyte differentiation (Verreth W et al. Circulation 2004;110:3259-3269). Recently, adiponectin was found to be positively related to SOD expression in diabetic patients because it decreases the expression of the inflammatory TNFalpha (Adachi T et al. J Endocrinol 2004; 181:413-417). Interestingly, we have decreased expression of iNOS in the aorta of stevioside-treated mice. iNOS generally produces much higher quantities of NO than does eNOS, that was not affected by stevioside, and together with reactive oxygen species (ROS) in oxidized LDL plays a role in cellular damage and inflammation (Luoma JS et al. Arteriosclerosis, Thrombosis and Vascular Biology 1998; 18:157-167). iNOS expression is regulated by TNFalpha (Juan CC et al. Obesity 2008 (Epub ahead of pub). The second possible mechanism starts with the decrease of ROS resulting from increased SOD expression. Previously, ROS were found to decrease IRS-1 expression (Taniyama Y et al. Arteriosclerosis, Thrombosis and Vascular Biology 2005; 25:1142-1147; HitomiH et al. Hypertension 2007; 50:750-755). Thus, we can expect that the decrease in ROS was associated with increased IRS expression. The latter can be associated with increased FOXO expression that induces SOD expression (Bartke A. Aging cell 2008 Epub ahead of print).

In aggregate, this is the first report showing a reduction of atherosclerosis by treatment with stevioside. Several processes contribute and interact in the development of atherosclerosis: loss of endothelial function, infiltration of monocytes into the arterial wall where they differentiate into macrophages, reduction of the antioxidant capacity and production of oxidized LDL, lipid accumulation and foam cell generation, reducing plaque stability by reducing the smooth muscle cell-to-macrophage ratio. We have demonstrated that stevioside affects all these processes: it improves endothelial function, evidenced by decreased iNOS production; it reduces the macrophage accumulation; it increases the SOD production indicating increased antioxidant defence associated with decreased oxLDL accumulation; it reduces foam cell formation evidenced by decreased lipid accumulation; it increases plaque stability evidenced by the increased smooth muscle cell-to-macrophage ratio. Essential for this application is that all these processes can be activated by oxidized LDL (for review Holvoet P and Mertens A. Faseb J 2001; 15:2073-2084), but not by LDL cholesterol, free fatty acids or triglycerides.

Previously, it was demonstrated that stevioside lowers serum concentrations of triglycerides and LDL cholesterol and increases HDL-cholesterol in the blood of diabetic rats (Jeppesen et al Rev Diabet Stud. 2006 3(4):189-199; and WO2001056959 B1, WO2002060419 A3, WO2006116814 A1, WO2006116815 A1 and WO2008031439 A2) It was concluded that therefore and thereby stevioside would decrease atherosclerosis without showing its actual effect on atherosclerosis. However, several arguments support the causal roles of oxLDL in cardiovascular disease processes which are not shared by other lipids such as LDL-cholesterol, fatty acids or triglycerides:
1) Oxidized LDL is only a minor fraction of LDL ranging from 0.001% in healthy controls (Shoji T et al. Atherosclerosis 2000; 148:171-177) to approximately 5% in patients with acute coronary events (Holvoet P et al. Circulation 1998;98:1487-1494).
2) Oxidized LDL, independent of other cardiovascular risk factors including other lipids, predicts future cardiovascular events (Holvoet P et al. Diabetes 2004;53:1068-1073; and JohnstonN et al. Int J Cardiol 2006;113:167-173; and Meisinger C et al. Circulation 2005;112:651-657). In addition, elevated oxLDL concentrations were associated with ischemic damage in acute stroke patients (Uno M et al. Neurol Res 2005;27:94-102).
3) We have demonstrated that high concentrations of oxidized LDL, but not LDL-cholesterol, were associated with the development of the metabolic syndrome in the general human population. In particular, we have demonstrated a relation with the development of abdominal obesity (characterized by impired adipogenesis and increased visceral adipose tissue accumulation), glycaemia (characterized by high glucose concentrations) and hypertriglycaemia (evidenced by serum high triglycerides). (Holvoet P et al. JAMA to be published on May 21, 2008).
4) We have demonstrated that rosuvastatin, at a dosage that did not decrease LDL-cholesterol, inhibited the development of atherosclerosis by increasing SOD production and decreasing the accumulation of oxidized LDL in DKO mice (Verreth W et al. Br J Pharmacol 2007; 151:347-355 en Desjardins F et al. Eur Heart J 2008; 29: 128-137).
5) We have demonstrated that a PPAR agonist, which decreased FFA and triglycerides but not oxidized LDL, did NOT inhibit the development of atherosclerosis in DKO mice (Verreth W. et al. Arterioscler Thromb Vasc Biol 2006;26:922-928).
6) We have demonstrated that reducing oxidative stress in the absence of an effect on LDL-cholesterol, FFA and triglycerides inhibited the development of atherosclerosis (Mackness B et al. Arterioscler Thromb Vasc Biol 2006; 26:1545-1550 and Guns PJ et al. Br J Pharmacol 2008;153:508-516). In addition, we have demonstrated that increasing the antioxidant capacity of HDL particles, without affecting HDL-cholesterol levels, inhibited the accumulation of oxLDL and thereby the progression of atherosclerosis in DKO mice (Mertens et al. Circulation 2003;107:1640-1646).
7) We have demonstrated that oxidized LDL, but not LDL-cholesterol, is associated with coronary calcification that predicts the development of cardiovascular diseases (Holvoet P et al. Atherosclerosis 2007; 194: 245-252). Vascular calcification increases vascular stiffness that does not only contribute to the development of cardiovascular diseases. For example, deterioration of vascular stiffness and inflammation-related structural vascular wall abnormalities are hallmarks of systemic sclerosis, a rheumatic disease with high case-specific mortality (Abou-Raya et al. Ann N Y Acad Sci. 2007, 10:670-680). Increased concentrations of oxidized LDL were also associated with worse fibro-calcific remodelling in valvular tissue in aortic stenosis (Cote C et al. Heart 2007; Oct 11, Epub ahead of print).

Oxidized LDL can also play causal roles in the development of other diseases which are different from other lipids.
1) Oxidized LDL contributes to the development of inflammation (Chou MY et al. J Intern Med 2008; 263; 479-488 en Holvoet P et al. Arteriosclerosis, Thrombosis and Vascular Biology 2006; 26:1558-1565). Its contribution to inflammation can be important for other diseases as well. For example, chronically inflamed intestinal microvessels also contribute to the pathology of inflammatory bowel disease (Papa et al. Dig Dis. 2008;26(2):149-15).
2) The predominant factor contributing to the development of atherosclerosis and cardiovascular diseases is ageing. It has been demonstrated that the production of SOD is inversely related with the resistance against ageing (Csiszar A et al. Aging Cell 2007; 6:783-797). Serum oxidized LDL, but not LDL-cholesterol, was inversely related to telomerase activity; it is generally accepted that telomere shortening is a measure of ageing (Tsirpanlis G et al. 2006; 11: 506-509). In addition, ageing-related oxidative effects in ear arteries may contribute to age-related deterioration of hearing (Syka et al. Neurosci Lett. 2007, 411(2):112-116).

Oxidized LDL also affects adipogenesis different from other lipids.
1) Oxidized LDL, but not LDL cholesterol, stimulates adipogenesis by inducing adipocyte hyperplasia and adipocyte hypertrophy (Parhami F et al. J Bone Miner Res 1999;14:2067-2078; and Masella R et al. FEBS Lett 2006;580:2421-2429). Thus, the effect of stevioside on the expression of SOD and adipogenesis is novel and important.
2) The effect of stevioside on adipogenesis is also important because it has been demonstrated that visceral adipose tissue of obese men increases the atherogenicity of their LDL-cholesterol by increasing their susceptibility to oxidation (Paradis ME et al. Int J Obes 2006;30:1615-1622). SOD protects LDL against oxidation.

Thus, these data are supporting our hypothesis that stevioside restores the impaired adipogenesis by reducing the oxidative stress in the adipose tissue. The decrease in FFA and triglycerides is a consequence of the improved adipogenesis; they do not play a causal role and the improved adipogenesis could not be predicted from the previously reported stevioside-induced reduction of FFA and triglycerides.

We describe a novel mechanism of stevioside-associated increase in expression of PPARalpha and PPARgamma resulting in improved adipocyte differentiation and adipogenesis, decreased hyperlipidemia, insulin resistance and atherosclerosis. The latter can be attributed by the increase insulin signalling in the vessel wall, increased expression of antioxidant enzymes SOD1 and SOD2, associated with decreased accumulation of oxidized LDL, and decreased monocyte/macrophage infiltration and lipid deposition. The latter is primarily determined by the uptake of oxidized LDL by macrophages. Because of their wide range of actions on glucose homeostasis, lipid metabolism and vascular inflammation, peroxisome proliferator-activated receptors (PPARs) are promising targets for the development of new drugs for the treatment of metabolic disorders such as diabetes, dyslipidemia and atherosclerosis. In clinical practice, PPARalpha agonists, such as the already available fibrates, improve dyslipidemia, while PPARgamma agonists, such as thiazolidinediones, improve insulin resistance and diabetes. The complementary action of simultaneous activation of each PPAR in patients suffering from metabolic syndrome and type-2 diabetes has led to new pharmacological strategies focused on the development of agonists targeting more than one receptor such as the dual PPARalpha/gamma agonists. Interestingly, steviol improved the blood profile and exerted similar inhibitory effects on athersoclerosis as did stevioside.

However, despite the proven benefits of targeting PPARs, safety concerns have recently led to late stage development failures of various PPAR agonists including novel specific PPARgamma agonists and dual PPARalpha/gamma agonists. These safety concerns include potential carcinogenicity in rodents, signs of myopathy and rhabdomyolysis, increase in plasma creatinine and homocysteine, weight gain, fluid retention, peripheral edema and potential increased risk of cardiac failure. Although the discontinued compounds shared common side effects, the reason for discontinuation was always compound specific and the toxicological or adverse effects which have motivated the discontinuation could be either due to the activation of PPARgamma, PPARalpha or both (class effect) or due to a PPAR unrelated effect.

Thus, the risk evaluation of each adverse effect should be viewed on a case by case basis considering both the PPAR profile of the drug, its absorption/distribution profile, the nature of the side effect and the putative PPAR-related mechanism of action (for review see Rubenstrunk, Hanf, Hum, Fruchart and Staels in Biochimica et Biophysica Acta -Molecular and Cell Biology of Lipids 2007; 1771, 1065-1081). Thus, the targeted action of stevioside and derivatives to PPARs in adipose tissues can be an advantage.

### EXAMPLE 5: Effects of stevioside and derivatives on adipocyte differentiation in vitro

### Methods

### The cellular model of (de)differentiation with 3T3-L1 adipocytes

3T3-L1 mouse preadipocytes were purchased from the American Type Culture Collection (ATCC). They are cultivated in DHG medium (Dulbecco's modified Eagle's medium + High Glucose 4.5 g/l, GibcoBRL) supplemented with 10 % FCS (foetal calf serum, GibcoBRL) and incubated a 37C + 5% CO2 incubator in a humidified atmosphere. For pre-adipocyte differentiation, 3T3-L1 cells were seeded at 50 % confluence 3 days before the beginning of the differentiation programme. Confluent cell monolayers (day 0) are switched for 48 h to media containing the adipogenic cocktail: "DHG-L1" medium (DHG that contains 1.5 g/l NaHCO3) supplemented with 10 % FCS, 5 µg/ml insulin, 300 µM dibutyryl-cyclicAMP (dbcAMP) and 1 µM dexamethasone. The cells were then refeeded every other day with DHG-L1 containing 10% FCS and 5µg/ml insulin. At the end, 80-90% of cells were differentiated. Stevioside, rebaudioside, steviol, and steviol glucuronide were added at 50-micromolar concentration during the 7-days differentiation process. Because stevioside, rebaudioside, and steviol did not affect lipid deposition in adipocytes, these compounds were also added at 4X higher concentrations.

### Triglyceride staining and quantitative analysis

To characterize the state of (de)differentiation obtained in the presence of the molecules of interest, triglycerides present in the cells were stained with Oil red O (a dye of the neutral lipids). The absorbance of cell monolayers at 490 nm was measured in a spectrophotometer.

### Results

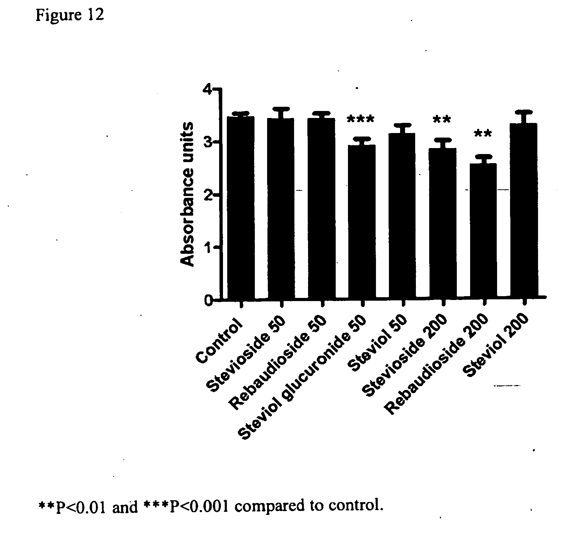

Figure 12 shows that stevioside, rebaudioside, and steviol did not significantly reduce triglyceride accumulation in adipocytes *in vitro* at the lowest concentration. In contrast steviol glucoronide did inhibit this accumulation. At 4X higher concentrations, stevoside and rebaudioside inhibited lipid deposition. Steviol did not have an effect possibly because interference with DMSO that was required to dissolve steviol.

### Discussion

Our in vitro data showed that at 200-micromolar concentrations, stevioside and rebaudioside inhibited lipid deposition in differentiated s. These data support the hypothesis that stevioside acts directly on adipocytes and inhibits triglyceride accumulation. Four times lower concentrations of steviol glucuronide were required to obtain significant inhibition.

Thus, our in vitro data support the above-mentioned hypothesis that when stevioside is converted to glucuronide this derivative actively triglyceride accumulation. Importantly rebaudioside had the same effect as stevioside. The lack of effect of steviol is most likely due to the rather high concentrations of DMSO which were required to dissolve steviol in the cell culture medium.

### Drawing Description

### BRIEF DESCRIPTION OF THE DRAWINGS

The present invention will become more fully understood from the detailed description given herein below and the accompanying drawings which are given by way of illustration only, and thus are not limitative of the present invention, and wherein:
Figure 1: demonstrates the IR of steviol glucuronide (KBr pellet).
Figure 2: demonstrates the ESI (-) MS of steviol glucuronide
Figure 3: provides the structure of steviol glucuronide and important two and three bonds coupling (HMBC) interactions
Figure 4: provides a 1H nmr spectrum of steviol glucuronide
Figure 5: demonstrates the hypothetical route from dietary stevioside to steviol glucuronide in human urine.

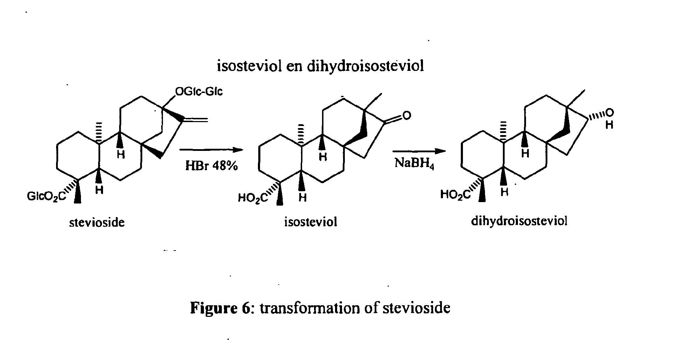

Figure 6: provides the structure of steviol, isosteviol and dihydroisosteviol.
Figure 7 is a graphic display that demonstrates the effects of an oral delivery of stevioside (10 mg/kg body weight per day for 12 weeks) in obese, dyslipidemic and diabetic mice* on the plasma glucose and insulin levels and on the HOMA Index to Assess Insulin Resistance, the plasma FFA, plasma triglycerides and plasma cholesterol.
   * For the mouse model: (Arteriosclerosis, Thrombosis, and Vascular Biology. 2006;26:922 Wim Verreth; et al and Circulation. 2003;107:1640. Ann Mertens et al.)
Figure 8 is a graphic display that compares the effects of an oral delivery of steviol (5 mg/kg body weight per day) with that of stevioside (10 mg/kg body weight per day) for 12 weeks in obese, dyslipidemic and diabetic mice* on the plasma glucose and glucose tolerance.
Figure 9 is a graphic display that demonstrates the effects of an oral treatment of obese, dyslipidemic and diabetic mice with stevioside 10 mg/kg body weight for 12 weeks on the expression of PPARalpha, PPARgamma, LXRalpha, the fatty acid binding protein 4 (FABP-4), the glucose transporter 4 (GLUT-4), the insulin receptor substrates 1 and 2 (IRS1 and IRS2) and the antioxidant superoxide dismutase 3 (SOD3) in adipose tissue.
Figure 10 is a graphic display that demonstrates the effect of an oral treatment of obese, dyslipidemic and diabetic mice with stevioside (10mg/kg body weight) or steviol (5mg/kg body weight) for 12 weeks on atherosclerosis more particular on plaque area (atherosclerotic plaque formation), lipid area (foam cell formation), macrophage area (macrophage infiltration as an indication of inflammation), oxidized LDL (oxidative stress), smooth muscle cells (SMC) and the SMC-to-macrophage ratio (proxy for plaque stability) in de atherosclerotic plaques in the aortic arch.
Figure 11 is a graphic display that demonstrates the effect of an oral treatment of obese, dyslipidemic and diabetic mice with stevioside 10 mg/kg body weight for 12 weeks increased the expression of PPARgamma that was associated with increased expression of IRS2 (insulin signalling) and SOD1 and SOD2 (antioxidant capacity). SOD1, but not SOD2, correlated significantly with IRS2. Stevioside also decreased the expression of the inflammatory iNOS (inflammation) in the aortic arch.
Figure 12 is a graphic display that shows that stevioside and rebaudioside at 200-micromolar, but not at 50-micromolar concentrations, inhibits triglyceride accumulation in 3T3-L1 adipocytes. In contrast, steviol glucuronide inhibited significantly lipid accumulation at the lowest concentration. Steviol did not affect lipid accumulation possibly because of interference with DMSO that is required to dissolve steviol.

## Claims

1. A diterpene or diterpene glycoside compound represented by the following general formula (I): 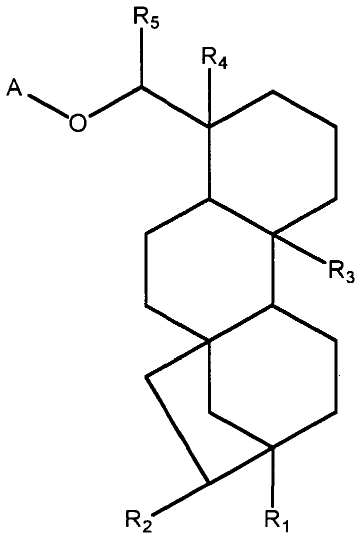 wherein
R1 is methyl, hydroxyl, hydrogen, or a -O-glycosyl,
R2 is =O, methyl, oxymethyl, hydroxyl, or forms an ethene, ethanol, 2-methoxy-propene, formaldehyde or 1-methoxy-ethanol bound or hydrogen,
R3 is methyl, ethyl, or hydrogen,
R4 is methyl, ethyl, or hydrogen,
R5 is =O, hydroxyl or hydrogen,
A is a glycosyl, a hydrogen or a tetrahydro-pyran group represented by the following general formula (II) 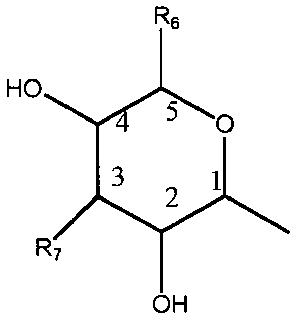
R6 is hydroxyl, ethyl, methylene, hydrogen, oxymethyl, nitrite, carboxyl, aldehyde, amino, or oxyamino,
R7 is hydroxyl, carboxyl or hydrogen
or a pharmaceutically acceptable salt of such a compound for use in a treatment to induce adipocyte differentiation (differentiation of pre-adipocytes to adipocytes) for reducing the size of the adipocytes and for prevention of adipose mass increase by a direct action of said compound on adipocytes.

2. Claim 1, whereby the treatment further comprises simultaneous or separate administration of a statin.

3. Any of the previous claims, **characterised in** compound is represented by the following general formula (I): 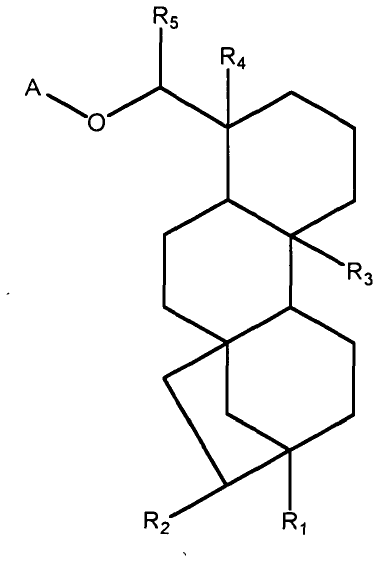 wherein
R1 is methyl, hydroxyl, hydrogen, a tetrahydro-pyran compound represented by the following general formula 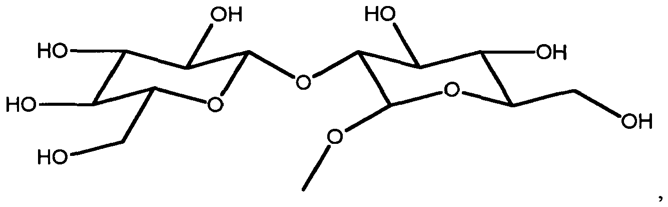 or R1 is a tetrahydro-pyran compound represented by the following general formula (II) 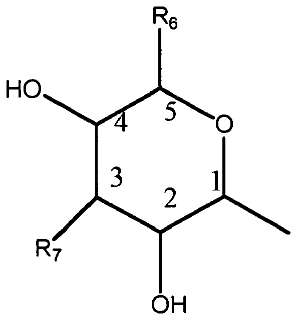
wherein R6 is hydroxyl, ethyl, hydrogen, methylene, oxymethyl, nitrite, carboxyl, aldehyde, amino, or oxyamino
or R1 is a -O-beta- Glc-beta- Glc (2-1) or has the following formula 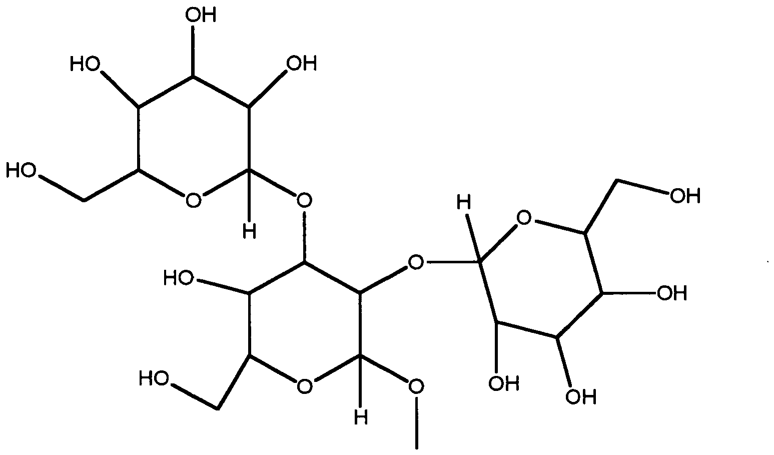
R2 is =O, methyl, oxymethyl, hydroxyl, or forms an ethene, ethanol, 2-methoxy-propene, formaldehyde or 1-methoxy-ethanol bound or hydrogen,
R3 is methyl, ethyl, or hydrogen,
R4 is methyl, ethyl, or hydrogen,
R5 is =O or hydroxyl or hydrogen,
and
A is hydrogen or a tetrahydro-pyran compound represented by the following general formula (II) 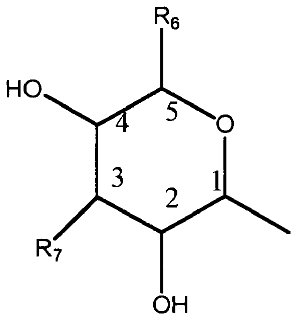
R6 is hydroxyl, ethyl, methylene, oxymethyl, nitrite, carboxyl, aldehyde, amino, or oxyamino.
R7 is hydroxyl or carboxyl

4. Claim 1, **characterised in that** A is a group selected from D-Galactopyranosiduronic acid, D-Glucopyranosiduronic acid, D-Glucopyranoside, D-Glucopyranose, D-gluco-Hexodialdo-1,5-pyranoside, L-Mannopyranose, D-Xylopyranoside, L-Xylopyranose, D-galacto-Heptopyranoside and D-Galactopyranoside.

5. Any of the previous claims, whereby the compound of claim 1, represented by the following general formula (III): 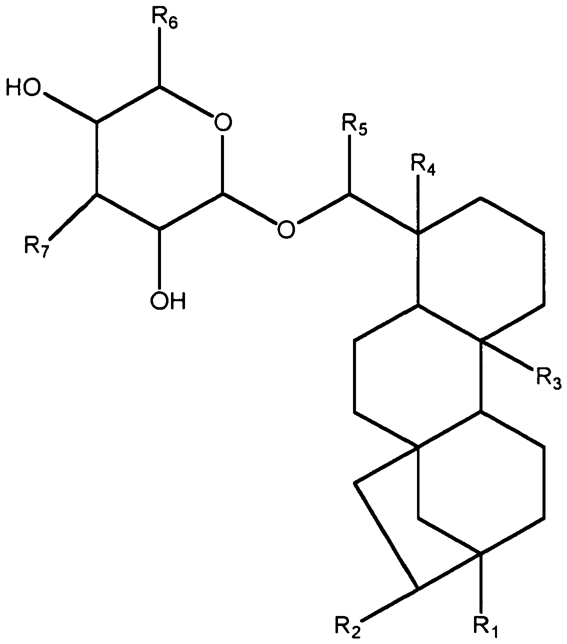 wherein
R1 is methyl, hydrogen, or hydroxyl,
R2 is =O, methyl, oxymethyl, hydroxyl, or forms an ethene, ethanol, 2-Methoxy-propene, formaldehyde or 1-methoxy-ethanol bound.
R3 is methyl, ethyl, or hydrogen,
R4 is methyl, ethyl, or hydrogen,
R5 is =O, hydrogen, or hydroxyl
R6 is hydroxyl, ethyl, hydrogen, methylene, oxymethyl, nitrite, carboxyl, aldehyde, amino, or oxyamino.
R7 is hydroxyl, hydrogen, carboxyl
or a pharmaceutically acceptable salt of such a compound.

6. Any of the previous claims, **characterised in that** the compound is represented by one of the following general formula (IV), (V), (VI), (VII), (IIX), or (IX) 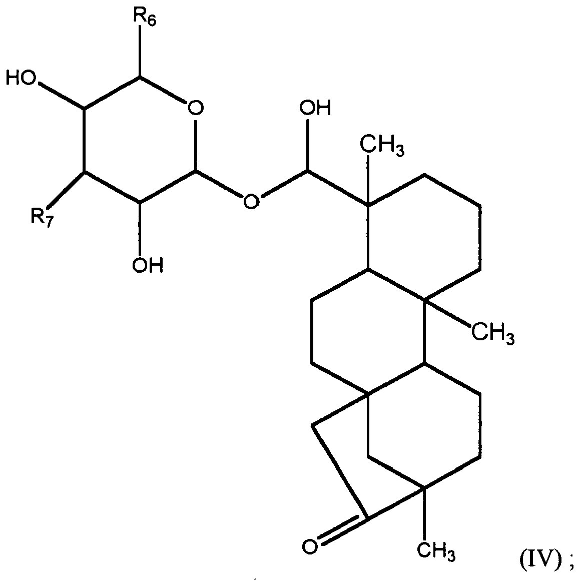 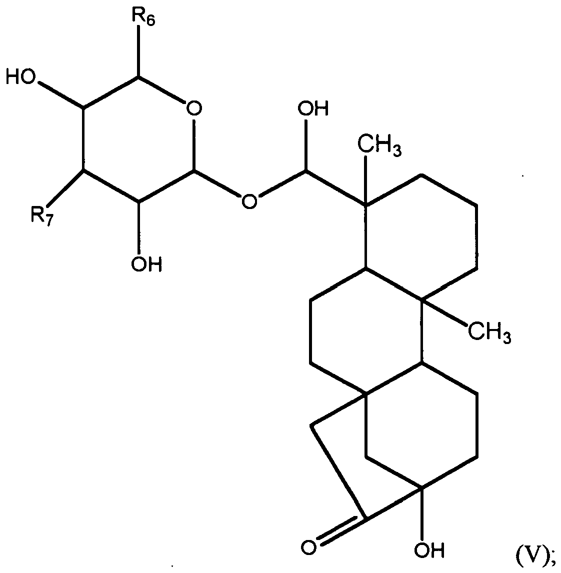 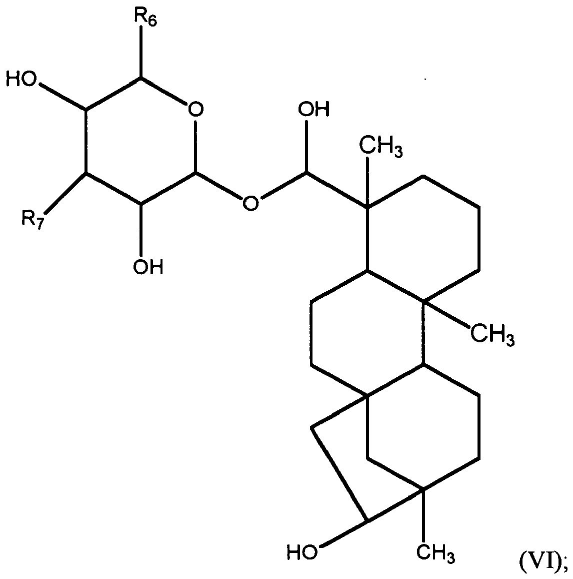 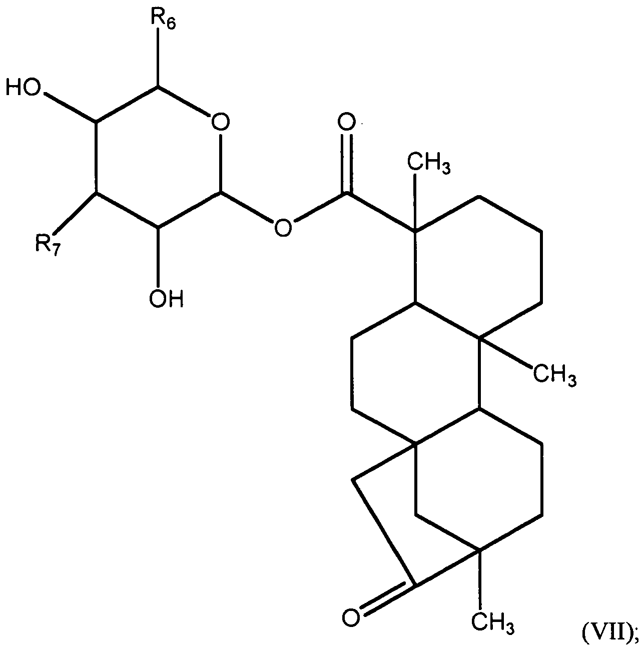 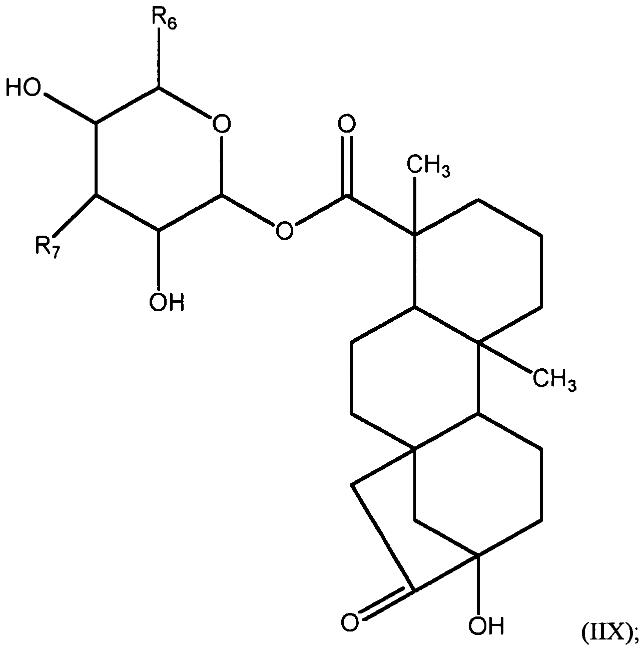 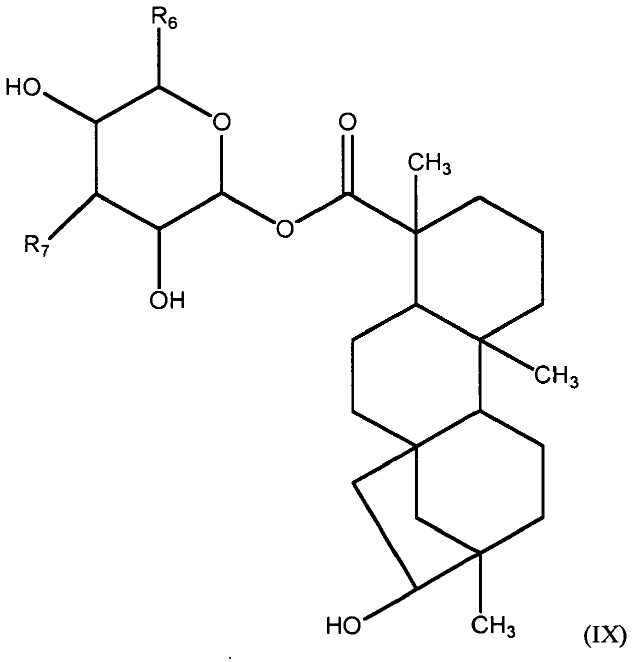 wherein R6 is hydroxyl, ethyl, hydrogen, methylene, oxymethyl, nitrite, carboxyl, aldehyde, amino, or oxyamino and R7 is hydroxyl or carboxyl

7. Any of the previous claims, **characterised in that** the compound is by the following general formula (X): 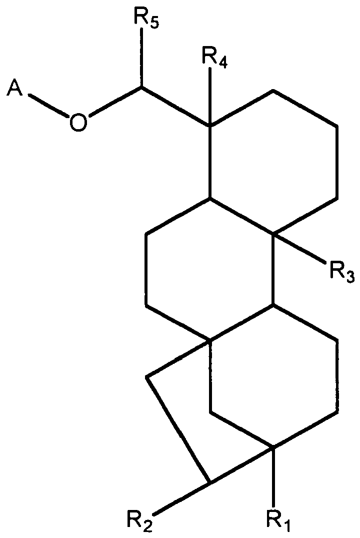 wherein
R1 is a tetrahydro-pyran compound represented by the following general formula 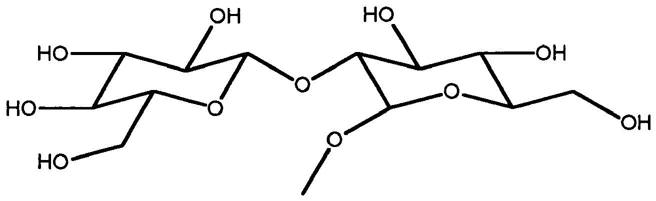
R2 is oxygen, methyl, hydrogen, oxymethyl, hydroxyl, or forms an ethene, ethanol, 2-methoxy-propene, formaldehyde or 1-methoxy-ethanol bound.
R3 is methyl, ethyl, or hydrogen,
R4 is methyl, ethyl, or hydrogen,
R5 is oxygen, hydrogen, or hydroxyl
A is hydrogen
R6 is hydroxyl, ethyl, hydrogen, methylene, oxymethyl, nitrite, carboxyl, aldehyde, amino, or oxyamino.
R7 is hydroxyl, hydrogen, or carboxyl
or a pharmaceutically acceptable salt of such a compound.

8. Any of the previous claims, **characterised in that** the compound is steviol, isosteviol, steviol-glucuronide, isosteviol-glucuronide, dihydroisosteviol-glucuronide steviol- galacturonide, isosteviol-galacturonide or dihydroisosteviol-galacturonide or pharmaceutically-acceptable acid- addition or cationic salt thereof.

9. Any of the previous claims, **characterised in that** the compound is selected from the group consisting of steviol-O-β-D-glucuronide, dihydroisosteviol-O-β-D-glucuronide, isosteviol-O-β-D-glucuronide steviol-O-β-D-galacturonide, dihydroisosteviol-O-β-D-galacturonide, isosteviol-O-β-D-galacturonide, dihydroisosteviol-O-α-D-glucuronide, steviol-O- α-D-glucuronide, isosteviol-O-α-D-glucuronide, steviol-O-α3-D-galacturonide, dihydroisosteviol-O-α-D-galacturonide, isosteviol-O-α-D-galacturonide, dihydroisosteviol-O-β-L-glucuronide, steviol-O-β-L-glucuronide, isosteviol-O-β-L-glucuronide, steviol-O-β-L-galacturonide, dihydroisosteviol-O-β-L-galacturonide, isosteviol-O-β-L-galacturonide, dihydroisosteviol-O-α-L-glucuronide, steviol-O- α-L-glucuronide, isosteviol-O-α-L-glucuronide, steviol-O-α-L-galacturonide, dihydroisosteviol-O-α-L-galacturonide, isosteviol-O-α-L-galacturonide, and steviol-octan-O-β-D-galacturonide.

10. Any of the previous claims, **characterised in that** the compound is selected from the group consisting of steviol-O-β-D-Glucopyranosiduronic acid, steviol-O-β-D-Glucopyranoside, steviol-O-α-D-Glucopyranoside, steviol-O-β-D-Glucopyranose, steviol-O-β-D-gluco-Hexodialdo-1,5-pyranoside, steviol-O-β-L-Mannopyranose, steviol-O-β-D-Xylopyranoside, steviol-O-β-L-Xylopyranose, steviol-O-β-D-galacto-Heptopyranoside, steviol-O-β-D-Galactopyranoside.

11. Any of the previous claims, **characterised in that** the compound is Steviol-19-O-β-D-glucuronide.

12. Any of the previous claims, **characterised in that** the compound is of the group consisting of 7a,15a-Methano-8H-naphth[2',1':5,6]azuleno[2,1-d]phenanthrene-4,7-dione, 1,2,2a,3,5,5a,9,9a,10,11,12,13,13a,13b,14,15-hexadecahydro-1,16-dihydroxy-17-methoxy-3,3,10,10,13a-pentamethyl-18-(1-methylethyl)-, (1R,2aR,5aS,13aR)-rel-(-)- (9Cl), Kauran-16-ol, 13-methyl-, (8**β**,13**β**)- (9Cl), Kaurane-16,18-diol, 13-methyl-, (4**α**,8**β**,13**β**)- (9Cl), Kauran-18-oic acid, 16-hydroxy-13-methyl-, (4a,8b,13b)- (9Cl) or ent-16b-Hydroxy-16a-methylbeyeran-19-oic acid, Kauran-18-oic acid, 11,13,16,17-tetrahydroxy-, (4**a**,11**a**)-, Kaur-16-en-18-oic acid, 7,11,13-trihydroxy-, (4**α**,7**β**,11**α**)-, Kauran-18-oic acid, 16,17-epoxy-11,13-dihydroxy-, (4**a**,11**a**)-, Kauran-18-oic acid, 17-(acetyloxy)-13,16-dihydroxy-, (4**α**)-, Kaur-16-en-18-oic acid, 13-hydroxy-7-oxo-, (4**α**)-, 1H-2,10a-Ethanophenanthrene-8-carboxylic acid, dodecahydro-10-hydroxy-2-(hydroxymethyl)-4b,8-dimethyl-12-oxo-(2S,4aS,4bS,8R,8aS,10R,10aS)- , 17-Norkauran-18-oic acid, 16-hydroxy-13-methyl-, methyl ester, (4**α**,8**β**,13**β**,16**α**)-, Kaur-16-ene-3,18-diol, 13-methyl-, (3**α**,4**α**,8**β**,13**β**)-(**±**)- (9Cl), 17-Norkauran-18-oic acid, 13-methyl-16-(2-oxopropylidene)-, (4**α**,8**β**,13**β**)-(**±**)- (9Cl), Kaur-16-en-18-oic acid, 13-methyl-3-oxo-, ethyl ester, (4**β**,8**β**,13**β**)-(**±**)- (9Cl), Kaur-16-en-18-oic acid, 3-hydroxy-13-methyl-, ethyl ester, (3**α**,4**α**,8**β**,13**β**)-(**±**)- (9Cl), and Kaur-16-en-18-oic acid, 13-methyl-3-oxo-, ethyl ester, (4**α**,8**β**,13**β**)-(**±**)- (9Cl) or functional derivatives thereof

13. Any of the previous claims, **characterised in that** the compound is rabaudioside A (Kaur-16-en-18-oic acid or 13-[(O-**β**-D-glucopyranosyl-(1→2)-O-[**β**-D-glucopyranosyl-(1**→**3)]-**β**-D-glucopyranosyl)oxy]-, **β**-D-glucopyranosyl ester, (4**α**)-) or stevioside (Kaur-16-en-18-oic acid or 13-[(2-O-**β**-D-glucopyranosyl-**β**-D-glucopyranosyl)oxy]-, **β**-D-glucopyranosyl ester, (4**α**)-)

14. Any of the previous claims, **characterised in that** the compound is of the group consisting of stevioside, rebaudioside A, rebaudioside C, Dulcoside A, steviolbioside, rebaudioside B, rebaudioside D, rebaudioside E, steviol, isosteviol, steviol-glucuronide, isosteviol-glucuronide, and dihydroisosteviol-glucuronide

## Patentansprüche

1. Eine Diterpen- oder Diterpen-Glykosid-Verbindung, die durch die folgende, allgemeine Formel (I) dargestellt wird: 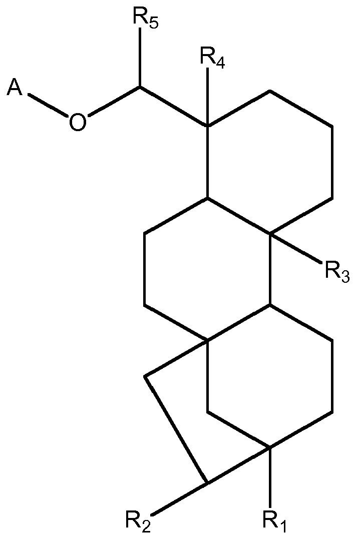 wobei
R1 Methyl, Hydroxyl, Wasserstoff oder ein -O-Glykosyl ist,
R2 =O, Methyl, Oxymethyl, Hydroxyl ist oder einen Ethylen-, Ethanol-, 2-Methoxy-Propen-, Formaldehyd- oder 1-Methoxy-Ethanol-Verbund oder Wasserstoff bildet,
R3 Methyl, Ethyl oder Wasserstoff ist,
R4 Methyl, Ethyl oder Wasserstoff ist,
R5 =O, Hydroxyl oder Wasserstoff ist,
A eine Glykosyl-, Wasserstoff- oder Tetrahydro-Pyran-Gruppe ist, die durch die folgende, allgemeine Formel (II) dargestellt wird: 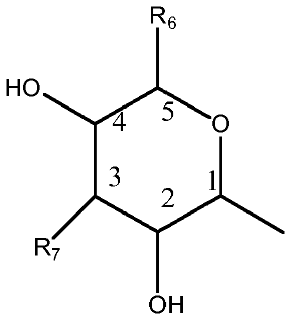
R6 Hydroxyl, Ethyl, Methylen, Wasserstoff, Oxymethyl, Nitrit, Carboxyl, Aldehyd, Amino oder Oxyamino ist,
R7 Hydroxyl, Carboxyl oder Wasserstoff ist
oder ein pharmazeutisch akzeptables Salz einer entsprechenden Verbindung ist, das sich zur Verwendung in einer Behandlung zur Induzierung der Adipozytendifferenzierung (Differenzierung der Prä-Adipozyten und Adipozyten) durch direkte Einwirkung der besagten Verbindung auf die Adipozyten mit dem Ziel einer Verkleinerung der Adipozyten und einer Verhinderung der Vergrößerung der adipösen Masse eignet.

2. Anspruch 1, wobei die Behandlung ferner die gleichzeitige oder separate Verabreichung eines Statins einschließt.

3. Jeder der vorherigen Ansprüche, **dadurch gekennzeichnet, dass** die Verbindung durch die folgende, allgemeine Formel (I) dargestellt wird: 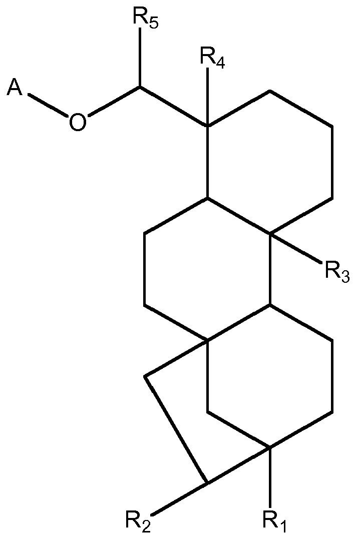 wobei
R1 Methyl, Hydroxyl, Wasserstoff, eine Tetrahydro-Pyran-Verbindung ist, die durch die folgende, allgemeine Formel dargestellt wird: 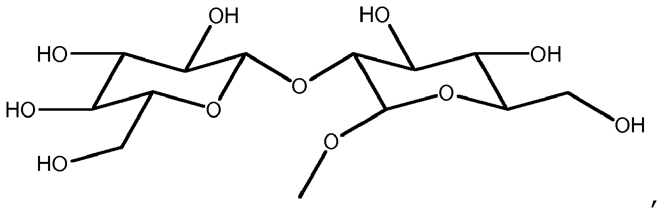 oder R1 eine Tetrahydro-Pyran-Verbindung ist, die durch die folgende, allgemeine Formel (II) dargestellt wird: 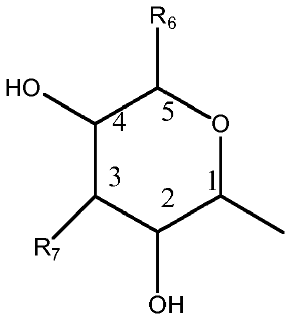
wobei R6 Hydroxyl, Ethyl, Wasserstoff, Methylen, Oxymethyl, Nitrit, Carboxyl, Aldehyd, Amino oder Oxyamino ist
oder R1 entweder eine -0-beta- Glc-beta- Glc (2-1) ist oder durch die folgende Formel dargestellt wird: 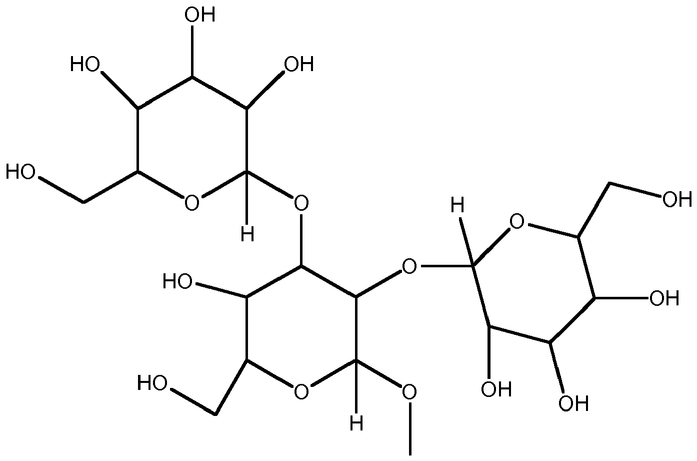
R2 =O, Methyl, Oxymethyl, Hydroxyl ist oder einen Ethen-, Ethanol-, 2-Methoxy-Propen-, Formaldehyd- oder 1-Methoxy-Ethanol-Verbund oder Wasserstoff bildet,
R3 Methyl, Ethyl oder Wasserstoff ist,
R4 Methyl, Ethyl oder Wasserstoff ist,
R5 =O oder Hydroxyl oder Wasserstoff ist,
und
A Wasserstoff oder eine Tetrahydro-Pyran-Verbindung ist, die durch die folgende, allgemeine Formel (II) dargestellt wird: 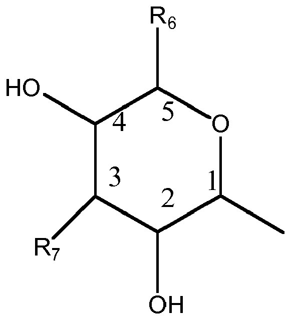
R6 Hydroxyl, Ethyl, Methylen, Oxymethyl, Nitrit, Carboxyl, Aldehyd, Amino oder Oxyamino ist,
R7 Hydroxyl oder Carboxyl ist.

4. Anspruch 1, **dadurch gekennzeichnet, dass** A aus der Gruppe der folgenden Substanzen gewählt wird: D-Galactopyrano-siduronische Säure, D-Glucopyranosiduronische Säure, D-Glucopyranosid, D-Glucopyranose, D-Gluco-Hexodialdo-1,5-Pyranosid, L-Mannopyranose, D-Xylopyranosid, L-Xylopyranose, D-Galacto-Heptopyranosid und D-Galactopyranosid.

5. Jeder der vorherigen Ansprüche, wobei die Verbindung von Anspruch 1 durch die folgende, allgemeine Formel (III) dargestellt wird: 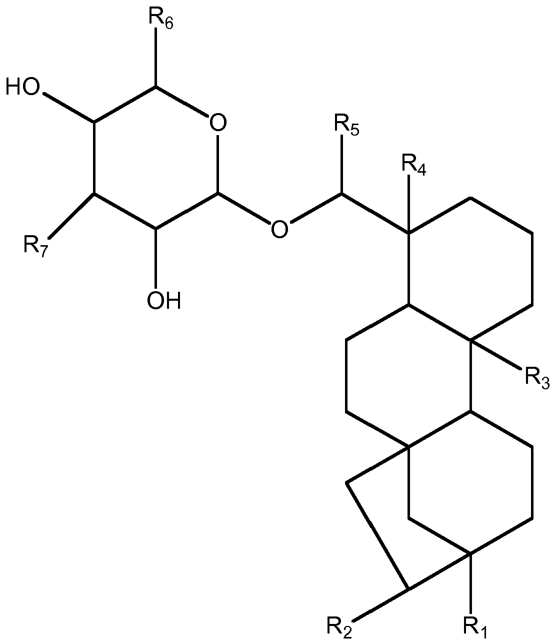 wobei
R1 Methyl, Wasserstoff oder Hydroxyl ist,
R2 =O, Methyl, Oxymethyl, Hydroxyl ist oder einen Ethylen-, Ethanol-, 2-Methoxy-Propen-, Formaldehyd- oder 1-Methoxy-Ethanol-Verbund bildet,
R3 Methyl, Ethyl oder Wasserstoff ist,
R4 Methyl, Ethyl oder Wasserstoff ist,
R5 =O, Wasserstoff oder Hydroxyl ist,
R6 Hydroxyl, Ethyl, Wasserstoff, Methylen, Oxymethyl, Nitrit, Carboxyl, Aldehyd, Amino oder Oxyamino ist,
R7 Hydroxyl, Wasserstoff oder Carboxyl ist oder ein pharmazeutisch akzeptables Salz der betreffenden Verbindung.

6. Jeder der vorherigen Ansprüche, **gekennzeichnet dadurch, dass** die Verbindung durch eine der folgenden, allgemeinen Formeln - (IV), (V), (VI), (VII), (IIX) oder (IX) - dargestellt wird: 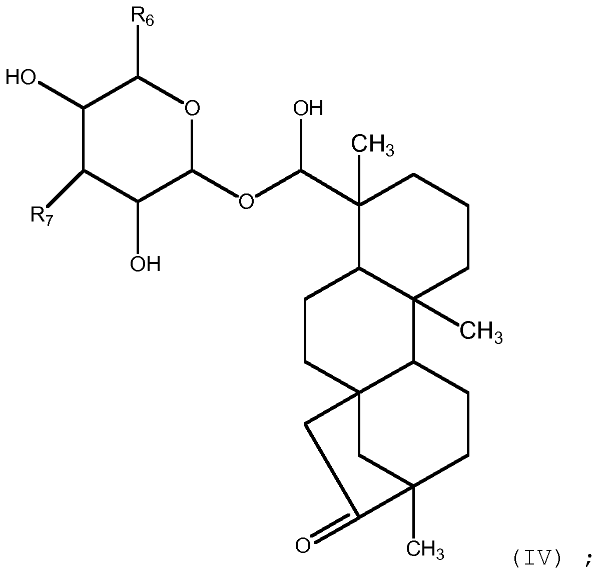 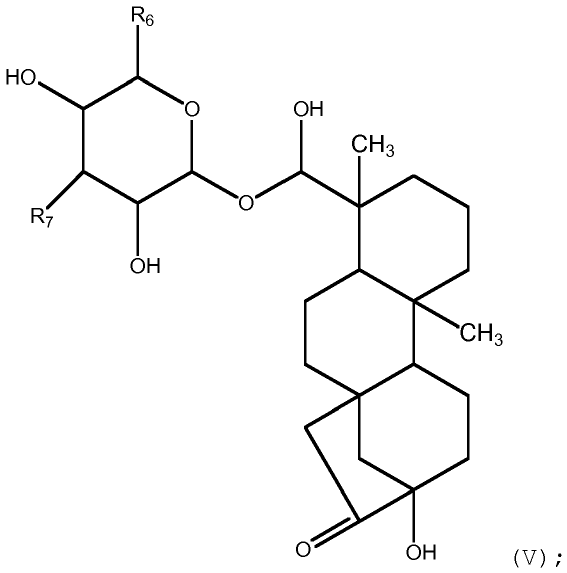 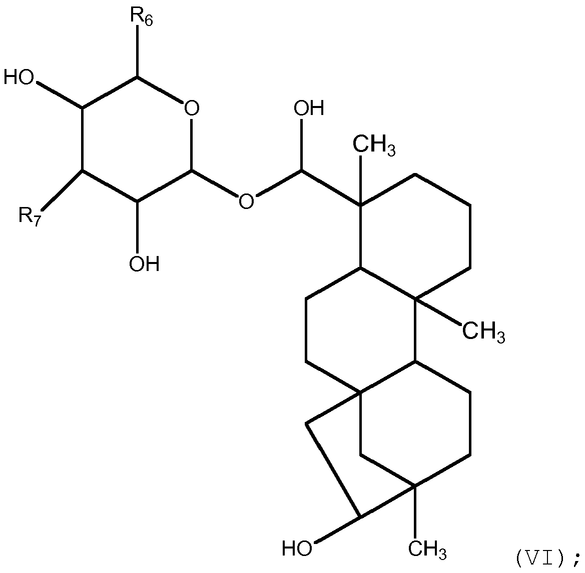 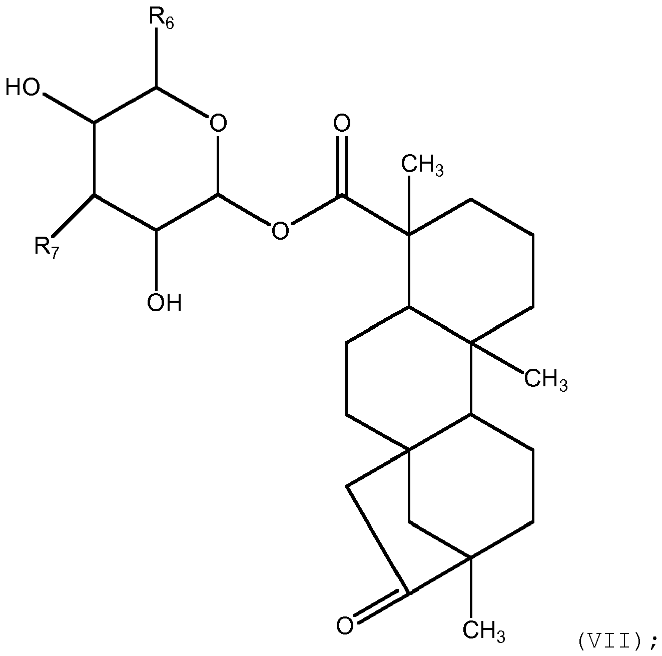 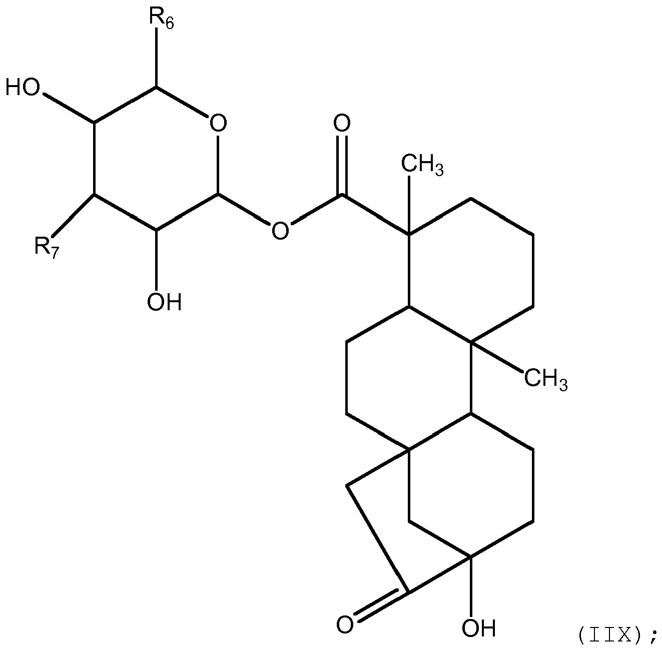 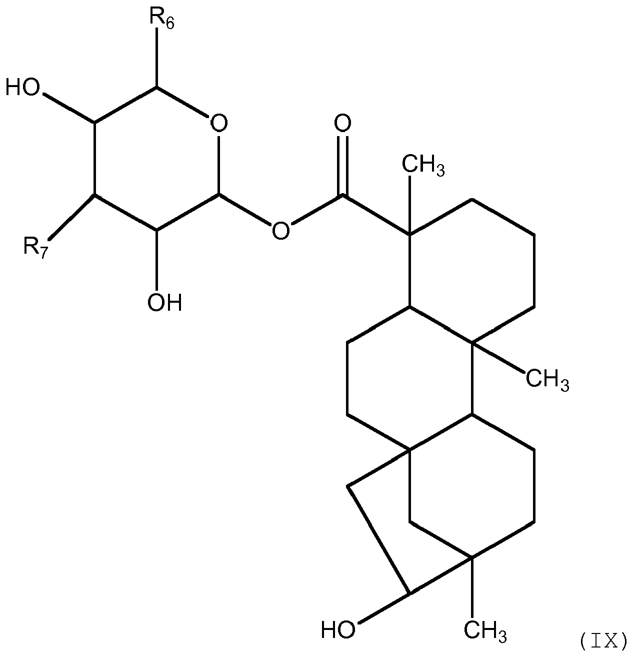 wobei R6 Hydroxyl, Ethyl, Wasserstoff, Methylen, Oxymethyl, Nitrit, Carboxyl, Aldehyd, Amino oder Oxyamino ist und R7 Hydroxyl oder Carboxyl ist.

7. Jeder der vorherigen Ansprüche, wobei die Verbindung durch die folgende, allgemeine Formel (X) dargestellt wird: 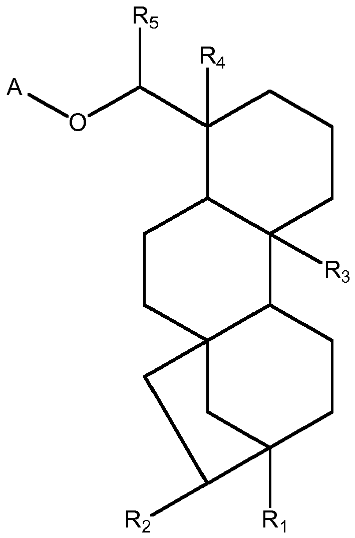 wobei
R1 eine Tetrahydro-Pyran-Verbindung ist, die durch die folgende, allgemeine Formel dargestellt wird: 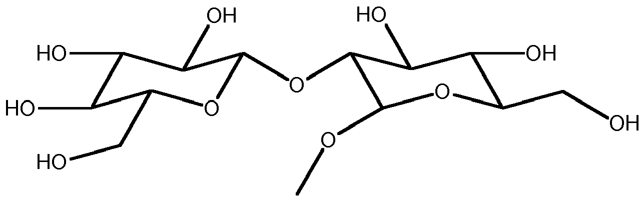
R2 Sauerstoff, Methyl, Wasserstoff, Oxymethyl, Hydroxyl ist oder einen Ethen-, Ethanol-, 2-Methoxy-Propen-, Formaldehyd- oder 1-Methoxy-Ethanol-Verbund bildet.
R3 Methyl, Ethyl oder Wasserstoff ist,
R4 Methyl, Ethyl oder Wasserstoff ist,
R5 Sauerstoff, Wasserstoff oder Hydroxyl ist,
A Wasserstoff ist,
R6 Hydroxyl, Ethyl, Wasserstoff, Methylen, Oxymethyl, Nitrit, Carboxyl, Aldehyd, Amino oder Oxyamino ist,
R7 Hydroxyl, Wasserstoff oder Carboxyl ist
oder ein pharmazeutisch akzeptables Salz der betreffenden Verbindung.

8. Jeder der vorherigen Ansprüche, **gekennzeichnet dadurch, dass** die Verbindung Steviol, Isosteviol, Steviol-Glucuronid, Isosteviol-Glucuronide, Dihydroisosteviol-Glucuronid, Steviol-Galacturonid, Isosteviol-Galacturonid oder Dihydroisosteviol-Galacturonid oder eine pharmazeutisch akzeptable Säureaddition bzw. ein kationisches Salz hiervon ist.

9. Jeder der vorherigen Ansprüche, **gekennzeichnet dadurch, dass** die Verbindung aus der Gruppe der folgenden Substanzen gewählt wird: Steviol-O-β-D-Glucuronid, Dihydroisosteviol-O-β-D-Glucuronid, Isosteviol-O-β-D-Glucuronid, Steviol-O-β-D-Galacturonid, Dihydroisosteviol-O-β-D-Galacturonid, Isosteviol-O-β-D-Galacturonid, Dihydroisosteviol-O-α-D-Glucuronid, Steviol-O- α-D-Glucuronid, Isosteviol-O-α-D-Glucuronid, Steviol-O-α-D-Galacturonid, Dihydroisosteviol-O-α-D-Galacturonid, Isosteviol-O-α-D-Galacturonid, Dihydroisosteviol-O-β-L-Glucuronid, Steviol-O-β-L-Glucuronid, Isosteviol-O-β-L-Glucuronid, Steviol-O-β-L-Galacturonid, Dihydroisosteviol-O-β-L-Galacturonid, Isosteviol-O-β-L-Galacturonid, Dihydroisosteviol-O-α-L-Glucuronid, Steviol-O- α-L-Glucuronid, Isosteviol-O-α-L-Glucuronid, Steviol-O-α-L-Galacturonid, Dihydroisosteviol-O-α-L-Galacturonid, Isosteviol-O-α-L-Galacturonide und Steviol-Octan-O-β-D-Galacturonid.

10. Jeder der vorherigen Ansprüche, **gekennzeichnet dadurch, dass** die Verbindung aus der Gruppe der folgenden Substanzen gewählt wird: Steviol-O-**β**-D-Glucopyranosiduronische Säure, Steviol-O-**β**-D-Glucopyranosid, Steviol-O-**α**-D-Glucopyranosid, Steviol-O-**β**-D-Glucopyranose, Steviol-O-**β**-D-Gluco-Hexodialdo-1,5-Pyranosid, Steviol-O-**β**-L-Mannopyranose, Steviol-O-**β**-D-Xylopyranosid, Steviol-O-β-L-Xylopyranose, Steviol-O-**β**-D-Galacto-Heptopyranosid, Steviol-O-**β**-D-Galactopyranosid.

11. Jeder der vorherigen Ansprüche, **gekennzeichnet dadurch, dass** die Verbindung Steviol-19-O- β-D-Glucuronid ist.

12. Jeder der vorherigen Ansprüche, **gekennzeichnet dadurch, dass** die Verbindung aus der Gruppe der folgenden Substanzen gewählt wird: 7a,15a-Methano-8H-naphth[2',1':5,6]azuleno[2,1-d]phenanthrene-4,7-Dione, 1, 2, 2a, 3, 5, 5a, 9, 9a, 10, 11, 12, 13, 13a, 13b, 14, 15-Hexadecahydro-1,16-Dihydroxy-17-Methoxy-3,3,10,10,13a-Pentamethyl-18-(1-Methylethyl)-, (1R,2aR,5aS,13aR)-rel-(- )- (9CI), Kauran-16-ol, 13-Methyl-, (8**β**,13**β**)- (9CI), Kaurane-16,18-diol, 13-Methyl-, (4**α**,8**β**,13**β**)- (9CI), Kauran-18-Säure, 16-Hydroxy-13-Methyl-, (4α,8β,13β) - (9CI) oder ent-16b-Hydroxy-16a-Methylbeyeran-19-Säure, Kauran-18-Säure, 11,13,16,17-Tetrahydroxy-, (4**α**,11**α**) -, Kaur-16-en-18-Säure, 7,11,13-Trihydroxy-, (4**α**,7**β**,11**α**) -, Kauran-18-Säure, 16,17-Epoxy-11,13-Dihydroxy-, (4**α**,11**α**) -, Kauran-18-Säure, 17-(Acetyloxy)-13,16-Dihydroxy-, (4**α**) -, Kaur-16-en-18-Säure, 13-Hydroxy-7-Oxo-, (4**α**) -, 1H-2,10a-Ethanophenanthren-8-Carbonsäure, Dodecahydro-10-Hydroxy-2-(Hydroxymethyl)-4b,8-Dimethyl-12-Oxo-(2S,4aS,4bS,8R,8aS,10R,10aS)- , 17-Norkauran-18-Säure, 16-Hydroxy-13-Methyl-, Methylester, (4**α**,8**β**,13**β**,16**α**) -, Kaur-16-ene-3,18-diol, 13-Methyl-, (3**α**,4**α**,8**β**,13**β**) - (**±**) - (9CI), 17-Norkauran-18-Säure, 13-Methyl-16-(2-Oxopropyliden) -, (4**α**, 8**β**, 13**β**) - (**±**) - (9CI) , Kaur-16-en-18-Säure, 13-Methyl-3-oxo-, Ethylester, (4**β**,8**β**,13**β**) - (**±**) - (9CI), Kaur-16-en-18-Säure, 3-Hydroxy-13-Methyl-, Ethylester, (3**α**,4**α**,8**β**,13**β**) - (**±**) - (9CI) und Kaur-16-en-18-Säure, 13-Methyl-3-Oxo-, Ethylester, (4**α**,8**β**,13**β**) - (**±**) - (9CI) oder funktionale Derivate der betreffenden Substanzen.

13. Jeder der vorherigen Ansprüche, **gekennzeichnet dadurch, dass** die Verbindung Rabaudioside A (Kaur-16-en-18-Säure oder 13-[(O-**β**-D-Glucopyranosyl-(1→2)-O-[**b**-D-Glucopyranosyl-(1→3)]-**β**-D-Glucopyranosyl)oxy]-, **β**-D-Glucopyranosylester, (4**α**)-**)** oder Steviosid (Kaur-16-en-18-Säure oder 13-[(2-O-**β**-D-Glucopyranosyl-**β**-D-Glucopyranosyl)oxy]-, **β**-D-Glucopyranosylester, **(4α)- )** ist.

14. Jeder der vorherigen Ansprüche, **gekennzeichnet dadurch, dass** die Verbindung aus der Gruppe der folgenden Substanzen gewählt wird: Steviosid, Rebaudiosid A, Rebaudiosid C, Dulcosid A, Steviolbiosid, Rebaudiosid B, Rebaudiosid D, Rebaudiosid E, Steviol, Isosteviol, Steviol-Glucuronid, Isosteviol-Glucuronid und Dihydroisosteviol-Glucuronid.

## Revendications

1. Un composé diterpénique de glucoside ou un diterpène représenté par la formule suivante (I): 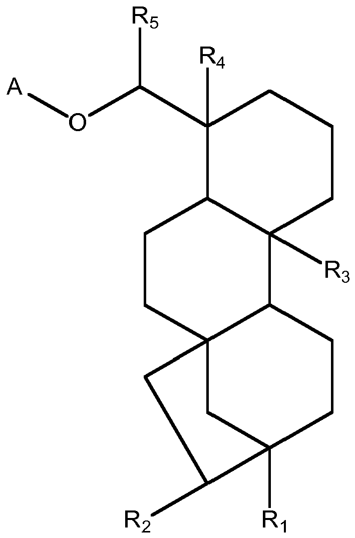 ou
R1 est un méthyle, un hydroxyle, un hydrogène, ou un -O-glycosyle,
R2 est =O, méthyle, oxyméthyle, hydroxyle, ou des formes d'éthène, éthanol, 2-méthoxy-propène, formaldéhyde ou 1-méthoxy-éthanol lié ou de l'hydrogène,
R3 est un méthyle, un éthyle, ou de l'hydrogène,
R4 est un méthyle, un éthyle, ou de l'hydrogène,
R5 est =O, hydroxyle ou hydrogène,
A est un glycosyle, un hydrogène ou un groupe tétrahydro - pyranne représenté par la formule générale suivante (II) 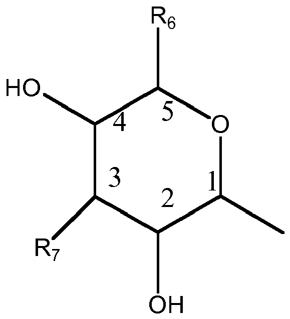
R6 est un hydroxyle, éthyle, méthylène, hydrogène, oxyméthyle, nitrite, carboxyle, aldéhyde, amino, ou oxyamino,
R7 est un hydroxyle, un carboxyle ou un hydrogène
Ou un sel pharmaceutiquement acceptable d'un tel composé, utilisé dans le traitement pour induire la différenciation des adipocytes (différentiation des préadipocytes des adipocytes) afin de réduire la taille des adipocytes et pour la prévention de l'augmentation de la masse adipeuse par une action directe dudit composé sur les adipocytes.

2. La revendication 1, où le traitement postérieur comprend une administration simultanée ou séparée de statine.

3. N'importe laquelle des revendications précédentes, **caractérisées par le fait que** le composé est représenté par la formule générale suivante (I): 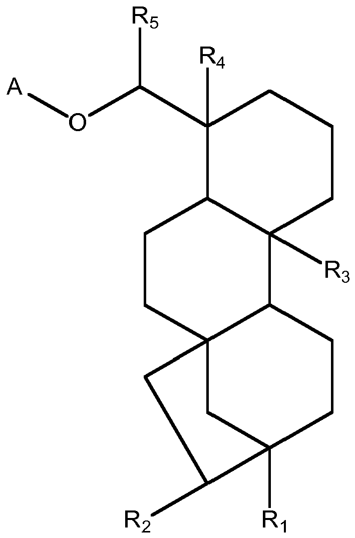 ou
R1 est un méthyle, un hydroxyle, un hydrogène, un composé tétrahydro-pyranne représenté par la formule générale suivante 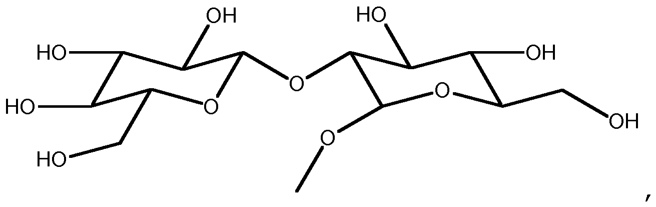 ou R1 est un composé de tétrahydro-pyranne représenté par la formule générale suivante (II) 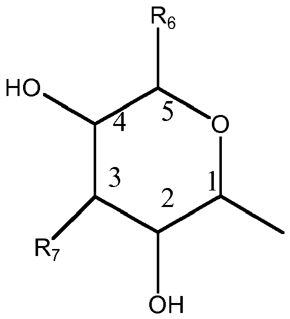
ou R6 est un hydroxyle, un éthyle, un hydrogène, un méthylène, un oxyméthyle, un nitrite, un carboxyle, un aldéhyde, un amino, ou un oxyamino
ou R1 est un -O-beta- Glc-beta- Glc (2-1), avec la formule suivante 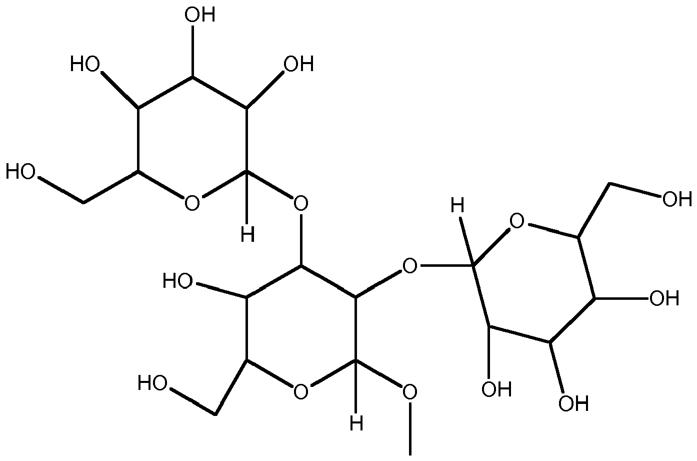
R2 est =O, méthyle, oxyméthyle, hydroxyle, ou des formes d'éthène, éthanol, 2-méthoxy-propène, formaldéhyde ou 1-méthoxy-éthanol lié ou un hydrogène,
R3 est un méthyle, un éthyle, ou un hydrogène,
R4 est un méthyle, un éthyle, ou un hydrogène,
R5 est un =O ou un hydroxyle ou un hydrogène,
et
A est un hydrogène ou un composé de tétrahydro-pyranne représenté par la formule générale suivante (II) 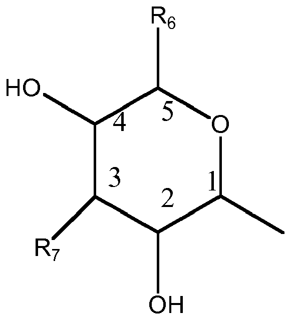
R6 est un hydroxyle, un éthyle, un méthylène, un oxyméthyle, un nitrite, un carboxyle, un aldéhyde, un amino, ou un oxyamino.
R7 est un hydroxyle ou un carboxyle

4. La revendication 1, **caractérisée par le fait que** A est un groupe sélectionné à partir de D-Galactopyranosiduronique acide, D-Glucopyranosiduronique acide, D-Glucopyranoside, D-Glucopyranose, D-gluco-Hexodialdo-1,5-pyranoside, L-Mannopyranose, D-Xylopyranoside, L-Xylopyranose, D-galacto-Heptopyranoside et D-Galactopyranoside.

5. N'importe laquelle des revendications précédentes, à savoir le composé de la revendication 1, représenté par la formule générale suivante (III): 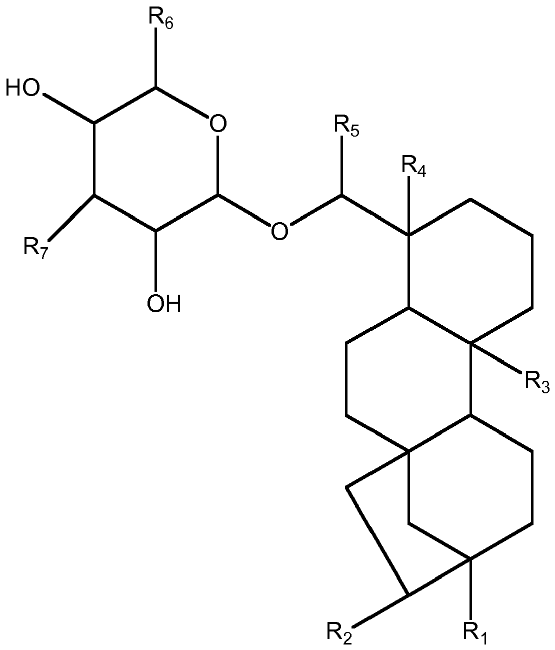 ou
R1 est un méthyle, un hydrogène, ou un hydroxyle,
R2 est =O, méthyle, oxyméthyle, hydroxyle, ou des formes d'éthène, éthanol, 2-Méthoxy-propène, formaldéhyde ou 1-méthoxy-éthanol lié.
R3 est un méthyle, un éthyle, ou un hydrogène,
R4 est un méthyle, un éthyle, ou un hydrogène,
R5 est =O, un hydrogène, ou un hydroxyle
R6 est un hydroxyle, un éthyle, un hydrogène, un méthylène, un oxyméthyle, un nitrite, un carboxyle, de l'aldéhyde, un amino, ou un oxyamino.
R7 est un hydroxyle, un hydrogène, un carboxyle ou un sel pharmaceutiquement acceptable d'un tel composé.

6. N'importe laquelle des revendications précédentes, **caractérisées par le fait que** le composé est représenté par l'une des formules générales suivantes (IV) (V) (VI) (VII) (IIX), ou (IX) 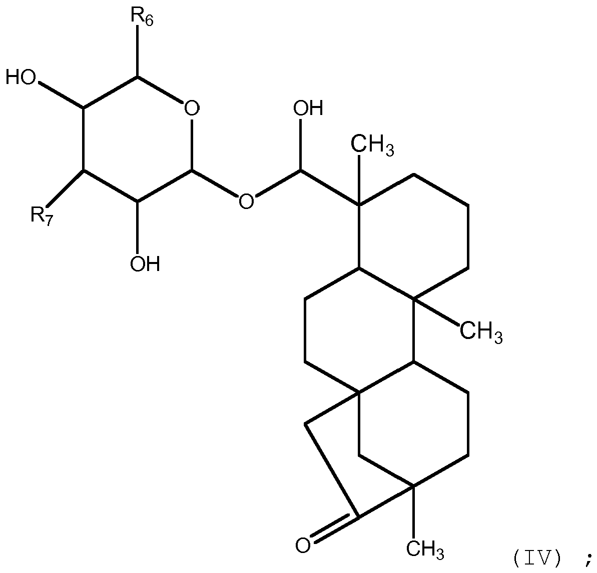 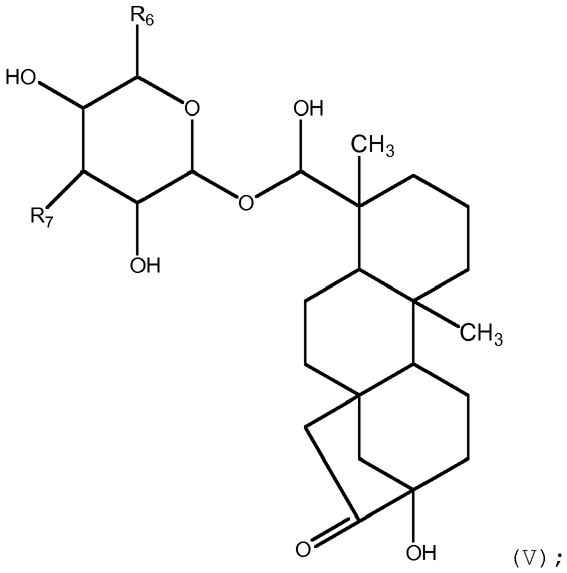 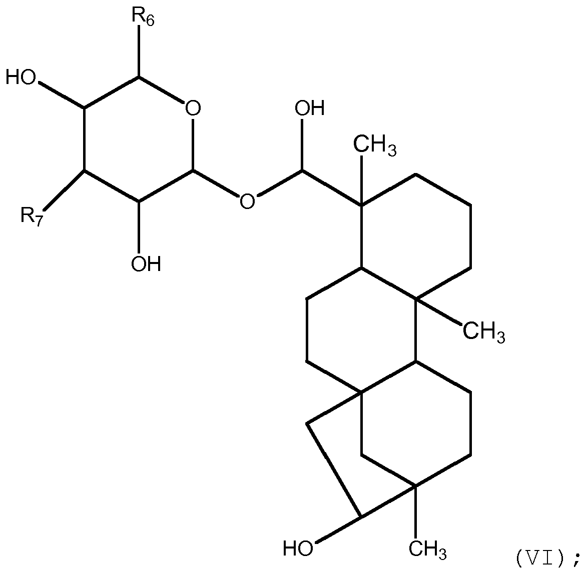 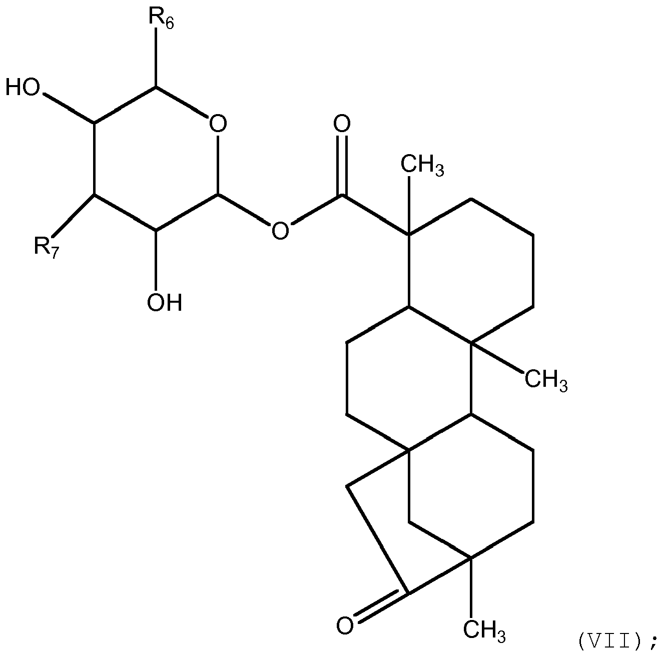 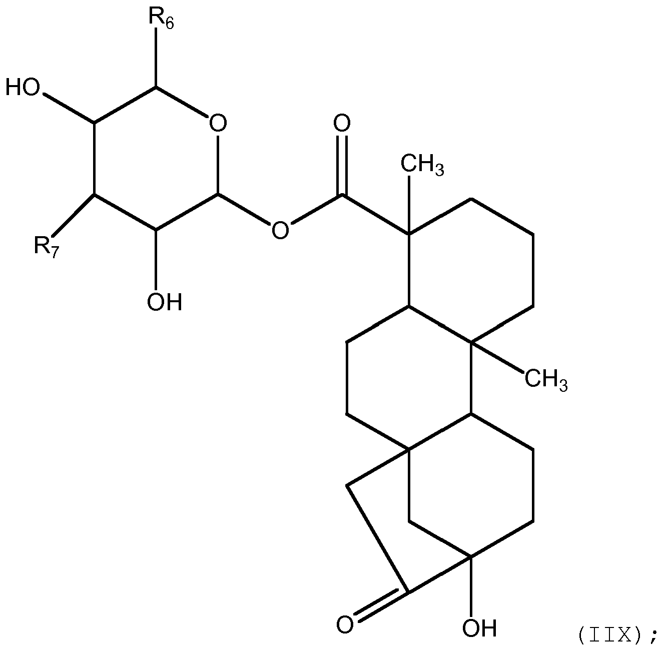 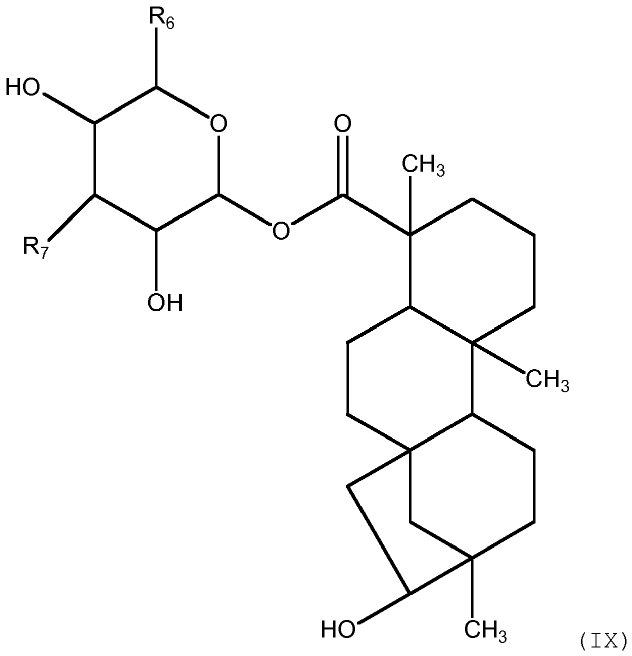 ou R6 est un hydroxyle, un éthyle, un hydrogène, un méthylène, un oxyméthyle, du nitrite, un carboxyle, un aldéhyde, un amino, ou un oxyamino et R7 est un hydroxyle ou un carboxyle

7. N'importe laquelle des revendications précédentes, **caractérisées par le fait que** le composé est l'une des formules générales suivantes (X): 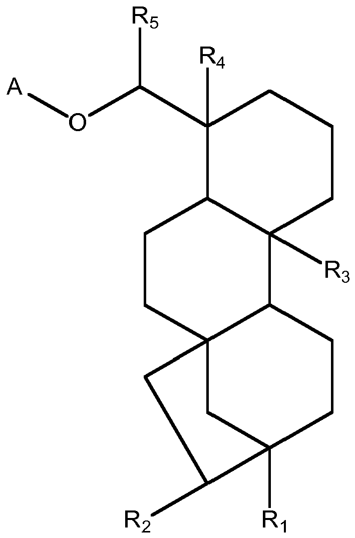 ou
R1 est un composé de tétrahydro-pyranne représenté par la formule générale suivante 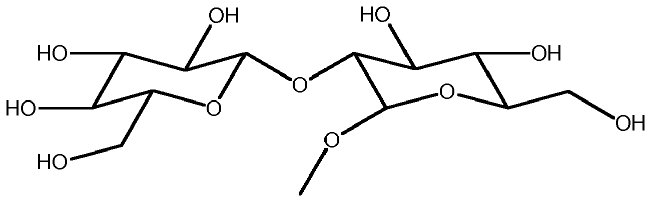
R2 est un oxygène, un méthyle, un hydrogène, de l'oxyméthyle, un hydroxyle, ou des formes d'éthène, éthanol, 2-méthoxy-propène, formaldéhyde ou 1-méthoxy-éthanol lié.
R3 est un méthyle, un éthyle, ou un hydrogène,
R4 est un méthyle, un éthyle, ou un hydrogène,
R5 est un oxygène, un hydrogène, ou un hydroxyle
A est un hydrogène
R6 est un hydroxyle, un éthyle, un hydrogène, un méthylène, un oxyméthyle, un nitrite, un carboxyle, de l'aldéhyde, un amino, ou un oxyamino.
R7 est un hydroxyle, un hydrogène, ou un carboxyle
ou un sel pharmaceutiquement acceptable d'un tel composé.

8. N'importe laquelle des revendications précédentes, **caractérisées par le fait que** le composé est un stéviol, un isostéviol, stéviol-glucuronide, stéviol-glucuronide, dihydroisosteviol-glucuronide stéviol- galacturonide, stéviol-galacturonide ou dihydroisostéviol-galacturonide ou un acide pharmaceutiquement acceptable- addition ou un sel cationique qui en résulte.

9. N'importe laquelle des revendications précédentes, **caractérisées par le fait que** le composé est sélectionné dans un groupe qui consiste en un stéviol-O-β-D-glucuronide, dihydroisostéviol-O-β-D-glucuronide,isostéviol-O-β-D-glucuronide.stéviol-O-β-D-galacturonide, dihydroisostéviol-O-β-D- galacturonide, isostéviol-O-β-D-galacturonide, dihydroisostéviol-O-α-D-glucuronide, stéviol-O- α-D-glucuronide, stéviol-O-α-D-glucuronide, stéviol-O-α-D-galacturonide, dihydroisostéviol-O-α-D-galacturonide, isostéviol-O-α-D-galacturonide,dihydrostéviol-O-β-L-glucuronide,stéviol-O-β-L-glucuronide, isostéviol-O-β-L-glucuronide,stéviol-O-β-L-galacturonide,dihydroisostéviol-O-β-L-galacturonide, stéviol-O-β-L-galacturonide, dihydroisostéviol-O-α-L-glucuronide, stéviol-O- α-L-glucuronide, isostéviol-O-α-L-glucuronide, stéviol-O-α-L-galacturonide,dihydroisostéviol-O-α-L-galacturonide, isostéviol-O-α-L-galacturonide, et stéviol-octan-O-β-D-galacturonide.

10. N'importe laquelle des revendications précédentes **caractérisées par le fait que** le composé est sélectionné à partir d'un groupe qui consiste en un stéviol-O-**β**-D-Acide Glucopyranosiduronique,stéviol-O-**β**-D-Glucopyranoside,stéviol-O-α-D-Glucopyranoside, stéviol-O-**β**-D-Glucopyranose, stéviol-O-**β-**D-gluco-Héxodialdo-1,5-pyranoside, stéviol-O-**β**-L-Mannopyranose, stéviol-O-**β**-D-Xylopyranoside, stéviol-O-**β**-L-Xylopyranose, stéviol-O-**β**-D-galacto-Heptopyranoside, stéviol-O-**β**-D-Galactopyranoside.

11. N'importe laquelle des revendications précédentes, **caractérisées par le fait que** le composé est un stéviol-19-O-β-D-glucuronide.

12. N'importe laquelle des revendications précédentes, **caractérisées par le fait que** le composé du groupe consiste en 7a,15a-Méthano-8H-naphth[2',1':5,6]azuleno[2,1-d]phenanthrene-4,7-dione,1,2,2a,3,5,5a,9,9a,10,11,12,13,13a,13b,14,15-hexadecahydro-1,16-dihydroxy-17-méthoxy-3,3,10,10,13a-pentaméthyle-18-(1-méthyleethyl)-, (1R,2aR,5aS,13aR)-rel-(-)-(9CI), Kauran-16-ol, 13-méthyle-, (8**β**,13**β**) - (9CI), Kaurane-16,18-diol, 13-méthyle-, (4**α**,8**β**,13**β**)- (9CI), Kauran-18-acide oïque, 16-hydroxy-13-méthyle-, (4**α**,8**β**,13**β**)- (9CI) ou ent-16b-Hydroxy-16a-méthylebeyeran-19-acide oïque, Kauran-18-acide oïque, 11,13,16,17-tétrahydroxy-, (4**α**,11**α**)-, Kaur-16-en-18-acide oïque, 7,11,13-trihydroxy-, (4**α**,7**β**,11**α**)-, Kauran-18-acide oïque, 16,17-epoxy-11,13-dihydroxy-, (4**α**,11**α**)-, Kauran-18-acide oïque, 17-(acétyloxy)-13,16-dihydroxy-, (4**α**) -, Kaur-16-en-18-acide oïque, 13-hydroxy-7-oxo-, (4α)-, 1H-2,10a-Ethan phénanthrène-8-acide carboxylique , dodécahydro-10-hydroxy-2-(hydroxyméthyle)-4b,8-diméthyle-12-oxo-(2S,4aS,4bS,8R,8aS,10R,10aS) - , 17-Noukauran-18-acide oïque, 16-hydroxy-13-méthyle-, ester méthylique, (4**α**,8**β**,13**β**,16**α**) -, Kaur-16-ene-3,18-diol, 13-méthyle-, (3**α**,4**α**,8**β**,13**β**)-(**±**)- (9CI), 17-Noukauran-18-acide oïque, 13-méthyle-16-(2-oxopropylidène)-, (4**α**,8**β**,13**β**)-(**±**)- (9CI), Kaur-16-en-18-acide oïque, 13-méthyle-3-oxo-, ester éthylique, (4**β**,8**β**,13**β**)-(**±**)- (9CI), Kaur-16-en-18-acide oïque, 3-hydroxy-13-méthyle-, ester éthylique, (3**α**,4**α**,8**β**,13**β**)-(**±**)- (9CI), et Kaur-16-en-18-acide oïque, 13-méthyle-3-oxo-, ester éthylique, (4**α**,8**β**,13**β**)-(**±**)- (9CI) ou des dérivés fonctionnels qui en résultent.

13. N'importe laquelle des revendications précédentes, **caractérisées par le fait que** le composé est un rebaudioside A (Kaur-16-en-18-acide oïque ou 13-[(O-**β**-D-glucopyranosyl-(1**→**2)-O-[**β**-D-glucopyranosyl-(1**→**3)]-**β**-D-glucopyranosyl)oxy]-, **β**-D-ester glucopyranosyl , (4**α**)-) ou stévioside (Kaur-16-en-18-acide oïque ou 13-[(2-O-**β**-D-glucopyranosyl-**β**-D-glucopyranosyl)oxy]-, **β**-D-ester glucopyranosyl , (4**α**)- )

14. N'importe laquelle des revendications précédentes, **caractérisées par le fait que** le composé appartient à un groupe constitué de stévioside, de rebaudioside A, rebaudioside C, Dulcoside A, stéviolbioside, rebaudioside B, rebaudioside D, rebaudioside E, stéviol, isostéviol, stéviol-glucuronide, isostéviol-glucuronide, et de dihydroisostéviol-glucuronide
